(19) Europäisches Patentamt
European Patent Office
Office européen des brevets

(11) EP 2 518 054 A1

(12) EUROPEAN PATENT APPLICATION
published in accordance with Art. 153(4) EPC

(43) Date of publication:
**31.10.2012 Bulletin 2012/44**

(21) Application number: **10839588.0**

(22) Date of filing: **24.12.2010**

(51) Int Cl.:
*C07D 231/56* (2006.01) *A61K 31/4025* (2006.01)
*A61K 31/4155* (2006.01) *A61K 31/4178* (2006.01)
*A61K 31/422* (2006.01) *A61K 31/427* (2006.01)
*A61P 21/02* (2006.01) *A61P 25/00* (2006.01)
*A61P 25/14* (2006.01) *A61P 25/16* (2006.01)
*A61P 25/18* (2006.01) *A61P 25/22* (2006.01)
*A61P 25/24* (2006.01) *A61P 25/28* (2006.01)
*A61P 43/00* (2006.01) *C07D 405/04* (2006.01)
*C07D 409/04* (2006.01) *C07D 413/04* (2006.01)
*C07D 417/04* (2006.01)

(86) International application number:
**PCT/JP2010/073426**

(87) International publication number:
**WO 2011/078360 (30.06.2011 Gazette 2011/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.12.2009 JP 2009293279**

(71) Applicant: **Takeda Pharmaceutical Company Limited**
**Osaka-shi, Osaka 541-0045 (JP)**

(72) Inventors:
- **SETOH, Masaki**
  **Kanagawa 251-0012 (JP)**
- **TOKUMARU, Kazuyuki**
  **Ama-shi,**
  **Aichi 490-1204 (JP)**
- **NAKAHATA, Takashi**
  **Kanagawa 251-0012 (JP)**
- **ISHII, Naoki**
  **Kanagawa 251-0012 (JP)**
- **KORI, Masakuni**
  **Osaka-shi**
  **Osaka 532-8686 (JP)**

(74) Representative: **Held, Stephan et al**
**Meissner, Bolte & Partner GbR**
**Chemistry**
**Widenmayerstraße 47**
**80538 München (DE)**

# (54) AMIDE COMPOUND

(57) A compound represented by the formula (I)

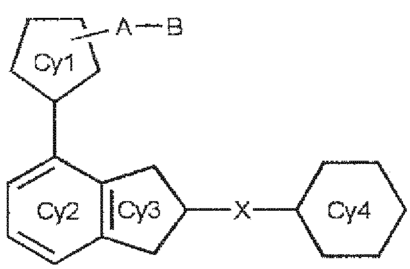

(I)

wherein
ring Cy1 is a 5-membered aromatic heterocycle optionally further substituted besides a group represented by -A-B,
ring Cy2 is an optionally substituted benzene ring,
ring Cy3 is an optionally substituted 5-membered aromatic heterocycle,
ring Cy4 is an optionally substituted 6-membered aromatic ring,
A is $-CONR^a-$ or $-NR^aCO-$,
$R^a$ is a hydrogen atom or a substituent,
B is a hydrogen atom or an optionally substituted $C_{1-6}$ alkyl-group,
X is an optionally substituted $C_{1-2}$ alkylene, -Y-, $-Y-CH_2-$ or $-CH_2-Y-$,
Y is an oxygen atom, $-NR^b-$ or $-S(O)_m-$,
$R^b$ is a hydrogen atom or a substituent, and
m is 0, 1 or 2,
provided that
N-methyl-4-[2-[3,4,5-trimethoxyphenyl)amino]-1,3-benzoxazol-7-yl]thiophene-2-carboxamide is excluded,
or a salt thereof,has an agonistic action on GPR52, and

EP 2 518 054 A1

is useful as an agent for the prophylaxis or treatment of schizophrenia and the like.

## Description

## Technical Field

The present invention relates to a novel amide compound and a production method of the same, and a medicament containing the same. More specifically, the present invention relates to a compound having an agonistic action on GPR52, which is effective as a medicament for the prophylaxis or treatment of mental disorders such as schizophrenia and the like.

[Background of the Invention]

Schizophrenia is a disease that occurs in people from adolescence to adulthood and shows characteristic thinking disturbances, disturbances of ego, and behavioral abnormalities associated therewith. The incidence of the disease is allegedly about 1% of the entire population. Most of them are chronic, so that the initiative or interpersonal contact of patients, and the like may be decreased, thereby seriously interfering with the social lives of the patients. The core symptoms of schizophrenia are broadly classified into (1) positive symptoms such as delusions and hallucination, (2) negative symptoms such as hypesthesia, social withdrawal, diminished motivation, and loss of concentration, and (3) cognitive dysfunction. In these core symptoms, the expression of positive symptoms is intimately involved in over activity of the dopamine nervous system in the mesolimbic system. The expression of the negative symptoms and impaired cognitive function are intimately involved in deterioration of the function of the nervous system such as the glutamic acid nervous system in the cortex of frontal lobe.

In addition, a typical antipsychotic agent having an antagonistic action on a dopamine D2 receptor, such as chlorpromazine, has favorable effects on the positive symptoms. On the other hand, drugs effective to multiple receptors, such as clozapine and olanzapine have certain effects on negative symptoms and cognitive dysfunction. However, it is known that many patients have poor response on these drugs. Also, the typical antipsychotic agent has controversial side effects such as the occurrence of extrapyramidal syndromes, for example, akathisia, dystonia and Parkinson-like movement disorders, and the occurrence of hyperprolactinemia. Furthermore, clozapine may cause agranulocytosis as a grave side effect. An atypical antipsychotic agent such as olanzapine may cause side effects, such as weight gain, lipidosis, excessive sedative effect, and prolonged cardiac QT interval.

Human GPR52 (Sawzdargo et al., Molecular Brain Research, vol.64: p.193-198, 1999) has been known as one of GPCRs. In recent years, because of an increase in cellular cAMP level in nerve cells expressing GPR52 or the like, any of agonists and ligands against GPR52 has been considered to have an effect of improving the positive symptoms of schizophrenia by suppressing the hyperactivation of dopamine pathway in the mesolimbic region, one of the causes of the positive symptoms of schizophrenia. In addition, it has been also found that the agonists and ligands against GPR52 can improve the negative symptoms of schizophrenia and cognitive deficiency by an improvement in decreased function of NMDA receptors in the cerebral cortex, which has been considered as one of the causes of the negative symptoms of schizophrenia and cognitive deficiency (WO 2006/098520).

Therefore, it has been demanded to develop a compound having an agonistic action on GPR52 and useful as a medicament for the prophylaxis or treatment of mental diseases such as schizophrenia.

As compounds having agonist action on GPR52, for example, WO 2009/107391 discloses a compound represented by the formula

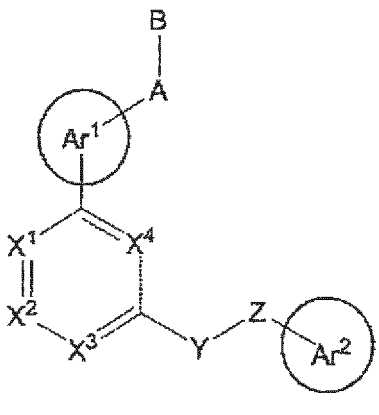

WO 2010/018874 discloses a compound represented by the formula

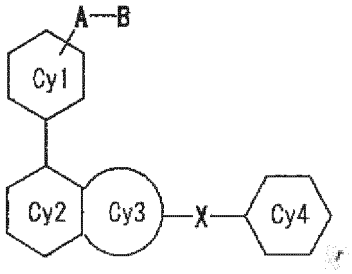

which encompasses amide compounds, and WO 2009/157196 discloses a compound represented by the formula

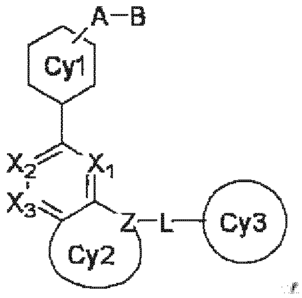

which encompasses amide compounds.

On the other hand, as to amide compounds, for example, WO 2006/116412 discloses a compound represented by the formula

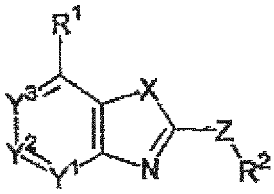

which encompasses amide compounds, WO 2006/138695 discloses a compound represented by the formula

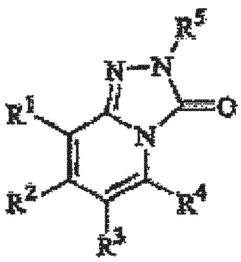

which encompasses amide compounds, US 2005/143381 discloses a compound represented by the formula

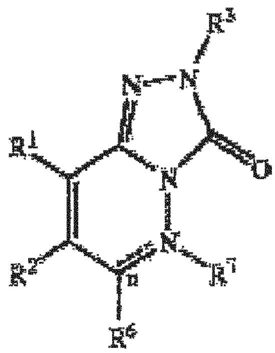

which encompasses amide compounds, and WO 2008/031594 discloses a compound represented by the formula

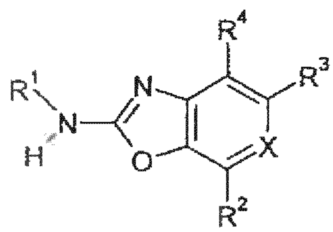

which encompasses amide compounds, specifically, a compound represented by the formula

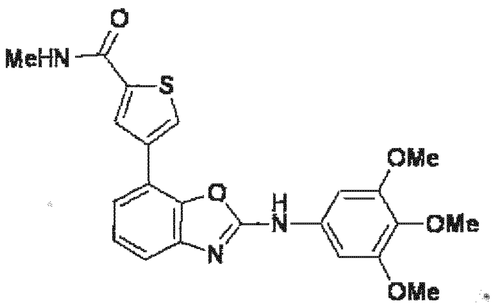

As for the definition of the abbreviations in the formulas showing the compounds of the above-mentioned respective patent documents, the specifications of the respective patent documents are to be referred to.

## Document List

### Patent Documents

Patent Document 1: WO 2009/107391
Patent Document 2: WO 2010/018874
Patent Document 3: WO 2009/157196
Patent Document 4: WO 2006/116412
Patent Document 5: WO 2006/138695
Patent Document 6: US 2005/143381
Patent Document 7: WO 2008/031594

**Non-Patent Document**

Non-Patent Document 1: Sawzdargo et al., Molecular Brain Research, vol. 64, p.193-198, 1999

## Summary of the Invention

### Problems to be Solved by the Invention

An object of the present invention is to provide a compound having an agonistic action on GPR52 and useful as a medicament for the prophylaxis or treatment of mental diseases such as schizophrenia.

### Means of Solving the Problems

The present inventors have found that compounds represented by the below formula (I) or salts thereof (herein also referred to as compounds (I)) have an agonistic action on GPR52 and finally completed the present invention by further investigations.
In the present specification, compound (I) or prodrugs thereof will be herein also referred to as the compounds of the present invention.
Accordingly, the present invention provides

[1] a compound represented by the formula (I)

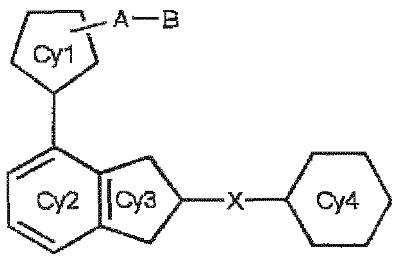

(I)

wherein
ring Cy1 is a 5-membered aromatic heterocycle optionally
further substituted besides a group represented by -A-B,
ring Cy2 is an optionally substituted benzene ring,
ring Cy3 is an optionally substituted 5-membered aromatic heterocycle,
ring Cy4 is an optionally substituted 6-membered aromatic ring, A is -CONR$^a$- or -NR$^a$CO-,
R$^a$ is a hydrogen atom or a substituent,
B is a hydrogen atom or an optionally substituted $C_{1-6}$ alkyl group,
X is an optionally substituted $C_{1-2}$ alkylene, -Y-, -Y-$CH_2$- or - $CH_2$-Y-,
Y is an oxygen atom, -NR$^b$- or -S(O)$_m$-,
R$^b$ is a hydrogen atom or a substituent, and
m is 0, 1 or 2,
provided that
N-methyl-4-[2-[(3,4,5-trimethoxyphenyl)amino]-1,3-benzoxazol-7-yl]thiophene-2-carboxamide is excluded,
or a salt thereof;

[2] the compound or salt of the aforementioned [1], wherein R$^b$ is a substituent;
[3] the compound or salt of the aforementioned [1], wherein ring Cy1 is a 5-membered nitrogen-containing aromatic heterocycle optionally further substituted by substituent(s) selected from

(i) optionally substituted lower alkyl, and
(ii) optionally substituted $C_{3-8}$ cycloalkyl,

besides a group represented by -A-B;
[4] the compound or salt of the aforementioned [3], wherein the 5-membered nitrogen-containing aromatic heterocycle is pyrrole, imidazole, pyrazole, thiazole or oxazole;
[5] the compound or salt of the aforementioned [1], wherein ring Cy2 is a benzene ring optionally substituted by substituent(s) selected from (i) a halogen atom and (ii) $C_{1-6}$ alkyl;
[6] the compound or salt of the aforementioned [1], wherein ring Cy3 is a 5-membered aromatic heterocycle optionally substituted by $C_{1-6}$ alkyl;
[7] the compound or salt of the aforementioned [6], wherein the 5-membered aromatic heterocycle is thiophene, furan, pyrazole or thiazole;
[8] the compound or salt of the aforementioned [1], wherein ring Cy4 is a 6-membered aromatic ring optionally substituted by substituent(s) selected from

(i) a halogen atom,
(ii) cyano,
(iii) optionally substituted lower alkyl,
(iv) optionally substituted lower alkoxy, and
(v) optionally substituted lower alkylsulfonyl;

[9] the compound or salt of the aforementioned [8], wherein the 6-membered aromatic ring is benzene, pyridine, pyridazine, pyrimidine, pyrazine or triazine;
[10] the compound or salt of the aforementioned [8], wherein the 6-membered aromatic ring is benzene;
[11] the compound or salt of the aforementioned [I], wherein A is -CONR$^a$- wherein R$^a$ is a hydrogen atom, an optionally substituted $C_{1-6}$ alkyl group or $C_{3-6}$ cycloalkyl, or -NHCO-;
[12] the compound or salt of the aforementioned [1], wherein B is

(i) a hydrogen atom, or
(ii) a $C_{1-6}$ alkyl group optionally substituted by substituent(s) selected from

(a) hydroxy,
(b) $C_{1-6}$ alkoxy, and
(c) carbamoyl;

[13] the compound or salt of the aforementioned [1], wherein is an $C_{1-2}$ alkylene;
[14] N-(2-methoxyethyl)-3-methyl-1-[2-[3-(trifluoromethyl)benzyl]-1-benzothiophen-7-yl]-1H-pyrazole-4-carboxamide or a salt thereof;
[15] N-(2-hydroxyethyl)-3-methyl-1-[2-[3-(trifluoromethyl)benzyl]-1-benzothiophen-7-yl]-1H-pyrazole-4-carboxamide or a salt thereof;
[16] 3-methyl-1-[2-[3-(trifluoromethyl)benzyl]-1-benzothiophen-7-yl]-1H-pyrazole-4-carboxamide or a salt thereof;
[17] N-(2-amino-2-oxoethyl)-7-[4-fluoro-2-[3-(trifluoromethyl)benzyl]-1-benzothiophen-7-yl]-3,5-dimethyl-1H-pyrazole-4-carboxamide or a salt thereof;
[18] N-(2-hydroxyethyl)-5-(hydroxymethyl)-3-methyl-1-[2-[3-(trifluoromethyl)benzyl]-1-benzothiophen-7-yl]-1H-pyrazole-4-carboxamide or a salt thereof;
[19] a medicament comprising the compound or salt of the aforementioned [1];
[20] a GPR52 activating agent comprising the compound or salt of the aforementioned [1];
[21] an agent for the prophylaxis or treatment of schizophrenia, which comprises the compound or salt of the aforementioned [1];
[22] a method of activating GPR52, which comprises administering an effective amount of the compound or salt of the aforementioned [1] to a mammal;
[23] a method for the prophylaxis or treatment of schizophrenia, which comprises administering an effective amount of the compound or salt of the aforementioned [1] to a mammal;
[24] use of the compound or salt of the aforementioned [1] for the production of a GPR52 activating agent;
[25] use of the compound or salt of the aforementioned [1] for the production of an agent for the prophylaxis or treatment of schizophrenia; and
[26] the compound or salt of the aforementioned [1] for use in the prophylaxis or treatment of schizophrenia and the like.

**Effect of the Invention**

The compound of the present invention has an agonistic action on GPR52 and is advantageously used as a medicament for the prophylaxis or treatment of mental diseases such as schizophrenia.

[Detailed Description of the Invention]

The present invention is explained in detail in the following.

In the present specification, unless otherwise specified, examples of the "halogen atom" include fluorine, chlorine, bromine and iodine.

In the present specification, unless otherwise specified, the "optionally halogenated" means optionally having one or more (e.g., 1 to 3) halogen atoms as substituent.

In the present specification, unless otherwise specified, examples of the "optionally esterified carboxyl (group)" include carboxyl, optionally substituted lower alkoxy-carbonyl, optionally substituted $C_{6-14}$ aryloxy-carbonyl, optionally substituted $C_{7-16}$ aralkyloxy-carbonyl, optionally substituted silyloxy-carbonyl (e.g., TMS-O-CO-, TES-O-CO-, TBS-O-CO-, TIPS-O-CO-, TBDPS-O-CO-) and the like.

In the present specification, unless otherwise specified, examples of the "lower alkoxy-carbonyl (group)" include methoxycarbonyl, ethoxycarbonyl, propoxycarbonyl, isopropoxycarbonyl, sec-butoxycarbonyl, tert-butoxycarbonyl, pentyloxycarbonyl, isopentyloxycarbonyl, neopentyloxycarbonyl, and hexyloxycarbonyl.

In the present specification, unless otherwise specified, examples of the "$C_{6-14}$ aryloxy-carbonyl (group)" include phenoxycarbonyl, 1-naphthyloxycarbonyl and 2-naphthyloxycarbonyl.

In the present specification, unless otherwise specified, examples of the "$C_{7-16}$ aralkyloxy-carbonyl (group)" include benzyloxycarbonyl and phenethyloxycarbonyl,

In the present specification, unless otherwise specified, examples of the "lower alkyl (groups)" include $C_{1-6}$ alkyl (group).

In the present specification, unless otherwise specified, examples of the "$C_{1-6}$ alkyl (group)" include methyl, ethyl, propyl, isopropyl, butyl, isobutyl, sec-butyl, tert-butyl, pentyl, isopentyl, neopentyl and hexyl.

In the present specification, unless otherwise specified, the "optionally halogenated $C_{1-6}$ alkyl (group)" means $C_{1-6}$ alkyl (group) optionally substituted by halogen atom(s), and examples thereof include trifluoromethyl.

In the present specification, unless otherwise specified, examples of the "lower alkenyl (group)" include $C_{2-6}$ alkenyl (group).

In the present specification, unless otherwise specified, examples of the "$C_{2-6}$ alkenyl (group)" include vinyl, 1-propen-1-yl, 2-propen-1-yl, isopropenyl, 2-buten-1-yl, 4-penten-1-yl and 5-hexen-1-yl.

In the present specification, unless otherwise specified, examples of the "lower alkynyl (group)" include $C_{2-6}$ alkynyl (group).

In the present specification, unless otherwise specified, examples of the "$C_{2-6}$ alkynyl (group)" include ethynyl, 1-propyn-1-yl, 2-propyn-1-yl, 4-pentyn-1-yl and 5-hexyn-1-yl.

In the present specification, unless otherwise specified, examples of the "$C_{3-8}$ cycloalkyl (group)" include cyclopropyl, cyclobutyl, cyclopentyl and cyclohexyl.

In the present specification, unless otherwise specified, examples of the "$C_{6-14}$ aryl (group)" include phenyl, 1-naphthyl, 2-naphthyl, 2-biphenylyl, 3-biphenylyl, 4-biphenylyl and 2-anthryl.

In the present specification, unless otherwise specified, examples of the "$C_{7-16}$ aralkyl (group)" include benzyl, phenethyl, diphenylmethyl, 1-naphthylmethyl, 2-naphthylmethyl, 2,2-diphenylethyl, 3-phenylpropyl, 4-phenylbutyl, 5-phenylpentyl, 2-biphenylylmethyl, 3-biphenylylmethyl and 4-biphenylylmethyl.

In the present specification, unless otherwise specified, examples of the "$C_{6-14}$ aryl-$C_{2-6}$ alkenyl (group) " include styryl.

In the present specification, unless otherwise specified, examples of the "heterocyclic group" (and the heterocyclic moiety contained in the substituent) include a 3- to 14-membered (monocyclic, bicyclic or tricyclic) heterocyclic group containing, besides carbon atoms, one to five of one to three kinds of heteroatoms selected from an oxygen atom, a sulfur atom and a nitrogen atom. Examples of such heterocyclic group include

aromatic heterocyclic groups such as pyrrolyl (e.g., 1-pyrrolyl, 2-pyrrolyl, 3-pyrrolyl), furyl (e.g., 2-furyl, 3-furyl), thienyl (e.g., 2-thienyl, 3-thienyl), pyrazolyl (e.g., 1-pyrazolyl, 3-pyrazolyl, 4-pyrazolyl), imidazolyl (e.g., 1-imidazolyl, 2-imidazolyl, 4-imidazolyl), isoxazolyl (e.g., 3-isoxazolyl, 4-isoxazolyl, 5-isoxazolyl), oxazolyl (e.g., 2-oxazolyl, 4-oxazolyl, 5-oxazolyl), isothiazolyl (e.g., 3-isothiazolyl, 4-isothiazolyl, 5-isothiazolyl), thiazolyl (e.g., 2-thiazolyl, 4-thiazolyl, 5-thiazolyl), triazolyl (1,2,3-triazol-4-yl, 1,2,4-triazol-3-yl), oxadiazolyl (1,2,4-oxadiazol-3-yl, 1,2,4-oxadiazol-5-yl), thiadiazolyl (1,2,4-thiadiazol-3-yl, 1,2,4-thiadiazol-5-yl), tetrazolyl, pyridyl (e.g., 2-pyridyl, 3-pyridyl, 4-pyridyl), pyridazinyl (e.g., 3-pyridazinyl, 4-pyridazinyl), pyrimidinyl (e.g., 2-pyrimidinyl, 4-pyrimidinyl, 5-pyrimidinyl), pyrazinyl, isoindolyl (e.g., 1-isoindolyl, 2-isoindolyl, 3-isoindolyl, 4-isoindolyl, 5-isoindolyl, 6-isoindolyl, 7-isoindolyl), indolyl (e.g., 1-indolyl, 2-indolyl, 3-indolyl, 4-indolyl,

5-indolyl, 6-indolyl, 7-indolyl), benzo[b]furanyl (e.g., 2-benzo[b]furanyl, 3-benzo[b]furanyl, 4-benzo[b]furanyl, 5-benzo[b]furanyl, 6-benzo[b]furanyl, 7-benzo[b]furanyl), benzo[c]furanyl (e.g., 1-benzo[c]furanyl, 4-benzo[c]furanyl, 5-benzo[c]furanyl), benzo[b]thienyl (e.g., 2-benzo[b]thienyl, 3-benzo[b]thienyl, 4-benzo[b]thienyl, 5-benzo[b]thienyl, 6-benzo[b]thienyl, 7-benzo[b]thienyl), benzo[c]thienyl (e.g., 1-benzo[c]thienyl, 4-benzo[c]thienyl, 5-benzo[c]thienyl), indazolyl (e.g., 1-indazolyl, 2-indazolyl, 3-indazolyl, 4-indazolyl, 5-indazolyl, 6-indazolyl, 7-indazolyl), benzimidazolyl (e.g., 1-benzimidazolyl, 2-benzimidazolyl, 4-benzimidazolyl, 5-benzimidazolyl), 1,2-benzisoxazolyl (e.g., 1,2-benzisoxazol-3-yl, 1,2-benzisoxazol-4-yl, 1,2-benzisoxazol-5-yl, 1,2-benzisoxazol-6-yl, 1,2-benzisoxazol-7-yl), benzoxazolyl (e.g., 2-benzoxazolyl, 4-benzoxazolyl, 5-benzoxazolyl, 6-benzoxazolyl, 7-benzoxazolyl), 1,2-benzisothiazolyl (e.g., 1,2-benzisothiazol-3-yl, 1,2-benzisothiazol-4-yl, 1,2-benzisothiazol-5-yl, 1,2-benzisothiazol-6-yl, 1,2-benzisothiazol-7-yl), benzothiazolyl (e.g., 2-benzothiazolyl, 4-benzothiazolyl, 5-benzothiazolyl, 6-benzothiazolyl, 7-benzothiazolyl), isoquinolyl (e.g., 1-isoquinolyl, 3-isoquinolyl, 4-isoquinolyl, 5-isoquinolyl), quinolyl (e.g., 2-quinolyl, 3-quinolyl, 4-quinolyl, 5-quinolyl, 8-quinolyl), cinnolinyl (e.g., 3-cinnolinyl, 4-cinnolinyl, 5-cinnolinyl, 6-cinnolinyl, 7-cinnolinyl, 8-cinnolinyl), phthalazinyl (e.g., 1-phthalazinyl, 4-phthalazinyl, 5-phthalazinyl, 6-phthalazinyl, 7-phthalazinyl, 8-phthalazinyl), quinazolinyl (e.g., 2-quinazolinyl, 4-quinazolinyl, 5-quinazolinyl, 6-quinazolinyl, 7-quinazolinyl, 8-quinazolinyl), quinoxalinyl (e.g., 2-quinoxalinyl, 3-quinoxalinyl, 5-quinoxalinyl, 6-quinoxalinyl, 7-quinoxalinyl, 8-quinoxalinyl), pyrazolo[1,5-a]pyridyl(pyrazolo[1,5-a]pyridin-2-yl, pyrazolo[1,5-a]pyridin-3-yl, pyrazolo[1,5-a]pyridin-4-yl, pyrazolo[1,5-a]pyridin-5-yl, pyrazolo[1,5-a]pyridin-6-yl, pyrazolo[1,5-a]pyridin-7-yl), imidazo[1,2-a]pyridyl (imidazo[1,2-a]pyridin-2-yl, imidazo[1,2-a]pyridin-3-yl, imidazo[1,2-a]pyridin-5-yl, imidazo[1,2-a]pyridin-6-yl, imidazo[1,2-a]pyridin-7-yl, imidazo[1,2-a]pyridine-8-yl) and the like;

non-aromatic heterocyclic groups such as imidazolinyl (e.g., imidazolinyl (e.g., 1-imidazolinyl, 2-imidazolinyl, 4-imidazolinyl), aziridinyl (e.g., 1-aziridinyl, 2-aziridinyl), azetidinyl (e.g., 1-azetidinyl, 2-azetidinyl), pyrrolidinyl (e.g., 1-pyrrolidinyl, 2-pyrrolidinyl, 3-pyrrolidinyl), piperidinyl (e.g., 1-piperidinyl, 2-piperidinyl, 3-piperidinyl), azepanyl (e.g., 1-azepanyl, 2-azepanyl, 3-azepanyl, 4-azepanyl), azocanyl (e.g., 1-azocanyl, 2-azocanyl, 3-azocanyl, 4-azocanyl), piperazinyl (e.g., 1,4-piperazin-1-yl, 1,4-piperazin-2-yl), diazepanyl (e.g., 1,4-diazepan-1-yl, 1,4-diazepan-2-yl, 1,4-diazepan-5-yl, 1,4-diazepan-6-yl), diazocanyl(1,4-diazocan-1-yl, 1,4-diazocan-2-yl, 1,4-diazocan-5-yl, 1,4-diazooan-6-yl, 1,5-diazocan-1-yl, 1,5-diazocan-2-yl, 1,5-diazocan-3-yl), 1-morpholinyl, 4-thiomorpholinyl, 2-oxazolidinyl and the like;

heterocyclic groups wherein the above-mentioned aromatic heterocyclic group is partially hydrogenated (e.g., heterocyclic groups such as indolyl, dihydroquinolyl and the like); and

heterocyclic groups wherein the above-mentioned non-aromatic heterocyclic group is partially dehydrogenated (e.g., dihydrofuranyl).

In the present specification, unless otherwise specified, examples of the "lower alkoxy (group)" include $C_{1-6}$ alkoxy.

In the present specification, unless otherwise specified, examples of the "$C_{1-6}$ alkoxy (group)" include methoxy, ethoxy, propoxy, isopropoxy, butoxy, isobutoxy, sec-butoxy, tert-butoxy, pentyloxy, isopentyloxy, neopentyloxy and hexyloxy.

In the present specification, unless otherwise specified, examples of the "$C_{3-8}$ cycloalkoxy (group)" include cyclopropoxy, cyclobutoxy, cyclopentyloxy and cyclohexyloxy.

In the present specification, unless otherwise specified, examples of the "$C_{5-14}$ aryloxy (group)" include phenyloxy, 1-naphthyloxy and 2-naphthyloxy.

In the present specification, unless otherwise specified, examples of the "$C_{7-16}$ aralkyloxy (group)" include benzyloxy and phenethyloxy.

In the present specification, unless otherwise specified, examples of the "lower alkyl-carbonyloxy (group)" include $C_{1-6}$ alkyl-carbonyloxy.

In the present specification, unless otherwise specified, examples of the "$C_{1-6}$ alkyl-carbonyloxy (group)" include acetoxy and propionyloxy.

In the present specification, unless otherwise specified, examples of the "lower alkoxy-carbonyloxy (group)" include $C_{1-6}$ alkoxy-carbonyloxy (group).

In the present specification, unless otherwise specified, examples of the "$C_{1-6}$ alkoxy-carbonyloxy (group)" include methoxycarbonyloxy, ethoxycarbonyloxy, propoxycarbonyloxy and butoxycarbonyloxy.

In the present specification, unless otherwise specified, examples of the "mono-lower alkyl-carbamoyloxy (group)" include mono-$C_{1-6}$ alkyl-carbamoyloxy (group).

In the present specification, unless otherwise specified, examples of the "mono-$C_{1-5}$ alkyl-carbamoyloxy (group)" include methylcarbamoyloxy and ethylcarbamoyloxy.

In the present specification, unless otherwise specified, examples of the "di-lower alkyl-carbamoyloxy (group)" include di-$C_{1-6}$ alkyl-carbamoyloxy (group).

In the present specification, unless otherwise specified, examples of the "di-$C_{1-6}$ alkyl-carbamoyloxy (group)" include dimethylcarbamoyloxy and diethylcarbamoyloxy.

In the present specification, unless otherwise specified, examples of the "$C_{6-14}$ aryl-carbonyloxy (group)" include benzoyloxy, 1-naphthylcarbonyloxy and 2-naphthylcarbonyloxy.

In the present specification, unless otherwise specified, examples of the "mono- or di-$C_{6-14}$ aryl-carbamoyloxy

(group)" include phenylcarbamoyloxy, 1-naphthylcarbamoyloxy and 2-naphthylcarbamoyloxy.

In the present specification, unless otherwise specified, examples of the heterocyclic moiety of the "heterocycleoxy (group)" include those similar to the aforementioned "heterocyclic group". Specific examples of the "heterocycleoxy (group)" include 3- to 14-membered heterocyclyl-oxy (group) containing, besides carbon atoms, one to five of one to three kinds of heteroatoms selected from a nitrogen atom, a sulfur atom and an oxygen atom.

In the present specification, unless otherwise specified, examples of the aromatic heterocyclic moiety of the "aromatic heterocycleoxy (group)" include those similar to the "aromatic heterocyclic group" exemplified as the aforementioned "heterocyclic group". Specific examples of the "aromatic heterocycleoxy (group)" include 3- to 14-membered aromatic heterocyclyl-oxy containing, besides carbon atoms, one to five of one to three kinds of heteroatoms selected from a nitrogen atom, a sulfur atom and an oxygen atom.

In the present specification, unless otherwise specified, examples of the "lower alkylthio (group)" include $C_{1-5}$ alkylthio (group).

In the present specification, unless otherwise specified, examples of the "$C_{1-6}$ alkylthio (group)" include methylthio, ethylthio, propylthio, isopropylthio, butylthio, sec-butylthio and tert-butylthio.

In the present specification, unless otherwise specified, examples of the "$C_{3-8}$ cycloalkylthio (group)" include cyclopropylthio, cyclobutylthio, cyclopentylthio and cyclohexylthio.

In the present specification, unless otherwise specified, examples of the "$C_{6-14}$ arylthio (group)" include phenylthio, 1-naphthylthio and 2-naphthylthio.

In the present specification, unless otherwise specified, examples of the "$C_{7-16}$ aralkylthio (group)" include benzylthio and phenethylthio.

In the present specification, unless otherwise specified, examples of the heterocyclic moiety of the "heterocyclethio (group)" include those similar to the aforementioned "heterocyclic group". Specific examples of the "heterocyclethio (group)" include 5- to 14-membered heterocyclyl-thio (group) containing, besides carbon atoms, one to five of one to three kinds of heteroatoms selected from a nitrogen atom, a sulfur atom and an oxygen atom.

In the present specification, unless otherwise specified, examples of the "lower alkyl-carbonyl (group)" include $C_{1-6}$ alkyl-carbonyl.

In the present specification, unless otherwise specified, examples of the "$C_{1-6}$ alkyl-carbonyl (group)" include acetyl, propionyl and pivaloyl.

In the present specification, unless otherwise specified, examples of the "$C_{3-8}$ cycloalkyl-carbonyl (group)" include cyclopropylcarbonyl, cyclobutylcarbonyl, cyclopentylcarbonyl and cyclohexylcarbonyl.

In the present specification, unless otherwise specified, examples of the "$C_{6-14}$ aryl-carbonyl (group)" include benzoyl, 1-naphthoyl and 2-naphthoyl.

In the present specification, unless otherwise specified, examples of the "$C_{7-16}$ aralkyl-carbonyl (group)" include phenylacetyl and 3-phenylpropionyl.

In the present specification, unless otherwise specified, examples of the heterocyclic moiety of the "heterocyclic-carbonyl (group)" include those similar to the aforementioned "heterocyclic group". Specific examples of the "heterocyclic-carbonyl (group)" include 3- to 14-membered heterocyclic-carbonyl (group) containing, besides carbon atoms, one to five of one to three kinds of heteroatoms selected from a nitrogen atom, a sulfur atom and an oxygen atom. More specific examples of thereof include picolinoyl, nicotinoyl, isonicotinoyl, 2-thenoyl, 3-thenoyl, 2-furoyl, 3-furoyl, 1-morpholinylcarbonyl, 4-thiomorpholinylcarbonyl, aziridin-1-ylcarbonyl, aziridin-2-ylcarbonyl, azetidin-1-ylcarbonyl, azetidin-2-ylcarbonyl, pyrrolidin-1-ylcarbonyl, pyrrolidin-2-ylcarbonyl, pyrrolidin-3-ylcarbonyl, piperidin-1-ylcarbonyl, piperidin-2-ylcarbonyl, piperidin-3-ylcarbonyl, azepan-1-ylcarbonyl, azepan-2-ylcarbonyl, azepan-3-ylcarbonyl, azepan-4-ylcarbonyl, azocan-1-ylcarbonyl, azocan-2-ylcarbonyl, azocan-3-ylcarbonyl, azocan-4-ylcarbonyl, 1,4-piperazin-1-ylcarbonyl, 1,4-piperazin-2-ylcarbonyl, 1,4-diazepan-1-ylcarbonyl, 1,4-diazepan-2-ylcarbonyl, 1,4-diazepan-5-ylcarbonyl, 1,4-diazepan-6-ylcarbonyl, 1,4-diazocan-1-ylcarbonyl, 1,4-diazocan-2-ylcarbonyl, 1,4-diazocan-5-ylcarbanyl, 1,4-diazocan-6-ylcarbonyl, 1,5-diazocan-1-ylcarbonyl, 1,5-diazocan-2-ylcarbonyl and 1,5-diazocan-3-ylcarbonyl.

In the present specification, unless otherwise specified, examples of the "lower alkylsulfonyl (group)" include $C_{1-6}$ alkylsulfonyl (group).

In the present specification, unless otherwise specified, examples of the "$C_{1-6}$ alkylsulfonyl (group)" include methylsulfonyl, ethylsulfonyl, propylsulfonyl, isopropylsulfonyl, butylsulfonyl, isobutylsulfonyl, sec-butylsulfonyl, tert-butylsulfonyl, pentylsulfonyl, isopentylsulfonyl, neopentylsulfonyl and hexylsulfonyl.

In the present specification, unless otherwise specified, examples of the "$C_{3-8}$ cycloalkylsulfonyl (group)" include cyclopropylsulfonyl, cyclobutylsulfonyl, cyclopentylsulfonyl and cyclohexylsulfonyl.

In the present specification, unless otherwise specified, examples of the "$C_{6-14}$ arylsulfonyl (group)" include phenylsulfonyl, 1-naphthylsulfonyl and 2-naphthylsulfonyl.

In the present specification, unless otherwise specified, examples of the heterocyclic moiety of the "heterocyclylsulfonyl (group)" include those similar to the aforementioned "heterocyclic group". Specific examples of the "heterocyclylsulfonyl (group)" include 3- to 14-membered heterocyclylsulfonyl (group) containing, besides carbon atoms,

one to five of one to three kinds of heteroatoms selected from a nitrogen atom, a sulfur atom and an oxygen atom.

In the present specification, unless otherwise specified, examples of the "lower alkylsulfinyl (group)" include $C_{1-6}$ alkylsulfinyl (group).

In the present specification, unless otherwise specified, examples of the "$C_{1-6}$ alkylsulfinyl (group)" include methylsulfinyl, ethylsulfinyl, propylsulfinyl, isopropylsulfinyl, butylsulfinyl, isobutylsulfinyl, sec-butylsulfinyl, tert-butylsulfinyl, pentylsulfinyl, isopentylsulfinyl, neopentylsulfinyl and hexylsulfinyl.

In the present specification, unless otherwise specified, examples of the "$C_{3-8}$ cycloalkylsulfinyl (group)" include cyclopropylsulfinyl, cyclobutylsulfinyl, cyclopentylsulfinyl and cyclohexylsulfinyl.

In the present specification, unless otherwise specified, examples of the "$C_{6-14}$ arylsulfinyl (group)" include phenylsulfinyl, 1-naphthylsulfinyl and 2-naphthylsulfinyl.

In the present specification, unless otherwise specified, examples of the heterocyclic moiety of the "heterocyclesulfinyl (group)" include those similar to the aforementioned "heterocyclic group". Specific examples of the "heterocyclesulfinyl (group)" include 3- to 14-membered heterocyclyl-sulfinyl (group), containing, besides carbon atoms, one to five of one to three kinds of heteroatoms selected from a nitrogen atom, a sulfur atom and an oxygen atom.

In the present specification, unless otherwise specified, examples of the "lower alkyl-carbamoyl (group)" include $C_{1-6}$ alkyl-carbamoyl.

In the present specification, unless otherwise specified, examples of the "$C_{1-6}$ alkyl-carbamoyl (group)" include methylcarbamoyl, ethylcarbamoyl, propylcarbamoyl, isopropylcarbamoyl, butylcarbamoyl, isobutylcarbamoyl, sec-butylcarbamoyl, tert-butylcarbamoyl, pentylcarbamoyl, isopentylcarbamoyl, neopentylcarbamoyl and hexylcarbamoyl.

In the present specification, unless otherwise specified, examples of the "mono- or di-lower alkylamino (group)" include mono- or di-$C_{1-6}$ alkylamino (group).

In the present specification, unless otherwise specified, examples of the "mono- or di-$C_{1-6}$ alkylamino (group)" include methylamino, ethylamino, propylamino, isopropylamino, butylamino, isobutylamino, sec-butylamino, tert-butylamino, pentylamino, isopentylamino, neopentylamino, hexylamino, dimethylamino and diethylamino.

In the present specification, unless otherwise specified, examples of the "lower alkyl-carbonylamino (group)" include $C_{1-6}$ alkyl-carbonylamino.

In the present specification, unless otherwise specified, examples of the "$C_{1-6}$ alkyl-carbonylamino (group)" include acetylamino, propionylamino and pivaloylamino.

In the present specification, unless otherwise specified, examples of the heterocyclic moiety of the "heterocyclylamino (group)" include those similar to the above-mentioned "heterocyclic group". Specific examples of the "heterocyclylamino" include 3- to 14-membered heterocyclyl-amino (group) containing, besides carbon atoms, one to five of one to three kinds of heteroatoms selected from a nitrogen atom, a sulfur atom and an oxygen atom (e.g., 2-pyridyl-amino).

In the present specification, unless otherwise specified, examples of the heterocyclic moiety of the "heterocyclylcarbonylamino (group)" include those similar to the above-mentioned "heterocyclyl-carbonyl". Specific examples of the "heterocyclyl-carbonylamino" include 3- to 14-membered heterocyclyl-carbonyl (group) containing, besides carbon atoms, one to five of one to three kinds of heteroatoms selected from a nitrogen atom, a sulfur atom and an oxygen atom (e.g., pyridyl-carbonylamino).

In the present specification, unless otherwise specified, examples of the heterocyclic moiety of the "heterocyclyloxycarbonylamino (group)" include those similar to the above-mentioned "heterocyclic group". Specific examples of the "heterocyclyl-oxycarbonylamino" include 3- to 14-membered heterocyclyl-oxycarbonylamino (group) containing, besides carbon atoms, one to five of one to three kinds of heteroatoms selected from a nitrogen atom, a sulfur atom and an oxygen atom (e.g., 2-pyridyl-oxycarbonylamino)

In the present specification, unless otherwise specified, examples of the heterocyclic moiety of the "heterocyclylsulfonylamino (group)" include those similar to the above-mentioned "heterocyclic group". Specific examples of the "heterocyclic group-sulfonylamino" include 3- to 14-membered heterocyclyl-(sulfonylamino group) containing, besides carbon atoms, one to five of one to three kinds of heteroatoms selected from a nitrogen atom, a sulfur atom and an oxygen atom (e.g., 2-pyridyl-sulfonylamino).

In the present specification, unless otherwise specified, examples of the "lower alkoxy-carbonylamino (group)" include $C_{1-6}$ alkoxy-carbonylamino (group).

In the present specification, unless otherwise specified, examples of the "$C_{1-6}$ alkoxy-carbonylamino (group)" include methoxycarbonylamino, ethoxycarbonylamino, propoxycarbonylamino, isopropoxycarbonylamino, butoxycarbonylamino, isobutoxycarbonylamino, sec-butoxycarbonylamino, tert-butoxycarbonylamino, pentyloxycarbonylamino, isopentyloxycarbonylamino, neopentyloxycarbonylamino and hexyloxycarbonylamino.

In the present specification, unless otherwise specified, examples of the "lower alkylsulfonylamino (group)" include $C_{1-6}$ alkylsulfonylamino (group).

In the present specification, unless otherwise specified, examples of the "$C_{1-6}$ alkylsulfonylamino (group)" include methylsulfonylamino, ethylsulfonylamino, propylsulfonylamino, isopropylsulfonylamino, butylsulfonylamino, isobutylsulfonylamino, sec-butylsulfonylamino, tert-butylsulfonylamino, pentylsulfonylamino, isopentylsulfonylamino, neopentylsulfonylamino and hexylsulfonylamino.

In the present specification, unless otherwise specified, examples of the "mono- or di-$C_{3-8}$ cycloalkylamino (group)" include cyclopropylamino, cyclobutylamino, cyclopentylamino and cyclohexylamino.

In the present specification, unless otherwise specified, examples of the "$C_{3-8}$ cycloalkyl-carbonylamino (group)" include cyclopropylcarbonylamino, cyclobutylcarbonylamino; cyclopentylcarbonylamino and cyclohexylcarbonylamino.

In the present specification, unless otherwise specified, examples of the "$C_{3-8}$ cycloalkoxy-carbonylamino (group)" include cyclopropoxycarbonylamino, cyclobutoxycarbonylamino, cyclopentyloxycarbonylamino and cyclohexyloxycarbonylamino.

In the present specification, unless otherwise specified, examples of the "$C_{3-8}$ cycloalkyl-sulfonylamino (group)" include cyclopropylsulfonylamino, cyclobutylsulfonylamino, cyclopentylsulfonylamino and cyclohexylsulfonylamino.

In the present specification, unless otherwise specified, examples of the "mono- or di-$C_{6-14}$ arylamino (group)" include phenylamino and diphenylamino.

In the present specification, unless otherwise specified, examples of the "mono- or di-$C_{7-16}$ aralkylamino (group)" include benzylamino.

In the present specification, unless otherwise specified, examples of the "$C_{6-14}$ aryl-carbonylamino" of the "$C_{6-14}$ aryl-carbonylamino" include benzoylamino, 1-naphthoylamino and 2-naphthoylamino.

In the present specification, unless otherwise specified, examples of the "$C_{6-14}$ arylsulfonylamino" of the "$C_{6-14}$ arylsulfonylamino" include phenylsulfonylamino, 2-naphthylsulfonylamino and 1-naphthylsulfonylamino.

The symbols in the formula (I) is explained in the following.

In the formula (I), ring Cy1 is a 5-membered aromatic heterocycle optionally further substituted besides a group represented by -A-B.

Examples of the "5-membered aromatic heterocycle" of the "5-membered aromatic heterocycle optionally further substituted besides a group represented by -A-B" for ring Cy1 include a 5-membered aromatic heterocycle containing, besides carbon atoms, 1 to 3 heteroatoms selected from an oxygen atom, a sulfur atom and a nitrogen atom (e.g., furan, thiophene, pyrrole, imidazole, pyrazole, thiazole, isothiazole, oxazole, isoxazole, 1,2,3-oxadiazole, 1,2,4-oxadiazole, furazan, 1,2,3-thiadiazole, 1,2,4-thiadiazole, 1,2,3-triazole, 1,2,4-triazole).

The "5-membered aromatic heterocycle" of the "5-membered aromatic heterocycle optionally further substituted besides a group represented by -A-B" for ring Cy1 is preferably a 5-membered nitrogen-containing aromatic heterocycle containing, besides carbon atoms, at least one nitrogen atom and optionally containing 1 or 2 heteroatoms selected from an oxygen atom, a sulfur atom and a nitrogen atom (e.g., pyrrole, imidazole, pyrazole, thiazole, oxazole).

Ring Cy1 preferably has a group represented by -A-B at the following position.

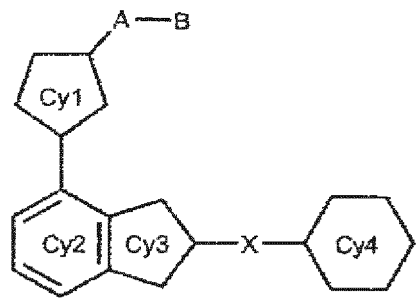

The symbols A and B are explained below.

The "5-membered aromatic heterocycle" of the "5-membered aromatic heterocycle optionally further substituted besides a group represented by -A-B" for ring Cy1 optionally has, addition to the group represented by -A-B, 1 to the maximum substitutable number (preferably 1 or 2) of substituents at substitutable positions. Examples of the substituent include substituents selected from the following Substituent Group A.

[Substituent Group A]

(1) a halogen atom;
(2) nitro;
(3) cyano;
(4) optionally esterified carboxyl [e.g., optionally substituted lower alkoxy-carbonyl, optionally substituted $C_{6-14}$ ary-

loxy-carbonyl, optionally substituted $C_{7-16}$ aralkyloxy-carbonyl and the like];

(5) optionally substituted lower alkyl;
(6) optionally substituted lower alkenyl;
(7) optionally substituted lower alkynyl;
(8) optionally substituted $C_{3-8}$ cycloalkyl;
(9) optionally substituted $C_{6-14}$ aryl;
(10) optionally substituted $C_{7-16}$ aralkyl;
(11) optionally substituted $C_{6-14}$ aryl-$C_{2-6}$ alkenyl;
(12) an optionally substituted heterocyclic group;

(13) hydroxy;
(14) optionally substituted lower alkoxy;
(15) optionally substituted $C_{3-8}$ cycloalkoxy;
(16) optionally substituted $C_{6-14}$ aryloxy;
(17) optionally substituted $C_{7-16}$ aralkyloxy;
(18) optionally substituted lower alkyl-carbonyloxy;
(19) optionally substituted lower alkoxy-carbonyloxy;
(20) optionally substituted mono-lower alkyl-carbamoyloxy;
(21) optionally substituted di-lower alkyl-carbamoyloxy;
(22) optionally substituted $C_{6-14}$ aryl-carbonyloxy;
(23) optionally substituted mono- or di-$C_{6-14}$ aryl-carbamoyloxy;
(24) optionally substituted heterocycleoxy (e.g., optionally substituted aromatic heterocycleoxy);

(25) mercapto;
(26) optionally substituted lower alkylthio;
(27) optionally substituted $C_{3-8}$ cycloalkylthio;
(28) optionally substituted $C_{6-14}$ arylthio;
(29) optionally substituted $C_{7-16}$ aralkylthio;
(30) optionally substituted heterocyclethio;

(31) formyl;
(32) optionally substituted lower alkyl-carbonyl;
(33) optionally substituted $C_{3-8}$ cycloalkyl-carbonyl;
(34) optionally substituted $C_{6-14}$ aryl-carbonyl;
(35) optionally substituted $C_{7-16}$ aralkyl-carbonyl;
(36) optionally substituted heterocyclyl-carbonyl;

(37) optionally substituted lower alkylsulfonyl;
(38) optionally substituted $C_{3-8}$ cycloalkylsulfonyl;
(39) optionally substituted $C_{6-14}$ arylsulfonyl;
(40) optionally substituted heterocyclesulfonyl;
(41) optionally substituted lower alkylsulfinyl;
(42) optionally substituted $C_{3-8}$ cycloalkylsulfinyl;
(43) optionally substituted $C_{6-14}$ arylsulfinyl;
(44) optionally substituted heterocyclesulfinyl;
(45) sulfo;
(46) sulfamoyl;

(47) sulfinamoyl;
(48) sulfenamoyl;
(49) thiocarbamoyl;
(50) an optionally substituted carbamoyl group [e.g., optionally substituted lower alkyl-carbamoyl etc.]; and

(51) an optionally substituted amino group [e.g., amino, optionally substituted mono- or di-lower alkylamino, optionally substituted mono- or di-$C_{3-8}$ cycloalkylamino, optionally substituted mono- or di-$C_{6-14}$ arylamino, optionally substituted mono- or di-$C_{7-16}$ aralkylamino, optionally substituted heterocyclyl-amino, optionally substituted $C_{6-14}$ aryl-carbonylamino, formylamino, optionally substituted lower alkyl-carbonylamino, optionally substituted $C_{3-8}$ cycloalkyl-carbonylamino, optionally substituted heterocyclyl-carbonylamino, optionally substituted lower alkoxy-carbonylamino, optionally substituted $C_{3-8}$ cycloalkoxy-carbonylamino, optionally substituted heterocyclyl-oxycarbonylamino, optionally substituted carbamoylamino, optionally substituted lower alkylsulfonylamino, optionally substituted $C_{3-8}$ cycloalkyl-sulfonylamino, optionally substituted heterocyclyl-sulfonylamino, optionally substituted $C_{6-14}$ arylsulfonylamino]

Examples of the substituent of the above-mentioned
"optionally substituted lower alkoxy-carbonyl",
"optionally substituted lower alkyl",
"optionally substituted lower alkenyl",
"optionally substituted lower alkynyl",
"optionally substituted lower alkoxy",
"optionally substituted lower alkyl-carbcnyloxy",
"optionally substituted lower alkoxy-carbonyloxy",
"optionally substituted mono-lower alkyl-carbamoyloxy",
"optionally substituted di-lower alkyl-carbamoyloxy",
"optionally substituted lower alkylthio",
"optionally substituted lower alkyl-carbonyl",
"optionally substituted lower alkylsulfonyl",
"optionally substituted lower alkylsulfinyl",
"optionally substituted lower alkyl-carbamoyl",
"optionally substituted mono- or di-lower alkylamino",
"optionally substituted lower alkyl-carbonylamino",
"optionally substituted lower alkoxy-carbonylamino", and
"optionally substituted lower alkylsulfonylamino"
include substituents selected from the following Substituent Group B. The number of the substituents is 1 to the maximum substitutable number, more preferably 1 to 3, more preferably 1.

[Substituent Group B]

a halogen atom;
hydroxy;
nitro;
cyano;

$C_{6-14}$ aryl (the $C_{6-14}$ aryl is optionally substituted by a halogen atom, hydroxy, cyano, amino, optionally halogenated $C_{1-6}$ alkyl, mono- or di-$C_{1-6}$ alkylamino, mono- or di-$C_{6-14}$ arylamino, mono- or di-$C_{7-16}$ aralkylamino, $C_{3-8}$ cycloalkyl, $C_{1-6}$ alkoxy, formyl, $C_{1-6}$ alkyl-carbonyl, $C_{3-8}$ cycloalkyl-carbonyl, $C_{6-14}$ aryl-carbonyl, $C_{7-16}$ aralkyl-carbonyl, $C_{1-6}$ alkoxy-carbonyl, $C_{6-14}$ aryloxy-carbonyl, $C_{7-16}$ aralkyloxy-carbonyl, $C_{1-6}$ alkylthio, $C_{1-6}$ alkylsulfinyl, $C_{1-6}$ alkylsulfonyl, carbamoyl, thiocarbamoyl, mono- or di-$C_{1-6}$ alkyl-carbamoyl, mono- or di-$C_{6-14}$ aryl-carbamoyl and the like);
$C_{6-14}$ aryloxy (the $C_{6-14}$ aryloxy is optionally substituted by a halogen atom, hydroxy, cyano, amino, optionally halogenated $C_{1-6}$ alkyl, mono- or di-$C_{1-6}$ alkylamino, mono- or di-$C_{6-14}$ arylamino, mono- or di-$C_{7-16}$ aralkylamino, $C_{3-8}$ cycloalkyl, $C_{1-6}$ alkoxy, formyl, $C_{1-6}$ alkyl-carbonyl, $C_{3-8}$ cycloalkyl-carbonyl, $C_{6-14}$ aryl-carbonyl, $C_{7-16}$ aralkyl-carbonyl, $C_{1-6}$ alkoxy-carbonyl, $C_{6-14}$ aryloxy-carbonyl, $C_{7-16}$ aralkyloxy-carbonyl, $C_{1-6}$ alkylthio, $C_{1-6}$ alkylsulfinyl, $C_{1-6}$ alkylsulfonyl, carbamoyl, thiocarbamoyl, mono- or di-$C_{1-6}$ alkyl-carbamoyl, mono- or di-$C_{6-14}$ aryl-carbamoyl and the like);

$C_{7-16}$ aralkyloxy (the $C_{7-16}$ aralkyloxy is optionally substituted by a halogen atom, hydroxy, cyano, amino, optionally halogenated $C_{1-6}$ alkyl, mono- or di-$C_{1-6}$ alkylamino, mono- or di-$C_{6-14}$ arylamino, mono- or di-$C_{7-16}$ aralkylamino, $C_{3-8}$ cycloalkyl, $C_{1-6}$ alkoxy, formyl, $C_{1-6}$ alkyl-carbonyl, $C_{3-8}$ cycloalkyl-carbonyl, $C_{6-14}$ aryl-carbonyl, $C_{7-16}$ aralkyl-carbonyl, $C_{1-6}$ alkoxy-carbonyl, $C_{6-14}$ aryloxy-carbonyl, $C_{7-16}$ aralkyloxy-carbonyl, $C_{1-6}$ alkylthio, $C_{1-6}$ alkylsulfinyl, $C_{1-6}$ alkylsulfonyl, carbamoyl, thiocarbamoyl, mono- or di-$C_{1-6}$ alkyl-carbamoyl, mono- or di-$C_{6-14}$ aryl-carbamoyl and the like);

a mono- or di- 5- to 10-membered heterocyclic group containing, besides carbon atoms, one to four of one or two kinds of heteroatoms selected from a nitrogen atom, a sulfur atom and an oxygen atom (e.g., furyl, pyridyl, thienyl, pyrrolidino, 1-piperidinyl, 4-piperidyl, piperazyl, 1-morpholinyl, 4-thiomorpholinyl, azepan-1-yl, azocan-1-yl, azonan-1-yl, 3,4-dihydroisoquinolin-2-yl etc.) (the heterocyclic group is optionally substituted by a halogen atom, hydroxy, cyano, amino, optionally halogenated $C_{1-6}$ alkyl, mono- or di-$C_{1-6}$ alkylamino, mono- or di-$C_{6-14}$ arylamino, mono- or di-$C_{7-16}$ aralkylamino, $C_{3-8}$ cycloalkyl, $C_{1-6}$ alkoxy, formyl, $C_{1-6}$ alkyl-carbonyl, $C_{3-8}$ cycloalkyl-carbonyl, $C_{6-14}$ aryl-carbonyl, $C_{7-16}$ aralkyl-carbonyl, $C_{1}$-$_{6}$ alkoxy-carbonyl, $C_{6-14}$ aryloxy-carbonyl, $C_{7-16}$ aralkyloxy-carbonyl, $C_{1-6}$ alkylthio, $C_{1-6}$ alkylsulfinyl, $C_{1-6}$ alkylsulfonyl, carbamoyl, thiocarbamoyl, mono- or di-$C_{1-6}$ alkyl-carbamoyl, mono- or di-$C_{6-14}$ aryl-carbamoyl and the like);

an optionally substituted amino group [e.g., an amino group optionally substituted by 1 or 2 substituents selected from $C_{1-6}$ alkyl, $C_{2-6}$ alkenyl, $C_{6-14}$ aryl, $C_{7-16}$ aralkyl, a heterocyclic group and heterocyclyl-lower alkyl (the $C_{1-6}$ alkyl, $C_{2-6}$ alkenyl, $C_{6-14}$ aryl, $C_{7-16}$ aralkyl, heterocyclic group and heterocyclyl-lower alkyl are each optionally substituted by a halogen atom, hydroxy, cyano, amino, optionally halogenated $C_{1-6}$ alkyl (it is not a substituent for the alkyl or alkenyl), mono- or di-$C_{1-6}$ alkylamino, mono- or di-$C_{6-14}$ arylamino, mono- or di-$C_{7-16}$ aralkylamino, $C_{3-8}$ cycloalkyl, $C_{1-6}$ alkoxy, formyl, $C_{1-6}$ alkyl-carbonyl, $C_{3-8}$ cycloalkyl-carbonyl, $C_{6-14}$ aryl-carbonyl, $C_{7-16}$ aralkyl-carbonyl, $C_{1-6}$ alkoxy-carbonyl, $C_{3-8}$ cycloalkoxy-carbonyl, $C_{6-14}$ aryloxy-carbonyl, $C_{7-16}$ aralkyloxy-carbonyl, $C_{1-6}$ alkylthio, $C_{3-8}$ cycloalkylthio, $C_{1-6}$ alkylsulfinyl, $C_{3-8}$ cycloalkylsulfinyl, $C_{1-6}$ alkylsulfonyl, $C_{3-8}$ cycloalkylsulfonyl, carbamoyl, thiocarbamoyl, mono- or di-$C_{1-6}$ alkyl-carbamoyl, mono- or di-$C_{6-14}$ aryl-carbamoyl and the like. Examples of the "heterocycle" and the "heterocycle" of the heterocyclyl-lower alkyl" include those similar to the above-mentioned "heterocyclic group".];

$C_{3-8}$ cycloalkyl;
$C_{1-6}$ alkoxy (the $C_{1-6}$ alkoxy is optionally substituted by a halogen atom, hydroxy, amino, mono- or di-$C_{1-6}$ alkylamino, mono- or di-$C_{6-14}$ arylamino, $C_{3-8}$ cycloalkyl, $C_{1-6}$ alkoxy, formyl, $C_{1-8}$ alkyl-carbonyl, $C_{3-8}$ cycloalkyl-carbonyl, $C_{6-14}$ aryl-carbonyl, $C_{7-16}$ aralkyl-carbonyl, $C_{1-6}$ alkoxy-carbonyl, $C_{6-14}$ aryloxy-carbonyl, $C_{7-16}$ aralkyloxy-carbonyl, $C_{1-6}$ alkylthio, $C_{1-6}$ alkylsulfinyl, $C_{1-6}$ alkylsulfonyl, carbamoyl, thiocarbamoyl, mono- or di-$C_{1-6}$ alkyl-carbamoyl, mono- or di-$C_{6-14}$ aryl-carbamoyl and the like);

formyl,
$C_{1-6}$ alkyl-carbonyl (e.g., acetyl);
$C_{3-8}$ cycloalkyl-carbonyl;
$C_{6-24}$ aryl-carbonyl;
$C_{7-16}$ aralkyl-carbonyl;
$C_{1-6}$ alkoxy-carbonyl;
$C_{6-14}$ aryloxy-carbonyl;
$C_{7-16}$ aralkyloxy-carbonyl;
$C_{1-6}$ alkylthio;
alkylsulfinyl;
$C_{1-6}$ alkylsulfonyl;
carbamoyl;
thiocarbamoyl;
mono-$C_{1-6}$ alkyl-carbamoyl (e.g., methylcarbamoyl, ethylcarbamoyl etc.);
di-$C_{1-6}$ alkyl-carbamoyl (e.g., dimethylcarbamoyl, diethylcarbamoyl, ethylmethylcarbamoyl etc.);

mono- or di-$C_{6-14}$ aryl-carbamoyl (e.g., phenylcarbamoyl, 1-naphthylcarbamoyl, 2-naphthylcarbamoyl etc.); and mono- or di- 5- to 7-membered heterocyclyl-carbamoyl containing, besides carbon atoms, one to four of one or two kinds of heteroatoms selected from a nitrogen atom, a sulfur atom and an oxygen atom (e.g., 2-pyridylcarbamoyl, 3-pyridylcarbamoyl, 4-pyridylcarbamoyl, 2-thienylcarbamoyl, 3-thienylcarbamoyl etc.)

Examples of the substituent of the above-mentioned
"optionally substituted $C_{6-14}$ aryloxy-carbonyl",
"optionally substituted $C_{7-16}$ aralkyloxy-carbonyl",
"optionally substituted $C_{3-8}$ cycloalkyl",
"optionally substituted $C_{6-14}$ aryl",
"optionally substituted $C_{7-16}$ aralkyl",

"optionally substituted $C_{6-14}$ aryl-$C_{2-6}$ alkenyl",
"optionally substituted heterocyclic group",
"optionally substituted $C_{3-8}$ cycloalkoxy",
"optionally substituted $C_{6-14}$ aryloxy",
"optionally substituted $C_{7-16}$ aralkyloxy",
"optionally substituted $C_{6-14}$ aryl-carbonyloxy",
"optionally substituted mono- or di-$C_{6-14}$ aryl-carbamoyloxy",
"optionally substituted heterocycleoxy",
"optionally substituted aromatic heterocycleoxy",
"optionally substituted $C_{3-8}$ cycloalkylthio",
"optionally substituted $C_{6-14}$ arylthio",
"optionally substituted $C_{7-16}$ aralkylthio",
"optionally substituted heterocyclethio",
"optionally substituted $C_{3-8}$ cycloalkyl-carbonyl",
"optionally substituted $C_{6-14}$ aryl-carbonyl",
"optionally substituted $C_{7-16}$ aralkyl-carbonyl",
"optionally substituted heterocyclyl-carbonyl",
"optionally substituted $C_{3-8}$ cycloalkylsulfonyl",
"optionally substituted $C_{6-14}$ arylsulfanyl",
"optionally substituted heterocyclesulfonyl",
"optionally substituted $C_{3-8}$ cycloalkylsulfinyl",
"optionally substituted $C_{6-14}$ arylsulfinyl",
"optionally substituted heterocyclesulfinyl",
"optionally substituted carbamoyl group",
"optionally substituted amino group",
"optionally substituted mono- or di-$C_{3-8}$ cycloalkylamino",
"optionally substituted mono- or di-$C_{6-14}$ arylamino",
"optionally substituted mono- or di-$C_{7-16}$ aralkylamino",
"optionally substituted heterocyclyl-amino",
"optionally substituted $C_{6-14}$ aryl-carbonylamino",
"optionally substituted $C_{3-8}$ cycloalkyl-carbonylamino",
"optionally substituted heterocyclyl-carbonylamino",
"optionally substituted $C_{3-8}$ cycloalkoxy-carbonylamino",
"optionally substituted heterocyclyl-oxycarbonylamino",
"optionally substituted carbamoylamino",
"optionally substituted lower alkylsulfonylamino",
"optionally substituted $C_{3-8}$ cycloalkyl-sulfonylamino",
"optionally substituted heterocyclyl-sulfonylamino", and
"optionally substituted $C_{6-14}$ arylsulfonylamino"
include substituents selected from the above-mentioned Substituent Group B and the following Substituent Group B'. The number of the substituents is 1 to the maximum substitutable number, more preferably 1 to 3, still more preferably 1.

[Substituent Group B']

$C_{1-6}$ alkyl (the $C_{1-6}$ alkyl is optionally substituted by a halogen atom, hydroxy, cyano, amino, mono- or di-$C_{1-6}$ alkylamino, mono- or di-$C_{6-14}$ arylamino, mono- or di-$C_{7-16}$ aralkylamino, $C_{3-8}$ cycloalkyl, $C_{1-6}$ alkoxy, formyl, $C_{1-6}$ alkyl-carbonyl, $C_{3-8}$ cycloalkyl-carbonyl, $C_{6-14}$ aryl-carbonyl, $C_{7-16}$ aralkyl-carbonyl, $C_{1-6}$ alkoxy-carbonyl, $C_{6-14}$ aryloxy-carbonyl, $C_{7-16}$ aralkyloxy-carbonyl, $C_{1-6}$ alkylthio, $C_{1-6}$ alkylsulfinyl, $C_{1-6}$ alkylsulfonyl, carbamoyl, thiocarbamoyl, mono- or di-$C_{1-6}$ alkyl-carbamoyl, mono- or di-$C_{1-14}$ aryl-carbamoyl and the like); $C_{2-6}$ alkenyl (the $C_{2-6}$ alkenyl is optionally substituted by a halogen atom, hydroxy, cyano, amino, mono- or di-$C_{1-6}$ alkylamino, mono- or di-$C_{6-14}$ arylamino, mono- or di-$C_{7-16}$ aralkylamino, $C_{3-8}$ cycloalkyl, $C_{1-6}$ alkoxy, formyl, $C_{1-6}$ alkyl-carbonyl, $C_{3-8}$ cycloalkyl-carbonyl, $C_{6-14}$ aryl-carbonyl, $C_{7-16}$ aralkyl-carbonyl, $C_{1-6}$ alkoxy-carbonyl, $C_{6-14}$ aryloxy-carbonyl, $C_{7-16}$ aralkyloxy-carbonyl, $C_{1-6}$ alkylthio, $C_{1-6}$ alkylsulfinyl, $C_{1-6}$ alkylsulfonyl, carbamoyl, thiocarbamoyl, mono- or di-$C_{1-6}$ alkyl-carbamoyl, mono- or di-$C_{6-14}$ aryl-carbamoyl and the like); and $C_{2-6}$ alkynyl (the $C_{2-6}$ alkynyl is optionally substituted by a halogen atom, hydroxy, cyano, amino, mono- or di-$C_{1-6}$ alkylamino, mono- or di-$C_{6-14}$ arylamino, mono- or di-$C_{7-16}$ aralkylamino, $C_{3-8}$ cycloalkyl, $C_{1-6}$ alkoxy, formyl, $C_{1-6}$ alkyl-carbonyl, $C_{3-8}$ cycloalkyl-carbonyl, $C_{6-14}$ aryl-carbonyl, $C_{7-16}$ aralkyl-carbonyl, $C_{1-6}$ alkoxy-carbonyl, $C_{6-14}$ aryloxy-carbonyl, $C_{7-16}$ aralkyloxy-carbonyl, $C_{1-6}$ alkylthio, $C_{1-6}$ alkylsulfinyl, $C_{1-6}$ alkylsulfonyl, carbamoyl, thiocarbamoyl, mono- or di-$C_{1-6}$ alkyl-carbamoyl, mono- or di-$C_{6-14}$ aryl-carbamoyl and the like)

Preferable examples of the "substituent" that the "5-membered aromatic heterocycle" of the "5-membered aromatic heterocycle optionally further substituted besides a group represented by -A-B" for ring Cy1 optionally has besides a group represented by -A-B include

(i) optionally substituted lower alkyl, and
(ii) optionally substituted $C_{3-8}$ cycloalkyl,

and more preferable examples thereof include

(i) $C_{1-6}$ alkyl optionally substituted by 1 to 3 substituents selected from a halogen atom, hydroxy and $C_{1-6}$ alkoxy, and
(ii) $C_{3-8}$ cycloalkyl.

In another embodiment, the "5-membered aromatic heterocycle" of the "5-membered aromatic heterocycle optionally further substituted besides a group represented by -A-B" for ring Cy1 preferably does not have a substituent other than -A-B.

Ring Cy1 is preferably a 5-membered nitrogen-containing aromatic heterocycle (e.g., pyrrole, imidazole, pyrazole, thiazole, oxazole) optionally further substituted by 1 or 2 substituents selected from

(i) optionally substituted lower alkyl, and
(ii) optionally substituted $C_{3-8}$ cycloalkyl,

besides a group represented by -A-B.

Ring Cy1 is more preferably a 5-membered nitrogen-containing aromatic heterocycle containing, besides carbon atoms, at least one nitrogen atom and optionally containing 1 or 2 heteroatoms selected from an oxygen atom, a sulfur atom and a nitrogen atom (e.g., pyrrole, imidazole, pyrazole, thiazole, oxazole) and optionally further substituted by 1 or 2 substituents selected from

(i) $C_{1-6}$ alkyl (e.g., methyl, isopropyl) optionally substituted by 1 to 3 substituents selected from a halogen atom (e.g., fluorine atom), hydroxy and $C_{1-6}$ alkoxy (e.g., methoxy), and
(ii) $C_{3-8}$ cycloalkyl (e.g., cyclopropyl),

besides a group represented by -A-B.

In the formula (I), ring Cy2 is an optionally substituted benzene ring.

The "benzene ring" of the "optionally substituted benzene ring" for ring Cy2 optionally has 1 to the maximum substitutable number (preferably 1 or 2) of substituents at substitutable positions. Examples of the substituent include substituents selected from the above-mentioned Substituent Group A.

Ring Cy2 is preferably a benzene ring optionally substituted by 1 or 2 substituents selected from

(i) a halogen atom (e.g., fluorine atom), and
(ii) $C_{1-6}$ alkyl.

Ring Cy2 is more preferably a benzene ring optionally substituted by 1 or 2 halogen atoms.

In the formula (I), ring Cy3 is an optionally substituted 5-membered aromatic heterocycle.

Examples of the "5-membered aromatic heterocycle" of the "optionally substituted 5-membered aromatic heterocycle" for ring Cy3 include those similar to the "5-membered aromatic heterocycle" of the "5-membered aromatic heterocycle optionally further substituted besides a group represented by -A-B" for ring Cy1.
The "5-membered aromatic heterocycle" of the "optionally substituted 5-membered aromatic heterocycle" for ring Cy3 is preferably thiophene, furan, pyrazole or thiazole, more preferably thiophene, furan or pyrazole.

The "5-membered aromatic heterocycle" of the "optionally substituted 5-membered aromatic heterocycle" for ring Cy3 optionally has 1 to the maximum substitutable number (preferably 1 or 2) of substituents at substitutable positions. Examples of the substituent include substituents selected from the above-mentioned Substituent Group A.
The substituent is preferably $C_{1-6}$ alkyl.
In another embodiment, the "5-membered aromatic heterocycle" of the "optionally substituted 5-membered aromatic heterocycle" for ring Cy3 preferably does not have a substituent.

Ring Cy3 is preferably a 5-membered aromatic heterocycle optionally substituted by $C_{1-6}$ alkyl,
more preferably a 5-membered aromatic heterocycle containing, besides carbon atoms, 1 to 3 (preferably 1 or 2) heteroatoms selected from an oxygen atom, a sulfur atom and a nitrogen atom (e.g., thiophene, furan, pyrazole, thiazole) and optionally substituted by 1 or 2 $C_{1-6}$ alkyl.

Ring Cy3 is more preferably a 5-membered aromatic heterocycle containing, besides carbon atoms, 1 to 3 (preferably 1 or 2) heteroatoms selected from an oxygen atom, a sulfur atom and a nitrogen atom (e.g., thiophene, furan, pyrazole) (does not have a substituent).

In the formula (I), ring Cy4 is an optionally substituted 6-membered aromatic ring.

Examples of the "6-membered aromatic ring" of the "optionally substituted 6-membered aromatic ring" for ring Cy4 include a 6-membered aromatic ring containing, besides carbon atoms, 1 to 3 (preferably 1 or 2) nitrogen atoms. Preferable examples thereof include benzene, pyridine, pyridazine, pyrimidine, pyrazine and triazine. Of these, benzene is preferable.

The "6-membered aromatic ring" of the "optionally substituted 6-membered aromatic ring" for ring Cy4 optionally has 1 to the maximum substitutable number (preferably 1 to 3, more preferably 1 or 2) of substituents at substitutable positions. Examples of the substituent include substituents selected from the above-mentioned Substituent Group A. Preferable examples of the substituent include

(i) a halogen atom,
(ii) cyano,
(iii) optionally substituted lower alkyl,
(iv) optionally substituted lower alkoxy, and
(v) optionally substituted lower alkylsulfonyl,

more preferable examples thereof include

(i) a halogen atom,
(ii) $C_{1-6}$ alkyl optionally substituted by 1 to 3 halogen atoms, and
(iii) $C_{1-6}$ alkoxy, and

still more preferable examples thereof include

(i) a halogen atom, and
(ii) $C_{1-6}$ alkyl optionally substituted by 1 to 3 halogen atoms.

Ring Cy4 is preferably a 6-membered aromatic ring (e.g., benzene, pyridine, pyridazine, pyrimidine, pyrazine, triazine) optionally substituted by 1 to 3 substituents selected from (i) a halogen atom,

(ii) cyano,
(iii) optionally substituted lower alkyl,
(iv) optionally substituted lower alkoxy, and
(v) optionally substituted lower alkylsulfonyl.

Ring Cy4 is more preferably a benzene ring optionally substituted by 1 to 3 substituents selected from

(i) a halogen atom (e.g., fluorine atom, chlorine atom),
(ii) $C_{1-6}$ alkyl optionally substituted by 1 to 3 halogen atoms (e.g., methyl, trifluoromethyl), and
(iii) $C_{1-6}$ alkoxy (e.g., methoxy),

more preferably a benzene ring optionally substituted by 1 to 3 substituents selected from

(i) a halogen atom, and
(ii) $C_{1-6}$ alkyl optionally substituted by 1 to 3 halogen atoms.

In the formula (I), A is $-CONR^a-$ or $-NR^aCO-$.
A is preferably $-CONR^a-$ or -NHCO-, more preferably - $CONR^a-$.

In the formula (I), $R^a$ is a hydrogen atom or a substituent.
Examples of the "substituent" for $R^a$ include substituents selected from the above-mentioned Substituent Group A.
$R^a$ is preferably a hydrogen atom, an optionally substituted $C_{1-6}$ alkyl group or a $C_{3-8}$ cycloalkyl group.

$R^2$ is more preferably

(i) a hydrogen atom,
(ii) a $C_{1-6}$ alkyl group optionally substituted by one cyano group (e.g., 2-cyanoethyl), or

(iii) a $C_{3-8}$ cycloalkyl group (e.g., cyclopropyl).

$R^a$ is more preferably a hydrogen atom or a $C_{1-6}$ alkyl group.

A is preferably -CCNR$^a$- wherein $R^a$ is a hydrogen atom, an optionally substituted $C_{1-6}$ alkyl group or a $C_{3-8}$ cycloalkyl group, preferably (i) a hydrogen atom, (ii) a $C_{1-6}$ alkyl group optionally substituted by one cyano group (e.g., 2-cyanoethyl), or (iii) a $C_{3-8}$ cycloalkyl group (e.g., cyclopropyl).

In the formula (I), B is a hydrogen atom or an optionally substituted $C_{1-6}$ alkyl group (preferably methyl, ethyl). The "$C_{1-6}$ alkyl group" of the "optionally substituted $C_{1-6}$ alkyl group" for B optionally has 1 to the maximum substitutable number (preferably 1 to 3) of substituents at substitutable positions.
Examples of the substituent include substituents selected from the above-mentioned Substituent Group A.
B is preferably

(i) a hydrogen atom, or
(ii) a $C_{1-6}$ alkyl group (e.g., methyl, ethyl) optionally substituted by 1 to 3 substituents selected from

(a) hydroxy,
(b) $C_{1-6}$ alkoxy (e.g., methoxy, isopropoxy), and
(c) carbamoyl.

In the formula (I), X is an optionally substituted $C_{1-2}$ alkylene, -Y-, -Y-$CH_2$- or -$CH_2$Y-.
The "optionally substituted $C_{1-2}$ alkylene" for X optionally has 1 to the maximum substitutable number (preferably 1 to 3) of substituents at substitutable positions.
Examples of the substituent include substituents selected from the above-mentioned Substituent Group A.
The "$C_{1-2}$ alkylene" of the "optionally substituted $C_{1-2}$ alkylene" for X is preferably unsubstituted.
X is preferably an optionally substituted $C_{1-2}$ alkylene, or -$CH_2$-Y-, more preferably a $C_{1-2}$ alkylene (e.g., methylene).

In the formula (I), Y is an oxygen atom (-O-), -NR$^b$- or - S(O)$_m$-.
In the formula (I), $R^b$ is a hydrogen atom or a substituent.
Examples of the substituent for $R^b$ include substituents selected from the above-mentioned Substituent Group A.
$R^b$ is preferably a hydrogen atom or a $C_{1-6}$ alkyl group.
In another embodiment, $R^b$ is preferably a substituent, more preferably a $C_{1-6}$ alkyl group.
In the formula (I), m is 0, 1 or 2.

The substituent, moiety and ring in the formula (I) exemplified above are more preferably used in combination.

Compound (I) is preferably, for example, the following compound.

[Compound A]

A compound wherein
ring Cy1 is a 5-membered nitrogen-containing aromatic heterocycle containing, besides carbon atoms, at least one nitrogen atom and optionally containing 1 or 2 heteroatoms selected from an oxygen atom, a sulfur atom and a nitrogen atom (e.g., pyrrole, imidazole, pyrazole, thiazole, oxazole) and optionally substituted by 1 or 2 substituents selected from

(i) $C_{1-6}$ alkyl optionally substituted by 1 to 3 substituents selected from a halogen atom, hydroxy and $C_{1-6}$ alkoxy, and
(ii) $C_{3-8}$ cycloalkyl,

besides a group represented by -A-B,
ring Cy2 is a benzene ring optionally substituted by 1 or 2 substituents selected from

(i) a halogen atom, and
(ii) $C_{1-6}$ alkyl,

ring Cy3 is a 5-membered aromatic heterocycle containing, besides carbon atoms, 1 to 3 heteroatoms selected from an oxygen atom, a sulfur atom and a nitrogen atom (e.g., thiophene, furan, pyrazole, thiazole) and optionally substituted by 1 or 2 $C_{1-6}$ alkyl groups,
ring Cy4 is a benzene ring optionally substituted by 1 to 3 substituents selected from

(i) a halogen atom, and
(ii) $C_{1-6}$ alkyl optionally substituted by 1 to 3 halogen atoms, A is -CONH-,

B is

(i) a hydrogen atom, or
(ii) a $C_{1-6}$ alkyl group optionally substituted by 1 to 3 substituents selected from

(a) hydroxy,
(b) $C_{1-6}$ alkoxy, and
(c) carbamoyl,

X is $C_{1-2}$ alkylene or $-CH_2-Y-$,
Y is an oxygen atom (-O-), $-NR^b-$ or $-S(O)_m-$,
$R^b$ is a hydrogen atom or a $C_{1-6}$ alkyl group, and
m is 0, 1 or 2.

[Compound B]

ring Cy1 is a 5-membered nitrogen-containing aromatic heterocycle containing, besides carbon atoms, at least one nitrogen atom and optionally containing 1 or 2 heteroatoms selected from an oxygen atom, a sulfur atom and a nitrogen atom (e.g., pyrrole, imidazole, pyrazole, thiazole, oxazole) and optionally substituted by 1 or 2 substituents selected from

(i) $C_{1-6}$ alkyl optionally substituted by 1 to 3 substituents selected from a halogen atom, hydroxy and $C_{1-6}$ alkoxy, and
(ii) $C_{3-8}$ cycloalkyl,

besides a group represented by -A-B,
ring Cy2 is a benzene ring optionally substituted by 1 or 2 halogen atoms,
ring Cy3 is a 5-membered aromatic heterocycle containing, besides carbon atoms, 1 to 3 heteroatoms selected from an oxygen atom, a sulfur atom and a nitrogen atom (e.g., thiophene, furan, pyrazole),
ring Cy4 is a benzene ring optionally substituted by 1 to 3 substituents selected from

(i) a halogen atom, and
(ii) $C_{1-6}$ alkyl optionally substituted by 1 to 3 halogen atoms, and
(iii) $C_{1-6}$ alkoxy (e.g., methoxy),

A is $-CONR^2-$ wherein $R^a$ is a hydrogen atom, a $C_{1-6}$ alkyl group optionally substituted by one cyano group, or a $C_{3-8}$ cycloalkyl group, or -NHCO-,
B is

(i) a hydrogen atom, or
(ii) a $C_{1-6}$ alkyl group optionally substituted by 1 to 3 substituents selected from

(a) hydroxy,
(b) $C_{1-6}$ alkoxy, and
(c) carbamoyl, and

X is a $C_{1-2}$ alkylene.
Specifically, compound (I) is preferably the below-mentioned compounds of Examples 1 to 111, particularly preferably
N-(2-methoxyethyl)-3-methyl-1-[2-[3-(trifluoromethyl)benzyl]-1-benzothiophen-7-yl]-1H-pyrazole-4-carboxamide,
N-(2-hydroxyethyl)-3-methyl-1-[2-[3-(trifluoromethyl)benzyl]-1-benzothiophen-7-yl]-1H-pyrazole-4-carboxamide,
3-methyl-1-[2-[3-(trifluoromethyl)benzyl]-1-benzothiophen-7-yl]-1H-pyrazole-4-carboxamide,
N-(2-amino-2-oxoethyl)-1-[4-fluoro-2-[3-(triflioromethy1)benzyl]-1-benzothiophen-7-yl]-3,5-dimethyl-1H-pyrazole-4-carboxamide,
N-(2-hydroxyethyl)-5-(hydroxymethyl)-3-methyl-1-[2-[3-(trifluoromethyl)benzyl]-1-benzothiophen-7-yl]-1H-pyrazole-4-carboxamide,
or a salt thereof.
When compound (I) is a salt, examples thereof include metal salts, ammonium salt, salts with organic base, salts with inorganic acid, salts with organic acid, salts with basic or acidic amino acid, and the like. Preferable examples

of the metal salt include alkaline metal salts such as sodium salt, potassium salt and the like; alkaline earth metal salts such as calcium salt, magnesium salt, barium salt and the like; aluminum salt and the like. Preferable examples of the salt with organic base include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine and the like. Preferable examples of the salt with inorganic acid include salts with hydrochloric acid, hydrobromic acid, nitric acid, sulfuric acid, phosphoric acid and the like. Preferable examples of the salt with organic acid include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like. Preferable examples of the salt with basic amino acid include salts with arginine, lysine, ornithine and the like. Preferable examples of the salt with acidic amino acid include salts with aspartic acid, glutamic acid and the like. Among them, pharmaceutically acceptable salts are preferable. For example, if the compound has an acidic functional group therein, examples of the salt include inorganic salts such as alkaline metal salts (e.g., sodium salt, potassium salt etc.), alkaline earth metal salt (e.g., calcium salt, magnesium salt, barium salt etc.); ammonium salt and the like. If the compound has a basic functional group therein, examples of the salt thereof include salts with inorganic acid such as hydrochloric acid, hydrobromic acid, nitric acid, sulfuric acid, phosphoric acid and the like, and salts with organic acid such as acetic acid, phthalic acid, fumaric acid, oxalic acid, tartaric aid, maleic acid, citric aid, succinic acid, methanesulfonic acid, p-toluenesulfonic acid and the like.

When compound (I) has an isomer such as tautomer, optical isomer, stereoisomer, positional isomer, rotational isomer and the like, any isomer and a mixture thereof are encompassed in the of the present invention. In addition, when compound (I) has an optical isomer, an optical isomer resolved from a racemate is also encompassed in compound (I).

Compound (I) may be a crystal, and both a single crystal and crystal mixtures are encompassed in compound (I). Compound (I) may be a solvate (e.g., hydrate etc.) or a non-solvate, and both are encompassed in compound (I).
A compound labeled or substituted with an isotope (e.g., $^{2}H$, $^{3}H$, $^{11}C$, $^{14}C$, $^{18}F$, $^{35}S$, $^{125}I$ etc.) is also encompassed in compound (I).

[Production Method]

The production method of the compound of the present invention is explained in the following.
Compound (I) and compound (Ia) - (Id) which are encompassed in compound (I) are obtained, for example, according to the methods shown in the following reaction schemes, a method analogous thereto or the like. Each symbol for the compound in reaction scheme is as defined above. The compound in reaction scheme may be in the form of a salt. Examples thereof include those similar to the salt of compound (I), and the like. In addition, the compound obtained in each step can be used directly as the reaction mixture or as a crude product for the next reaction, or can be isolated from the reaction mixture according a conventional method, and can be easily purified according to known separation means, for example, extraction, concentration, neutralization, filtration, distillation, recrystallization, chromatography and the like. When the compound in the reaction scheme is commercially available, the commercially available product can also be used directly.

Compound (Ia) can be produced according to the method described in the following Reaction Scheme 1.

Reaction Scheme 1

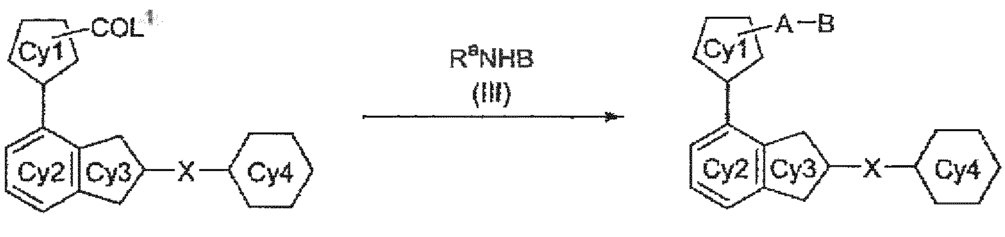


(IIa) (Ia)

wherein $L^1$ is a leaving group.

Compound (Ia) can be produced by reacting compound (IIa) with compound (III) in the presence of a base or an acid if desired.
Compound (III) may be commercially available products, or can be produced according to a method known per se or a method analogous thereto.
Examples of the "leaving group" for $L^1$ include a hydroxy group, a halogen atom (e.g. fluorine, chlorine, bromine, iodine),

$C_{1-6}$ alkoxy (e.g., methoxy, ethoxy, propoxy, isopropoxy, butoxy, isobutoxy, sec-butoxy, pentyloxy, hexyloxy etc.) which may be halogenated, an optionally substituted $C_{1-5}$ alkylsulfonyloxy group (e.g., methanesulfonyloxy, ethanesulfonyloxy, trichloromethanesulfonyloxy etc.), an optionally substituted $C_{6-10}$ arylsulfonyloxy group, an optionally substituted phenyloxy group, an optionally substituted benzothiazol-2-ylthio group and the like.

Examples of the "optionally substituted $C_{6-10}$ arylsulfonyloxy group" include $C_{6-10}$ arylsulfonyloxy (e.g., phenylsulfonyloxy, naphthylsulfonyloxy etc.) optionally having 1 to 3 substituents selected from $C_{1-6}$ alkyl (e.g., methyl, ethyl, propyl, isopropyl, butyl, isobutyl, sec-butyl, tert-butyl, pentyl, hexyl etc.), $C_{1-6}$ alkoxy (e.g., methoxy, ethoxy, propoxy, isopropoxy, butoxy, isobutoxy, sec-butoxy, pentyloxy, hexyloxy etc.) and nitro, and the like, and specific examples thereof include benzenesulfonyloxy, m-nitrobenzenesulfonyloxy, p-toluenesulfonyloxy and the like.

Examples of the "optionally substituted phenyloxy group" include a phenyloxy group optionally having 1 to 3 substituents selected from $C_{1-6}$ alkyl (e.g., methyl, ethyl, propyl, isopropyl, butyl, isobutyl, sec-butyl, tert-butyl, pentyl, hexyl etc.), $C_{1-6}$ alkoxy (e.g., methoxy, ethoxy, propoxy, isopropoxy, butoxy, isobutoxy, sec-butoxy, pentyloxy, hexyloxy etc.) and nitro, and the like, and specific examples thereof include phenyloxy, 4-nitrophenoxy and the like.

Examples of the optionally substituted benzothiazol-2-ylthio group include a benzothiazol-2-ylthio group optionally having 1 to 3 substituents selected from $C_{1-6}$ alkyl (e.g., methyl, ethyl, propyl, isopropyl, butyl, isobutyl, sec-butyl, tert-butyl, pentyl, hexyl etc.), $C_{1-6}$ alkoxy (e.g., methoxy, ethoxy, propoxy, isopropoxy, butoxy, isobutoxy, sec-butoxy, pentyloxy, hexyloxy etc.) and nitro, and the like, and specific examples thereof include benzothiazol-2-ylthio and the like.

The amount of compound (III) to be used is about 1 to 10 mol, preferably about 1 to 2 mol, per 1 mol of compound (IIa).

Examples of the "base" include basic salts such as sodium carbonate, potassium carbonate, cesium carbonate, sodium hydrogen carbonate and the like; aromatic amines such as pyridine, lutidine and the like; tertiary amines such as triethylamine, N,N-diisopropylethylamine, tripropylamine, tributylamine, cyclohexyldimethylamine, 4-dimethylaminopyridine, N-methylpiperidine, N-methylpyrrolidine, N-methylmorpholine and the like; alkali metal hydrides such as sodium hydride, potassium hydride and the like; metal amides such as sodium amide, lithium diisopropylamide, lithium hexamethyldisilazide and the like; metal alkoxides such as sodium methoxide, sodium ethoxide, potassium tert-butoxide and the like; and the like.

The amount of the "base" to be used is generally about 0.1 to 10 equivalents, preferably 0.8 to 2 equivalents, relative to compound (IIa).

Examples of the "acid" include methanesulfonic acid, p-toluenesulfonic acid, camphorsulfonic acid and the like.

The amount of the "acid" to be used is generally about 0.1 to 10 equivalents, preferably 0.8 to 3 equivalents, relative to compound (IIa).

This reaction is advantageously carried out without a solvent or in a solvent inert to the reaction. The solvent is not particularly limited as long as the reaction proceeds, and preferable examples thereof include water; ethers such as diethyl ether, tetrahydrofuran, 1,4-dioxane, 1,2-dimethoxyethane and the like; hydrocarbons such as benzene, toluene, cyclohexane, hexane and the like; amides such as N,N-dimethylformamide, N,N-dimethylacetamide and the like; halogenated hydrocarbons such as dichloromethane, chloroform, carbon tetrachloride, 1,2-dichloroethane and the like; nitriles such as acetonitrile, propionitrile and the like; sulfoxides such as dimethylsulfoxide and the like; nitrogen-containing aromatic hydrocarbons such as pyridine, lutidine, quinoline and the like, a mixed solvent thereof, and the like.

The reaction temperature is generally about -40 to 150°C, preferably 0 to 100°C.

The reaction time is generally 5 min to 24 hr, preferably 10 min to 5 hr.

Alternatively, When $L^1$ is OH, compound (IIa) may be reacted with compound (III) in the presence of an appropriate condensing agent.

The amount of compound (III) to be used is generally about 0.8 to about 10 mol, preferably about 0.8 to about 2 mol, per 1 mol of compound (IIa).

Examples of the "condensing agent" include N,N'-carbodiimides such as N,N'-dicyclohexylcarbodiimide, 1-ethyl-3-(3-dimethylaminopropyl)carbodiimide hydrochloride (WSC) and the like; azorites such as N,N'-carbonylimidazole and the like; 2-halogenopyridinium salts such as 2-chloro-1-methylpyridinium iodide, 2-fluoro-1-methylpyridinium iodide and the like; N-ethoxycarbonyl-2-ethoxy-1,2-dihydroquinoline, 2-(7-aza-1H-benzotriazol-1-yl)-1,1,3,3-tetramethyluronium hexafluorophosphate (HATU), 4-(4,6-dimethoxy-1,3,5-triazin-2-yl)-4-methylmorpholinium chloride n-hydrate (DMTMM), diethyl phosphorocyanidate, phosphorus oxychloride, acetic anhydride and the like.

The amount of the "condensing agent" to be used is generally about 0.8 to about 5 mol, preferably about 1 to about 3 mol, per 1 mol of compound (IIa).

The reaction may be carried out in the presence of a base if desired. Examples of the "base" include basic salts such as potassium acetate, sodium acetate and the like; tertiary amines such as triethylamine, N,N-diisopropylethylamine, tripropylamine, tributylamine, cyclohexyldimethylamine, 4-dimethylaminopyridine, N-methylpiperidine, N-methylpyrrolidine, N-methylmorpholine and the like; and the like. In addition, the reaction may be carried out in the presence of a condensation promoter such as 1-hydroxy-1H-benzotriazole (HOBt) monohydrate and the like if desired.

The amount of the "base" to be used is generally about 0.5 to about 5 mol, preferably about 2 to about 3 mol, per 1 mol

of compound (IIa).

This reaction is advantageously carried out in a solvent inert to the reaction. Preferable examples of the solvent include alcohols such as methanol, ethanol, propanol and the like; hydrocarbons such as hexane, cyclohexane, benzene, toluene, xylene and the like; ethers such as diethyl ether, diisopropyl ether, tetrahydrofuran, 1,4-dioxane, 1,2-dimethoxyethane and the like; amides such as N,N-dimethylformamide, N,N-dimethylacetamide, hexamethylphosphoric triamide, 1-methylpyrrolidin-2-one and the like; sulfoxides such as dimethylsulfoxide and the like; halogenated hydrocarbons such as dichloromethane, chloroform, carbon tetrachloride, 1,2-dichloroethane and the like; nitriles such as acetonitrile, propionitrile and the like; acid anhydrides such as acetic anhydride and the like; a mixed solvent thereof; and the like.

The reaction time is generally about 10 min to about 48 hr, preferably about 30 min to about 24 hr.

The reaction temperature is generally about -20 to about 150°C, preferably about 0 to about 100°C.

The reaction time can be shortened using a microwave reactor or the like.

Compound (Ia) thus obtained can be isolated from the reaction mixture according a conventional method, and can be isolated and purified according to known separation and purification means, for example, concentration, concentration under reduced pressure, solvent extraction, crystallization, recrystallization, phase transfer, chromatography and the like.

Compound (Ib) can be produced according to the method described in the following Reaction Scheme 2.

Reaction Scheme 2

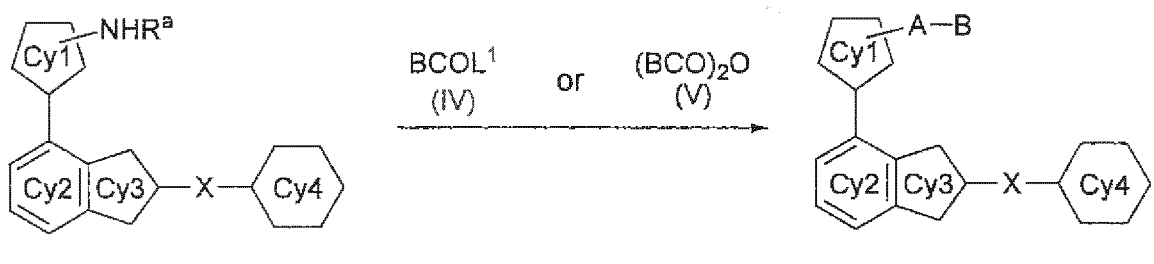


(IIb) (Ib)

wherein the symbols are as defined above.

Compound (Ib) can be produced by reacting compound (IIb) with compound (IV) or compound (V) in the presence of a base or an acid if desired.

Compound (IV) or compound (V) may be commercially available product, or can be produced according to a method known per se or a method analogous thereto.

The amount of compound (IV) or compound (V) to be used is about 1 to 10 mol, preferably about 1 to 2 mol, per 1 mol of compound (IIb).

Examples of the "base" include basic salts such as sodium carbonate, potassium carbonate, cesium carbonate, sodium hydrogen carbonate and the like; aromatic amines such as pyridine, lutidine and the like; tertiary amines such as triethylamine, N,N-diisopropylethylamine, tripropylamine, tributylamine, cyclohexyldimethylamine, 4-dimethylaminopyridine, N-methylpiperidine, N-methylpyrrolidine, N-methylmorpholine and the like; alkali metal hydrides such as sodium hydride, potassium hydride and the like; metal amides such as sodium amide, lithium diisopropylamide, lithium hexamethyldisilazide and the like; metal alkoxides such as sodium methoxide, sodium ethoxide, potassium tert-butoxide and the like; and the like.

The amount of the "base" to be used is generally about 0.1 to 10 equivalents, preferably 0.8 to 2 equivalents, relative to compound (IIb).

Examples of the "acid" include methanesulfonic acid, p-toluenesulfonic acid, camphorsulfonic acid and the like. The amount of the "acid" to be used is generally about 0.1 to 10 equivalents, preferably 0.8 to 3 equivalents, relative to compound (IIb).

This reaction is advantageously carried out without a solvent or in a solvent inert to the reaction. The solvent is not particularly limited as long as the reaction proceeds, and preferable examples thereof include water; ethers such as diethyl ether, tetrahydrofuran, 1,4-dioxane, 1,2-dimethoxyethane and the like; hydrocarbons such as benzene, toluene, cyclohexane, hexane and the like; amides such as N,N-dimethylformamide, N,N-dimethylacetamide and the like; halogenated hydrocarbons such as dichloromethane, chloroform, carbon tetrachloride, 1,2-dichloroethane and the like; nitriles such as acetonitrile, propionitrile and the like; sulfoxides such as dimethylsulfoxide and the like; nitrogen-containing

aromatic hydrocarbons such as pyridine, lutidine, quinoline and the like; a mixed solvent thereof; and the like.
The reaction temperature is generally about -40 to 150°C, preferably 0 to 100°C.
The reaction time is generally 5 min to 24 hr, preferably 10 min to 5 hr.

Alternatively, compound (II) may be reacted with BCOOH in the presence of an appropriate condensing agent. The amount of the BCOOH to be used is generally about 0.8 to about 10 mol, preferably about 0.8 to about 2 mol, per 1 mol of compound (IIb).

Examples of the "condensing agent" include N,N'-carbodiimides such as N,N'-dicyclohexylcarbodiimide, 1-ethyl-3-(3-dimethylaminopropyl)carbodiimide hydrochloride (WSC) and the like; azorites such as N,'N'-carbonylimidazole and the like; 2-halogenopyridinium salts such as 2-chloro-1-methylpyridinium iodide, 2-fluoro-1-methylpyridinium iodide and the like; N-ethoxycarbonyl-2-ethoxy-1,2-dihydroquinoline, diethyl phosphorocyanidate, phosphorus oxychloride, acetic anhydride and the like.
The amount of the "condensing agent" to be used is generally about 0.8 to about 5 mol, preferably about 1 to about 3 mol, per 1 mol of compound (IIb).

The reaction may be carried out in the presence of a base if desired. Examples of the "base" include basic salts such as potassium acetate, sodium acetate and the like; tertiary amines such as triethylamine, N,N-diisopropylethylamine, tripropylamine, tributylamine, cyclohexyldimethylamine, 4-dimethylaminopyridine, N-methylpiperidine, N-methylpyrrolidine, N-methylmorpholine and the like; and the like. In addition, the reaction may be carried out in the presence of a condensation promoter such as 1-hydroxy-1H-benzotriazole (HOBt) monohydrate and the like if desired.
The amount of the "base" to be used is generally about 0.5 to about 5 mol, preferably about 2 to about 3 mol, per 1 mol of compound (IIb).

This reaction is advantageously carried out in a solvent inert to the reaction. Preferable examples of the solvent include alcohols such as methanol, ethanol, propanol and the like; hydrocarbons such as hexane, cyclohexane, benzene, toluene, xylene and the like; ethers such as diethyl ether, diisopropyl ether, tetrahydrofuran, 1,4-dioxane, 1,2-dimethoxyethane and the like; amides such as N,N-dimethylformamide, N,N-dimethylacetamide, hexamethylphosphoric triamide, 1-methylpyrrolidin-2-one and the like; sulfoxides such as dimethylsulfoxide and the like; halogenated hydrocarbons such as dichloromethane, chloroform, carbon tetrachloride, 1,2-dichloroethane and the like; nitriles such as acetonitrile, propionitrile and the like; acid anhydrides such as acetic anhydride and the like; a mixed solvent thereof; and the like.
The reaction time is generally about 10 min to about 48 hr, preferably about 30 min to about 24 hr.
The reaction temperature is generally about -20 to about 150°C, preferably about 0 to about 100°C.
The reaction time can be shortened using a microwave reactor or the like.

Compound (Ib) thus obtained can be isolated from the reaction mixture according a conventional method, and can be isolated and purified according to known separation and purification means, for example, concentration, concentration under reduced pressure, solvent extraction, crystallization, recrystallization, phase transfer, chromatography and the like.

Compound (I) can be produced according to the method described in the following Reaction Scheme 3.

Reaction Scheme 3

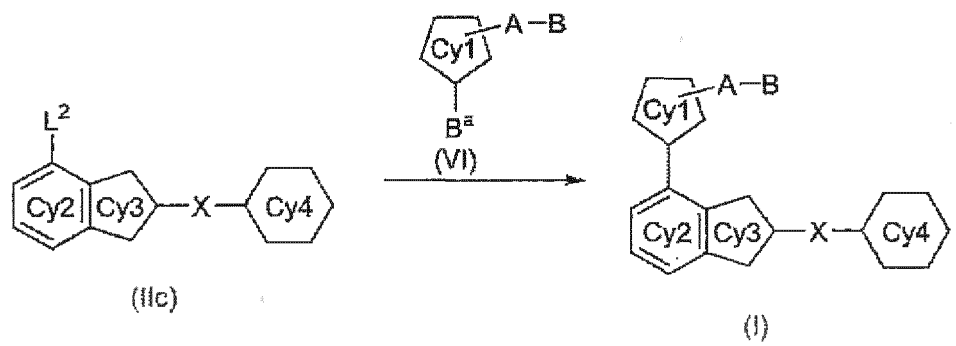

wherein $L^2$ is a leaving group, $B^a$ is $B(OR^c)_2$ wherein each of $R^c$ is independently a hydrogen atom or a $C_{1-6}$ alkyl group, or two $R^c$ in combination form a $C_{2-6}$ alkylene chain, and other symbols are as defined above.

Examples of the $C_{2-6}$ alkylene chain formed by two $R^c$ in combination include $-CH_2-CH_2-$, $-C(CH_3)_2-C(CH_3)_2-$, $-CH_2-CH_2-CH_2-$and $-CH_2-C(CH_3)_2-CH_2-$ and the like.

Compound (I) is produced by subjecting compound (IIc) to the Suzuki coupling with compound (VI).

The reaction is carried out by reacting compound (IIc) with boronic acid (VI) in the presence of a base and a transition metal catalyst in a solvent.
Compound (VI) may be commercially available product, or can be produced according to a method known per se or a method analogous thereto.
Examples of the "leaving group" for $L^2$ include a halogen atom (e.g., chlorine, bromine, iodine), an optionally halogenated $C_{1-5}$alkylsulfonyloxy group (e.g., trifluoromethanesulfonyloxy etc.) and the like.
Examples of the functional group represented by $B(OR^c)_2$ include boronic acid, borates (e.g., 4,4,5,5-tetramethyl-1,3,2-dioxaborolan-2-yl etc.) and the like.
The amount of the "boronic acid (VI)" to be used is about 0.5 to about 10 mol, preferably about 0.9 to about 3 mol, per 1 mol of compound (IIc).

Examples of the "base" include basic salts such as sodium carbonate, potassium carbonate, cesium carbonate, sodium hydrogen carbonate and the like; aromatic amines such as pyridine, lutidine and the like; tertiary amines such as triethylamine, N,N-diisopropylethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like; metal alkoxides such as sodium methoxide, sodium ethoxide, sodium tert-butoxide, potassium tert-butoxide and the like; and the like.

Examples of the "transition metal catalyst" include palladium catalyst such as palladium acetate, palladium chloride, tetrakis(triphenylphosphine)palladium, 1,1-bis(diphenylphosphino)ferrocenedichloropalladium, dichlorobis (triphenylphosphine)palladium and the like; and the like. The amount of the transition metal catalyst to be used is about 0.001 to about 3 mol, preferably about 0.02 to about 0.2 mol, per 1 mol of compound (IIc).

Examples of the solvent include ethers such as diethyl ether, diisopropyl ether, tetrahydrofuran, dioxane, 1,2-dimethoxyethane and the like; alcohols such as methanol, ethanol, propanol and the like; hydrocarbons such as benzene, toluene, carbon disulfide, cyclohexane, hexane and the like: amides such as N,N-dimethylformamide, N,N-dimethylacetamide and the like; halogenated hydrocarbons such as dichloromethane, chloroform, carbon tetrachloride, 1,2-dichloroethane and the like; nitriles such as acetonitrile, propionitrile and the like; sulfoxides dimethylsulfoxide and the like; water; a mixed solvent thereof, and the like.
The reaction temperature is generally 0 to 250°C, preferably 50 to 150°C. The reaction time is generally about 5 min to about 48 hr, preferably about 30 min to about 24 hr.
The reaction time can be shortened using a microwave reactor or the like.

The resultant product can be used directly as the reaction mixture or as a crude product for the next reaction, or can be isolated from the reaction mixture according a conventional method, and can be isolated and purified according to known separation and purification means, for example, concentration, concentration under reduced pressure, solvent extraction, crystallization, recrystallization, phase transfer, chromatography and the like.

Compound (I) can be produced according to the method described in the following Reaction Scheme 4.

Reaction Scheme 4

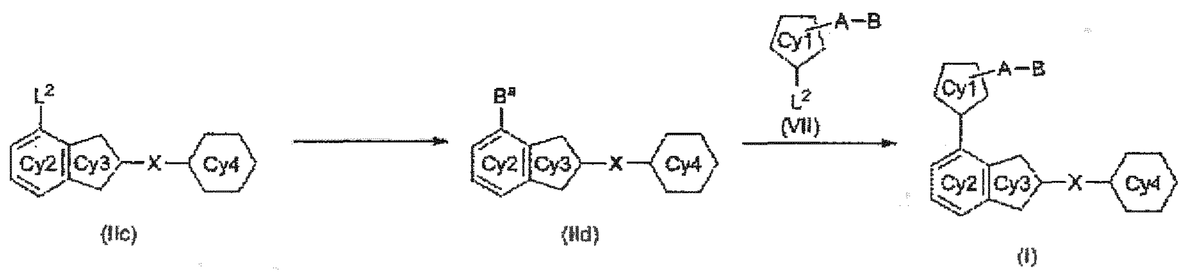

wherein the symbols are as defined above.

Compound (IId) is produced from compound (IIc) according to method known per se or a method analogous thereto.
Compound (I) is produced from compound (IId) and compound (VII) in the same manner as in the production of compound (I) from compound (IIc) described in Reaction Scheme 3.
Compound (VII) may be commercially available product, or can be produced according to a method known per se or a method analogous thereto.
The resultant product can be used directly as the reaction mixture or as a crude product for the next reaction, or can be isolated from the reaction mixture according a conventional method, and can be isolated and purified according to known separation and purification means, for example, concentration, concentration under reduced pressure, solvent extraction,

crystallization, recrystallization, phase transfer, chromatography and the like.

Compound (Ic) wherein the nitrogen atom of Cy1 ring is bonded to Cy2, can be produced according to the method described in the following Reaction Scheme 5.

Reaction Scheme 5

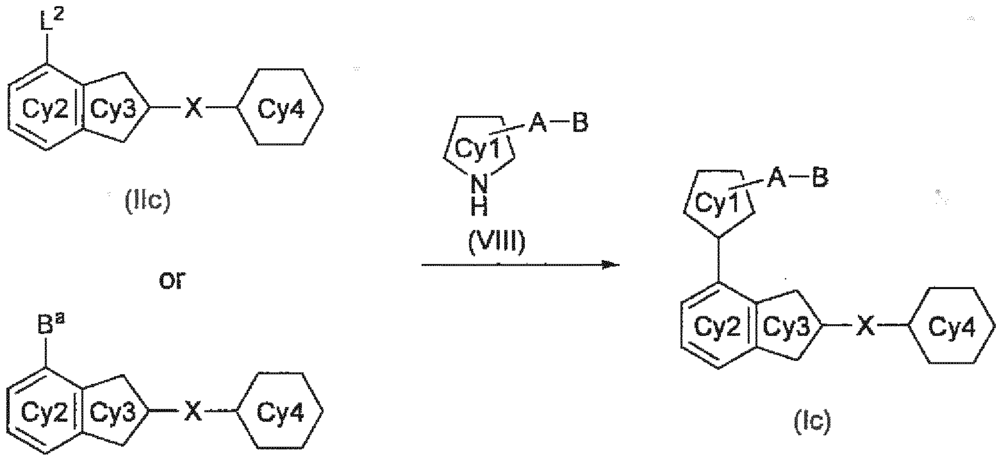

(IIc)

or

(IId)

(VIII)

(Ic)

wherein the symbols are as defined above.

Compound (Ic) is produced by subjecting compound (IIc) or compound (IId) to the Buchwald-Hartwig reaction or Ullmann reaction with compound (VIII).

Compound (VIII) may be commercially available product, or can be produced according to a method known per se or a method analogous thereto.

Compound (IIc) or compound (IId) is reacted with compound (VIII) in the presence of a base and a transition metal catalyst, in a solvent.

The amount of compound (VIII) to be used is about 0.5 to about 10 mol, preferably about 0.9 to about 3 mol, per 1 mol of compound (IIc) or compound (IId).

Examples of the "base" include basic salts such as sodium carbonate, potassium carbonate, cesium carbonate, sodium hydrogen carbonate and the like; aromatic amines such as pyridine, lutidine and the like; tertiary amines such as triethylamine, N,N-diisopropylethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like; metal alkoxides such as sodium methoxide, sodium ethoxide, sodium tert-butoxide, potassium tert-butoxide and the like; and the like.

Examples of the "transition metal catalyst" include palladium catalysts such as palladium acetate, palladium chloride, tetrakis(triphenylphosphine)palladium, 1,1-bis(diphenylphosphino)ferrocenedichloropalladium, dichlorobis (triphenylphosphine)palladium and the like; copper catalysts such as copper, copper acetate, copper chloride, copper iodide, copper oxide and the like; and the like. The amount of the transition metal catalyst to be used is about 0.001 to about 3 mol, preferably about 0.02 to about 0.2 mol, per 1 mol of compound (IIc) or compound (IId).

Examples of the solvent include ethers such as diethyl ether, diisopropyl ether, tetrahydrofuran, dioxane, 1,2-dimethoxyethane and the like; alcohols such as methanol, ethanol, propanol and the like; hydrocarbons such as benzene, toluene, carbon disulfide, cyclohexane, hexane and the like; amides such as N,N-dimethylformamide, N,N-dimethylacetamide and the like; halogenated hydrocarbons such as dichloromethane, chloroform, carbon tetrachloride, 1,2-dichloroethane and the like; nitriles such as acetonitrile, propionitrile and the like; sulfoxides such as dimethylsulfoxide and the like; aromatic amines such as pyridine, lutidine and the like; water; a mixed solvent thereof; and the like

The reaction temperature is generally 0 to 250°C, preferably 50 to 150°C. The reaction time is generally about 5 min to about 48 hr, preferably about 30 min to about 24 hr.

The reaction time can be shortened using a microwave reactor or the like.

The resultant product can be used directly as the reaction mixture or as a crude product for the next reaction, or can be isolated from the reaction mixture according a conventional method, and can be isolated and purified according to known separation and purification means, for example, concentration, concentration under reduced pressure, solvent extraction, crystallization, recrystallization, phase transfer, chromatography and the like.

Compound (Id) wherein the nitrogen atom of Cy3 ring is bonded to X, and X is alkylene, can be produced according to the method described in the following Reaction Scheme 6. Reaction Scheme 6

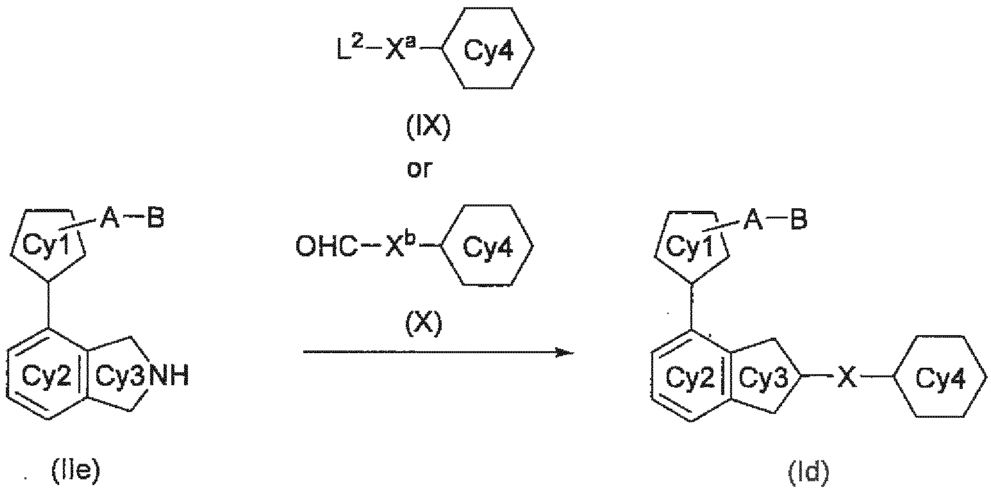

wherein $X^a$ is $-CH_2-$ or $-(CH_2)_2-$, $X^b$ is a bond or $-CH_2-$, and the other symbols are as defined above.

Compound (Id) can be produced by reacting compound (IIe) with compound (IX) in the presence of a base, if desired.

Compound (IX) may be commercially available product, or can be produced according to a method known per se or a method analogous thereto.

The amount of compound (IX) to be used is about 0.8 to about 5.0 mol, preferably about 1.0 to about 2.0 mol, per 1 mol of compound (IIe).

Examples of the "base" include basic salts such as sodium carbonate, potassium carbonate, cesium carbonate, sodium hydrogen carbonate and the like; aromatic amines such as pyridine, lutidine and the like; tertiary amines such as triethylamine, N,N-diisopropylethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like; alkali metal hydrides such as sodium hydride, potassium hydride and the like; metal amides such as sodium amide, lithium diisopropylamide, lithium hexamethyldisilazide and the like; metal alkoxides such as sodium methoxide, sodium ethoxide, potassium tert-butoxide and the like; and the like.

The amount of the base to be used is about 0.8 to about 5.0 mol, preferably about 1.0 to about 2.0 mol, per 1 mol of compound (IIe).

This reaction is advantageously carried out in a solvent inert to the reaction. The solvent is not particularly limited as long as the reaction proceeds, and preferable examples thereof include alcohols such as methanol, ethanol, propanol and the like; ethers such as diethyl ether, tetrahydrofuran, dioxane, 1,2-dimethoxyethane and the like; hydrocarbons such as benzene, toluene, cyclohexane, hexane and the like; amides such as N,N-dimethylformamide, N,N-dimethylacetamide and the like; halogenated hydrocarbons such as dichloromethane, chloroform, carbon tetrachloride, 1,2-dichloroethane and the like; nitriles such as acetonitrile, propionitrile and the like; sulfoxides such as dimethylsulfoxide and the like; a mixed solvent thereof; and the like.

The reaction time is generally about 30 min to about 48 hr, preferably about 1 hr to about 24 hr. The reaction temperature is generally about -20 to about 200°C, preferably about 0 to about 150°C.

Alternatively, compound (Id) can be synthesized from compound (IIe) and compound (X) by employing a reductive amination reaction using a reducing agent instead of the aforementioned reaction.

Compound (X) may be commercially available product, or can be produced according to a method known per se or a method analogous thereto.

The amount of compound (X) to be used is about 0.8 to about 5.0 mol, preferably about 1.0 to about 2.0 mol, per 1 mol of compound (IIe).

Examples of the "reducing agent" include metal hydrides such as sodium borohydride, sodium cyanoborohydride, sodium triacetoxyborohydride, lithium aluminum hydride and the like; boranes such as boranetetrahydrofuran complex and the like; hydrosilanes such as triethylsilane and the like; formic acid; and the like. In addition, an acid catalyst may be used together with a reducing agent, if desired. Examples of the acid catalyst include mineral acids such as hydrochloric acid, hydrobromic acid, sulfuric acid and the like; sulfonic acids such as methanesulfonic acid, p-toluenesulfonic acid and the like; organic acids such as acetic acid, propionic acid, trifluoroacetic acid and the like; Lewis

acids such as zinc chloride, aluminum chloride and the like; and the like.
The amount of the "reducing agent" to be used is about 0.25 to about 5.0 mol, preferably about 0.5 to about 2.0 mol, per 1 mol of compound (IIe).
The amount of the acid catalyst to be used is, for example, in the case of a mineral acid, generally about 1 to about 100 mol, preferably about 1 to about 20 mol, per 1 mol of compound (IIe).

This reaction is advantageously carried out in a solvent inert to the reaction. The solvent is not particularly limited as long as the reaction proceeds, and preferable examples thereof include alcohols such as methanol, ethanol, propanol and the like; ethers such as diethyl ether, tetrahydrofuran, dioxane, 1,2-dimethoxyethane and the like; hydrocarbons such as benzene, toluene, cyclohexane, hexane and the like; amides such as N,N-dimethylformamide, N,N-dimethylacetamide and the like; a mixed solvent thereof; and the like.
The reaction time is generally about 5 min to about 48 hr, preferably about 30 min to about 24 hr. The reaction temperature is generally about -20 to about 200°C, preferably about 0 to about 100°C.

Alternatively, the reaction is carried out, after the condensation of compound (IIe) and compound (X), by employing a catalytic hydrogenation reaction under a hydrogen atmosphere in the presence of a catalyst, instead of reduction using a reducing agent. Examples of the catalyst to be used include platinum oxide, platinum on activated carbon, palladium on activated carbon, nickel, copper-chromium oxide, rhodium, cobalt, ruthenium and the like. The amount of the catalyst to be used is about 1 to about 1000 wt%, preferably about 5 to about 50 wt%, relative to compound (IIe).

This reaction is advantageously carried out in a solvent inert to the reaction. The solvent is not particularly limited as long as the reaction proceeds, and preferable examples thereof include alcohols such as methanol, ethanol, propanol and the like; ethers such as diethyl ether, tetrahydrofuran, dioxane, 1,2-dimethoxyethane and the like; hydrocarbons such as benzene, toluene, cyclohexane, hexane and the like; amides such as N,N-dimethylformamide, N,N-dimethylacetamide and the like; water; a mixed solvent thereof; and the like.
The reaction time is generally about 30 min to about 48 hr, preferably about 30 min to about 24 hr. The reaction temperature is generally about 0 to about 120°C, preferably about 20 to about 80°C.
The resultant product can be used directly as the reaction mixture or as a crude product for the next reaction, or can be isolated from the reaction mixture according a conventional method, and can be isolated and purified according to known separation and purification means, for example, concentration, concentration under reduced pressure, solvent extraction, crystallization, recrystallization, phase transfer, chromatography and the like.

Compound (IIe) can be produced according to the method described in the following Reaction Scheme 7.

Reaction Scheme 7

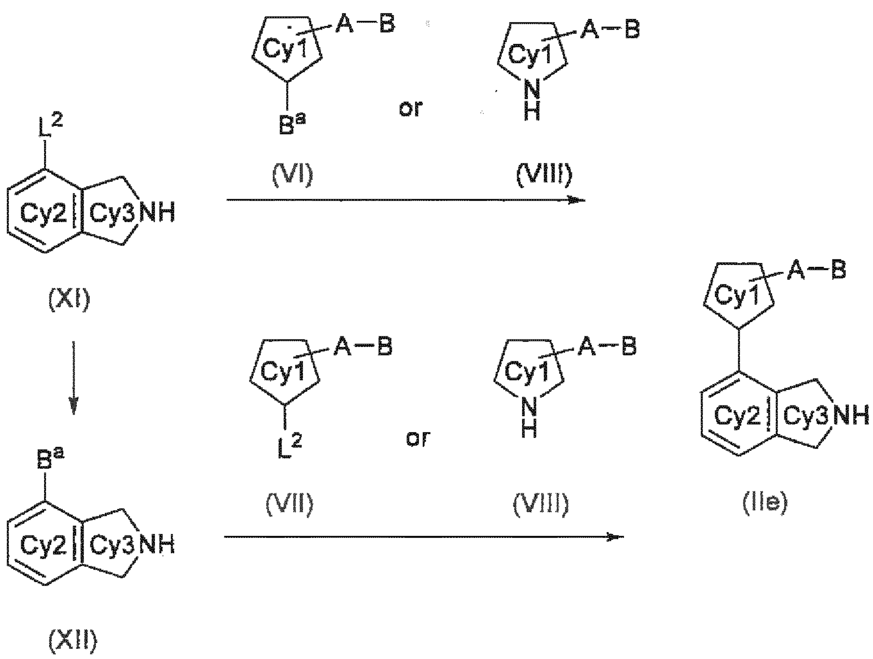
(XI) (VI) or (VIII) (XII) (VII) or (VIII) (IIe)

wherein the symbols are as defined above.

Compound (IIe) can be produced from compound (XI) in the same manner as in the production of compound

(I) from compound (IIc) described in Reaction Scheme 3 or the production of compound (I) from compound (IIc) described in Reaction Scheme 5.
Alternatively, Compound (IIe) can be produced from compound (XII) in the same manner as in the production of compound (I) from compound (IId) described in Reaction Scheme 4 or the production of compound (I) from compound (IId) described in Reaction Scheme 5.
Compound (XI) may be commercially available product, or can be produced according to a method known per se or a method analogous thereto.
Compound (XII) may be commercially available product, or can be produced from compound (XI) in the same manner as in the production of compound (IId) from compound (IIc) described in Reaction Scheme 4.
Compound (IIe) thus obtained can be isolated from the reaction mixture according a conventional method, and can be isolated and purified according to known separation and purification means, for example, concentration, concentration under reduced pressure, solvent extraction, crystallization, recrystallization, phase transfer, chromatography and the like.
Compound (I) can be produced according to the method described in the following Reaction Scheme 8.

Reaction Scheme 8

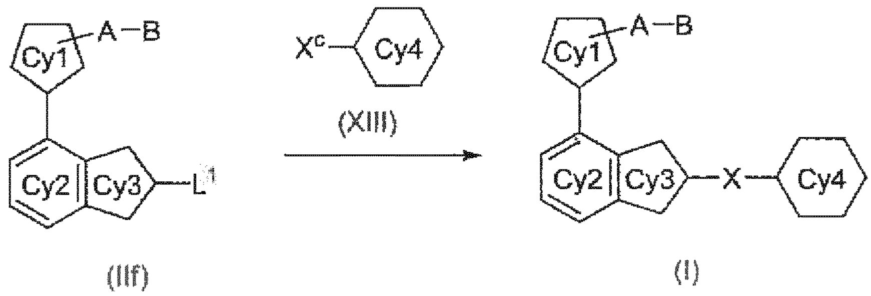

wherein $X^c$ is a substituent selected from a hydroxy group, an amino group optionally substituted by substituent $R^b$, and a mercapto group, and the other symbols are as defined above.
Compound (I) can be produced by reacting compound (IIf) with compound (XIII) in the presence of a base, if desired.
Compound (XIII) may be commercially available product, or can be produced according to a method known per se or a method analogous thereto.
The amount of compound (XIII) to be used is about 0.8 to about 5.0 mol, preferably about 1.0 to about 2.0 mol, per 1 mol of compound (IIf).
Examples of the "base" include basic salts such as sodium carbonate, potassium carbonate, cesium carbonate, sodium hydrogen carbonate and the like; aromatic amines such as pyridine, lutidine and the like; tertiary amines such as triethylamine, N,N-diisopropylethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like; alkali metal hydrides such as sodium hydride, potassium hydride and the like; metal amides such as sodium amide, lithium diisopropylamide, lithium hexamethyldisilazide and the like; metal alkoxides such as sodium methoxide, sodium ethoxide, potassium tert-butoxide and the like; and the like.
The amount of the base to be used is about 0.8 to about 5.0 mol, preferably about 1.0 to about 2.0 mol, per 1 mol of compound (IIf).
This reaction is advantageously carried out in a solvent inert to the reaction. The solvent is not particularly limited as long as the reaction proceeds, and preferable examples thereof include alcohols such as methanol, ethanol, propanol and the like; ethers such as diethyl ether, tetrahydrofuran, dioxane, 1,2-dimethoxyethane and the like; hydrocarbons such as benzene, toluene, cyclohexane, hexane and the like; amides such as N,N-dimethylformamide, N,N-dimethylacetamide and the like; halogenated hydrocarbons such as dichloromethane, chloroform, carbon tetrachloride, 1,2-dichloroethane and the like; nitriles such as acetonitrile, propionitrile and the like; sulfoxides such as dimethylsulfoxide and the like; a mixed solvent thereof; and the like.
The reaction time is generally about 30 min to about 48 hr, preferably about 1 hr to about 24 hr. The reaction temperature is generally about -20 to about 200°C, preferably about 0 to about 150°C.
The resultant product can be used directly as the reaction mixture or as a crude product for the next reaction, or can be isolated from the reaction mixture according a conventional method, and can be isolated and purified according to known separation and purification means, for example, concentration, concentration under reduced pressure, solvent extraction,

crystallization, recrystallization, phase transfer, chromatography and the like.

Compound (IIf) can be produced from compound (XIVa) or compound (XIVb) according to the method described in the following Reaction Scheme 9.

Reaction Scheme 9

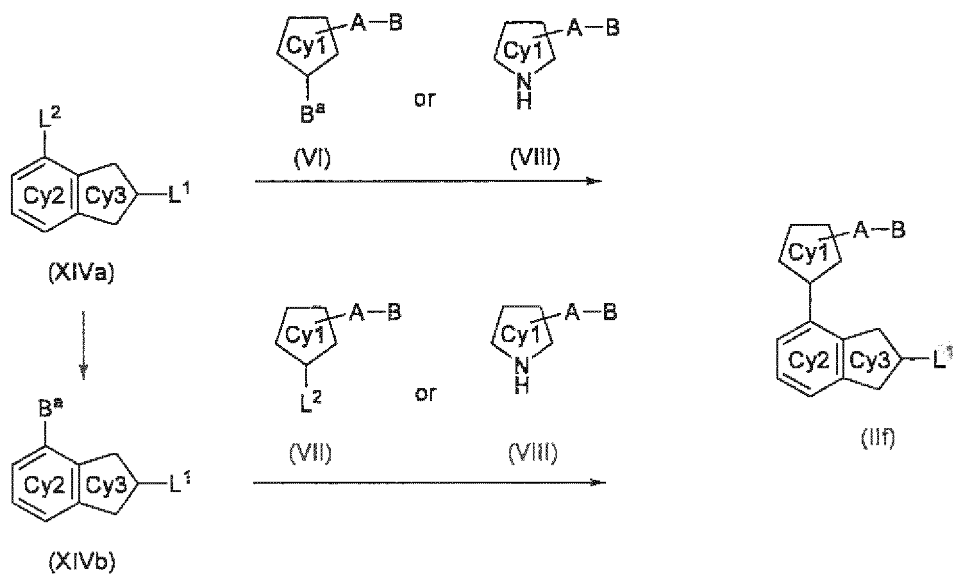

wherein the symbols are as defined above.

Compound (IIf) can be produced from compound (XIVa) in the same manner as in the production of compound (I) from compound (IIc) described in Reaction Scheme 3 or the production of compound (I) from compound (IIc) described in Reaction Scheme 5.

Alternatively, Compound (IIf) can be produced from compound (XIVb) in the same manner as in the production of compound (I) from compound (IId) described in Reaction Scheme 4 or the production of compound (I) from compound (IId) described in Reaction Scheme 5.

Compound (XIVa) may be commercially available product, or can be produced according to a method known per se or a method analogous thereto.

Compound (XIVb) can be produced from compound (XIVa) in the same manner as in the production of compound (IId) from compound (IIc) described in Reaction Scheme 4.

Compound (IIf) thus obtained can be isolated from the reaction mixture according a conventional method, and can be isolated and purified according to known separation and purification means, for example, concentration, concentration under reduced pressure, solvent extraction, crystallization, recrystallization, phase transfer, chromatography and the like.

Compound (IIa) can be produced according to the method described in the following Reaction Scheme 10.

Reaction Scheme 10

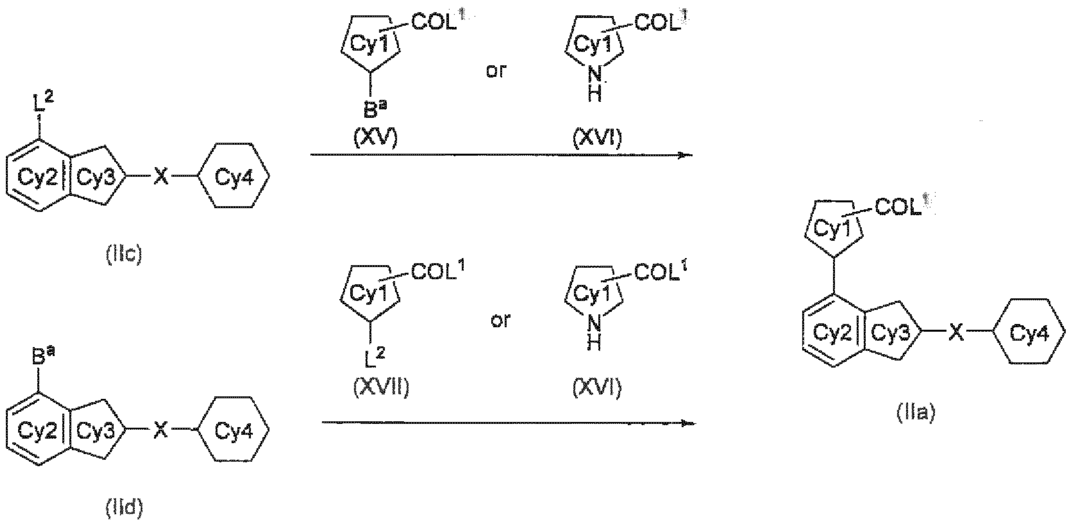


wherein the symbols are as defined above.

Compound (IIa) can be produced from compound (IIc) and compound (XV) in the same manner as in the production of compound (I) from compound (IIc) described in Reaction Scheme 3, or can be produced from compound (IIc) and compound (XVI) in the same manner as in the production of compound (I) from compound (IIc) described in Reaction Scheme 5.

Alternatively, compound (IIa) can be produced from compound (IId) and compound (XVII) in the same manner as in the production of compound (I) from compound (IId) described in Reaction Scheme 4, or can be produced from compound (IId) and compound (XVI) in the same manner as in the production of compound (I) from compound (IId) described in Reaction Scheme 5.

Compound (XV), compound (XVI) and compound (XVII) may be commercially available product, or can be produced according to a method known per se or a method analogous thereto.

Compound (IIa) thus obtained can be isolated from the reaction mixture according a conventional method, and can be isolated and purified according to known separation and purification means, for example, concentration, concentration under reduced pressure, solvent extraction, crystallization, recrystallization, phase transfer, chromatography and the like.

Compound (IIa) can be produced according to the method described in the following Reaction Scheme 11.

Reaction Scheme 11

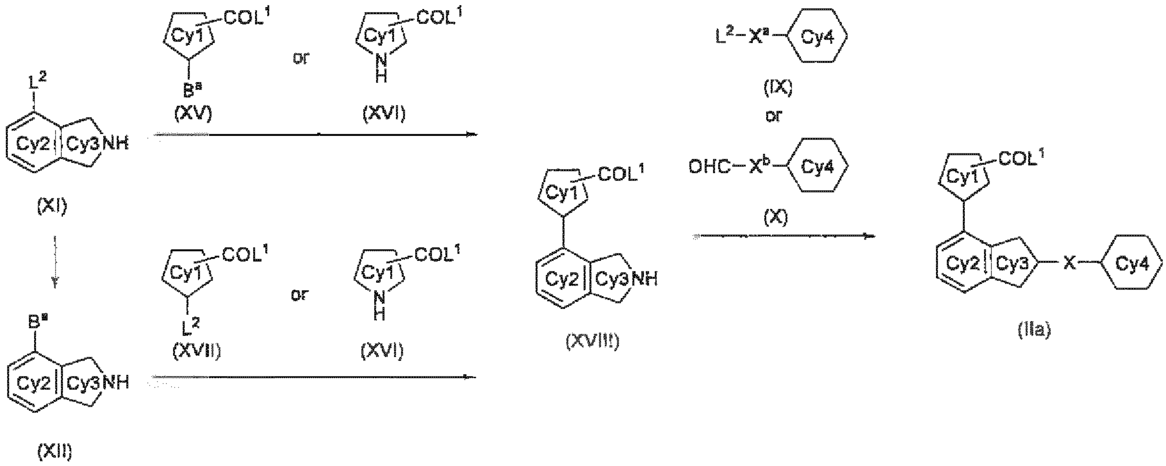


wherein the symbols are as defined above.

Compound (IIa) can be produced from compound (XVIII) in the same manner as in the production of compound (I) from compound (IIe) described in Reaction Scheme 6.

Compound (XVIII) can be produced from compound (XI) and compound (XV) or compound (XVI), or from compound

(XII) and compound (XVI) or compound (XVII), in the same manner as in the production of compound (IIe) described in Reaction Scheme 7.
Compound (IIa) thus obtained can be isolated from the reaction mixture according a conventional method, and can be isolated and purified according to known separation and purification means, for example, concentration, concentration under reduced pressure, solvent extraction, crystallization, recrystallization, phase transfer, chromatography and the like.
Compound (IIa) can be produced according to the method described in the following Reaction Scheme 12.

Reaction Scheme 12

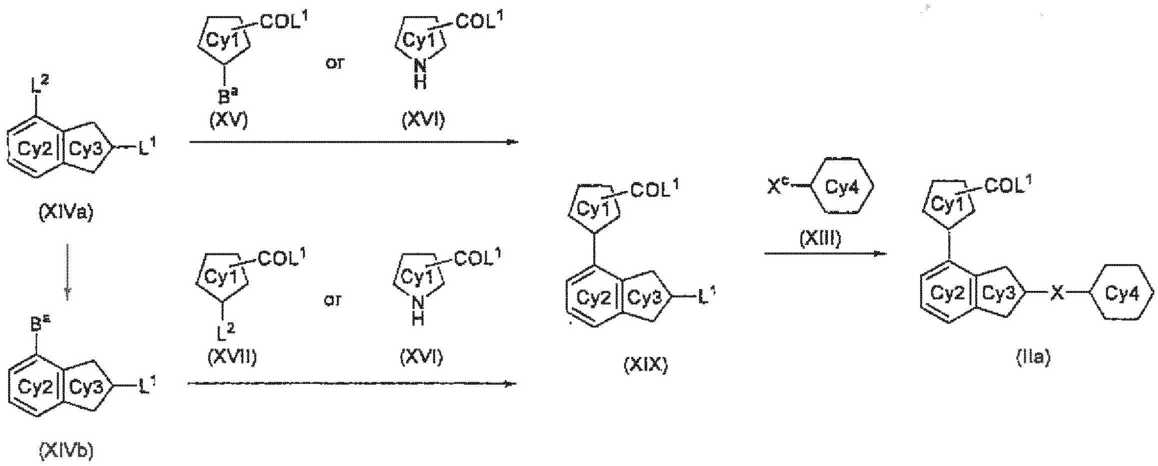

wherein the symbols are as defined above.
Compound (IIa) can be produced from compound (XIX) and compound (XIII) in the same manner as in the production of compound (I) from compound (IIf) described in Reaction Scheme 8.
Compound (XIX) can be produced from compound (XIVa) and compound (XV) or compound (XVI), or from compound (XIVb) and compound (XVI) or compound (XVII), in the same manner as in the production of compound (IIf) from compound (XIVa) and compound (XIVb) described in Reaction Scheme 9.
Compound (IIa) thus obtained can be isolated from the reaction mixture according a conventional method, and can be isolated and purified according to known separation and purification means, for example, concentration, concentration under reduced pressure, solvent extraction, crystallization, recrystallization, phase transfer, chromatography and the like.
Compound (IIa) can be produced according to the method described in the following Reaction Scheme 13.

Reaction Scheme 13

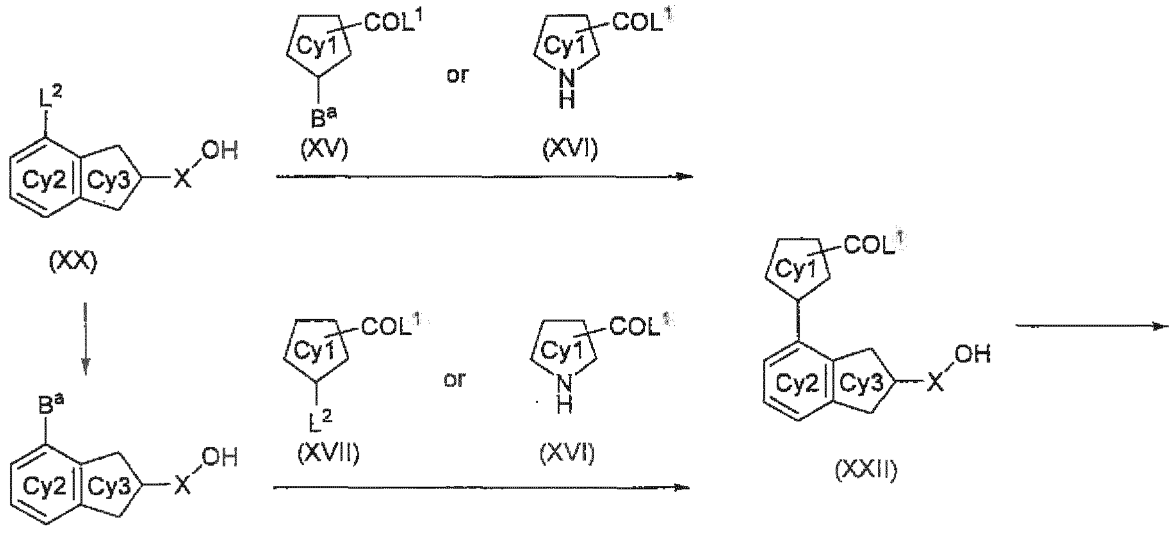

(XX) (XV) or (XVI) (XXI) (XVII) or (XVI) (XXII)

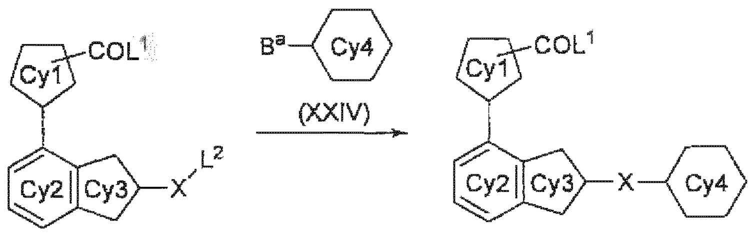

(XXIII) (XXIV) (IIa)

wherein the symbols are as defined above.

Compound (IIa) can be produced from compound (XXIII) and compound (XXIV) in the same manner as in the production of compound (I) from compound (IIc) described in Reaction Scheme 3.

Compound (XXIII) can be produced by subjecting compound (XXII) to halogenation or sulfonyloxylation using optionally halogenated $C_{1-5}$ alkyl.

The halogenation can also be carried out according to a method known per se, for example, the method described in 4th Edition Jikken Kagaku Kouza, 19 (The Chemical Society of Japan ed.), publication by MARUZEN.

Compound (XXII) can be produced from compound (XX) and compound (XV) or compound (XVI), or from compound (XXI) and compound (XVI) or compound (XVII), in the same manner as in the production of compound (IIf) from compound in (XIVa) and compound (XIVb) described Reaction Scheme 9.

Compound (XX), compound (XIVa) and compound (XIVb) may be commercially available product, or can be produced according to a method known per se or a method analogous thereto.

Compound (XXI) may be commercially available product, or can be produced from compound (XX) in the same manner as in the production of compound (IId) from compound (IIc) described in Reaction Scheme 4.

Compound (IIa) thus obtained can be isolated from the reaction mixture according a conventional method, and can be isolated and purified according to known separation and purification means, for example, concentration, concentration under reduced pressure, solvent extraction, crystallization, recrystallization, phase transfer, chromatography and the like.

Compound (IIaa), which is compound (IIa) wherein Cy1 ring is a 2-oxazolyl group, can be produced according to the method described in the following Reaction Scheme 14.

Reaction Scheme 14

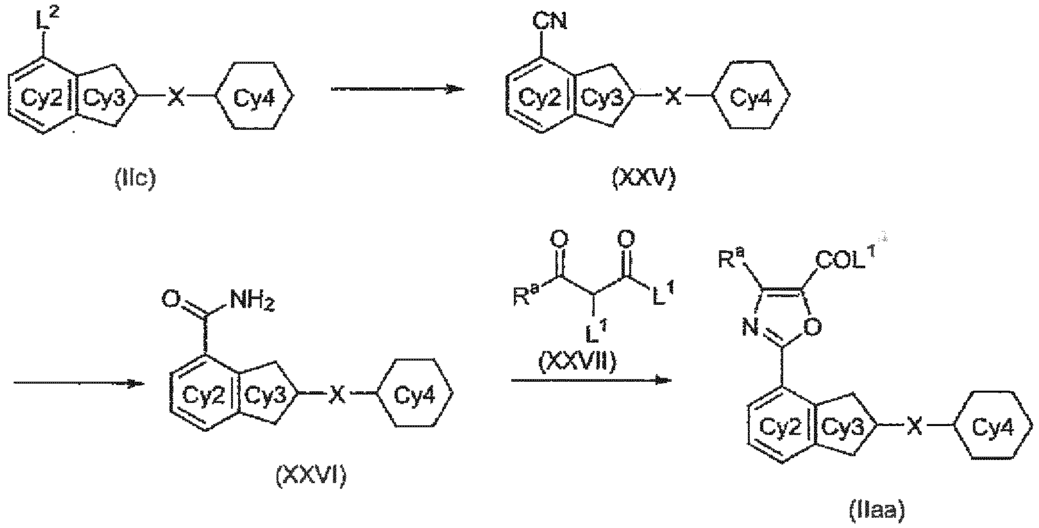


(IIc) (XXV) (XXVI) (IIaa)

wherein the symbols are as defined above.

Compound (XXV) is produced by reacting compound (IIc) with a metal cyanide such as copper cyanide, zinc cyanide and the like, in the presence of a transition metal catalyst such as palladium acetate, palladium chloride, tetrakis (triphenylphosphine)palladium, 1,1-bis(diphenylphosphino)ferrocenedichloropalladium, dichlorobis(triphenylphosphine) palladium and the like, if desired.

This reaction is advantageously carried out without a solvent or in a solvent inert to the reaction. The solvent is not particularly limited as long as the reaction proceeds, and preferable examples thereof include water; ethers such as diethyl ether, tetrahydrofuran, 1,4-dioxane, 1,2-dimethoxyethane and the like; hydrocarbons such as benzene, toluene, cyclohexane, hexane and the like; amides such as N,N-dimethylformamide, N,N-dimethylacetamide and the like; halogenated hydrocarbons such as dichloromethane, chloroform, carbon tetrachloride, 1,2-dichloroethane and the like; nitriles such as acetonitrile, propionitrile and the like; sulfoxides such as dimethyl sulfoxide and the like; nitrogen-containing aromatic hydrocarbons such as pyridine, lutidine, quinoline and the like; a mixed solvent thereof; and the like.

The reaction temperature is generally about -40 to 200°C, preferably 80 to 150°C.

The reaction time is generally 5 min to 24 hr, preferably 10 min to 5 hr.

Compound (XXVI) is produced by subjecting compound (XXV) to hydrolysis in the presence of an acid or a base. This reaction is advantageously carried out without a solvent or in a solvent inert to the reaction. The solvent is not particularly limited as long as the reaction proceeds, and preferable examples thereof include water; ethers such as tetrahydrofuran, 1,4-dioxane, 1,2-dimethoxyethane and the like; hydrocarbons such as benzene, toluene, cyclohexane, hexane and the like; amides such as N,N-dimethylformamide, N,N-dimethylacetamide and the like; halogenated hydrocarbons such as dichloromethane, chloroform, carbon tetrachloride, 1,2-dichloroethane and the like; sulfoxides such as dimethylsulfoxide and the like; nitrogen-containing aromatic hydrocarbons such as pyridine, lutidine, quinoline and the like; a mixed solvent thereof; and the like.

The reaction temperature is generally about 0°C to 200°C, preferably 50 to 120°C.

The reaction time is generally 5 min to 24 hr, preferably 10 min to 5 hr.

Examples of the "base" include alkali metal hydroxides such as sodium hydroxide, potassium hydroxide and the like; basic salts such as sodium carbonate, potassium carbonate, cesium carbonate, sodium hydrogen carbonate and the like; alkali metal hydrides such as sodium hydride, potassium hydride and the like; metal amides such as sodium amide, lithium diisopropylamide, lithium hexamethyldisilazide and the like; metal alkoxides such as sodium methoxide, sodium ethoxide, potassium tert-butoxide and the like; and the like.

The amount of the "base" to be used is generally about 0.1 to 10 equivalents, preferably 0.8 to 2 equivalents, relative to compound (XXVI).

Examples of the "acid" include organic acids such as hydrogen chloride, hydrogen bromide, methanesulfonic acid, p-toluenesulfonic acid, camphorsulfonic acid and the like; mineral acids such as sulfuric acid, hydrochloric acid, hydrobromic acid and the like; and the like.

The amount of the "acid" to be used is generally about 0.1 to 10 equivalents, preferably 0.8 to 3 equivalents, relative to compound (IIa).

Alternatively, compound (XXVI) can be synthesized by converting compound (XXV) to the corresponding carboxylic acid by hydrolysis in the presence of an acid or a base, and then converting the carboxylic acid to the corresponding amidate.

Compound (IIaa) is produced by reacting compound (XXVI) with compound (XXVII). Compound (XXVII) may be commercially available product, or can be produced according to a method known per se or a method analogous thereto.

This reaction is advantageously carried out without a solvent or in a solvent inert to the reaction. The solvent is not particularly limited as long as the reaction proceeds, and preferable examples thereof include water; ethers such as diethyl ether, tetrahydrofuran, 1,4-dioxane, 1,2-dimethoxy ethane and the like; hydrocarbons such as benzene, toluene, cyclohexane, hexane and the like; amides such as N,N-dimethylformamide, N,N-dimethylacetamide and the like; halogenated hydrocarbons such as dichloromethane, chloroform, carbon tetrachloride, 1,2-dichloroethane and the like; nitriles such as acetonitrile, propionitrile and the like; sulfoxides such as dimethyl sulfoxide and the like; nitrogen-containing aromatic hydrocarbons such as pyridine, lutidine, quinoline and the like; a mixed solvent thereof; and the like.
The reaction temperature is generally about -40 to 200°C, preferably 80 to 150°C.
The reaction time is generally 5 min to 24 hr, preferably 10 min to 5 hr.
The resultant product can be used directly as the reaction mixture or as a crude product for the next reaction, or can be isolated from the reaction mixture according a conventional method, and can be isolated and purified according to known separation and purification means, for example, concentration, concentration under reduced pressure, solvent extraction, crystallization, recrystallization, phase transfer, chromatography and the like.

Compound (IIab), which is compound (IIa) wherein Cy1 ring is a 1-imidazolyl group, can be produced according to the method described in the following Reaction Scheme 15. Reaction Scheme 15

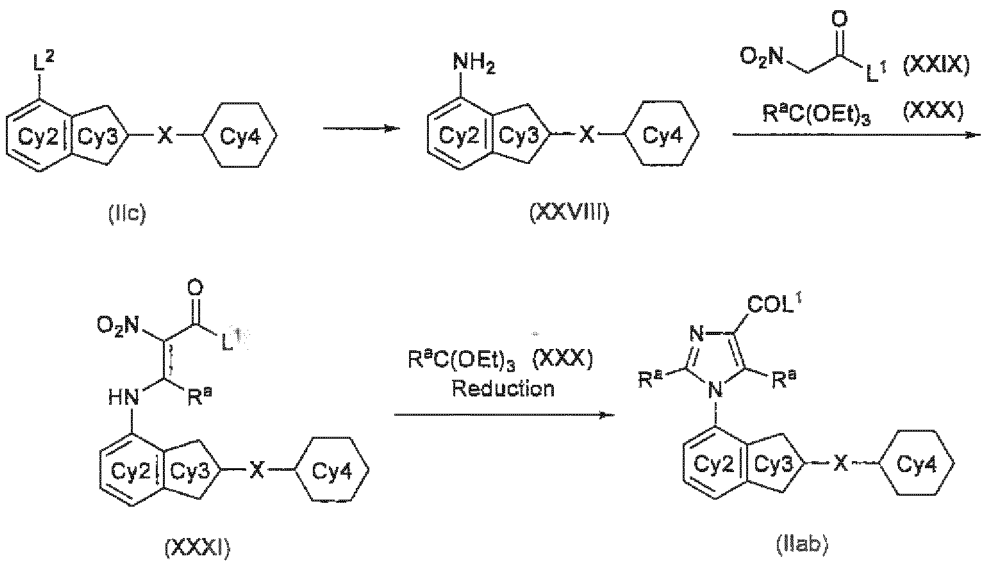

(IIc) (XXVIII) (XXXI) (IIab)

wherein the symbols are as defined above.

Compound (XXVIII) is produced from compound (IIc) according to a method known per se or a method analogous thereto.
Compound (XXXI) is produced by reacting compound (XXVIII) with compound (XXIX) and compound (XXX).
Compound (XXIX) and compound (XXX) may be commercially available product, or can be produced according to a method known per se or a method analogous thereto.

Examples of solvent include ethers such as diethyl ether, diisopropyl ether, tetrahydrofuran, dioxane, 1,2-dimethoxyethane and the like; alcohols such as methanol, ethanol, propanol and the like; hydrocarbons such as benzene, toluene, carbon disulfide, cyclohexane, hexane and the like; amides such as N,N-dimethylformamide, N,N-dimethylacetamide and the like; halogenated hydrocarbons such as dichloromethane, chloroform, carbon tetrachloride, 1,2-dichloroethane and the like; nitriles such as acetonitrile, propionitrile and the like; sulfoxides such as dimethyl sulfoxide and the like; water; a mixed solvent thereof; and the like.
The reaction temperature is generally 0 to 250°C, and preferably 50 to 150°C. The reaction time is generally about 5 min to about 48 hr, and preferably about 30 min to about 24 hr.
The reaction time can be shortened using a microwave reactor or the like.

Compound (IIab) is produced by subjecting compound (XXXI) to a catalytic hydrogenation reaction with to compound (XXX) in the presence of a catalyst under a hydrogen atmosphere. Examples of the catalyst to be used include platinum oxide, platinum on activated carbon, palladium on activated carbon, nickel, copper-chromium oxide, rhodium, cobalt, ruthenium and the like. The amount of the catalyst to be used is about 1 to about 1000 wt%, preferably about 5 to about 50 wt%, relative to compound (XXXI).

This reaction is advantageously carried out in a solvent inert to the reaction. The solvent is not particularly

limited as long as the reaction proceeds, and preferable examples thereof include alcohols such as methanol, ethanol, propanol and the like; ethers such as diethyl ether, tetrahydrofuran, dioxane, 1,2-dimethoxyethane and the like; hydrocarbons such as benzene, toluene, cyclohexane, hexane and the like; amides such as N,N-dimethylformamide, N,N-dimethylacetamide and the like; water; a mixed solvent thereof; and the like.
The reaction time is generally about 30 min to about 48 hr, preferably about 30 min to about 24 hr. The reaction temperature is generally about 0 to about 150°C, preferably about 50 to about 120°C.
The resultant product can be used directly as the reaction mixture or as a crude product for the next reaction, or can be isolated from the reaction mixture according a conventional method, and can be isolated and purified according to known separation and purification means, for example, concentration, concentration under reduced pressure, solvent extraction, crystallization, recrystallization, phase transfer, chromatography and the like.
Compound (IIb) can be produced according to the method described in the following Reaction Scheme 16.
Reaction Scheme 16
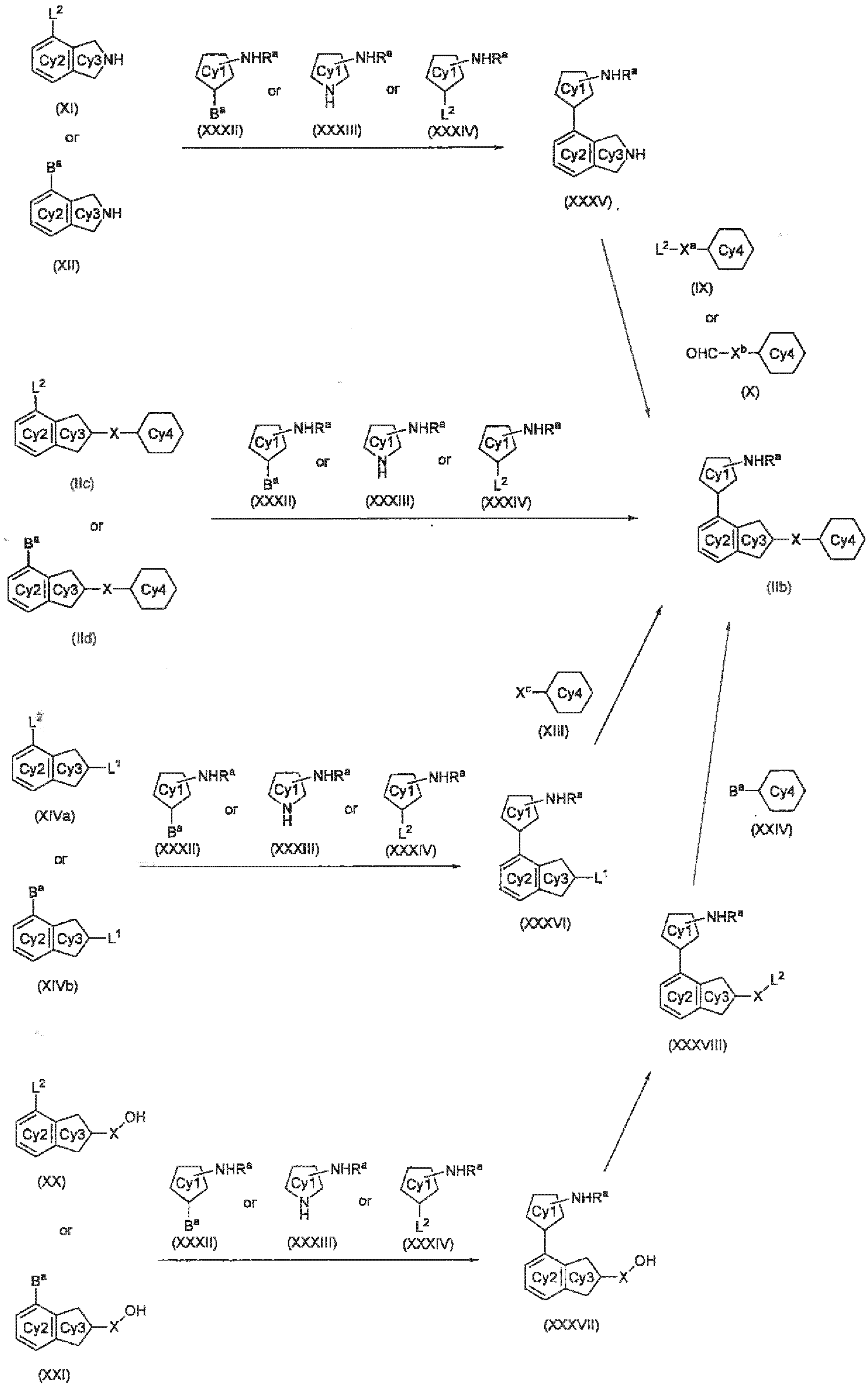

(XI) or (XII)

(XXXII) or (XXXIII) or (XXXIV)

(XXXV)

(IX) or (X)

(IIc) or (IId)

(XXXII) or (XXXIII) or (XXXIV)

(IIb)

(XIII)

(XIVa) or (XIVb)

(XXXII) or (XXXIII) or (XXXIV)

(XXXVI)

(XXIV)

(XXXVIII)

(XX) or (XXI)

(XXXII) or (XXXIII) or (XXXIV)

(XXXVII)

wherein the symbols are as defined above.

Compound (IIb) can be produced from compound (IIc) or compound (IId), and compound (XXXII) or compound (XXXIII) or compound (XXXIV), in the same manner as in the production of compound (I) from compound (IIc) described in Reaction Scheme 3, or the production of compound (I) from compound (IId) described in Reaction Scheme 4, or the production of compound (I) from compound (IIc) or compound (IId) described in Reaction Scheme 5; or can be'produced from compound (XI) or compound (XII) in the same manner as in the production of compound (IIcompound (IIe) described in Reaction Scheme 7 to give compound (XXXV), and then from compound (XXXV) in the production of compound (I) from compound (IIe) described in Reaction Scheme 6; or can produced from compound (XIVa) or compound (XIVb) in the same manner as in the production of compound (IIf) described in Reaction Scheme 9 to give compound (XXXVI), and then from compound (XXXVI) in the same manner as in the production of compound (I) from compound (IIf) described in Reaction Scheme 8; or

can produced from compound (XX) or compound (XXI) in the same manner as in the production of compound (IIa) described in Reaction Scheme 13.

Compound (XXXII), compound (XXXIII) and compound (XXXIV) may be commercially available product, or can be produced according to a method known per se or a method analogous thereto.

Compound (IIb) thus obtained can be isolated from the reaction mixture according a conventional method, and can be isolated and purified according to known separation and purification means, for example, concentration, concentration under reduced pressure, solvent extraction, crystallization, recrystallization, phase transfer, chromatography and the like.

Compound (IIc) is produced according to a method known per se or a method analogous thereto.

Compound (IIc) is produced according to the method described in the following Reaction Scheme 17.

Reaction Scheme 17

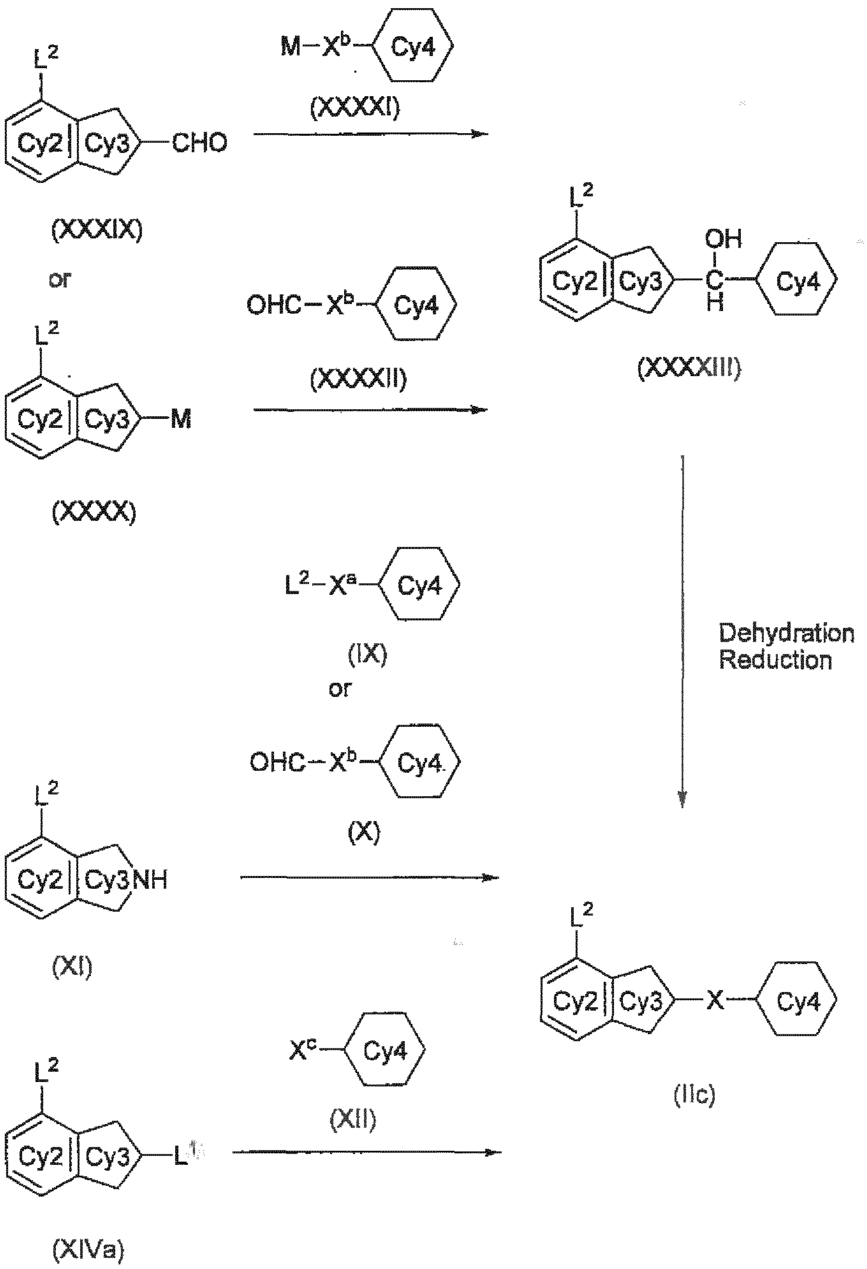


(XXXIX) or (XXXX) (XXXXI) (XXXXII) (XXXXIII) (IX) or (X) (XI) (XII) (XIVa) (IIc)

wherein M is $MgL^3$ or Li, $L^3$ is halogen (e.g., chlorine, bromine, iodine), and the other symbols are as defined above.

Compound (XXXXIII) is produced by reacting compound (XXXIX) with a Grignard reagent or an organic lithium reagent (XXII), or by reacting a Grignard reagent or an organic lithium reagent (XXXX) with compound (XXXXII). Compound (XXXIX) and compound (XXXXII) may be commercially available product, or can be produced according to a method known per se or a method analogous thereto.

The Grignard reagent or organic lithium reagent (XXXX) and (XXXXI) may be commercially available product, can be produced according to a method known per se, for example, the method described in 4th Edition Jikken Kagaku Kouza, 25 (the Chemical Society of Japan ed.), publication by MARUZEN.

The amount of the Grignard reagent or organic lithium reagent (XXXX) or (XXXXI) to be used is about 0.8 to about 30 mol, preferably about 1.0 to about 20 mol, per 1 mol of compound (XXXIX) or compound (XXXXII).

This reaction is advantageously carried out without a solvent or in a solvent inert to the reaction. The solvent is not particularly limited as long as the reaction proceeds, and examples thereof include alcohols such as methanol, ethanol, propanol and the like; hydrocarbons such as hexane, cyclohexane, benzene, toluene, xylene and the like; ethers such as diethyl ether, diisopropyl ether, tetrahydrofuran, dioxane, 1,2-dimethoxyethane and the like; amides such as N, N-dimethylformamide, N,N-dimethylacetamidehexamethylphosphoric triamide and the like; sulfoxides such as dimethylsulfoxide and the like; halogenated carbons such as dichloromethane, chloroform, carbon tetrachloride, 1,2-dichloroethane and the like; a mixed solvent thereof; and the like.
The reaction time is generally about 10 min to about 24 hr, preferably about 30 min to about 12 hr. The reaction temperature is generally about -100 to about 120°C, preferably about -80 to about 60°C.
The resultant product can be used directly as the reaction mixture or as a crude product for the next reaction, or can be isolated from the reaction mixture according a conventional method, and can be isolated and purified according to known separation and purification means, for example, concentration, concentration under reduced pressure, solvent extraction, crystallization, recrystallization, phase transfer, chromatography and the like.
Alternatively, compound (IIc) can be produced by subjecting compound (XXXXIII) to a reductive dehydrating reaction.
Examples of the reductive dehydrating reaction include a catalytic reduction known per se, methods using an organic silyl reagent (an alkylsilane reagent etc.) and the like.
In the catalytic reduction, compound (IIc) can be produced by reacting compound (XXXXIII) with a metal catalyst under a hydrogen atmosphere. An appropriate acid catalyst may be added the reaction system if desired.
Examples of the "metal catalyst" include Raney-nickel, platinum oxide, metal palladium, palladium on activated carbon and the like. The amount of the "metal catalyst" to be used is generally about 1 to about 1000 wt%, preferably about 5 to about 20 wt%, relative to compound (XXXXIII).
Examples of the "acid catalyst" include organic acids such as formic acid, acetic acid, trifluoroacetic acid, p-toluenesulfonic acid and the like; mineral acids such as sulfuric acid, hydrochloric acid, hydrobromic acid and the like; and the like. The amount of the "acid catalyst" to be used is about 0.1 to excess amount, per 1 mol of compound (XXXXIII).
This reaction is advantageously carried out in a solvent inert to the reaction. The solvent is not particularly limited as long as the reaction proceeds, and preferable examples thereof include such as alcohols methanol, ethanol, propanol and the like; ethers such as diethyl ether, tetrahydrofuran, dioxane, 1,2-dimethoxyethane and the like; hydrocarbons such as benzene, toluene, cyclohexane, hexane and the like; amides such as N,N-dimethylformamide, N,N-dimethylacetamide and the like; organic acids such as acetic acid and the like; water; a mixed solvent thereof; and the like. The hydrogen pressure is generally about 1 to about 100 pressure, preferably about 1 to about 5 pressure. The reaction time is generally about 30 min to about 48 hr, preferably about 1 to 24 hr. The reaction temperature is generally about 0 to about 120°C, preferably about 20 to about 80°C.
After removal of the catalyst, the resultant product can be isolated from the reaction mixture according a conventional method, and can be isolated and purified according to known separation and purification means, for example, concentration, concentration under reduced pressure, solvent extraction, crystallization, recrystallization, phase transfer, chromatography and the like.
In the method using an organic silyl reagent (an alkylsilane reagent), compound (IIc) can be produced by reacting compound (XXXXIII) with an alkylsilane reagent and an acid.
Examples of the alkylsilane reagent include triethylsilane, phenyldimethylsilane and the like. The amount of the "alkylsilane reagent" to be used is about 0.8 to about 20 mol, preferably about 1 to about 10 mol, per 1 mol of compound (XXXXIII).
Examples of the acid include organic acids such as trifluoroacetic acid and the like. The amount of the acid to be used is about 0.1 to excess amount, per 1 mol of compound (XXXXIII).
This reaction is advantageously carried out without a solvent or in a solvent inert to the reaction. The solvent is not particularly limited as long as the reaction proceeds, and preferable examples thereof include ethers such as diethyl ether, tetrahydrofuran, dioxane, 1,2-dimethoxyethane and the like; hydrocarbons such as benzene, toluene, cyclohexane, hexane and the like; organic acids such as acetic acid, trifluoroacetic acid and the like; a mixed solvent thereof; and the like.
The resultant product can be isolated according to a conventional method according to reaction mixture, and can be isolated and purified according to known separation and purification means, for example, concentration, concentration under reduced pressure, solvent extraction, crystallization, recrystallization, phase transfer, chromatography and the like.
Alternatively, compound (IIc) can be produced from compound (XI) in the same manner as in the production of compound (I) from compound (IIe) described in Reaction Scheme 6.
Alternatively, compound (IIc) can be produced from compound (XIVa) in the same manner as in the production of compound (I) from compound (IIf) described in Reaction Scheme 8.
Compound (I) can also be synthesized, if desired, by a combination of the above-mentioned reaction and each or two more of known protection and deprotection, acylation reaction, alkylation reaction, hydrogenation reaction, oxidation reaction, reduction reaction, hydrolysis reaction, carbon chain extension reaction, substituent exchange reaction and the like.
When the compound is obtained as a free compound in the above-mentioned reaction, it can be converted to a desired salt according to a conventional method. Conversely, when the compound is obtained as a salt, it can be converted to a free form or other desired salt according to a conventional method. Compound (I) thus obtained can be isolated and purified according to a means known per se, such as phase transfer, concentration, solvent extraction, fractionation, crystallization, recrystallization, chromatography and the like.

Furthermore, if compound (I) is present as a configurational isomer (configuration isomer), a diastereomer, a conformer and the like, it may be isolated by any of the separation and purification techniques if desired. In addition, when compound (I) is present as a racemic body, it can be separated into a d-form and an 1-form using a usual optical resolution technique.

Compound (I) may be used as a prodrug. A prodrug of compound (I) means a compound converted to compound (I) by a reaction due to an enzyme, a gastric acid, etc. under the physiological condition in the living body, that is, a compound converted to compound (I) by oxidation, reduction, hydrolysis, etc. due to an enzyme, a compound converted to compound (I) by hydrolysis etc. due to gastric acid, and the like.

Examples of the prodrug of compound (I) include a compound obtained by subjecting amino in compound (I) to an acylation, alkylation or phosphorylation (e.g., a compound obtained by subjecting amino in compound (I) to eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation or tert-butylation); a compound obtained by subjecting hydroxy in compound (I) to acylation, alkylation, phosphorylation or boration (e.g., a compound obtained by subjecting hydroxy in the compound (I) to acetylation, palmitoylation, propanoylation, pivaloylation, succinylation, fumarylation, alanylation or dimethylaminoacethylation); a compound obtained by subjecting carboxy in compound (I) to esterification or amidation (e.g., a compound obtained by subjecting carboxy in compound (I) to ethyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification or methylamidation) and the like. Any one of these compounds can be produced from compound (I) by a method known per se. A prodrug of compound (I) may also be a compound converted into compound (I) under physiological conditions, such as those described in IYAKUHIN no KAIHATSU (Development of Pharmaceuticals), Vol. 7, Design of Molecules, p.163-198, Published by HIROKAWA SHOTEN (1990).

The compound of the present invention has an excellent GPR52 agonist activity and is useful, for example, as an agent for the prophylaxis or treatment of diseases or symptoms in described in the following (1) to (10) to mammals (e.g., humans, cows, horses, dogs, cats, monkeys, mice and rats, particularly humans).

(1) mental diseases [e.g., depression, major depression, bipolar depression, dysthymic disorder, emotional disorder (seasonal emotional disorder and the like), recurrent depression, postpartum depression, stress disorder, symptom of depression, mania, anxiety, generalized anxiety disorder, anxiety syndrome, panic disorder, phobia, social phobia, social anxiety disorder, obsessive disorder, post-traumatic stress syndrome, post-traumatic stress disorder, Tourette syndrome, autism, adjustment disorder, bipolar disorder, neurosis, schizophrenia, neurosis, chronic fatigue syndrome, anxiety neurosis, compulsive neurosis, phobic disorder, epilepsy, anxiety, anxious mental state, dysthymia, cyclothymia, nervous erethism, faint, addiction, low sex drive, attention deficit hyperactivity disorder (ADHD), psychotic major depression, intractable major depression, treatment-resistant depression],

(2) neurodegenerative diseases [e.g., Alzheimer's disease, alzheimer-type senile dementia, Parkinson's disease, Huntington chorea, spinocerebellar degeneration, multi-infarct dementia, frontotemporal dementia, Parkinson's type frontotemporal dementia, progressive supranuclear palsy, Pick's syndrome, Niemann-Pick's syndrome, corticobasal degeneration, Down's disease, vascular dementia, postencephalitic parkinsonism, Lewy body dementia, HIV dementia, amyotrophic lateral sclerosis (ALS), motor neurogenesis disease (MND), Creutzfeldt-Jakob disease or prion disease, cerebral palsy, progressive supranuclear palsy, multiple sclerosis],

(3) age-related cognition·memory disorders [e.g., age-related memory disorders, senile dementia],

(4) sleep disorders [e.g., intrinsic sleep disorder (e.g., psychophysiological insomnia and the like), extrinsic sleep disorder, circadian rhythm disorder (e.g., time zone change syndrome (jet lag), shift work sleep disorder, irregular sleep-wake pattern, delayed sleep phase syndrome, advanced sleep phase syndrome, non-24-hour sleep-wake and the like), parasomnia, sleep disorder with internal or psychiatric disorder (e.g., chronic obstructive pulmonary diseases, Alzheimer's disease, Parkinson's disease, cerebrovascular dementia, schizophrenia, depression, anxiety neurosis), stress-related insomnia, insomnia, insomnia-related neurosis, sleep apnea syndrome],

(5) anesthetics (e.g., morphine), traumatic disease or respiratory depression caused by neurodegenerative disease and the like,

(6) traumatic brain injury, cerebral apoplexy, and complications, which are depression, dysuria, pain and the like, with them,

(7) neurotic anorexia, eating disorder, anorexia nervosa, hyperorexia, other eating disorder, alcohol dependence, alcohol abuse, alcoholic amnesia, alcohol paranoia, alcohol preference, alcohol withdrawal, alcoholic mental disease, alcohol addiction, alcoholic jealousy, alcoholic mania, alcohol-dependent mental disorder, alcoholic insanity, nicotine addiction, pharmacophilia, pharmacophobia, pharmacomania, drug withdrawal, Meniere's disease, autonomic ataxia, as other neurological diseases

(8) neuropathic pain, postoperative pain, carcinomatous pain, inflammatory pain, migraine, stress headache, tension headache, dysesthesia (e.g., hypesthesia etc.),

(9) diabetic neuropathy, obesity, diabetes, muscular spasm, alopecia, glaucoma, hypertension, cardiac disease, tachycardia, cardiac failure, hyperventilation, bronchial asthma, apnea, inflammatory disease, allergic disease, impotence, infertility, immunodeficiency syndrome, acromegaly, incontinence, metabolic syndrome, osteoporosis, gastrointestinal disorder, peptic ulcer, irritable bowel syndrome, inflammatory bowel disease, ulcerative colitis, Crohn's disease, vomiting, diarrhea, constipation and postoperative ileus, caused by stress and the like,
(10) others, climacteric disorder, sudden infant death syndrome, brain tumor and the like malignant tumor, immune deficiency syndrome caused by HIV infection, cerebrospinal meningitis.

The compound of the present invention is particularly useful as a prophylactic or therapeutic drug for symptoms of mental disorders (e.g., schizophrenia, depression, anxiety, bipolar disorders or PTSD, anxiety neuroses, obsessive-compulsive neuroses etc.), neurodegenerative diseases (e.g., Alzheimer's disease, mild cognitive impairment (MCI), Parkinson's disease, amyotrophic lateral sclerosis (ALS), Huntington's disease, spinocerebellar degeneration, multiple sclerosis (MS), Pick disease etc.), among them, schizophrenia, including (1) positive symptoms such as delusion and hallucination, (2) negative symptoms such as hypesthesia, social withdrawal, and diminished motivation/loss of concentration, and (3) cognitive dysfunction.

The compound of the present invention is superior in metabolic stability, so that the compound of this invention can be expected to have an excellent therapeutic effect on the above-mentioned diseases even in a low dose.

The compound of the present invention has low toxicity (which is a medicament superior to others in, for example, acute toxicity, chronic toxicity, genetic toxicity, reproductive toxicity, cardiotoxicity, drug interactions, carcinogenicity etc.). The compound of the present invention is directly used as a medicament or a pharmaceutical composition mixed with a pharmaceutically acceptable carrier or the like to be orally or parenterally administered to mammals (e.g., humans, monkeys, cows, horses, pigs, mice, rats, hamsters, rabbits, cats, dogs, sheep, goats etc.) in safety.

The medicament containing the compound of the present invention can be safely administered in the compound of the present invention as the sole regimen or as mixed with pharmacologically acceptable carrier according to method known per se as a production method for pharmaceutical preparation (e.g., the Japanese Pharmacopoeia the method described in etc.). Specifically, for example, it can be orally or parenterally (e.g., intravenous, intramuscular, subcutaneous, intraorgan, intranasal, intradermal, instillation, intracerebral, intrarectal, vaginal, intraperitoneal, interior of a tumor, proximal of a tumor and the like, and directly administration to the lesion) in the form of a tablet (inclusive of a sugar-coated tablet, a film-coated tablet, a sublingual tablet, an orally disintegrating tablet, a buccal tablet and the like), a pill, powders, granules, a capsule (inclusive of a soft capsule, a microcapsule), a troche, syrups, a liquid drug, emulsions, a suspension drug, controlled-release preparation (e.g., immediate-release preparation, sustained-release preparation, sustained-release microcapsule), aerosols, films (e.g., orally disintegrable films, oral mucosal adhesive film), an injection drug (e.g., subcutaneous injection, intravenous injection, intramuscular injection, intraperitoneal injection), drip infusion, transdermal absorption type preparation, ointment, lotion, adhesive preparation, suppository (e.g., rectal suppository, vaginal suppository), pellet, nasal preparations, pulmonary preparation (inhalant), eye drop and the like.

Here, examples of the pharmacologically acceptable carrier include various organic or inorganic carrier substances conventionally used as preparation materials, which are added as excipient, lubricant, binder or disintegrant for solid dosage forms; as solvent, solubilizing agent, suspending agent, isotonicity agent, buffer or soothing agent for liquid preparation, and the like. Where necessary, preparation additives such as preservative, antioxidant, colorant, sweetener and the like can also be used.

Preferable examples of the excipient include lactose, sucrose, D-mannitol, D-sorbitol, starch, pregelatinized starch, dextrin, crystalline cellulose, low-substituted hydroxypropyl cellulose, sodium carboxymethyl cellulose, gum arabic, pullulan, light anhydrous silicic acid, synthetic aluminum silicate, magnesium aluminometasilicate and the like.

Preferable examples of the lubricant include magnesium stearate, calcium stearate, talc, colloidal silica and the like.

Preferable examples of the binder include pregelatinized starch, saccharose, gelatin, gum arabic, methylcellulose, carboxymethyl cellulose, sodium carboxymethyl cellulose, crystalline cellulose, sucrose, D-mannitol, trehalose, dextrin, pullulan, hydroxypropyl cellulose, hydroxypropyl methylcellulose, polyvinyl pyrrolidone and the like.

Preferable examples of the disintegrant include lactose, sucrose, starch, carboxymethyl cellulose, carboxymethyl cellulose calcium, croscarmellose sodium, sodium carboxymethyl starch, light anhydrous silicic acid, low-substituted hydroxypropyl cellulose and the like.

Preferable examples of the solvent include water for injection, physiological saline, Ringer's solution, alcohol, propylene glycol, polyethylene glycol, sesame oil, corn oil, olive oil, cottonseed oil and the like.

Preferable examples of the solubilizing agents include polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate and the like.

Preferable examples of the suspending agent include surfactants such as stearyltriethanolamine, sodium lauryl sulfate, lauryl aminopropionate, lecithin, benzalkonium chloride, benzethonium chloride, glycerol monostearate and the like; hydrophilic polymers such as polyvinyl alcohol, polyvinyl pyrrolidone, sodium carboxymethyl cellulose, methylcellulose, hydroxymethyl cellulose, hydroxyethyl cellulose, hydroxypropyl cellulose and the like; polysorbates, polyoxyethylene hydrogenated castor oil and the like.

Preferable examples of an isotonicity agent include sodium chloride, glycerin, D-mannitol, D-sorbitol, glucose and the like.

Preferable examples of the buffer include buffers such as phosphate, acetate, carbonate, citrate and the like.

Preferable examples of the soothing agent include benzyl alcohol and the like.

Preferable examples of the preservative include p-hydroxybenzoic acid esters, chlorobutanol, benzyl alcohol, phenethyl alcohol, dehydroacetic acid, sorbic acid and the like.

Preferable examples of the antioxidant include sulfite, ascorbate and the like.

Preferable examples of the colorant include water-soluble edible tar pigments (e.g., food colors such as Food Color Red Nos. 2 and 3, Food Color Yellow Nos. 4 and 5 and Food Color Blue Nos. 1 and 2), water-insoluble lake pigments (e.g., aluminum salt of the aforementioned water-soluble edible tar pigment), natural pigments (e.g., β-carotene, chlorophil and colcothar) and the like.

Preferable examples of the sweetening agent include saccharin sodium, dipotassium glycyrrhizinate, aspartame, stevia and the like.

The pharmaceutical composition of the present invention can be produced according to a method conventionally used in the technical field of pharmaceutical preparation, for example, the method described in the Japanese Pharmacopoeia and the like. Hereinafter, a method for preparing a pharmaceutical preparation is explained in detail.

While the content of the compound of the present invention in the pharmaceutical composition of the present invention varies depending on the dosage form, the dose of the compound of the present invention and the like, for example, it is about 0.01 to 100% by weight, preferably 0.1 to 95% by weight with respect to the total amount of the composition.

While the dose of the compound of the present invention varies depending on the subject of administration, administration route, target disease, symptom and the like, for example, for oral administration to an adult diabetic patient (adult, about 60 kg in weight), it is generally about 0.1 to about 20 mg/kg body weight, preferably 0.2 to about 10 mg/kg body weight, more preferably 0.5 to about 10 mg/kg body weight for one dose, which is desirably administered once to several times (e.g., three times) a day.

The compound of the present invention may be used in combination with other active ingredients. Examples of such active ingredients (hereinafter to be referred as concomitant drug) include:

(1) atypical antipsychotic agents (such as clozapine, olanzapine, risperidone, aripiprazole, iloperidone, asenapine, ziprasidone, quetiapine, and zotepine);
(2) typical antipsychotic agents (such as haloperidol and chlorpromazine);
(3) selective serotonin reuptake inhibitors (such as paroxetine, sertraline, fluvoxamine, and fluoxetine), selective serotonin-noradrenaline reuptake inhibitors (such as milnacipran and venlafaxine),
(4) selective noradrenaline-dopamine reuptake inhibitors (e.g., bupropion);
(5) tetracyclic antidepressants (such as amoxapine and clomipramine);
(6) tricyclic antidepressants (such as imipramine and amitriptyline);
(7) other antidepressant agents (such as NS-2359, Lu AA21004, and DOV21947);
(8) nicotinic acetylcholine receptor modulators (e.g., varenicline, SSR-180711, PNU-120596 etc.),
(9) muscarinic acetylcholine receptor modulators (e.g., sabcomeline etc.),
(10) ionotropic glutamic acid receptor modulators (e.g., D-serine,LY-451395 etc.),
(11) metabolic glutamic acid receptor modulators (e.g., CDPPB, MPEP, LY-2140023 etc.),
(12) GABA receptor modulators (e.g., valproic acid, MK-0777 etc.),
(13) histamine receptor modulators (e.g., GSK-239512 etc.),
(14) dopamine receptor modulators (e.g., cariprazine etc.),
(15) serotonin receptor modulators (e.g., pimavanserin, GSK-742457 etc.),
(16) noradrenaline receptor antagonist (e.g., idazoxan etc.),
(17) CRF receptor modulators (e.g., pexacerfont etc.),
(18) substance P receptor modulators (e.g., aprepitant, orvepitant etc.),
(19) orexin receptor modulators (e.g., almorexant, MK-4305 etc.),
(20) phosphodiesterase inhibitor (e.g., MP-10, WEB-3, rolipram etc.),
(21) glycine transporter 1 inhibitor (e.g., RG1678, ALX5407, SSR504734 etc.),
(22) anxiolytics [benzodiazepine-based agents (such as diazepam and etizolam) and serotonin 5-$HT_{1A}$ agonists (such as tandospirone)];
(23) sleep inducing agents [benzodiazepine-based agents (e.g... estazolam and triazolam);
(24) non-benzodiazepine-based agents (such as zolpidem),
(25) melatonin receptor agonists (such as ramelteon)];
(26) β-amyloid vaccines, β-amyloid antibody;

(27) β-amyloid degrading enzyme etc.,
(28) brain function activators (such as aniracetam and nicergoline);
(29) anti-Parkinson agents [such as dopamine receptor agonists (including L-DOPA, bromocriptine, pergolide, talipexole, pramipexole, cabergoline, and amantadine), mono-amine oxidase (MAO) inhibitors (such as deprenyl, selegiline, remacemide, and riluzole), anticholinergic agents (such as trihexyphenidyl and biperiden); and COMT inhibitors (such as entacapone)];
(30) therapeutic agents for amyotrophic lateral sclerosis (including neurotrophic factors and riluzole);
(31) antihyperlipidemic drugs such as cholesterol-lowering drugs [statins (such as pravastatin sodium, atorvastatin, simvastatin, and rosuvastatin), fibrates (such as clofibrate), and squalene synthase inhibitors];
(32) therapeutic agents for abnormal behaviors/wandering symptoms associated with dementia (such as sedatives and anxiolytics);
(33) apoptosis inhibitors (such as CPI-1189, IDN-6556, and CEP-1347);
(34) neuronal differentiation/regeneration accelerators (such as leteprinim and xaliproden (SR-57746-A), and SB-216763);
(35) antihypertensive agents;
(36) therapeutic agents for diabetes mellitus;
(37) nonsteroidal anti-inflammatory agents (such as meloxicam, tenoxicam, indomethacin, ibuprofen, celecoxib, rofecoxib, aspirin, and indomethacin);
(38) disease-modifying anti-rheumatic drugs (DMARDs);
(39) anticytokine agents (such as TNF inhibitors and MAP kinase inhibitors);
(40) steroid agents (such as dexamethasone, hexestrol, and cortisone acetate); sex hormones or the derivatives thereof (such as progesterone, estradiol, and estradiol benzoate);
(41) parathyroid hormone (PTH);
(42) calcium receptor blockers
(43) antiepileptic drugs (e.g., phenytoin, pregabalin, carbamazepine etc.),

can be mentioned.
As concomitant drug, various drugs acting on the central nervous system, or a therapeutic drug for easily-associated diseases with schizophrenia (therapeutic drug for diabetes etc.) is preferable.
The compound of the present invention can be used in combination with a concomitant drug which particularly does not act for GPR52.

The dosage forms of the compound of the present invention and the concomitant drugs thereof are not specifically limited. Any dosage form may be employed as long as the compound of the present invention is combined with any of the concomitant drugs. Exemplary dosage forms include:

(1) administration of a single medicament prepared by simultaneously formulating the compound of the present invention and the concomitant drug;
(2) administration of two different medicaments by the same administering route at same time, which are independently formulated from the compound of the present invention and the concomitant drug;
(3) administration of two different medicaments by the same administering route at different times, which are independently formulated from the compound of the present invention and the concomitant drug;
(4) administration of two different medicaments by different administering routes at same time, which are independently formulated from the compound of the present invention and the concomitant drug;
(5) administration of two different medicaments by different administering routes at different times, which are independently formulated from the compound of the present invention and the concomitant drug (e.g., the compound of the present invention and the concomitant drug are administered in this order and vice versa); and the like. Hereinafter, these dosage forms are collectively referred to as a combination agent of the present invention.

When the combination agent of the present invention is administered, both the concomitant drug and the compound of the present invention may be simultaneously administered. Alternatively, after the administration of a concomitant drug, the compound of the present invention may be administered. Alternatively, the concomitant drug may be administered after the administration of the compound of the present invention. In the case of administration at different times, the time difference may vary among active ingredients, dosage forms and medication methods. For instance, there is a method in which, when the concomitant drug is administered first, the compound of the present invention is administered after one minute or more but not more than three days, preferably 10 minutes to one day, more preferably 15 minutes to one hour from the administration of the concomitant drug. For instance, there is another method in which, when the concomitant drug is administered after one minute or more but not more than one day, preferably 10 minutes to 6 hours, more preferably 15 minutes to one hour from the administration of the compound of the present

invention.

The concomitant drug may be contained in any amount as long as a side effect does not pose a problem. The daily dose of the concomitant drug may vary depending on the target of administration, route of administration, target diseases, symptoms, and so on. For example, when orally administering to a schizophrenia patient (adult, about 60 kg in weight), it is desirable to administer the concomitant drug in general at a unit dose of abut 0.1 to about 20 mg/kg weight, preferably about 0.2 to about 10 mg/kg weight, more preferably about 0.5 to about 10 mg/kg weight. The unit dose of the concomitant drug may be preferably administered one to several times (e.g., three times) a day.
When the compound of the present invention is administered in combination with the concomitant drug, the amounts of the respective agents may be reduced within their safe ranges in consideration of their opposing effects.

The combination agent of the present invention is less toxic, so that it can be administered in safety in the form of a pharmaceutical composition prepared by mixing the compound of the present invention and/or the above concomitant drug with a pharmaceutically acceptable carrier according to a well-known method. Specifically, for example, it may be orally or parenterally (e.g., locally, rectal, or intravenous) administered in the form of a tablet (e.g., sugar-coated tablet or a film-coating tablet), powders, granules, a capsule (inclusive of a soft capsule), a liquid drug, an injection agent, a suppository agent, a sustained-release agent, or the like in safety.

The pharmaceutically acceptable carrier to be used in the production of the combination agent of the present invention may be any of those used for the pharmaceutical composition of the present invention.

A blending ratio of the compound of the present invention to the concomitant drug in the combination agent of the present invention can be appropriately determined depending on the target of administration, the route of administration, diseases and the like.
Two or more of the concomitant drugs as described above may be combined together at an appropriate ratio.
The dosage of the concomitant drug can be appropriately determined on the basis of a clinically used dosage. A blending ratio of the compound of the present invention to the concomitant drug can be appropriately determined depending on the target of administration, the route of administration, target diseases, symptoms, combination and the like. For example, if the target of administration is a human, 0.01 to 100 parts by weight of the concomitant drug may be used for one part by weight of the compound of the present invention.

For instance, the content of the compound of the present invention in the combination agent of the present invention varies among the dosage forms. In general, however, the content of the compound of the present invention is in the range of about 0.01 to 99.9% by weight, preferably about 0.1 to 50% by weight, more preferably about 0.5 to 20% by weight with respect to the whole amount of the medicament.

The content of the concomitant drug in the combination agent of the present invention varies among the dosage forms. In general, however, the content of the concomitant drug is in the range of about 0.01 to 99.9% by weight, preferably about 0.1 to 50% by weight, more preferably about 0.5 to 20% by weight with respect to the whole amount of the medicament.

The content of any additive such as a carrier in the combination agent of the present invention varies among the dosage forms. In general, however, the content of the additive is in the range of about 1 to 99.99% by weight, preferably about 10 to 90% by weight with respect to the whole amount of the medicament.

The contents of the compound of the present invention and the concomitant drug may be equal to those described above even if they are independently formulated.

As described above, the dosage varies under various conditions, so that the contents of the compound of the present invention and the concomitant drug may be less than the above dosages or may be higher than the above dosages in some cases.

## Examples

The present invention will be illustrated in further detail by the following Reference Examples, Examples, Formulation Example and Experimental Example, but these examples, which are merely embodiments, do not limit the present invention and may be modified without departing from the scope of the invention.

In the following Reference Examples and Examples, "room temperature" ordinarily indicates a temperature from about 10°C to about 35°C. Percentages for yield indicate mol/mol% and percentages for media used in chromatography indicate percent by volume, but otherwise indicate percent by weight. Broad peaks such as OH and NH protons that could not be confirmed in the proton NMR spectra are not included in the data. Kiesselgel 60 by Merck was used in silica gel chromatography and Chromatorex NH by Fuji Silysia Chemical Ltd. was used in basic silica gel chromatography.

Other abbreviations used in this document are defined below.

s: singlet
d: doublet

dd: doublet of doublets
dt: doublet of triplets
t: triplet
tt: triplet of triplets
td: triplet of doublets
q: quartet
septet
m: multiplet
br: broad
J: coupling constant
Hz: Hertz
$CDCl_3$: deuterated chloroform
DMSO-$d_6$: deuterated dimethyl sulfoxide
$^1$H-NMR: proton nuclear magnetic resonance HPLC: high performance liquid chromatography
THF: tetrahydrofuran
DMF: N,N-dimethylformamide
DMSO: dimethyl sulfoxide
NMP: N-methylpyrrolidone
HOBt: 1-hydroxybenzotriazole
WSC: 1-ethyl-3-(3-dimethylaminopropyl)carbodiimide hydrochloride
HATU: 2-(7-aza-1H-benzotriazol-1-yl)-1,1,3,3-tetramethyluronium hexafluorophosphate
DMTMM: 4-(4,6-dimethoxy-1,3,5-triazin-2-yl)-4-methylmorpholinium chloride n-hydrate
DBU: 1,8-diazabicyclo[5.4.0]-7-undecene
LC-MS: liquid chromatography-mass spectrometry
ESI: electrospray ionization

Reference Example 1

ethyl 2-[2-[3-(trifluoromethyl)benzyl]-1-benzothiophen-7-yl]-1,3-thiazole-5-carboxylate

A mixture of 4,4,5,5-tetramethyl-2-[2-[3-(trifluoromethyl)benzyl]-1-benzothiophen-7-yl]-1,3,2-dioxaborolane (1.05 g, 2.5 mmol), ethyl 2-bromo-1,3-thiazole-5-carboxylate (1.18 g, 5.0 mmol), tetrakis(triphenylphosphine)palladium (289 mg, 0.25 mmol) and 2N aqueous sodium carbonate solution (7.5 mL) in dimethoxyethane (20 mL) was stirred overnight at 80°C. The reaction mixture was diluted with ethyl acetate, and washed with saturated brine. The organic layer was dried over anhydrous sodium sulfate, and concentrated under reduced pressure. The residue was purified by silica gel column chromatography (hexane-ethyl acetate=9:1). The obtained solid was crystallized from ethanol-water to give the title compound (340 mg, yield 30 %).
$^1$H-NMR ($CDCl_3$) 5: 1.42 (3 H, t, J = 7.1 Hz), 4.33 (2 H, s), 4.41 (2 H, q, J = 7.1 Hz), 7.10 (1 H, s), 7.38 - 7.61 (5 H, m), 7.81 (1 H, d, J = 8.0 Hz), 7.86 (1 H, d, J = 7.7 Hz), 8.52 (1 H, s).

Reference Example 2

2-[2-[3-(trifluoromethyl)benzyl]-1-benzothiophen-7-yl]-1,3-thiazole-5-carboxylic acid

A mixture of ethyl 2-[2-[3-(trifluoromethyl)benzyl]-1-benzothiophen-7-yl]-1,3-thiazole-5-carboxylate obtained in Reference Example 1 (320 mg, 0.72 mmol) and 1N aqueous sodium hydroxide solution (2.1 mL, 2.1 mmol) in THF (3 mL)-ethanol (3 mL) was stirred for 3 hr at 80°C. The reaction mixture was neutralized with 1N hydrochloric acid, and the resulting solid was collected by filtration to give the title compound (272 mg, yield 90 %) as a colorless solid.
$^1$H-NMR (DMSO-$d_6$) $\delta$: 4.42 (2 H, s), 7.37 (1 H, s), 7.46 - 7.77 (6 H, m), 7.94 - 8.04 (2 H, m), 8.46 - 8.53 (1 H, m).

Reference Example 3

ethyl 1-[2-[3-(trifluoromethyl)benzyl]-1-benzothiophen-7-yl]-1H-pyrazole-4-carboxylate

A mixture of 4,4,5,5-tetramethyl-2-[2-[3-(trifluoromethyl)benzyl]-1-benzothiophen-7-yl]-1,3,2-dioxaborolane (1.25 g, 3.0 mmol), ethyl 1H-pyrazole-4-carboxylate (840 mg, 6.0 mmol) and copper(II) acetate hydrate (1.19 g, 6.0 mmol) in pyridine (10 mL) was stirred overnight at 100°C. The reaction mixture was diluted with ethyl acetate, and the mixture was washed with 1N hydrochloric acid and saturated brine. The organic layer was dried over anhydrous sodium

sulfate, and concentrated under reduced pressure. The residue was purified by silica gel column chromatography (hexane-ethyl acetate=4:1) to give the title compound (557 mg, yield 43%) as a colorless solid.
$^1$H-NMR ($CDCl_3$) δ: 1.39 (3 H, t, J = 7.1 Hz), 4.29 (2 H, s), 4.36 (2 H, q, J = 7.1 Hz), 7.08 - 7.11 (1 H, m), 7.38 - 7.57 (6 H, m), 7.68 (1 H, dd, J = 6.6, 2.2 Hz), 8.18 (1 H, s), 8.50 (1 H, s).

Reference Example 4

1-[2-[3-(trifluoromethyl)benzyl]-1-benzothiophen-7-yl]-1H-pyrazole-4-carboxylic acid

A mixture of ethyl 1-[2-[3-(trifluoromethyl)benzyl]-1-benzothiophen-7-yl]-1H-pyrazole-4-carboxylate obtained in Reference Example 3 (530 mg, 1.2 mmol) and 1N aqueous sodium hydroxide solution (3.7 mL, 3.7 mmol) in THF (5 mL)-ethanol (5 mL) was stirred for 3 hr at 80°C. The reaction mixture was neutralized with 1N hydrochloric acid, and the resulting solid was collected by filtration to give the title compound (460 mg, yield 95 %) as a brown solid.
$^1$H-NMR (DMSO-$d_6$) δ: 4.39 (2 H, s), 7.35 (1 H, s), 7.47 (1 H, t, J = 7.9 Hz), 7.53 - 7.91 (6 H, m), 8.15 (1 H, s), 9.08 (1 H, s).

Reference Example 5

2-[3-(trifluoromethyl)benzyl]-1-benzothiophen-7-amine

A mixture of 7-bromo-2-[3-(trifluoromethyl)benzyl]-1-benzothiophene (3.71 g, 10.0 mmol), benzophenonimine (3.35 mL, 20 mmol), palladium acetate (68 mg, 0.30 mmol), 2,2'-bis(diphenylphosphino)-1,1'-binaphthyl (373 mg, 0.60 mmol) and sodium tert-butoxide (1.92 g, 20 mmol) in toluene (50 mL) was heated under reflux overnight under a nitrogen atmosphere. The reaction mixture was allowed to cool to room temperature, and diluted with ethyl acetate. The mixture was washed with saturated brine. The organic layer was dried over anhydrous sodium sulfate, and concentrated under reduced pressure. The residue was dissolved in THF (30 mL), and 1N hydrochloric acid (10 mL) was added thereto. The mixture was stirred for 24 hr at 50°C. The reaction mixture was diluted with ethyl acetate, and washed with saturated aqueous sodium hydrogen carbonate solution and saturated brine. The organic layer was dried over anhydrous sodium sulfate, and concentrated under reduced pressure. The residue was purified by silica gel column chromatography (hexane-ethyl acetate=4:1) to give the title compound (2.85 g, yield 93%) as an oil.
$^1$H-NMR ($CDCl_3$) δ: 3.79 (2 H, br s), 4.28 (2 H, s), 6.57 - 6.66 (1 H, m), 7.02 (1 H, t, J = 0.9 Hz), 7.14 - 7.21 (2 H, m), 7.38 - 7.60 (4 H, m).

Reference Example 6

ethyl 1-[2-[3-(trifluoromethyl)benzyl]-1-benzothiophen-7-yl]-1H-imidazole-4-carboxylate

A solution of 2-[3-(trifluoromethyl)benzyl]-1-benzothiophen-7-amine obtained in Reference Example 5 (1.84 g, 6.0 mmol), ethyl orthoformate (2.0 mL, 12.0 mmol) and acetic acid (2.0 mL) in ethanol (20 mL) was stirred for 5 hr at 100°C. To the reaction mixture was added ethyl nitroacetate (1.3 mL), and the mixture was stirred overnight at 100°C. The reaction mixture was allowed to cool to room temperature, and the resulting solid was collected by filtration. The obtained solid was dissolved in ethyl orthoformate (10 mL). To the solution was added 10%Pd/C (300 mg), and the mixture was stirred overnight at 80°C under a hydrogen atmosphere. The reaction mixture was allowed to cool to room temperature, diluted with ethanol, and filtered. The filtrate was concentrated under reduced pressure, and the obtained residue was purified by silica gel column chromatography (ethyl acetate) to give the title compound (860 mg, yield 33%) as an oil.
$^1$H-NMR ($CDCl_3$) δ: 1.42 (3 H, t, J = 7.1 Hz), 4.29 (2 H, s), 4.41 (2 H, q, J = 7.1 Hz), 7.12 - 7.16 (1 H, m), 7.24 - 7.30 (1 H, m), 7.41 - 7.57 (5 H, m), 7.75 (1 H, dd, J = 8.0, 1.1 Hz), 7.90 (1 H, d, J = 1.4 Hz), 8.02 (1 H, d, J = 1.4 Hz).

Reference Example 7

1-[2-[3-(trifluoromethyl)benzyl]-1-benzothiophen-7-yl]-1H-imidazole-4-carboxylic acid

A mixture of ethyl 1-[2-[3-(trifluoromethyl)benzyl]-1-benzothiophen-7-yl]-1H-imidazole-4-carboxylate obtained in Reference Example 6 (860 mg, 2.0 mmol) and 1N aqueous sodium hydroxide solution (6.0 mL, 6.0 mmol) in THF (10 mL)-ethanol (10 mL) was stirred for 3 hr at 80°C. The reaction mixture was neutralized with 1N hydrochloric acid, and the resulting solid was collected by filtration to give the title compound (650 mg, yield 81 %) as a colorless solid.
$^1$H-NMR (DMSO-$d_6$) δ: 4.41 (2 H, s), 7.40 - 7.77 (7 H, m), 7.91 (1 H, dd, J = 7.0, 2.1 Hz), 8.19 (1 H, d, J = 1.5 Hz), 8.23 (1 H, d, J = 1.5 Hz), 12.60 (1 H, br s).

Reference Example 8

2-[3-(trifluoromethyl)benzyl]-1-benzothiophene-7-carbonitrile

A mixture of 7-bromo-2-[3-(trifluoromethyl)benzyl]-1-benzothiophene (1.0 g, 2.69 mmol) and copper cyanide (0.31 g, 3.50 mol) in DMF (5 ml) was stirred for 3.5 hr at 150°C. Copper cyanide (0.36 g, 1.4 mol) was added again thereto, and the mixture was stirred for 4.5 hr at 150°C. The reaction mixture was diluted with water, and extracted with ethyl acetate. The extract was washed with water, dried over anhydrous magnesium sulfate, and evaporated under reduced pressure. The residue was purified by silica gel column chromatography (hexane:ethyl acetate=10:1 - 2:3) to give the title compound (0.37 g, 43 %) as crystals.
$^1$H-NMR ($CDCl_3$) $\delta$: 4.32 (2 H, s), 7.08 (1 H, s), 7.39 (1 H, t, J = 8.1 Hz), 7.45 - 7.60 (4 H, m), 7.61 (1 H, d, J = 7.5 Hz), 7.87 (1 H, d, J =8.1 Hz).

Reference Example 9

2-[3-(trifluoromethyl)benzyl]-1-benzothiophene-7-carboxamide

To a solution of 2-[3-(trifluoromethyl)benzyl]-1-benzothiophene-7-carbonitrile obtained in Reference Example 8 (0.37 g, 1.17 mmol) in ethanol (20 mL) was added 1N aqueous sodium hydroxide solution (5.83 mL, 5.83 mmol), and the mixture was stirred for 15 hr at 80°C. The reaction mixture was acidified with 1N hydrochloric acid (10 mL), and the mixture was extracted with ethyl acetate. The extract was washed with water, dried over anhydrous magnesium sulfate, and evaporated under reduced pressure to give 2-[3-(trifluoromethyl)benzyl]-1-benzothiophene-7-carboxylic acid (0.39 g, yield 99%). To a solution of the carboxylic acid (0.39 g, 1.16 mmol) in DMF (5 mL) were added WSC (0.30 g, 1.74 mmol) and HOBt (0.24 g, 1.74 mmoL), and the mixture was stirred for 1 hr at room temperature. The reaction mixture was poured into aqueous ammonia (20 mL), and the mixture was stirred for 1 hr. The reaction mixture was extracted with ethyl acetate. The extract was washed with water, dried over anhydrous magnesium sulfate, and evaporated under reduced pressure to give the title compound (0.36 g, yield 93%) as crystals.
$^1$H-NMR ($CDCl_3$) $\delta$: 4.30 (2 H, s), 6. 00 (2 H, br s), 7. 05 (1 H, s), 7.38 - 7.48 (2 H, m), 7.49 (1 H, s), 7.51 (1 H, s), 7.54 (1 H, s), 7.57 (1 H, s), 7.84 (1 H, d, J = 7.8 Hz).

Reference Example 10

ethyl 4-methyl-2-[2-[3-(trifluoromethyl)benzyl]-1-benzothiophen-7-yl]-1,3-oxazole-5-carboxylate

A mixture of 2-[3-(trifluoromethyl)benzyl]-1-benzothiophene-7-carboxamide obtained in Reference Example 9 (0.5 g, 1.49 mmol) and ethyl 2-chloro-3-oxobutanoate (0.84 mL, 6.0 mmol) was stirred for 7 hr at 120°C. The reaction mixture was allowed to cool, and purified by silica gel column chromatography (ethyl acetate:methanol=10:1) to give the title compound (0.23 g, yield 34%) as crystals.
$^1$H-NMR ($CDCl_3$) $\delta$: 1.43 (3 H, t, J = 7.1 Hz), 2.60 (3 H, s), 4.33 (2 H, s), 4.42 (2 H, q, J = 7.1 Hz), 7.09 (1 H, s), 7.40 - 7.58 (4 H, m), 7.57 (1 H, s), 7.82 (1 H, d, J = 7.5 Hz), 8.14 (1 H, d, J = 7.5 Hz) .

Reference Example 11

ethyl 5-methyl-1-[2-[3-(trifluoromethyl)benzyl]-1-benzothiophen-7-yl]-1H-pyrazole-4-carboxylate

Reference Example 12

ethyl 3-methyl-1-[2-[3-(trifluoromethyl)benzyl]-1-benzothiophen-7-yl]-1H-pyrazale-4-carboxylate

A mixture of 4,4,5,5-tetramethyl-2-[2-[3-(trifluoromethyl)benzyl]-1-benzothiophen-7-yl]-1,3,2-dioxaborolane (1.25 g, 3.0 mmol), ethyl 3-methyl-1H-pyrazole-4-carboxylate (554 mg, 3.6 mmol) and copper(II) acetate hydrate (300 mg, 1.5 mmol) in pyridine (15 mL) was stirred overnight at 50°C, and the reaction mixture was concentrated under reduced pressure. The residue was purified by silica gel column chromatography (hexane-ethyl acetate=65:35) to give ethyl 5-methyl-1-[2-[3-(trifluoromethyl)benzyl]-1-benzothiophen-7-yl]-1H-pyrazole-4-carboxylate (150 mg, yield 11%) as brown amorphous and ethyl 3-methyl-1-[2-[3-(trifluoromethyl)benzyl]-1-benzothiophen-7-yl]-1H-pyrazole-4-carboxylate (884 mg, yield 66%) as a colorless solid. ethyl 5-methyl-1-[2-[3-(trifluoromethyl)benzyl]-1-benzothiophen-7-yl]-1H-pyrazole-4-carboxylate (Reference Example 11)
$^1$H-NMR ($CDCl_3$) $\delta$: 1.38 (3 H, t, J = 7.2 Hz), 2.60 (3 H, s), 4.29 (2 H, s), 4.33 (2 H, q, J = 6.9 Hz), 7.06 - 7.08 (1 H, m),

7.35 - 7.68 (7 H, m), 8.44 (1 H, s).
ethyl 3-methyl-1-[2-[3-(trifluoromethyl)benzyl]-1-benzothiophen-7-yl]-1H-pyrazole-4-carboxylate (Reference Example 12)
$^1$H-NMR ($CDCl_3$) $\delta$: 1.38 (3 H, t, J = 7.2 Hz), 2.51 (3 H, s), 4.26 (2 H, s), 4.34 (2 H, q, J = 6.9 Hz), 7.10 (1 H, s), 7.23 (1 H, dd, J = 7.6, 1.1 Hz), 7.39 - 7.55 (5 H, m), 7.76 (1 H, dd, J = 8.0, 1.1 Hz), 8.10 (1 H, s).

Reference Example 13

5-methyl-1-[2-[3-(trifluoromethyl)benzyl]-1-benzothiophen-7-yl]-1H-pyrazole-4-carboxylic acid

A mixture of ethyl 5-methyl-1-[2-[3-(trifluoromethyl)benzyl]-1-benzothiophen-7-yl]-1H-pyrazole-4-carboxylate obtained in Reference Example 11 (30 mg, 0.068 mmol) and 1N aqueous sodium hydroxide solution (0.20 mL, 0.20 mmol) in THF (1 mL)-ethanol (1 mL) was stirred overnight at 80°C. To the reaction mixture was added 1N hydrochloric acid, and the mixture was extracted with ethyl acetate. The organic layer was washed with saturated brine, dried over anhydrous sodium sulfate, and concentrated under reduced pressure to give the title compound (19 mg, yield 67 %) as a pale-yellow amorphous.
$^1$H-NMR ($CDCl_3$) $\delta$: 2.53 (3 H, s), 4.26 (2 H, s), 7.08 - 7.14 (1 H, m), 7.20 - 7.29 (1 H, m), 7.39 - 7.57 (5 H, m), 7.77 (1 H, dd, J = 8.0, 0.8 Hz), 8.18 (1 H, s).

Reference Example 14

3-methyl-1-[2-[3-(trifluoromethyl)benzyl]-1-benzothiophen-7-yl]-1H-pyrazole-4-carboylic acid

A mixture of ethyl 3-methyl-1-[2-[3-(trifluoromethyl)benzyl]-1-benzothiophen-7-yl]-1H-pyrazole-4-carboxylate obtained in Reference Example 12 (1.16 g, 2.6 mmol) and 1N aqueous sodium hydroxide solution (13.1 mL, 13.1 mmol) in THF (20 mL)-ethanol (20 mL) was stirred overnight at 80°C. To the reaction mixture was added 1N hydrochloric acid, and the mixture was extracted with ethyl acetate. The organic layer was washed with saturated brine, dried over anhydrous sodium sulfate, and concentrated under reduced pressure. To the residue was added isopropyl ether, and the mixture was stirred. The resulting solid was collected by filtration to give the title compound (855 mg, yield 79 %) as a colorless solid.
$^1$H-NMR (DMSO-$d_6$) 5: 2.46 (3 H, s), 4.40 (2 H, s), 7.31 (1 H, s), 7.45 (1 H, t, J = 7.8 Hz), 7.54 - 7.84 (6 H, m), 8.98 (1 H, s), 12.55 (1 H, br s).

Reference Example 15 ethyl 3-(trifluoromethyl-1-[2-[3-(trifluoromethyl)benzyl]-1-benzothiophen-7-yl]-1H-pyrazole-4-carboxylate

A mixture of 4,4,5,5-tetramethyl-2-[2-[3-(trifluoromethyl)benzyl]-1-benzothiophen-7-yl]-1,3,2-dioxaborolane (1.25 g, 3.0 mmol), ethyl 3-(trifluoromethyl)-1H-pyrazole-4-carboxylate (1.50 g, 7.2 mmol) and copper(II) acetate hydrate (600 mg, 3.0 mmol) in pyridine (15 mL) was stirred for 3 days at 50°C, and the reaction mixture was concentrated under reduced pressure. The residue was purified by silica gel column chromatography (hexane-ethyl acetate=7:3), and the obtained solid was washed with hexane to give the title compound (1.03 g, yield 69%) as a colorless solid. $^1$H-NMR ($CDCl_3$) $\delta$: 1.39 (3 H, t, J = 7.2 Hz), 4.31 (2 H, s), 4.38 (2 H, q, J = 7.2 Hz), 7.08 (1 H, t, J = 1.1 Hz), 7.40 - 7.58 (6 H, m), 7.73 (1 H, dd, J = 7.3, 1.7 Hz), 8.58 - 8.60 (1 H, m).

Reference Example 16

3-(trifluoromethyl)-1-[2-[3-(trifluoromethyl)benzyl]-1-benzothiophen-7-yl]-1H-pyrazole-4-carboxylic acid

A mixture of ethyl 3-(trifluoromethyl)-1-[2-[3-(trifluoromethyl)benzyl]-1-benzothiophen-7-yl]-1H-pyrazole-4-carboxylate obtained in Reference Example 15 (1.22 g, 2.5 mmol) and 1N aqueous sodium hydroxide solution (7.4 mL, 7.4 mmol) in THF (15 mL)-ethanol (15 mL) was stirred for 1 hr at 50°C. To the reaction mixture was added 1N hydrochloric acid, and the resulting solid was collected by filtration to give the title compound (1.08 g, yield 94 %) as a colorless solid. $^1$H-NMR (DMSO-$d_6$) $\delta$: 4.42 (2 H, s), 7.36 (1 H, s), 7.47 - 7.77 (5 H, m), 7.89 (1 H, d, J = 7.6 Hz), 7.95 (1 H, d, J = 7.6 Hz), 9.33 (1 H, s), 13.29 (1 H, br s).

Reference Example 17

2,2,2-trichloro-1-(1-methyl-1H-imidazol-2-yl)ethanone

To a solution of trichloroacetyl chloride (1.36 mL, 12.2 mmol) in toluene (25 mL) was added dropwise a solution of 1-methyl-1H-imidazole (971 μL, 12.2 mmol) in toluene (25 mL), and the mixture was stirred for 7 hr at room temperature. The reaction mixture was ice-cooled, and triethylamine (1.7 mL, 12.2 mL) was added thereto. The mixture was evaporated under reduced pressure. The residue was suspended in THF-toluene mixed solution, and the suspension was purified by filtration column chromatography using silica gel. The residue was concentrated to give the title compound (2.54 g, yield 91%). $^1$H-NMR ($CDCl_3$) δ: 4.07 (3 H, s), 7.17 (1 H, s), 7.36 (1 H, d, J = 0.8 Hz).

Reference Example 18

ethyl 4-bromo-1-methyl-1H-imidazole-2-carboxylate

To a solution of 2,2,2-trichloro-1-(1-methyl-1H-imidazol-2-yl)ethanone obtained in Reference Example 17 (1.0 g, 4.4 mmol) in THF (30 ) was added N-bromosuccinimide (1.56 g, 8.8 mmol) under ice-cooling, and the mixture was stirred for 1 day at room temperature. The reaction mixture was concentrated under reduced pressure to give crude 1-(4-bromo-1-methyl-1H-imidazol-2-yl)-2,2,2-trichloroethanone. To a solution of the obtained crude product in ethanol (40 mL) was added a solution of sodium ethoxide in 20% ethanol (1.0 mL), and the mixture was stirred for 4 hr at room temperature. The reaction mixture was concentrated under reduced pressure, and the residue was purified by silica gel column chromatography (hexane:ethyl acetate= 33:67→0:100, 90:10→50:50) to give the title compound (181 mg, yield 18%).
$^1$H-NMR ($CDCl_3$) δ : 1.42 (3 H, t, J = 7.1 Hz), 3.39 (3 H, s), 4.41 (2 H, q, J = 7.1 Hz), 7.01 (1 H, s).

Reference Example 19

ethyl 1-methyl-4-[2-[3-(trifluoromethyl)benzyl]-1-benzothiophen-7-yl]-1H-imidazole-2-carboxylate

A mixture of ethyl 4-bromo-1-methyl-1H-imidazole-2-carboxylate obtained in Reference Example 18 (221 mg, 0.95 mmol), 4,4,5,5-tetramethyl-2-[2-[3-(trifluoromethyl)benzyl]-1-benzothiophen-7-yl]-1,3,2-dioxaborolane (476 mg, 1.14 mmol), tetrakis(triphenylphosphine)palladium(0) (44 mg, 0.038 mmol) and 2N aqueous sodium carbonate solution (1.9 mL)-1,2-dimethoxyethane (8.0 mL) was heated under reflux overnight under a nitrogen atmosphere. To the reaction mixture was added saturated brine, and the mixture was extracted with ethyl acetate. The organic layer was dried over anhydrous sodium sulfate, and concentrated under reduced pressure. The residue was purified by silica gel column chromatography (hexane-ethyl acetate 95:5→67:33) to give the title compound (95 mg, yield 23%).
$^1$H-NMR ($CDCl_3$) δ : 1.47 (3 H, t, J = 7.1 Hz), 4.10 (3 H, s), 4.32 (2 H, s), 4.47 (2 H, q, J = 7.1 Hz), 7.09 (1 H, t, J = 1.1 Hz), 7.34 - 7.47 (2 H, m), 7.47 - 7.55 (3 H, m), 7.58 (1 H, s), 7.64 (1 H, dd, J = 8.0, 1.1 Hz), 7.82 (1 H, dd, J = 7.4, 1.1 Hz).

Reference Example 20

methyl 1-methyl-2-[2-[3-(trifluoromethyl)benzyl]-1-benzothiophen-7-yl]-1H-imidazole-5-carboxylate

In the same manner as in Reference Example 1 and using 4,4,5,5-tetramethyl-2-[2-[3-(trifluoromethyl)benzyl]-1-benzothiophen-7-yl]-1,3,2-dioxaborolane and methyl 2-bromo-1-methyl-1H-imidazole-5-carboxylate, the title compound was obtained as an oil. yield 74%
$^1$H-NMR ($CDCl_3$) δ: 3.89 (3 H, s), 3.95 (3 H, s), 4.28 (2 H, s) , 7.10 (1 H, s), 7.36 - 7.56 (6 H, m), 7.74 - 7.80 (1 H, m), 7.91 (1 H, s).

Reference Example 21

1-methyl-2-[2-[3-(trifluoromethyl)benzyl]-1-benzothiophen-7-yl]-1H-imidazole-5-carboxylic acid

To a mixture of methyl 1-methyl-2-[2-[3-(trifluoromethyl)benzyl]-1-benzothiophen-7-yl]-1H-imidazole-5-carboxylate obtained in Reference Example 20 (225 mg, 0.523 mmol) in THF (3.0 ml)-methanol (1.5 ml) was added 1N aqueous sodium hydroxide solution (0.784 ml) at room temperature, and the mixture was stirred overnight at room temperature. Water was poured into the reaction mixture. The mixture was adjusted pH2 - 3 with 1N hydrochloric acid, and extracted with ethyl acetate. The extract was washed with water, dried over anhydrous magnesium sulfate, and evaporated under

reduced pressure. To the residue were added diethyl ether and hexane, the title compound (177 mg, yield 81%) was collected by filtration as a solid.
$^1$H-NMR (DMSO-$d_6$) δ: 3.89 (3 H, s), 4.38 (2 H, s), 7.35 (1 H, s), 7.46 - 7.68 (5 H, m), 7.71 (1 H, s), 7.78 (1 H, s), 7.91 (1 H, d, J=7.6 Hz), 12.93 (1 H, br s).

Reference Example 22

ethyl 3-(1-methylethyl)-1-[2-[3-(trifluoromethyl)benzyl]-1-benzothiophen-7-yl]-1H-pyrazole-4-carboxylate

Reference Example 23

ethyl 5-(1-methylethyl)-1-[2-[3-(trifluoromethyl)benzyl]-1-benzothiophen-7-yl]-1H-pyrazole-4-carboxylate

To a solution of 4,4,5,5-tetramethyl-2-[2-[3-(trifluoromethyl)benzyl]-1-benzothiophen-7-yl]-1,3,2-dioxaborolane (0.96 g, 2.29 mmol) in pyridine (5 mL) were added ethyl 3-(1-methylethyl)-1H-pyrazole-4-carboxylate (0.5 g, 2.74 mmol) and copper(II) acetate monohydrate (0.23 g, 1.15 mmol), and the mixture was stirred overnight at 55°C. The reaction mixture was evaporated under reduced pressure. The residue was purified by silica gel column chromatography (ethyl acetate:hexane=1:4) to give ethyl 3-(1-methylethyl)-1-[2-[3-(trifluoromethyl)benzyl]-1-benzothiophen-7-yl]-1H-pyrazole-4-carboxylate (710 mg, yield 66%) as a solid and ethyl 5-(1-methylethyl)-1-[2-[3-(trifluoromethyl)benzyl]-1-benzothiophen-7-yl]-1H-pyrazole-4-carboxylate (20 mg, yield 2%) as an oil.
ethyl 3-(1-methylethyl)-[2-[3-(trifluoromethyl)benzyl]-1-benzothiophen-7-yl]-1H-pyrazole-4-carboxylate (Reference Example 22)
$^1$H-NMR ($CDCl_3$) δ: 1.36-1.45 (6 H, m), 3.53-3.72 (1 H, m), 4.24-4.41 (4 H, m), 7.03 (1 H, s), 7.32-7.68 (10 H, m), 8.44 (1 H, s).
ethyl 5-(1-methylethyl)-1-[2-[3-(trifluoromethyl)benzyl]-1-benzothiophen-7-yl]-1H-pyrazole-4-carboxylate (Reference Example 23)
$^1$H-NMR ($CDCl_3$) δ: 1.19-1.47 (9 H, m), 3.05-3.24 (1 H, m), 4.19-4.42 (4 H, m), 7.09 (1 H, s), 7.16-7.23 (1 H, m), 7.37-7.61 (5 H, m), 7.73-7.86 (1 H, m), 8.10 (1 H, s).

Reference Example 24

3-(1-methylethyl)-1-[2-[3-(trifluoromethyl)benzyl]-1-benzothiophen-7-yl]-1H-pyrazole-4-carboxylic acid

To a mixture of ethyl 3-(1-methylethyl)-1-[2-[3-(trifluoromethyl)benzyl]-1-benzothiophen-7-yl]-1H-pyrazole-4-carboxylate obtained in Reference Example 22 (0.71 g, 1.50 mmol) in THF (7 mL)-ethanol (7 mL) was added 1N aqueous sodium hydroxide solution (4.51 mL, 4.51 mmol), and the mixture was stirred for 3 hr at 85°C. The reaction mixture was neutralized with 1N hydrochloric acid. The mixture was diluted with water, and extracted with ethyl acetate. The organic layer was washed with water, dried over anhydrous magnesium sulfate, and concentrated under reduced pressure. To the precipitated crystals was added isopropyl alcohol, and the crystals were collected by filtration to give the title compound (0.63 g, yield 94%) as crystals.
$^1$H-NMR (DMSO-$d_6$) δ: 1. 30 (6 H, d, J = 6.8 Hz), 3.46-3.63 (1 H, m), 4.40 (2 H, s), 7.27 (1 H, s), 7.43 (1 H, t, J = 7.8 Hz), 7.53-7.70 (3 H, m), 7.71-7.78 (2 H, m), 7.82 (1 H, d, J = 7.6 Hz), 8.98 (1 H, s), 12.47 (1 H, br s).

Reference Example 25

5-(1-methylethyl)-1-[2-[3-(trifluoromethyl)benzyl]-1-benzothiophen-7-yl]-1H-pyrazole-4-carboxylic acid

In the same manner as in Reference Example 24 and using ethyl 5-(1-methylethyl)-1-[2-[3-(trifluoromethyl) benzyl]-1-benzothiophen-7-yl]-1H-pyrazole-4-carboxylate obtained in Reference Example 23, the title compound was obtained. yield quantitatively oil.
$^1$H-NMR ($CDCl_3$) 5: 1.20-1.38 (6 H, m), 3.04-3.25 (1 H, m), 4.26 (2 H, s), 7.10 (1 H, s), 7.21 (1 H, d, J = 7.7 Hz), 7.37-7.61 (5 H, m), 7.79 (1 H, d, J = 7.7 Hz) , 8. 19 (1 H, br s),1 H, unconfirmed.

Reference Example 26

ethyl 3-cyclopropyl-1-[2-[3-(trifluoromethyl)benzyl]-1-benzothiophen-7-yl]-1H-pyrazole-4-carboxylate Reference Example 27

ethyl 5-cyclopropyl-1-[2-[3-(trifluoromethyl)benzyl]-1-benzothiophen-7-yl]-1H-pyrazole-4-carboxylate

To a solution of 4,4,5,5-tetramethyl-2-[2-[3-(trifluoromethyl)benzyl]-1-benzothiophen-7-yl]-1,3,2-dioxaborolane (1.03 g, 2.45 mmol) in pyridine (5 mL) were added ethyl 3-cyclopropyl-1H-pyrazol-4-carboxylate (0.53 g, 2.94 mmol) and copper(II) acetate monohydrate (0.25 g, 1.23 mmol), and the mixture was stirred overnight at 55°C. The reaction mixture was evaporated under reduced pressure. The residue was purified by silica gel column chromatography (ethyl acetate:hexane=1:4) to give ethyl 3-cyclopropyl-1-[2-[3-(trifluoromethyl)benzyl]-1-benzothiophen-7-yl]-1H-pyrazole-4-carboxylate (990 mg, yield 86%) as a solid and ethyl 5-cyclopropyl-1-[2-[3-(trifluoromethyl)benzyl]-1-benzothiophen-7-yl]-1H-pyrazole-4-carboxylate (20 mg, yield 2%) as an oil. ethyl 3-cyclopropyl-1-[2-[3-(trifluoromethyl)benzyl]-1-benzothiophen-7-yl]-1H-pyrazole-4-carboxylate (Reference Example 26) $^1$H-NMR ($CDCl_3$) δ: 1.00-1.10 (2 H, m), 1.10-1.18 (2 H, m), 1.39 (3 H, t, J = 7.2 Hz), 2.57-2.70 (1 H, m), 4.29 (2 H, s), 4.35 (2 H, q, J = 7.2 Hz), 7.02 (1 H, s), 7.33-7.41 (2 H, m), 7.41-7.56 (3 H, m), 7.56-7.64 (2 H, m), 8.41 (1 H, s). ethyl 5-cyclopropyl-1-[2-[3-(trifluoromethyl)benzyl]-1-benzothiophen-7-yl]-1H-pyrazole-4-carboxylate (Reference Example 27)
$^1$H-NMR ($CDCl_3$) δ: 0.59-0.71 (2 H, m), 0.77-0.91 (2 H, m), 1.32-1.45 (3 H, m), 1.88-2.01 (1 H, m), 4.22-4.39 (4 H, m), 7.08 (1 H, s), 7.30-7.56 (6 H, m), 7.74 (1 H, d, J = 8.0 Hz), 8.07 (1 H, s).

Reference Example 28

3-cyclopropyl-1-[2-[3-(trifluoromethyl)benzyl]-1-benzothiophen-7-yl]-1H-pyrazole-4-carboxylic acid

To a solution of ethyl 3-cyclopropyl-1-[2-[3-(trifluoromethyl)benzyl]-1-benzothiophen-7-yl]-1H-pyrazole-4-carboxylate obtained in Reference Example 26 (0.99 g, 2.10 mmol) in THF (10 )-ethanol (10 mL) was added 8N aqueous sodium hydroxide solution (0.79 mL, 6.31 mmol), and the mixture was stirred for 6 hr at 85°C. The reaction mixture was neutralized with 1N hydrochloric acid. The mixture was diluted with water, and extracted with ethyl acetate. The organic layer was washed with water, dried over anhydrous magnesium sulfate, and concentrated under reduced pressure. To the precipitated was added isopropyl alcohol, and the crystals were collected by filtration to give the title compound (0.65 g, yield 70%) as crystals.
$^1$H-NMR (DMSO-$d_6$) δ: 0.91-1.05 (4 H, m), 2.53-2.69 (1 H, m), 4.39 (2 H, s), 7.25 (1 H, s), 7.42 (1 H, t, J = 8.0 Hz), 7.54-7.69 (3 H, m), 7.73 (2 H, d, J = 8. 0 Hz) , 7. 80 (1 H, d, J = 8.0 Hz), 8.95 (1 H, s), 12.59 (1 H, br s).

Reference Example 29

5-cyclopropyl-1-[2-[3-(trifluoromethyl)benzyl]-1-benzothiophen-7-yl]-1H-pyrazole-4-carboxylic acid

In the same manner as in Reference Example 28 and using ethyl 5-cyclopropyl-1-[2-[3-(trifluoromethyl)benzyl]-1-benzothiophen-7-yl]-1H-pyrazole-4-carboxylate obtained in Reference Example 27, the title compound was obtained. yield 53% oil.
$^1$H-NMR ($CDCl_3$) δ: 0.65-0.74 (2 H, m), 0.80-0.90 (2 H, m), 1.91-2.04 (1 H, m), 4.26 (2 H, s), 7.09 (1 H, s), 7.32-7.56 (6 H, m), 7.70-7.81 (1 H, m), 8.15 (1 H, s), 1 H, unconfirmed.

Reference Example 30

ethyl 4-methyl-1-[2-[3-(trifluoromethyl)benzyl]-1-benzothiophen-7-yl]-1H-pyrazole-3-carboxylate

A mixture of ethyl 4-methyl-1H-pyrazole-3-carboxylate (154 mg, 1.0 mmol), 4,4,5,5-tetramethyl-2-[2-[3-(trifluoromothyl)benzyl]-1-benzothiophen-7-yl]-1,3,2-dioxaborolane (627 mg, 1.2 mmol), pyridine (323 μL, 4.0 mmol) and copper (II) acetate (272 mg, 1.5 mmol) in toluene (5 mL)-1,2-dimethoxyethane (4 mL) was stirred for 4 hr at room temperature, and heated under reflux for 2 days. The reaction mixture was allowed to cool to room temperature, diluted with ethyl acetate, and filtered through celite. The filtrate was washed with saturated brine, dried over anhydrous sodium sulfate, and evaporated under reduced pressure. The residue was purified by silica gel column chromatography (hexane:ethyl acetate= 95:5→50:50) and preparative HPLC to give the title compound (42 mg, 9%).
$^1$H-NMR ($CDCl_3$) δ : 1.43 (3 H, t, J = 7.0 Hz), 2.39 (3 H, s), 4.29 (2 H, s), 4.43 (2 H, q, J = 7.1 Hz), 7.07 (1 H, s), 7.32 - 7.60 (6 H, m), 7.64 (1 H, dd, J = 7.3, 1.5 Hz), 7.86 (1 H, t, J = 0.8 Hz).

Reference Example 31

ethyl 3-methyl-1-[2-[3-(trifluoromethyl)benzyl]-1-benzothiophen-7-yl]-1H-pyrazole-5-carboxylate

Reference Example 32

ethyl 5-methyl-1-[2-[3-(trifluoromethyl)benzyl]-1-benzothiophen-7-yl]-1H-pyrazole-3-carboxylate

To a solution of 4,4,5,5-tetramethyl-2-[2-[3-(trifluoromethyl)benzyl]-1-benzothiophen-7-yl]-1,3,2-dioxaborolane (1.50 g, 3.59 mmol) in pyridine (7.5 mL) were added ethyl 3-methyl-1H-pyrazole-5-carboxylate (0.72 g, 4.66 mmol) and copper(II) acetate monohydrate (0.36 g, 1.80 mmol), and the mixture was stirred overnight at 55°C. The reaction mixture was evaporated under reduced pressure. The residue was purified by silica gel column chromatography (ethyl acetate: hexane=1:4) to give ethyl 3-methyl-1-[2-[3-(trifluoromethyl)benzyl]-1-benzothiophen-7-yl]-1H-pyrazole-5-carboxylate (382 mg, yield 24%) as an oil and ethyl 5-methyl-1-[2-[3-(trifluoromethyl)benzyl]-1-benzothiophen-7-yl]-1H-pyrazole-3-carboxylate (371 mg, yield 23%) as an oil. ethyl 3-methyl-1-[2-[3-(trifluoromethyl)benzyl]-1-benzothiophen-7-yl]-1H-pyrazole-5-carboxylate (Reference Example 31)
$^1$H-NMR ($CDCl_3$) δ: 1.07 (3 H, t, J = 7.1 Hz), 2.38 (3 H, s), 4.12 (2 H, q, J = 7.1 Hz), 4.23 (2 H, s), 6.84 (1 H, s), 7.06 (1 H, s), 7.24-7.57 (6 H, m), 7.71 (1 H, d, J = 8.0 Hz).
ethyl 5-methyl-1-[2-[3-(trifluoromethyl)benzyl]-1-benzothiophen-7-yl]-1H-pyrazole-3-carboxylate (Reference Example 32)
$^1$H-NMR ($CDCl_3$) δ: 1.39 (3 H, t, J = 7.1 Hz), 2.27 (3 H, s), 4.25 (2 H, s), 4.41 (2 H, q, J = 7.1 Hz), 6.77 (1 H, s), 7.08 (1 H, s), 7.23-7.56 (6 H, m), 7.74 (1 H, d, J = 8.0 Hz).

Reference Example 33

3-methyl-1-[2-[3-(trifluoromethyl)benzyl]-1-benzothiophen-7-yl]-1H-pyrazole-5-carboxylic acid

To a mixture of ethyl 3-methyl-1-[2-[3-(trifluoromethyl)benzyl]-1-benzothiophen-7-yl]-1H-pyrazole-5-carboxylate obtained in Reference Example 31 (0.38 g, 0.86 mmol) in THF (5 mL)-ethanol (5 mL) was added 8N aqueous sodium hydroxide solution (0.32 mL, 2.58 mmol), and the mixture was stirred for 3 hr at 85°C. The reaction mixture was neutralized with 1N hydrochloric acid. The mixture was diluted with water, and extracted with ethyl acetate. The organic layer was washed with water, dried over anhydrous magnesium sulfate, and concentrated under reduced pressure to give the title compound (0.36 g, yield quantitatively) as an uncrystallized solid. $^1$H-NMR ($CDCl_3$) δ: 2.38 (3 H, s), 4.23 (2 H, s), 6.89 (1 H, s), 7.04 (1 H, s), 7.22-7.57 (6 H, m), 7.70 (1 H, d, J = 8.0 Hz), 1 H, unconfirmed.

Reference Example 34

5-methyl-1-[2-[3-(trifluoromethyl)benzyl]-1-benzothiophen-7-yl]-lH-pyrazole-3-carboxylic acid

In the same manner as in Reference Example 33 and using ethyl 5-methyl-1-[2-[3-(trifluoromethyl)benzyl]-1-benzothiophen-7-yl]-lH-pyrazole-3-carboxylate obtained in Reference Example 32, the title compound was obtained. yield 44% solid.
$^1$H-NMR ($CDCl_3$) δ: 2.31 (3 H, s), 4.27 (2 H, s), 6.82 (1 H, s), 7.10 (1 H, s), 7.22-7.62 (6 H, m), 7.78 (1 H, d, J = 7.7 Hz), 1 H, unconfirmed.

Reference Example 35

ethyl 2-bromo-4-methyl-1,3-thiazole-5-carboxylate

A solution of copper(II) bromide (3.6 g, 16.11 mmol) and n-amyl nitrite (1.07 mL, 8.06 mmol) in acetonitrile (22 mL) was heated to 60°C, and a solution of ethyl 2-amino-4-methyl-1,3-thiazole-5-carboxylate (1.0 g, 5.37 mmol) in acetonitrile (30 mL) was added dropwise thereto. The mixture was stirred for 4 hr at same temperature. The reaction mixture was allowed to cool to room temperature, and added into ice-cooled 1N aqueous sodium hydroxide solution. The mixture was extracted with ethyl acetate. The organic layer was dried over anhydrous sodium sulfate, and evaporated under reduced pressure. The residue was purified by silica gel column chromatography (hexane:ethyl acetate= 95: 5→85:15) to give the title compound (1.1 g, 83%).
$^1$H-NMR ($CDCl_3$) δ : 1.37 (3 H, t, J = 7.1 Hz), 2.72 (3 H, s), 4.33 (2 H, q, J = 7.1 Hz).

Reference Example 36

ethyl 2-bromo-4-(trifluoromethyl)-1,3-thiazole-5-carboxylate

In the same manner as in Reference Example 35 and using ethyl 2-amino-4-(trifluoromethyl)-1,3-thiazole-5-carboxylate, the title compound was obtained. yield 84%.
$^1$H-NMR $CDCl_3$) δ : 1.38 (3 H, t, J = 7.1 Hz), 4.40 (2 H, q, J = 7.3 Hz).

Reference Example 37

ethyl 4-methyl-2-[2-[3-(trifluoromethyl)benzyl]-1-benzothiophen-7-yl]-1,3-thiazole-5-carboxylate

A mixture of ethyl 2-bromo-4-methyl-1,3-thiazole-5-carboxylate obtained in Reference Example 35 (125 mg, 0.50 mmol), 4,4,5,5-tetramethyl-2-[2-[3-(trifluoromethyl)benzyl]-1-benzothiophen-7-yl]-1,3,2-dioxaborolane (251 mg, 0.60 mmol), tetrakis(triphenylphosphine)palladium(0) (23 mg, 0.020 mmol) and 2N aqueous sodium carbonate solution (1.0 mL)-1,2-dimethoxyethane (5.0 mL) was heated under reflux for 13 hr under a nitrogen atmosphere. The reaction mixture was diluted with saturated brine and ethyl acetate, and the mixture was extracted with ethyl acetate. The organic layer was dried over anhydrous sodium sulfate, and concentrated under reduced pressure. The residue was purified by silica gel column chromatography (hexane-ethyl acetate 95:5→90:10) to give the title compound (213 mg, 92%).
$^1$H-NMR ($CDCl_3$) δ : 1.41 (3 H, t, J = 7.1 Hz), 2.85 (3 H, s), 4.30 - 4.42 (4 H, m), 7.07 (1 H, t, J = 1.1 Hz), 7.36 - 7.56 (4 H, m), 7.58 (1 H, s), 7.79 (1 H, dd, J = 8.0, 1.1 Hz), 7.84 (1 H, dd, J = 7.6, 1.0 Hz).

Reference Example 38

4-(4,4,5,5-tetramethyl-1,3,2-dioxaborolan-2-yl)-2-[3-(trifluoromethyl)benzyl]-1-benzofuran

To a solution of 4-bromo-2-[3-(trifluoromethyl)benzyl]-1-benzofuran (3.00 g, 8.45 mmol) in DMF (18 mL) were added 4,4,4',4',5,5,5',5'-octamethyl-2,2'-bi-1,3,2-dioxaborolane (3.21 g, 12.7 mmol), potassium acetate (1.66 g, 16.9 mmol) and [1,1'-bis(diphenylphosphino)ferrocene]diohloropalladium(II), dichloromethane adduct (0.35 g, 0.42 mmol), and the mixture was stirred overnight at 85°C under a nitrogen atmosphere. Water was poured into the reaction mixture, and the mixture was extracted with ethyl acetate. The extract was washed with water, dried over anhydrous magnesium sulfate, and evaporated under reduced pressure. The residue was purified by silica gel column chromatography (ethyl acetate:hexane=1:9) to give the title compound (2.8 g, yield 82%) as a solid.
$^1$H-NMR ($CDCl_3$) δ: 1.19-1.43 (12 H, m), 4.18 (2 H, s), 6.94 (1 H, s), 7.17-7.27 (1 H, m), 7.36-7.54 (4 H, m), 7.59 (1 H, s), 7.67 (1 H, d, J = 7.2 Hz).

Reference Example 39

ethyl 4-methyl-2-[2-[3-(trifluoromethyl)benzyl]-1-benzofuran 4-yl]-1,3-thiazole-5-carboxylate

A solution of copper(II) bromide (18.0 g, 80.55 mmol) and n-amyl nitrite (5.36 mL, 40.27 mmol) in acetonitrile (100 mL) was heated to 60°C, and ethyl 2-amino-4-methyl-1,3-thiazole-5-carboxylate (4.0 g, 21.48 mmol) was added dropwise thereto. The mixture was stirred for 5 hr at same temperature. The reaction mixture was allowed to cool to room temperature, added into ice-cooled 1N aqueous sodium hydroxide solution. The mixture was extracted with ethyl acetate. The organic layer was dried over anhydrous sodium sulfate, and evaporated under reduced pressure. The residue was purified by silica gel column chromatography (hexane:ethyl acetate= 95:5→80:20) to give a crude ethyl 2-bromo-4-methyl-1,3-thiazole-5-carboxylate (6.0 g). A mixture of the obtained crude ethyl 2-bromo-4-methyl-1,3-thiazole-5-carboxylate (500 mg), 4-(4,4,5,5-tetramethyl-1,3,2-dioxaborolan-2-yl)-2-[3-(trifluoromethyl)benzyl]-1-benzofuran obtained in Reference Example 38 (965 mg, 2.4 mmol), tetrakis(triphenylphosphine)palladium(0) (92 mg, 0.08 mmol) and 2N aqueous sodium carbonate solution (4.0 mL)-1,2-dimethoxyethane (20 mL) was heated under reflux for 7 hr under a nitrogen atmosphere. The reaction mixture was diluted with saturated brine and ethyl acetate, and the mixture was extracted with ethyl acetate. The organic layer was dried over anhydrous sodium sulfate, and concentrated under reduced pressure. The residue was purified by silica gel column chromatography (hexane-ethyl acetate 95:5→85:5, 97:3→90: 10) to give the title compound (668 mg, yield 84%, 2 steps). $^1$H-NMR ($CDCl_3$) δ : 1.41 (3 H, t, J = 7.1 Hz), 2.82 (3 H, s), 4.24 (2 H, s), 4.37 (2 H, q, J = 7.1 Hz), 7.31 (2 H, dd, J = 4.4, 3.6 Hz), 7.40 - 7.57 (4 H, m), 7.61 (1 H, s), 7.78 (1 H, dd, J = 7.7, 0.8 Hz).

Reference Example 40

ethyl 4-methyl-2-[2-[3-(trifluoromethyl)benzyl]-2H-indazol-4-yl]-1,3-thiazole-5-carboxylate

In the same manner as in Reference Example 39 and crude ethyl 2-bromo-4-methyl-1,3-thiazole-5-carboxylate was synthesized from ethyl 2-amino-4-methyl-1,3-thiazole-5-carboxylate, and Using the obtained crude product and 4-(4,4,5,5-tetramethyl-1,3,2-dioxaborolan-2-yl)-2-[3-(trifluoromethyl)benzyl]-2H-indazole, the title compound was obtained. yield 74% (2 steps).
$^1$H-NMR ($CDCl_3$) $\delta$ : 1.41 (3 H, t, J = 7.1 Hz), 2.82 (3 H, s), 4.37 (2 H, q, J = 7.1 Hz), 5.72 (2 H, s), 7.36 (1 H, dd, J = 8.5, 7.1 Hz), 7.43 - 7.53 (2 H, m), 7.54 - 7.64 (2 H, m), 7.71 (1 H, dd, J = 7.1, 0.5 Hz), 7.86 (1 H, dt, J = 8.5, 0.8 Hz), 8.80 (1 H, d, J = 0.8 Hz).

Reference Example 41

ethyl 4-(trifluoromethyl)-2-[2-[3-(trifluoromethyl)benzyl]-1-benzothiophen-7-yl]-1,3-thiazole-5-carboxylate

In the same manner as in Reference Example 37 and using ethyl 2-bromo-4-(trifluoromethyl)-1,3-thiazole-5-carboxylate obtained in Reference Example 36 and 4,4,5,5-tetramethyl-2-[2-[3-(trifluoromethyl)benzyl]-1-benzothiophen-7-yl]-1,3,2-dioxaborolane, the title compound was obtained. yield 16%. $^1$H-NMR ($CDCl_3$) $\delta$ : 1.43 (3 H, t, J = 7.1 Hz), 4.34 (2 H, s), 4.44 (2 H, q, J = 7.1 Hz), 7.07 (1 H, s), 7.39 - 7.62 (5 H, m), 7.85 (2 H, dd, J = 7.6, 4.5 Hz).

Reference Example 42

ethyl 1,4-dimethyl-2-[2-[3-(trifluoromethyl)benzyl]-1-benzothiophen-7-yl]-1H-imidazole-5-carboxylate

A mixture of 4,4,5,5-tetramethyl-2-[2-[3-(trifluoromethyl)benzyl]-1-benzothiophen-7-yl]-1,3,2-dioxaborolane (520 mg, 1.24 mmol), ethyl 2-bromo-1,4-dimethyl-1H-imidazole-5-carboxylate (369 mg, 1.49 mmol), tetrakis(triphenylphosphine)palladium(0) (172.4 mg, 0.149 mmol) and 2N aqueous sodium carbonate solution (1.9 ml)-1,2-dimethoxyethane (10 ml) was stirred for 5 hr at 95°C under a nitrogen atmosphere. Water was poured into the reaction mixture, and the mixture was extracted with ethyl acetate. The extract was dried over anhydrous magnesium sulfate, and evaporated under reduced pressure. The residue was purified by silica gel column chromatography (ethyl acetate:hexane=1: 4) to give the title compound (418 mg, yield 73%) as an oil. $^1$H-NMR ($CDCl_3$) $\delta$: 1.41 (3 H, t, J= 7.2 Hz), 2.57 (3 H, s), 3.85 (3 H, s), 4.27 (2 H, s), 4.37 (2 H, q, J= 7.2 Hz), 7.08 (1 H, s), 7.32 - 7.56 (6 H, m), 7.72 - 7.79 (1 H, m).

Reference Example 43

1,4-dimethyl-2-[2-[3-(trifluoromethyl)benzyl]-1-benzothiophen-7-yl]-1H-imidazolo-5-carboxylic acid

To a mixture of ethyl 1,4-dimethyl-2-[2-[3-(trifluoromethyl)benzyl]-1-benzothiophen-7-yl]-1H-imidazole-5-carboxylate obtained in Reference Example 42 (410 mg, 0.894 mmol) and THF (6.0 ml)-methanol (3.0 ml) was added 1N aqueous sodium hydroxide solution (1.34 ml) at room temperature, and the mixture was stirred overnight at room temperature. Water was poured into the reaction mixture, and the mixture was adjusted pH2 - 3 with 1N hydrochloric acid. The mixture was extracted with ethyl acetate. The extract was washed with water, dried over anhydrous magnesium sulfate, and evaporated under reduced pressure. To the residue were added diethyl ether and hexane, and the title compound (283 mg, yield 74%) was collected by filtration as a solid.
$^1$H-NMR (DMSO-$d_6$) $\delta$: 2.42 (3 H, s), 3.79 (3 H, s), 4.38 (2 H, s), 7.33 (1 H, s), 7.44 - 7.67 (5 H, m), 7.71 (1 H, s), 7.86 - 7.92 (1 H, m), 12.86 (1H, br s).

Reference Example 44

ethyl 2-bromo-1,5-dimethyl-1H-imidazole-4-carboxylate

To a solution of ethyl 1,5-dimethyl-1H-imidazole-4-carboxylate (410 mg, 2.43 mmol) in acetonitrile (14 ml) was added N-bromosuccinimide (521 mg, 2.92 mmol) at room temperature, and the mixture was stirred for 8 hr at room temperature, and evaporated under reduced pressure. The residue was dissolved in ethyl acetate. The solution was washed with water, dried over anhydrous magnesium sulfate, and evaporated under reduced pressure. The residue was purified by silica gel column chromatography (ethyl acetate:hexane=1:1) to give the title compound (598 mg, yield 99%) as a solid. $^1$H-NMR ($CDCl_3$) $\delta$: 1.38 (3 H, t, J=7.2 Hz), 2.57 (3 H, s), 3.54 (3 H, s), 4.36 (2 H, q, J=7.2 Hz).

Reference Example 45

ethyl 1,5-dimethyl-2-[2-[3-(trifluoromethyl)benzyl]-1-benzothiophen-7-yl]-1H-imidazole-4-carboxylate

In the same manner as in Reference Example 42 and using 4,4,5,5-tetramethyl-2-[2-[3-(trifluoromethyl)benzyl]-1-benzothiophen-7-yl]-1,3,2-dioxaborolane and ethyl 2-bromo-1,5-dimethyl-1H-imidazole-4-carboxylate obtained in Reference Example 44, the title compound was obtained as an oil. yield 64%
$^1$H-NMR ($CDCl_3$) δ: 1.39 (3 H, t, J=7.2 Hz), 2.63 (3 H, s), 3.55 (3 H, s), 4.27 (2 H, s), 4.39 (2 H, q, J=7.2 Hz), 7.07 (1H, s), 7.32 - 7.56 (6 H, m), 7.73 (1 H, dd, J=7.6, 1.5 Hz).

Reference Example 46

1,5-dimethyl-2-[2-[3-(trifluoromethyl)benzyl]-1-benzothiophen-7-yl]-1H-imidazole-4-carboxylic acid

In the same manner as in Reference Example 43 and using ethyl 1,5-dimethyl-2-[2-[3-(trifluoromethyl)benzyl]-1-benzothiophen-7-yl]-1H-imidazole-4-carboxylate obtained in Reference Example 45, the title compound was obtained as a solid. yield 79%
$^1$H-NMR (DMSO-$d_6$) δ: 2.55 (3H, s), 3.58 (3 H, s), 4.38 (2 H, s), 7.33 (1 H, s), 7.38 - 7.74 (6 H, m), 7.87 (1 H, dd, J=7.0, 1.7 Hz), 12. 14 (1 H, br s).

Reference Example 47

ethyl 3,5-dimethyl-1-[2-[3-(trifluoromethyl)benzyl]-1-benzothiophen-7-yl]-1H-pyrazale-4-carboxylate

A mixture of 4,4,5,5-tetramethyl-2-[2-[3-(trifluoromethyl)benzyl]-1-benzothiophen-7-yl]-1,3,2-dioxaborolane (1.25 g, 3.0 mmol), ethyl 3,5-dimethyl-1H-pyrazole-4-carboxylate (605 mg, 3.6 mmol) and copper(II) acetate hydrate (300 mg, 1.5 mmol) in pyridine (15 mL) was stirred for 3 days at 50°C, and the reaction mixture was concentrated under reduced pressure. The residue was purified by silica gel column chromatography (hexane-ethyl acetate=7:3) to give the title compound (940 mg, yield 68%) as an oil. $^1$H-NMR ($CDCl_3$) 5: 1.39 (3 H, t, J = 7.1 Hz), 2.46 (3 H, s), 2.52 (3 H, s), 4.26 (2 H, s), 4.34 (2 H, q, J = 7.1 Hz), 7.08 (1 H, t, J = 1.1 Hz), 7.20 (1 H, dd, J = 7.6, 1.0 Hz), 7.37 - 7.55 (5 H, m), 7.73 (1H, dd, J = 8.0, 1.1 Hz).

Reference Example 48

3,5-dimethyl-1-[2-[3-(trifluoromethyl)benzyl]-1-benzothiophen-7-yl]-1H-pyrazole-4-carboxylic acid

A mixture of ethyl 3,5-dimethyl-1-[2-[3-(trifluoromethyl)benzyl]-1-benzothiophen-7-yl]-1H-pyrazole-4-carboxylate obtained in Reference Example 47 (1.14 g, 2.5 mmol) and 1N aqueous sodium hydroxide solution (7.5 mL, 7.5 mmol) in THF (20 mL)-ethanol (20 mL) was stirred overnight at 80°C. To the reaction mixture was added 1N hydrochloric acid, and the resulting solid was collected by filtration to give the title compound (920 mg, yield 86 %) as a pale-yellow solid. $^1$H-NMR ($CDCl_3$) 5: 2.50 (3 H, s), 2.56 (3 H, s), 4.27 (2 H, s), 7.07 - 7.14 (1 H, m), 7.23 (1 H, dd, J = 7.6, 1.1 Hz), 7.40 - 7.57 (5 H, m), 7.76 (1 H, d, J = 8.0 Hz).

Reference Example 49

1-[2-(3-chloro-5-fluorobenzyl)-1-benzothiophen-7-yl]-3,5-dimethyl-1H-pyrazole-4-carboxylic acid

A mixture of 2-[2-(3-chloro-5-fluorobenzyl)-1-benzothiophen-7-yl]-4,4,5,5-tetramethyl-1,3,2-dioxaborolane (1.50 g, 3.72 mmol), ethyl 3,5-dimethyl-1H-pyrazole-4-carboxylate (0.76 g, 4.5 mmol) and copper(II) acetate monohydrate (0.37 g, 1.9 mmol) in pyridine (8 mL) was reacted for 20 hr at 50°C. The reaction solution was concentrated, and the residue was diluted with ethyl acetate. The organic layer was washed with 1N hydrochloric acid, dried over magnesium sulfate, and concentrated under reduced pressure. The residue was purified by silica gel column chromatography (ethyl acetate-hexane 1:10) to give ethyl 1-[2-(3-chloro-5-fluorobenzyl)-1-benzothiophen-7-yl]-3,5-dimethyl-1H-pyrazole-4-carboxylate (0.88 g). To a solution of the compound in methanol (20 mL) was added 1N aqueous sodium hydroxide solution (12 mL, 12 mmol) at room temperature, and the mixture was stirred for 1 days at 60°C. To the reaction mixture were added water and hydrochloric acid, and the aqueous layer was acidified. The mixture was extracted with ethyl acetate. The organic layer was dried over magnesium sulfate, and evaporated under reduced pressure. The obtained residue was crystallized from ether to give the title compound (630 mg, yield 41%). melting point 187 - 188°C.

$^1$H-NMR (DMSO-$d_6$)δ: 2.38 (6 H, s), 4.29 (2 H, s), 7.18 - 7.25 (1 H, m), 7.30 (1 H, s), 7.36 - 7.44 (2 H, m), 7.46 - 7.54 (1 H, m), 7.72 - 7.81 (1 H, m), 7.88 - 7.92 (1 H, m), 12.43 (1 H, br s).

Reference Example 50

[4-fluoro-7-(4,4,5,5-tetramethyl-1,3,2-dioxaborolan-2-yl)-1-benzothiophen-2-yl]methanol

To a solution of (7-bromo-9-fluoro-1-benzothiophen-2-yl)methanol (5.00 g, 19.2 mmol) in DMF (30 mL) were added 4,4,4',4',5,5,5',5'-octamethyl-2,2'-bi-1,3,2-dioxaborolane (7.30 g, 28.7 mol), potassium acetate (3.75 g, 38.3 mmol) and [1,1'-bis(diphenylphosphino)ferrocene]dichloropalladium(II), dichloromethane adduct (0.78 g, 0.96 mmol), and the mixture was stirred overnight at 85°C under a nitrogen atmosphere. Water was poured into the reaction mixture, and the mixture was extracted with ethyl acetate. The extract was washed with water, dried over anhydrous magnesium sulfate, and evaporated under reduced pressure. The residue was purified by silica gel column chromatography (ethyl acetate:hexane=1:9) to give the title compound (5.92 g, yield quantitatively) as a solid. $^1$H-NMR ($CDCl_3$) δ: 1.40 (12 H, s), 2.10 (1 H, s), 4.95 (2 H, s), 7.01 (1 H, dd, J = 10.2, 8.0 Hz), 7.33 (1 H, s), 7.76 (1 H, dd, J = 7.8, 5.6 Hz).

Reference Example 51

ethyl 1-[4-fluoro-2-(hydroxymethyl)-1-benzothiophen-7-yl]-3,5-dimethyl-1H-pyrazole-4-carboxylate

To a solution of [4-fluoro-7-(4,4,5,5-tetrethyl-1,3,2-dioxaborolan-2-yl)-1-benzothiophen-2-yl]methanol obtained in Reference Example 50 (3.00 g, 9.73 mmol) in pyridine (15 mL) were added ethyl 3,5-dimethyl-1H-pyrazole-4-carboxylate (1.96 g, 1.20 mmol) and copper(II) acetate monohydrate (0.39 g, 1.95 mmol), and the mixture was stirred overnight at 55°C. The reaction mixture was evaporated under reduced pressure. The residue was purified by silica gel column chromatography (ethyl acetate:hexane=1:4) to give the title compound (1.75 g, yield 52%) as a solid.
$^1$H-NMR ($CDCl_3$) δ: 1.40 (3 H, t, J = 7.1 Hz), 1.96 (1 H, br s), 2.43 (3 H, s), 2.53 (3 H, s), 4.34 (2 H, q, J = 7.1 Hz), 4.87 (2 H, s), 7.03-7.40 (3 H, m).

Reference Example 52

ethyl 1-[4-fluoro-2-[3-(trifluoromethyl)benzyl]-1-benzothiophen-7-yl]-3,5-dimethyl-1H-pyrazole-4-carboxylate

To a solution of ethyl 1-[4-fluoro-2-(hydroxymethyl)-1-benzothiophen-7-yl]-3,5-dimethyl-1H-pyrazole-4-carboxylate obtained in Reference Example 51 (1.65 g, 4.74 mol) in THF (20 mL) were added methanesulfonyl chloride (0.81 g, 7.10 mmol), triethylamine (0.96 g, 9.47 mmol) and 4-dimethylaminopyridine (6 mg, 0.05 mmol), and the mixture was stirred for 1 hr at room temperature under a nitrogen atmosphere. Saturated aqueous ammonium chloride solution was poured into the reaction mixture, and the mixture was extracted with ethyl acetate. The extract was washed with water and saturated brine, dried over anhydrous sodium sulfate, and evaporated under reduced pressure. To a solution of the obtained residue in THF (5 mL)-water (1 mL) were added m-(trifluoromethyl)phenylboronic acid (0.54 g, 2.84 mmol), cesium carbonate (1.93 g, 5.93 mmol) and [1,1'-bis(diphenylphosphino)ferrocene]dichloropalladium(II), dichloromethane adduct (77 mg, 0.09 mmol), and the mixture was stirred overnight at 60°C. Water was poured into the reaction mixture, and the mixture was extracted with ethyl acetate. The extract was washed with water, dried over anhydrous magnesium sulfate, and evaporated under reduced pressure. The residue was purified by silica gel column chromatography (ethyl acetate:hexane=1:9) to give the title compound (0.93 g, yield 74%) as a solid.
$^1$H-NMR ($CDCl_3$) δ: 1.35-1.41 (3 H, m), 2.43 (3 H, s), 2.51 (3 H, s), 4.26 (2 H, s), 4.33 (2 H, q, J = 7.2 Hz), 7.03-7.22 (3 H, m), 7.41-7.56 (4 H, m).

Reference Example 53

1-[4-fluoro-2-[3-(trifluoromethyl)benzyl]-1-benzothiophen-7-yl]-3,5-dimethyl-1H-pyrazole-4-carboxylic acid

To a solution of ethyl 1-[4-fluoro-2-[3-(trifluoromethyl)benzyl]-1-benzothiophen-7-yl]-3,5-dimethyl-1H-pyrazole-4-carboxylate obtained in Reference Example 52 (3.73 g, 7.83 mmol) in THF (40 mL)-ethanol (40 mL) was added 8N aqueous sodium hydroxide solution (2.94 mL, 0.02 mol), and the mixture was stirred for 3 hr at 85°C. The reaction mixture was neutralized with 6N hydrochloric acid, and the mixture was diluted with water. The mixture was extracted with ethyl acetate. The organic layer was washed with water, dried over anhydrous magnesium sulfate, and concentrated under reduced pressure. To the precipitated crystals was added isopropyl, alcohol, and the crystals were collected by filtration to give the title compound (2.94 g, yield 84%) as crystals. $^1$H-NMR ($CDCl_3$) δ: 2.47 (3 H, s), 2.54 (3 H, s), 4.27

(2 H, s), 7.05-7.31 (3 H, m), 7.41-7.60 (4H, m), 1 H, unconfirmed.

Reference Example 54 ethyl 1-[2-(3-chloro-5-fluorobenzyl)-4-fluoro-1-benzothiophen-7-yl]-3,5-dimethyl-1H-pyrazole-4-carboxylate

In the same manner as in Reference Example 52 and using (3-chloro-5-fluorophenyl)boronic acid, the title compound was obtained. yield 58% solid.
$^1$H-NMR ($CDCl_3$) $\delta$: 1.39 (3 H, t, J = 7.0 Hz), 2.44 (3 H, s), 2.51 (3 H, s), 4.16 (2 H, s), 4.34 (2 H, q, J = 7.2 Hz), 6.82-7.24 (6 H, m).

Reference Example 55

1-[2-(3-chloro-5-fluorobenzyl)-4-fluoro-1-benzothiophen-7-yl]-3,5-dimethyl-1H-pyrazole-4-carboxylic acid

In the same manner as in Reference Example 53 and using ethyl 1-[2-(3-chloro-5-fluorobenzyl)-4-fluoro-1-benzothiophen-7-yl]-3,5-dimethyl-1H-pyrazole-4-carboxylate obtained in Reference Example 54, the title compound was obtained. yield 84% solid.
$^1$H-NMR ($CDCl_3$) $\delta$: 2.48 (3 H, s), 2.55 (3 H, s), 4.17 (2 H, s), 6.82-7.31 (6 H, m), 1 H, unconfirmed.

Reference Example 56

ethyl 5-(hydroxymethyl)-3-methyl-1-[2-[3-(trifluoromethyl)benzyl]-1-benzothiophen-7-yl]-1H-pyrazole-4-carboxylate

To a solution of 4,4,5,5-tetramethyl-2-[2-[3-(trifluoromethyl)benzyl]-1-benzothiophen-7-yl]-1,3,2-dioxaborolane (1.03 g, 2.45 mmol) in pyridine (5 mL) were added ethyl 5-(hydroxymethyl)-3-methyl-1H-pyrazole-4-carboxylate (0.5 g, 2.71 mmol) and copper(II) acetate monohydrate (0.23 g, 1.13 mmol), and the mixture was stirred overnight at 55°C. The reaction mixture was evaporated under reduced pressure. The residue was purified by silica gel column chromatography (ethyl acetate:hexane=1:4) to give the title compound (160 mg, yield 15%) as a solid. $^1$H-NMR ($CDCl_3$) $\delta$: 1.43 (3 H, t, J = 7.1 Hz), 2.53 (3 H, s), 4.19-4.31 (2 H, m), 4.39 (2 H, q, J = 7.1 Hz), 4.64 (2 H, s), 7.08 (1 H, s), 7.23-7.34 (1 H, m), 7.36-7.57 (5 H, m), 7.74 (1 H, d, J = 8.0 Hz), 10.48 (1 H, br s).

Reference Example 57

5-(hydroxymethyl)-3-methyl-1-[2-[3-(trifluoromethyl)benzyl]-1-benzothiophen-7-yl]-1H-pyrazole-4-carboxylic acid

To a solution of ethyl 5-(hydroxymethyl)-3-methyl-1-[2-[3-(trifluoromethyl)benzyl]-1-benzothiophen-7-yl]-1H-pyrazole-4-carboxylate obtained in Reference Example 56 (0.16 g, 0.34 mmol) in THF (3 mL)-ethanol (3 mL) was added 1N aqueous sodium hydroxide solution (1.01 mL, 1.01 mmol), and the mixture was stirred for 6 hr at 85°C. The reaction mixture was neutralized with 1N hydrochloric acid, and the mixture was diluted with water, and extracted with ethyl acetate. The organic layer was washed with water, dried over anhydrous magnesium sulfate, and concentrated under reduced pressure. To the precipitated crystals was added isopropyl alcohol, and the crystals were collected by filtration to give the title compound (0.11 g, yield 76%) as crystals.
$^1$H-NMR (DMSO-$d_6$) 5: 3.31 (3 H, br s), 4.36 (2 H, s), 4.60 (2 H, br s), 5.28 (1 H, br s), 7.33 (1 H, s), 7.41-7.66 (5 H, m), 7.67-7.74 (1 H, m), 7.82-7.90 (1 H, m), 12.43 (1 H, br s).

Reference Example 58

ethyl 5-(tert-butoxymethyl)-3-methyl-1-[2-[3-(trifluoromethyl)benzyl]-1-benzothiophen-7-yl]-1H-pyrazole-4-carboxylate

Reference Example 59

ethyl 3-(tert-butoxethyl)-5-methyl-1-[2-[3-(trifluoromethyl)benzyl]-1-benzothiophen-7-yl]-1H-pyrazole-4-carboxylate

To a solution of 4,4,5,5-tetramethyl-2-[2-[3-(trifluoromethyl)benzyl]-1-benzothiophen-7-yl]-1,3,2-dioxaborolane (3.12 g, 7.45 mmol) in pyridine (15 mL) were added ethyl 5-(tert-butoxymethyl)-3-methyl-1H-pyrazole-4-carboxylate (1.79 g, 7.45 mmol) and copper(II) acetate monohydrate (0.30 g, 1.49 mmol), and the mixture was stirred overnight at 55°C. The reaction mixture was evaporated under reduced pressure. The residue was purified by silica gel column chromatography (ethyl acetate:hexane=1:4) to give ethyl 5-(tert-butoxymethyl)-3-methyl-1-[2-[3-(trifluoromethyl)benzyl]-1-benzothiophen-7-yl]-1H-pyrazole-4-carboxylate (1.11 g, yield 28%) as a solid and ethyl 3-(tert-butoxymethyl)-5-methyl-1-[2-[3-(trifluoromethyl)benzyl]-1-benzothiophen-7-yl]-1H-pyrazole-4-carboxylate (1.02 g, yield 26%) as an oil.
ethyl 5-(tert-butoxymethyl)-3-methyl-1-[2-[3-(trifluoromethyl)benzyl]-1-benzothiophen-7-yl]-1H-pyrazole-4-carboxylate (Reference Example 58)
$^1$H-NMR ($CDCl_3$) $\delta$: 1.08 (9 H, s), 1.35-1.43 (3 H, m), 2.53 (3 H, s), 4.25 (2 H, s), 4.35 (2 H, q, J = 7.1 Hz), 4.56 (2 H, s), 7.08 (1 H, s), 7.34-7.63 (6 H, m), 7.73 (1 H, d, J = 8.0 Hz). ethyl 3-(tert-butoxymethyl)-5-methyl-1-[2-[3-(trifluoromethyl) benzyl]-1-benzothiophen-7-yl]-1H-pyrazole-4-carboxylate (Reference Example 59)
$^1$H-NMR ($CDCl_3$) 5: 1.31 (9 H, s), 1.39 (3 H, t, J = 7.1 Hz), 2.45 (3 H, s), 4.25 (2 H, s), 4.35 (2 H, q, J = 7.1 Hz), 4.72 (2 H, s), 7.08 (1 H, s), 7.21 (1 H, d, J = 7.7 Hz), 7.36-7.47 (3 H, m), 7.48-7.55 (2 H, m), 7.73 (1 H, d, J = 8.0 Hz).

Reference Example 60

5-(tert-hutoxymethyl)-3-methyl-1-[2-[3-(trifluoromethyl)benzyl]-1-benzothiophen-7-yl]-1H-pyrazole-4-carboxylic acid

To a solution of ethyl 5-(tert-butoxymethyl)-3-methyl-1-[2-[3-(trifluoromethyl)benzyl]-1-benzothiophen-7-yl]-1H-pyrazole-4-carboxylate obtained in Reference Example 58 (1.11 g, 2.09 mmol) in THF (10 mL)-ethanol (10 mL) was added 8N aqueous sodium hydroxide solution (0.79 mL, 6.31 mmol), and the mixture was stirred for 6 hr at 85°C. The reaction mixture was neutralized with 1N hydrochloric acid. The mixture was diluted with water, and extracted with ethyl acetate. The organic layer was washed with water, dried over anhydrous magnesium sulfate, and concentrated under reduced pressure. To the precipitated crystals was added isopropyl alcohol, and the crystals were collected by filtration to give the title compound (1.05 g, yield quantitatively) as crystals. $^1$H-NMR ($CDCl_3$) 5: 1.13 (9 H, s), 2.57 (3 H, s), 4.26 (2 H, s), 4.56 (2 H, s), 7.11 (1 H, s), 7.37-7.56 (6 H, m), 7.76 (1 H, d, J = 7.6 Hz), 1 H, unconfirmed.

Reference Example 61

3-(tert-butoxymethyl)-5-methyl-1-[2-[3-(trifluoromethyl)benzyl]-1-benzothiophen-7-yl]-1H-pyrazole-4-carboxylic acid

To a solution of ethyl 3-(tert-butoxymethyl)-5-methyl-1-[2-[3-(trifluoromethyl)benzyl]-1-benzothiophen-7-yl]-1H-pyrazole-4-carboxylate obtained in Reference Example 59 (1.02 g, 1.92 mmol) in THF (10 mL)-ethanol (10 my) was added 8N aqueous sodium hydroxide solution (0.72 mL, 5.77 mmol), and the mixture was stirred for 6 hr at 85°C. The reaction mixture was neutralized with 1N hydrochloric acid. The mixture was diluted with water, and extracted with ethyl acetate. The organic layer was washed with water, dried over anhydrous magnesium sulfate, and concentrated under reduced pressure. To the precipitated was added isopropyl alcohol, and the crystals were collected by filtration to give the title compound (1.00 g, yield quantitatively) as crystals.
$^1$H-NMR ($CDCl_3$) 5: 1.38 (9 H, s), 2.52 (3 H, s), 4.26 (2 H, s), 4.79 (2 H, s), 7.11 (1 H, s), 7.19-7.27 (1 H, m), 7.39-7.56 (5 H, m), 7.77 (1 H, d, J = 6.8 Hz), 12.06 (1 H, br s).

Reference Example 62

ethyl 5-(tert-butoxymethyl)-1-[4-fluoro-2-[3-(trifluoromethyl)benzyl]-1-benzothiophen-7-yl]-3-methyl-1H-pyrazole-4-carboxylate

To a solution of [4-fluoro-7-(4,4,5,5-tetramethyl-1,3,2-dioxaborolan-2-yl)-1-benzothiophen-2-yl]methanol obtained in Reference Example 50 (3.20 g, 0.01 mol) in pyridine (12 mL) were added ethyl 5-(tert-butoxymethyl)-3-methyl-1H-pyrazole-4-carboxylate (2.50 g, 0.01 mol) and copper(II) acetate monohydrate (0.40 g, 2.00 mmol), and the mixture was stirred overnight at 55°C. The reaction mixture was evaporated under reduced pressure. The residue was simple-purified by silica gel column chromatography (ethyl acetate:hexane=1:4). To a solution of the obtained mixture in THF (20 mL) were added methanesulfonyl chloride (0.81 g, 7.10 mmol), triethylamine (0.96 g, 9.47 mmol) and 4-dimethylaminopyridine (12 mg, 0.10 mmol), and the mixture was stirred for 1 hr at room temperature under a nitrogen atmosphere. Into the reaction mixture was poured saturated aqueous ammonium chloride solution, and the mixture was extracted with ethyl acetate. The extract was washed with water and saturated brine, dried over anhydrous sodium sulfate, and evaporated under reduced pressure. To a solution of the obtained residue in THF (40 mL)-water (8 mL) were added m-(trifluoromethyl)phenylboronic acid (1.07 g, 5.62 mmol), cesium carbonate (3.81 g, 11.7 mmol) and [1,1'-bis(diphEnylphosphino)ferrocene]dichloropalladium(II), dichloromethane adduct (153 mg, 0.19 mmol), and the mixture was stirred overnight at 60°C. Water was poured into the reaction mixture, and the mixture was extracted with ethyl acetate. The extract was washed with water, dried over anhydrous magnesium sulfate, and evaporated under reduced pressure. The residue was purified by silica gel column chromatography (ethyl acetate:hexane=1:9) to give the title compound (0.98 g, yield 38%) as a solid.

$^1$H-NMR ($CDCl_3$) 5: 1.08 (9 H, s), 1.34-1.43 (3 H, m), 2.52 (3 H, s), 4.25 (2 H, s), 4.34 (2 H, q, J = 7.2 Hz), 4.54 (2 H, s), 702-7.11 (1 H, m), 7 .15--7. 34 (2 H, m) , z 65 (4 H, m)

Reference Example 63

1-[4-fluoro-2-[3-(trifluoromethy3)benzyl]-1--benzothzpphen-7-yl]-5-(hydroxymethyl)-3-methyl-1H-pyrazole-4-carboxylic acid

To ethyl 5-(tert-butoxymethyl)-1-[4-fluoro-2-[3-(trifluoromethyl)benzyl]-1-benzothiophen-7-yl]-3-methyl-1H-pyrazole-4-carboxylate obtained in Reference Example 62 (1.00 g, 1.82 mmol) was added 4N hydrochloric acid-ethyl acetate solution (15 mL), and the mixture was stirred overnight at room temperature. The reaction mixture was concentrated under reduced pressure. To a solution of the obtained residue in THF (20 mL)-ethanol (20 mL) was added 8N aqueous sodium hydroxide solution (0.68 mL, 5.46 mmol), and the mixture was stirred for 3 hr at 85°C. The reaction mixture was neutralized with 6N hydrochloric acid, and diluted with water. The mixture was extracted with ethyl acetate. The organic layer was washed with water, dried over anhydrous magnesium sulfate, and concentrated under reduced pressure. To the precipitated crystals was added isopropyl alcohol, and the crystals were collected by filtration to give the title compound (0.38 g, yield 48%) as crystals.
$^1$H-NMR ($CDCl_3$) 5: 2.57 (3 H, s), 4.27 (2 H, s), 4.66 (2 H, s), 7.12 (1 H, t, J = 8.7 Hz), 7.21 (1 H, s), 7.32 (1 H, dd, J = 8.5, 4.0 Hz), 7.43-7.50 (2 H, m), 7.53 (2 H, s), 2 H, unconfirmed.

Reference Example 64

ethyl 3,5-dimethyl-1-[2-[3-(trifluoromethyl)benzyl]-1-benzofuran-4-yl]-1H-pyrazole-4-carboxylate

To a solution of 4-(4,4,5,5-tetramethyl-1,3,2-dioxaborolan-2-yl)-2-[3-(trifluoromethyl)ben.zyl]-1-benzofuran obtained in Reference Example 38 (1.80 g, 4.48 mmol) in pyridine (8 mL) were added ethyl 3,5-dimethyl-1H-pyrazole-4-carboxylate (0.98 g, 5.82 mmol) and copper(II) acetate monohydrate (0.45 g, 2.24 mmol), and the mixture was stirred overnight at 55°C. The reaction mixture was evaporated under reduced pressure. The residue was purified by silica gel column chromatography (ethyl acetate:hexane=1:4) to give the title compound (1.69 g, yield 85%) as a solid. $^1$H-NMR ($CDCl_3$) $\delta$: 1.39 (3 H, t, J = 7.1 Hz), 2.46 (3 H, s), 2.51 (3 H, s), 4.16 (2 H, s), 4.28-4.40 (2 H, m), 6.35 (1 H, s), 7.16 (1 H, d, J = 7.7 Hz), 7.31 (1 H, t, J = 8.0 Hz), 7.39-7.60 (5 H, m).

Reference Example 65

3,5-dimethyl-1-[2-[3-(trifluoromethyl-)benzyl]-1-benzofuran-4-yl]-1H-pyrazole-4-carboxylic acid

To a solution of ethyl 3,5-dimethyl-1-[2-[3-(trifluoromethyl)benzyl]-1-benzofuran-4-yl]-1H-pyrazole-4-carboxylate obtained in Reference Example 64 (1.69 g, 3.82 mmol) in THF (10 mL)-ethanol (10 mL) was added BN aqueous sodium hydroxide solution (1.43 mL, 11.5 mmol), and the mixture was stirred for 3 hr at 85°C. The reaction mixture was neutralized with 6N hydrochloric acid, and diluted with water. The mixture was extracted with ethyl acetate. The organic layer was washed with water, dried over anhydrous magnesium sulfate, and concentrated under reduced pressure. To the precipitated crystals was added isopropyl alcohol, and the crystals were collected by filtration to give the title compound (1.45 g, yield 92%) as crystals.
$^1$H-NMR ($CDCl_3$) $\delta$: 2.50 (3 H, s), 2.55 (3 H, s), 4.17 (2 H, s), 6.37 (1 H, s), 7.13-7.38 (2 H, m), 7.41-7.63 (5 H, m), 1 H, unconfirmed.

Reference Example 66

4-bromo-2-[3-(trifluoromethyl)benzyl]-2H-indazole

A solution of 4-bromo-1H-indazole (3.24 g, 16.4 mol) and 1-(bromomethyl)-3-(trifluoromethyl)benzene (3.8 mL, 24.7 mmol) in DMF (6.4 mL) was stirred for 20 hr at 50°C. The reaction mixture was allowed to cool to room temperature, and water was added thereto. The mixture was extracted with ethyl acetate. The organic layer was washed with water and saturated brine, dried over anhydrous sodium sulfate, and concentrated under reduced pressure. The residue was purified by silica gel column chromatography (hexane-ethyl acetate 90:10$\rightarrow$80:20) to give the title compound (4.67 g, 80%).
$^1$H-NMR ($CDCl_3$) $\delta$ : 5.65 (2 H, s), 7.12 - 7.20 (1 H, m), 7.23 - 7.29 (1 H, m), 7.41 - 7.55 (2 H, m), 7.57 - 7.64 (2 H, m), 7.66 (1 H, dd, J = 8.5, 0.5 Hz), 7.97 (1 H, s).

Reference Example 67

4-bromo-2-(3-chloro-5-fluorobenzyly-2H-indazole

In the same manner as in Reference Example 66 and using 4-bromo-1H-indazole and 1-(bromomethyl)-3-chloro-5-fluorobenzene, the title compound was obtained. yield 75%. $^1$H-NMR ($CDCl_3$) 6 : 5.55 (2 H, s), 6.87 (1 H, d, J = 8.5 Hz), 7.01 - 7.10 (2 H, m), 7.12 - 7.21 (1 H, m), 7.25 - 7.31 (1 H, m), 7.66 (1 H, d, J = 8.5 Hz), 7.98 (1 H, s).

Reference Example 68

4-bromo-2-[3-fluoro-5-(trifluoromethyl)benzyl]-2H-indazole

In the same manner as in Reference Example 66 and using 4-bromo-1H-indazole and 1-(bromomethyl)-3-fluoro-5-(trifluoromethyl)benzene, the title compound was obtained. yield 72%.
$^1$H-NMR ($CDCl_3$) $\delta$ : 5.64 (2 H, s), 7.08 - 7.22 (2 H, m), 7.26 - 7.35 (2 H, m), 7.38 (1 H, s), 7.66 (1 H, d, J = 8.5 Hz), 8.01 (1 H, s).

Reference Example 69

2-(3-chloro-5-fluorobenzyl)-4-(4,4,5,5-tetramethyl-1,3,2-dioxaborolan-2-yl)-2H-indazole

A solution of 4-bromo-2-(3-chloro-5-fluorobenzyl)-2H-indazole obtained in Reference Example 67 (1.93 g, 5.68 mmol), 4,4,4',4',5,5,5',5'-octamethyl-2,2'-bi-1,3,2-dioxaborolane (2.16 g, 8.53 mmol), [1,1'-bis(diphenylphosphino)ferrocene]palladium(II) dichloride dichloromethane complex (1:1) (232 mg, 0.284 mol) and potassium acetate (1.12 g, 11.37 mmol) in DMF (50 mL) was stirred for 24 hr at 85°C, and further stirred for 16 hr at room temperature. The reaction mixture was diluted with saturated brine and ethyl acetate, and the mixture was filtered through celite. The organic layer of the filtrate was washed with water, dried over anhydrous sodium sulfate, and concentrated under reduced pressure. The residue was purified by silica gel column chromatography (hexane-ethyl acetate 90:10→67:33, 95:5→80:20) to give the title compound (507 mg, yield 23%).
$^1$H-NMR ($CDCl_3$) $\delta$ : 1.39 (12 H, s), 5.58 (2 H, s), 6.84 (1 H, d, J = 8.8 Hz), 6.97 - 7.09 (2 H, m), 7.32 (1 H, dd, J = 8.5, 6.6 Hz), 7.66 (1 H, d, J = 6.6 Hz), 7.83 (1 H, d, J = 8.5 Hz), 8.32 (1 H, s).

Reference Example 70

2-[3-fluoro-5-(trifluoromethyl)benzyl]-4-(4,4,5,5-tetramethyl-1,3,2-dioxaborolan-2-y1)-2H-indazole

In the same manner as in Reference Example 69 and using 4-bromo-2-[3-fluoro-5-(trifluoromethyl)bonzyl]-2H-indazole obtained in Reference Example 68 and 4,4,4',4',5,5,5',5'-octamethyl-2,2'-bi-1,3,2-dioxaborolane, the title compound was obtained. yield 34%.
$^1$H-NMR ($CDCl_3$) $\delta$ : 1.39 (12 H, s), 5.66 (2 H, s), 7.09 (1 H, d, J = 9.3 Hz), 7.23 - 7.30 (1 H, m), 7.33 (1 H, dd, J = 8.8, 6.6 Hz), 7.38 (1H, s), 7.66 (1 H, d, J = 6.6 Hz), 7.83 (1 H, d, J = 8.5 Hz), 8.34 (1 H, s).

Reference Example 71

ethyl 3,5-dimethyl-1-[2-[3-(trifluoromethyl)benzyl]-2H-indazol-4-yl]-1H-pyrazole-4-carboxylate

A solution of 4-bromo-2-[3-(trifluoromethyl)benzyl]-2H-indazole obtained in Reference Example 66 (4.67 g, 13.15 mmol), 4,4,4',4,5,5,5',5'-octamethyl-2,2'-bi-1,3,2-dioxaborolane (5.01 g, 19.72 mmol), [1,1'-bis(diphenylphosphino)ferrocene]palladium(II) dichloride dichloromethane complex (1:1) (537 mg, 0.678 mmol) and potassium acetate (2.58 g, 26.30 mmol) in DMF (100 mL) was stirred for 14 hr at 85°C. The reaction mixture was diluted with saturated brine and ethyl acetate, and the mixture was filtrated through celite. The organic layer of the filtrate was washed with water, dried over anhydrous sodium sulfate, and concentrated under reduced pressure. The residue was purified by silica gel column chromatography (hexane-ethyl acetate 90:1.0→50:50) to give crude 4-(4,4,5,5-tetramethyl-1,3,2-dioxaborolan-2-y1)-2-[3-(trifluoromethyl)benzyl]-2H-indazole (4.04 g).
$^1$H-NMR ($CDCl_3$) $\delta$ : 1.38 (12 H, s), 5.67 (2 H, s), 7.32 (1 H, dd, J = 8.7, 6.5 Hz), 7.37 - 7.51 (2 H, m), 7.54 - 7.61 (2 H, m), 7.65 (1 H, d, J = 6.6, 0.8 Hz), 7.83 (1 H, dt, J = 8.7, 1.0 Hz), 8.33 (1 H, d, J = 0.8 Hz).
A solution of the obtained crude 4-(4,4,5,5-tetramethyl-1,3,2-dioxaborolan-2-yl)-2-[3-(trifluoromethyl)benzyl]-2H-indazole (600 mg), ethyl 3,5-dimethyl-1H-pyrazole-4-carboxylate (300 mg, 1.78 mmol) and copper(II) acetate monohydrate (148 mg, 0.74 mmol) in pyridine (7.0 mL) was stirred for 9 hr at 80°C. The reaction mixture was allowed to cool to room

temperature, and diluted with saturated brine and ethyl acetate. The mixture was extracted with ethyl acetate. The organic layer was dried over anhydrous sodium sulfate, and evaporated under reduced pressure. The residue was purified by silica gel column chromatography (hexane:ethyl acetate= 80:20→ 60:40) to give the title compound (460 mg, yield 53%, 2 steps). $^1$H-NMR ($CDCl_3$) δ : 1.39 (3 H, t, J = 7.1 Hz), 2.53 (3 H, s), 2.55 (3 H, s), 4.34 (2 H, q, J = 7.1 Hz), 5.63 (2 H, s), 7.01 (1 H, dd, J = 7.1, 0.8 Hz), 7.36 (1 H, dd, J = 8.8, 7.1 Hz), 7.43 - 7.49 (2 H, m), 7.55 - 7.63 (2 H, m), 7.78 (1 H, dt, J= 8.8, 0.8 Hz), 8.00 (1 H d, J = 1.1 Hz).

Reference Example 72

ethyl 1-[2-(3-chloro-5-fluarobenzyl)-2H-indazol-4-yl]-3,5 dimethyl-1H-pyrazole-4-carboxylate

A solution of 2-(3-chloro-5-fluorobenzyl)-4-(4,4,5,5-tetramethyl-1,3,2-dioxaborolan-2-yl)-2H-indazole obtained in Reference Example 69 (330 mg, 0.85 mmol), ethyl 3,5-dimethyl-1H-pyrazole-4-carboxylate (172 mg, 1.02 mmol) and copper(II) acetate monohydrate (85 mg, 0.43 mmol) in pyridine (4.3 mL) was stirred overnight at 80°C. The reaction mixture was allowed to cool to room temperature, and diluted with saturated brine and ethyl acetate. The mixture was extracted with ethyl acetate. The organic layer was dried over anhydrous sodium sulfate, and evaporated under reduced pressure. The residue was purified by silica gel column chromatography (hexane:ethyl acetate= 90:10→67:33) to give the title compound (271 mg, yield 74%).
$^1$H-NMR ($CDCl_3$) δ : 1.40 (3 H, t, J = 7.1 Hz), 2.54 (3 H, s), 2.57 (3 H, s), 4.35 (2 H, q, J = 7.1 Hz), 5.54 (2 H, s), 6.84 - 6.92 (1 H, m), 6.98 - 7.10 (3 H, cm), 7.33 - 7.41 (1 H, m), 7.78 (1H, dd, J = 8.8, 0.5 Hz), 8.01 (1 H, s).

Reference Example 73

ethyl 1-[2-[3-fluoro-5-(trifluoromethyl)benzyl]-2H-indazol-4-yl]-3,5-dimethyl-1H-pyrazole-4-carboxylate

In the same manner as in Reference Example 72 and using 2-[3-fluoro-5-(trifluoromethyl)benzyl]-4-(4,4,5,5-tetramethyl-1,3,2-diaxaborolan-2-yl)-2H-indazole obtained in Reference Example 70 and ethyl 3,5-dimefihyl-1H-pyrazole-4-carboxylate, the title compound was obtained. yield 45%.
$^1$H-NMR ($CDCl_3$) δ : 1.40 (3 H, t, J = 7.1 Hz), 2.53 (3 H, s), 2.57 (3 H, s), 4.35 (2 H, q, J = 7.1 Hz), 5.62 (2 H, s), 7.03 (1H, d, J = 7.1 Hz), 7.15 (1 H, d, J = 8.8 Hz), 7.29 (1 H, d, J = 8.5 Hz), 7.33 - 7.44 (2 H, m), 7.78 (1 H, d, J = 8.8 Hz), 8.04 (1 H, d, J = 0.8 Hz).

Reference Example 74

ethyl 2-[2-(3-chloro-5-fluorobenzyl)-2H-indazol-4-yl]-4-methyl-1,3-thiazole-5-carboxylate

In the same manner as in Reference Example 39 and crude ethyl 2-bromo-4-methyl-1,3-thiazole-5-carboxylate was synthesized from ethyl 2-amino-4-methyl-1,3-thiazole-5-carboxylate, and using the obtained crude ethyl 2-bromo-4-methyl-1,3-thiazole-5-carboxylate and 2-(3-chloro-5-fluorobenzyl)-4-(4,4,5,5-tetramethyl-1,3,2-dioxaborolan-2-yl)-2H-indazole obtained in Reference Example 69, the title compound was obtained. yield 87% (2 steps).
$^1$H-NMR ($CDCl_3$) δ : 1.41 (3 H, t, J = 7.1 Hz), 2.83 (3 H, s), 4.38 (2 H, q, J = 7.1 Hz), 5.63 (2 H, s), 6.83 - 6.94 (1 H, m), 7.00 - 7.13 (2 H, m), 7.37 (1 H, dd, J = 8.8, 7.1 Hz), 7.71 (1 H, d, J = 7.1 Hz), 7.86 (1 H, d, J = 8.8 Hz), 8.81 (1 H, s).

Reference Example 75

ethyl 2-[2-[3-fluoro-5-(trifluoromethyl)benzyl]-2H-indazol-4-yl]-4-methyl-1,3-thiazole-5-carboxylate

In the same manner as in Reference Example 39 and crude ethyl 2-bromo-4-methyl-1,3-thiazole-5-carboxylate was synthesized from ethyl 2-amino-4-methyl-1,3-thiazole-5-carboxylate, and using the obtained crude product and 2-[3-fluoro-5-(trifluoromethyl)benzyl]-4-(4,4,5,5-tetramethyl-1,3,2-dioxaborolan-2-yl)-2H-indazole obtained in Reference Example 70, the title compound was obtained. yield 94% (2 steps).
$^1$H-NMR ($CDCl_3$) δ : 1.41 (3 H, t, J = 7.1 Hz), 2.83 (3 H, s), 4.38 (2 H, q, J = 7.0 Hz), 5-71 (2 H, s), 7.14 (1 H, d, J = 9.1 Hz), 7.29 (1 H, d, J = 8.2 Hz), 7.37 - 7.45 (2 H, m), 7.72 (1 H, d, J = 7.1 Hz), 7.86 (1 H, dd, J = 8.7, 0.7 Hz), 8.84 (1 H, s).

Reference Example 76

(4-bromo-1-benzofuran-2-yl)[3-(trifluoromethyl)phenyl]methanone

To a solution of 2-bromo-6-hydroxybenzaldehyde (4.0 g, 19.9 mmol) in acetonitrile (50 mL) were added 2-bromo-1-[3-(trifluoromethyl)phenyl]ethanone (6.38 g, 23.9 mmol) and potassium carbonate (3.30 g, 23.9 mmol) at room temperature, and the mixture was heated under reflux for 16 hr. The reaction mixture was added to water, and the mixture was extracted with ethyl acetate. The organic layer was washed with water and saturated brine, dried over anhydrous sodium sulfate, and concentrated under reduced pressure. The residue was purified by silica gel column chromatography (hexane-ethyl acetate 90:10→40:60) to give the title compound (4.4 g, yield 60%). melting point 91 - 92°C (methanol).
$^1$H-NMR ($CDCl_3$) δ : 7.39 (1 H, t, J = 8.1 Hz), 7.49 - 7.62 (3 H, m), 7.71 (1 H, t, J = 7.5 Hz), 7.91 (1 H, d, J = 7.8, 1.2 Hz), 8.25 (1 H, d, J = 7.8 Hz), 8.32 (1 H, s).

Reference Example 77

4-bromo-2-[3-(trifluoromethyl)benzyl]-1-benzofuran

A mixture of (4-bromo-1-benzofuran-2-yl)[3-(trifluoromethyl)phenyl]methanone obtained in Reference Example 76 (4.4 g, 11.9 mmol) and hydrazine monohydrate (2.38 g, 47.6 mmol) in ethyleneglycol (50 mL) was heated for 2 hr at 130°C. The mixture was allowed to cool to room temperature, and potassium hydroxide (2.00 g, 35.7 mmol) was added thereto. The mixture was heated for 2 hr at 160°C. The reaction mixture was added to water, and the mixture was extracted with ethyl acetate. The organic layer was washed with water and saturated brine, dried over anhydrous sodium sulfate, and concentrated under reduced pressure. The residue was purified by silica gel column chromatography (hexane-ethyl acetate 95:5→30:70) to give the title compound (3.4 g, yield 80%) as an oil.
$^1$H-NMR ($CDCl_3$) δ: 4.16 (2 H, s), 6.46 (1 H, s), 7.09 (1 H, t, J = 8.1 Hz), 7.31 - 7.36 (2 H, m), 7.40 - 7.61 (4 H, m).

Reference Example 78

3-[2-[3-(trifluoromethyl)benzyl]-1-benzofuran-4-yl]benzoic acid

A mixture of 4-bromo-2-[3-(trifluoromethyl)benzyl]-1-benzofuran obtained in Reference Example 77 (3.40 g, 9.58 mmol), (3-(ethoxycarbonyl)phenyl)boronic acid (2.23 g, 11.5 mmol), tetrakis(triphenylphosphine)palladium(0) (553 mg, 0.48 mmol) in 2N aqueous sodium carbonate solution (30 mL)-1,2-dimethoxyethane (30 mL) was reacted for 16 hr at 90°C under a nitrogen atmosphere. To the reaction mixture was added water, and the mixture was extracted with ethyl acetate. The organic layer was washed with saturated brine, dried over anhydrous sodium sulfate, and concentrated under reduced pressure. The residue was purified by silica gel column chromatography (ethyl acetate-hexane 2:3) to give ethyl 3-[2-[3-(trifluoromethyl)benzyl]-1-benzofuran-4-yl]benzoate (3.30 g). To a solution of the compound in ethanol (50 mL) was added 2N aqueous sodium hydroxide solution (8 mL, 16 mmol) at room temperature, and the mixture was stirred for 2 hr at 60°C, and concentrated under reduced pressure. To the reaction mixture were added water and hydrochloric acid to acidify the aqueous layer, and the mixture was extracted with ethyl acetate. The organic layer was washed with water and saturated brine, dried over anhydrous sodium sulfate, and evaporated under reduced pressure. The obtained residue was crystallized from ethyl acetate-hexane to give the title compound (2.3 g, yield 61%). melting point 138 - 139°C (ethyl acetate-hexane).
$^1$H-NMR ($CDCl_3$) δ: 4.19 (2 H, s), 6.63 (1 H, s), 7.20 - 7.78 (8 H, m), 7.85 (1 H, dd, J = 7.8, 1.2 Hz), 8.13 (1 H, dd, J = 7.8, 1.2 Hz), 8.37 (1 H, s), 1 H unconfirmed.

Reference Example 79

N-(2-hydroxyethyl)-3-[2-[3-(trifluoromethyl)benzyl]-1-benzofuran-4-yl]benzamide

To a solution of 3-[2-[3-(trifluoromethyl)benzyl]-1-benzofuran-4-yl]benzoic acid obtained in Reference Example 78 (300 mg, 0.76 mmol), WSC (159 mg, 0.83 mmol) and HOBt (112 mg, 0.83 mol) in DMF (2 mL) was added 2-aminoethanol (55 μL, 0.91 mmol), and the mixture was stirred for 5 hr at room temperature. To the reaction mixture was added saturated aqueous sodium hydrogen carbonate solution, and the mixture was extracted with ethyl acetate. The organic layer was washed with water, dried over anhydrous magnesium sulfate, and concentrated under reduced pressure. The residue was purified by silica gel column chromatography to give the title compound (208 mg, yield 63%). melting point 135 - 136°C (ethyl acetate-hexane).

$^1$H-NMR ($CDCl_3$) δ: 2.43 (1 H, t, J = 4.8 Hz), 3.66 (2 H, q, J = 4.8 Hz), 3.86 (2 H, q, J = 4.8 Hz), 4.18 (2 H, s), 6.60 (1 H, s), 6.64 (1 H, br s), 7.26 - 7.32 (2 H, m), 7.38 - 7.60 (6 H, m), 7.68 - 7.81 (2 H, m), 8.01 (1 H, s).

Reference Example 80

ethyl 5-(tert-butoxymethyl)-1-[2-(3-chloro-5-fluorobenzyl)-4-fluoro-1-benzothiophen-7-yl]-3-methyl-1H-pyrazole-4-carboxylate

To a solution of [4-fluoro-7-(4,4,5,5-tetrethyl-1,3,2-dioxaborolan-2-yl)-1-benzothiophen-2-yl]methanol obtained in Reference Example 50 (6.13 g, 0.02 mol) in pyridine (10 mL) were added 5-(tert-butoxymethyl)-3-methyl-1H-pyrazole-4-carboxylate (2.50 g, 0.01 mol) (4.78 g, 0.02 mol) and copper(II) acetate monohydrate (0.80 g, 4.00 mmol), and the mixture was stirred overnight at 55°C. The reaction mixture was evaporated under reduced pressure. To a solution of the obtained residue in THF (20 mL) were added methanesulfonyl chloride (1.55 g, 0.01 mol), triethylamine (3.34 g, 0.03 mol) and 4-dimethylaminopyridine (13 mg, 0.11 mmol), and the mixture was stirred for 1 hr at room temperature under a nitrogen atmosphere. To the reaction mixture was poured into saturated aqueous ammonium chloride solution, and the mixture was extracted with ethyl acetate. The extract was washed with water and saturated brine, dried over anhydrous sodium sulfate, and evaporated under reduced pressure. To a solution of the obtained residue in THF (50 mL)-water (10 mL) were added 3-chloro-5-fluorophenylboronic acid (1.92 g, 0.01 mol), cesium carbonate (7.17 g, 0.02 mol) and [1,1'-bis(diphenylphosphino)ferrocane]dichloropalladium(II) dichloromethane adduct (0.36 g, 0.4 mmol), and the mixture was stirred overnight at 60°C. Water was poured into the reaction mixture, and the mixture was extracted with ethyl acetate. The extract was washed with water, dried over anhydrous magnesium sulfate, and evaporated under reduced pressure. The residue was purified by silica gel column chromatography (ethyl acetate:hexane=1:9) to give the title compound (0.75 g, yield 7%) as a solid.
$^1$H-NMR (DMSO-$d_6$) δ: 0.86 (9H, s), 1.24-1.34 (3 H, m), 2.46 (3 H, brs), 4.20-4.36 (4 H, m), 4.46 (2 H, s), 7.14-7.45 (4 H, m), 7.45-7.64 (2 H, m).

Reference Example 81

1-[2-(3-chloro-5-fluorobenzyl)-4-fluoro-1-benzothiophen-7-yl-5-(hydroxymethyl)-3-methyl-1H-pyrazole-4-carboxylic acid

To ethyl 5-(tert-butoxymethyl)-1-[2-(3-chloro-5-fluorobenzyl)-4-fluoro-1-benzothiophen-7-yl]-3-methyl-1H-pyrazole-4-carboxylate obtained in Reference Example 80 (0.75 g, 1.00 mmol) was added 4N hydrochloric acid-ethyl acetate solution (15 mL), and the mixture was stirred overnight at room temperature. The reaction mixture was concentrated under reduced pressure. To a solution of the obtained residue in THE (10 mL)-ethanol (10 mL) was added 8N aqueous sodium hydroxide solution (0.68 mL, 5.46 mmol), and the mixture was stirred for 3 hr at 85°C. The reaction mixture was neutralized with 6N hydrochloric acid, and diluted with water. The mixture was extracted with ethyl acetate. The organic layer was washed with water, dried over anhydrous magnesium sulfate, and concentrated under reduced pressure. To the precipitated crystals was added isopropyl alcohol, and the crystals were collected by filtration to give the title compound (0.27 g, yield 61%) as crystals.
$^1$H-NMR ($CDCl_3$) δ 2.57 (3 H, s), 4.18 (2 H, s), 4.67 (2 H, s), 6.84-6.92 (1 H, m), 6.99 (1 H, d, J = 8.3 Hz), 7.06 (1 H, s), 7.09-7.17 (1 H, m), 7.23 (1 H, s), 7.33 (1 H, dd, J = 8.3, 4.2 Hz), 2 H, unconfirmed.

Reference Example 82

ethyl 1-[2-(hydroxymethyl)-1-benzothiophen-7-yl]-3,5-dimethyl-1H-pyrazole-4-carboxylate

In the same manner as in Reference Example 50, to a solution of 4,4,5,5-tetramethyl-2-[2-[3-(trifluoromethyl)benzyl]-1-benzothiophen-7-yl]-1,3,2-dioxaborolane (6.00 g, 0.02 mmol) obtained from (7-bromo-1-benzothiophen-2-yl)methanol in pyridine (50 mL) were added ethyl 3,5-dimethyl-1H-pyrazole-5-carboxylate (3.53 g, 0.02 mmol) and copper (II) acetate monohydrate (0.84 g, 4.00 mmol), and the mixture was stirred overnight at 55°C. The reaction mixture was evaporated under reduced pressure. The residue was purified by silica gel column chromatography (ethyl acetate: hexane=1:4) to give the title compound (4.55 g, yield 66%) as a solid.
$^1$H-NMR ($CDCl_3$) δ: 1.32-1.44 (3 H, m), 2.45-2.49 (4 H, m), 2.54 (3 H, s), 4.25-4.39 (2 H, m), 4.89 (2 H, s), 7.20-7.30 (2 H, m), 7.44 (1 H, s), 7.74-7.81 (1 H, m).

Reference Example 83

ethyl 3,5-dimethyl-1-[2-(3-methylbenzyl)-1-benzothiophen-7-yl]-1H-pyrazole-4-carboxylate

To a solution of ethyl 1-[2-(hydroxymethyl)-1-benzothiophen-7-yl]-3,5-dimethyl-1H-pyrazole-4-carboxylate obtained in Reference Example 82 (1.52 g, 4.67 mol) in THF (30 mL) were added methanesulfonyl chloride (0.63 g, 5.67 mol), triethylamine (0.94 g, 4.67 mol) and 4-dimethylaminopyridine (57 mg, 0.47 mmol), and the mixture was stirred overnight at room temperature under a nitrogen atmosphere. To the reaction mixture was poured into saturated aqueous ammonium chloride solution, and the mixture was extracted with ethyl acetate. The extract was washed with water and saturated brine, dried over anhydrous sodium sulfate, and evaporated under reduced pressure. To a solution of the obtained residue in THF (16.7 mL)-water (3.3 mL) were added 3-methylphenylboronic acid (0.68 g, 5.00 mmol), cesium carbonate (3.26 g, 10.0 mmol) and [1,1'-bis(diphenylphosphino)ferrocene]dichloropalladium(II), dichloromethane adduct (163 mg, 0.20 mmol), and the mixture was stirred overnight at 60°C. Water was poured into the reaction mixture, and the mixture was extracted with ethyl acetate. The extract was washed with water, dried over anhydrous magnesium sulfate, and evaporated under reduced pressure. The residue was purified by silica gel column chromatography (ethyl acetate:hexane=1:9) to give the title compound (0.47 g, yield 23%) as a solid.
$^1$H-NMR ($CDCl_3$) $\delta$: 1.38 (3 H, t, J = 7.2 Hz), 2.32 (3 H, s), 2.44 (3 H, s), 2.52 (3 H, s), 4.15 (2 H, s), 4.33 (2 H, q, J = 7.2 Hz), 7.02-7.12 (4 H, m), 7.15-7.24 (2 H, m), 7.39 (1 H, t, J = 7.7 Hz), 7.72 (1 H, d, J = 7.9 Hz).

Reference Example 84

ethyl 1-[2-(4-fluoro-3-methoxybenzyl)-1-benzothiophen-7-yl]-3,5-dimethyl-1H-pyrazole-4-carboxylate

In the same manner as in Reference Example 83 and using ethyl 1-[2-(hydroxymethyl)-1-benzothiophen-7-yl]-3,5-dimethyl-1H-pyrazole-4-carboxylate obtained in Reference Example 82 and 4-fluoro-3-methoxyphenylboronic acid, the title compound was obtained as a solid. yield 59%.
$^1$H-NMR ($CDCl_3$) $\delta$: 1.34-1.42 (3 H, m), 2.45 (3 H, s), 2.52 (3 H, s), 3.85 (3 H, s), 4.15 (2 H, s), 4.28-4.39 (2 H, m), 6.86 (2 H, s), 6.96-7.10 (2 H, m), 7.20 (1 H, d, J = 7.5 Hz), 7.41 (1 H, t, J = 7.7 Hz), 7.73 (1 H, d, J = 7.9 Hz).

Reference Example 85

ethyl 1-[2-(3,5-difluorobenzyl)-1-benzothiophen-7-yl]-3,5-dimethyl-1H-pyrazole-4-carboxylate

In the same manner as in Reference Example 83 and using ethyl 1-[2-(hydroxymethyl)-1-benzothiophen-7-yl]-3,5-dimethyl-1H-pyrazole-4-carboxylate obtained in Reference Example 82 and 3,5-difluorophenylboronic acid, the title compound was obtained as a solid. yield 25%.
$^1$H-NMR ($CDCl_3$) $\delta$: 1.39 (3 H, t, J = 7.2 Hz), 2.46 (3 H, s), 2.52 (3 H, s), 4.17 (2 H, s), 4.34 (2 H, q, J = 7.2 Hz), 6.63-6.74 (1 H, m), 6.75-6.83 (2 H, m), 7.11 (1 H, s), 7.21 (1 H, d, J = 7.9 Hz), 7.43 (1 H, t, J = 7.7 Hz), 7.75 (1 H, d, J = 8.3 Hz).

Reference Example 86

3,5-dimethyl-1-[2-(3-methylbenzyl)-1-benzothiophen-7-yl]-1H-pyrazole-4-carboxylic acid

To a solution of ethyl 3,5-dimethyl-1-[2-(3-methylbenzyl)-1-benzothiophen-7-yl]-1H-pyrazole-4-carboxylate obtained in Reference Example 83 (0.47 g, 1.16 mmol) in THF (12.5 mL)-ethanol (12.5 mL) was added 8N aqueous sodium hydroxide solution (0.44 mL, 3.49 mmol), and the mixture was stirred overnight at 85°C. The reaction mixture was neutralized with 6N hydrochloric acid, and diluted with water. The mixture was extracted with ethyl acetate. The organic layer was washed with water, dried over anhydrous magnesium sulfate, and concentrated under reduced pressure. To the precipitated crystals was added isopropyl alcohol, and the crystals were collected by filtration to give the title compound (0.41 g, yield 94%) as crystals.
$^1$H-NMR ($CDCl_3$) $\delta$: 2.33 (3 H, s), 2.48 (3 H, s), 2.55 (3 H, s), 4.16 (2 H, s), 7.02-7.12 (4 H, m), 7.15-7.24 (2 H, m), 7.41 (1 H, t, J = 7.9 Hz), 7.73 (1 H, d, J = 7.2 Hz), 11.24 (1 H, brs)

Reference Example 87

1-[2-(3,5-difluorobenzyl)-1-benzothiophen-7-yl]-3,5-dimethyl-1H-pyrazole-4-carboxylic acid

In the same manner as in Reference Example 86 and using ethyl 1-[2-(3,5-difluorobenzyl)-1-benzothiophen-7-yl]-3,5-dimethyl-1H-pyrazole-4-carboxylate obtained in Reference Example 85, the title compound was obtained as a solid. yield 81%.
$^1$H-NMR ($CDCl_3$) δ: 2.50 (3 H, s), 2.56 (3 H, s), 4.18 (2 H, s), 6.64-6.74 (1 H, m), 6.75-6.84 (2 H, m), 7.12 (1 H, s), 7.21-7.26 (1 H, m), 7.44 (1 H, t, J = 7.7 Hz), 7.76 (1 H, d, J = 7.2 Hz), 10.94 (1 H, brs).

Reference Example 88

1-[2-(4-fluoro-3-methoxybenzyl)-1-benzothiophen-7-yl]-3,5-dimethyl-lH-pyrazole-4-carboxylic acid

In the same manner as in Reference Example 86 and using ethyl 1-[2-(4-fluoro-3-methoxybenzyl)-1-benzothiophen-7-yl]-3,5-dimethyl-1H-pyrazole-4-carboxylate obtained in Reference Example 84, the title compound was obtained as a solid. yield 42%.
$^1$H-NMR ($CDCl_3$) δ: 2.48 (3 H, s), 2.55 (3 H, s), 3.86 (3 H, s), 4.16 (2 H, s), 6.75-6.89 (2 H, m), 7.02 (1 H, dd, J = 11.3, 8.3 Hz), 7.08 (1 H, s), 7.22 (1 H, d, J = 7.5 Hz), 7.42 (1 H, t, J = 7.7 Hz), 7.74 (1 H, d, J = 7.2 Hz), 1 H, unconfirmed.

Reference Example 89

ethyl 1-[2-(hydroxymethyl)-1-benzothiophen-4-yl]-3,5-dimethyl-1H-pyrazole-4-carboxylate

In the same manner as in Reference Example 50, to a solution of [4-(4,4,5,5-tetramethyl-1,3,2-dioxaborolan-2-yl)-1-benzothiophen-2-yl]methanol obtained from (4-bromo-1-benzothiophen-2-yl)methanol (2.30 g, 7.93 mmol) in pyridine (10 mL) were added ethyl 3,5-dimethyl-1H-pyrazole-5-carboxylate (1.11 g, 6.66 mmol) and copper(II) acetate monohydrate (0.27 g, 1.33 mmol), and the mixture was stirred overnight at 55°C. The reaction mixture was evaporated under reduced pressure. The residue was purified by silica gel column chromatography (ethyl acetate:hexane=1:4) to give the title compound (1.70 g, yield 65%) as a solid.
$^1$H-NMR ($CDCl_3$) δ: 1.40 (3 H, t, J = 7.2 Hz), 2.37-2.41 (3 H, m), 2.53 (3 H, s), 4.35 (2 H, q, J = 7.2 Hz), 4.85 (2 H, d, J = 8.7 Hz), 5.09 (1 H, s), 6.80-6.99 (1 H, m), 7.21-7.33 (1 H, m), 7.35-7.47 (1 H, m), 7.89 (1 H, d, J = 8.3 Hz).

Reference Example 90

ethyl 3,5-dimethyl-1-{2-[3-(trifluoromethyl)benzyl]-1-benzothiophen-4-yl}-1H-pyrazole-4-carboxylate

To a solution of ethyl 1-[2-(hydroxymethyl)-benzothiophen-4-yl]-3,5-dimethyl-1H-pyrazole-4-carboxylate obtained in Reference Example 89 (0.85 g, 2.58 mmol) in THF (5 mL) were added methanesulfonyl chloride (0.63 g, 5.67 mmol), triethylamine (0.35 g, 3.09 mmol) and 4-dimethylaminopyridine (31 mg, 0.26 mmol), and the mixture was stirred overnight at room temperature under a nitrogen atmosphere. To the reaction mixture was poured into saturated aqueous ammonium chloride solution, and the mixture was extracted with ethyl acetate. The extract was washed with water and saturated brine, dried over anhydrous sodium sulfate, and evaporated under reduced pressure. To a solution of the obtained residue in THF (20.8 mL)-water (4.2 mL) were added m-(trifluoromethyl)phenylboronic acid (0.49 g, 2.58 mmol), cesium carbonate (1.68 g, 5.15 mmol) and [1,1'-bis(diphenylphosphino)ferrocene]dichloropalladium(II), dichloromethane adduct (84 mg, 0.10 mmol), and the mixture was stirred overnight at 60°C. Water was poured into the reaction mixture, and the mixture was extracted with ethyl acetate. The extract was washed with water, dried over anhydrous magnesium sulfate, and evaporated under reduced pressure. The residue was purified by silica gel column'chromatography (ethyl acetate:hexane=1:9) to give the title compound (0.58 g, yield 49%) as a solid.
$^1$H-NMR ($CDCl_3$) δ: 1.40 (3 H, t, J = 7.0 Hz), 2.41 (3 H, s), 2.53 (3 H, s), 4.26 (2 H, s), 4.35 (2 H, q, J = 7.0 Hz), 6.83 (1 H, s), 7.21-7.29 (1 H, m), 7.33-7.40 (1 H, m), 7.41-7.47 (2 H, m), 7.48-7.56 (2 H, m), 7.80 (1 H, s).

Reference Example 91

ethyl 1-[2-(3-chloro-5-fluorobenzyl)-1-benzothiophen-4-yl]-3,5-dimethyl-1H-pyrazole-4-carboxylate

In the same manner as in Reference Example 90 and using ethyl 1-[2-(hydroxymethyl)-1-benzothiophen-4-yl]-3,5-dimethyl-1H-pyrazole-4-carboxylate obtained in Reference Example 89 and 3-chloro-5-fluorophenylboronic acid, the title compound was obtained as a solid. yield 66%.
$^1$H-NMR ($CDCl_3$) δ: 1.40 (3 H, t, J = 7.1 Hz), 2.42 (3 H, s), 2.54 (3 H, s), 4.16 (2 H, s), 4.36 (2 H, q, J = 7.1 Hz), 6.81-6.91 (2 H, m), 6.98 (1 H, d, J = 8.3 Hz), 7.05 (1 H, s), 7.22-7.29 (1 H, m), 7.34-7.44 (1 H, m), 7.83 (1 H, d, J = 8.0 Hz).

Reference Example 92

3,5-dimethyl-1-{2-[3-(trifluoromethyl)benzyl]-1-benzothiophen-4-yl)-1H-pyrazole-4-carboxylic acid

To a solution of ethyl 3,5-dimethyl-1-{2-[3-(trifloromethyl)benzyl]-1-benzothiophen-4-yl}-1H-pyrazole-4-carboxylate obtained in Reference Example 90 (0.58 g, 1.26 mmol) in THF (7.5 mL)-methanol (7.5 mL) was added 8N aqueous sodium hydroxide solution (0.47 mL, 3.79 mmol), and the mixture was stirred overnight at 65°C. The reaction mixture was neutralized with 6N hydrochloric acid, and diluted with water. The precipitated crystals were collected by filtration to give the title compound (0.47 g, yield 88%) as crystals. $^1$H-NMR (DMSO-$d_6$) δ: 2.32 (3 H, s), 2.40 (3 H, s), 4.39 (2 H, s), 6.95 (1 H, s), 7.27-7.85 (6 H, m), 7.98-8.12 (1 H, m), 12.39 (1 H, brs).

Reference Example 93

1-[2-(3-chloro-5-fluorobenzyl)-1-benzothiophen-4-yl]-3,5-dimethyl-1H-pyrazole-4-carboxylic acid

In the same manner as in Reference Example 92 and using ethyl 1-[2-(3-chloro-5-fluorobenzyl)-1-benzothiophen-4-yl]-3,5-dimethyl-1H-pyrazole-4-carboxylate obtained in Reference Example 91, the title compound (0.50 g, yield 71%) as crystals. $^1$H-NMR (DMSO-$d_6$) 5: 2.32 (3 H, s), 2.40 (3 H, s), 4.30 (2 H, s), 6.97 (1 H, s), 7.16-7.52 (5 H, m), 8.05 (1 H, d, J = 7.9 Hz), 12.40 (1 H, brs).

Reference Example 94

methyl 1-methyl-3-{2-[3-(trifluoromethyl)benzyl]-1-benzothiophen-7-yl}-1H-pyrazole-5-carboxylate

To a solution of 4,4,5,5-tetramethyl-2-{2-[3-(trifluoromethyl)benzyl]-1-benzothiophen-7-yl}-1,3,2-dioxaborolane (0.80 g, 1.92 mmol) and methyl 1-methyl-3-{[(trifluoromethyl)sulfonyl]oxy}-1H-pyrazole-5-carboxylate (0.37 g, 1.28 mmol) in DMF (10 mL) were added 2N aqueous sodium carbonate solution (1.28 mL) and tetrakistriphenylphosphinepalladium (0.15 g, 0.13 mmol), and the mixture was stirred overnight at 95°C under a nitrogen atmosphere. Water was poured into the reaction mixture, and the mixture was extracted with ethyl acetate. The extract was washed with water, dried over anhydrous magnesium sulfate, and evaporated under reduced pressure. The residue was purified by silica gel column chromatography (ethyl acetate:hexane=1:9) to give the title compound (0.14 g, yield 25%) as a solid. $^1$H-NMR ($CDCl_3$) δ: 3.93 (3 H, s), 4.29 (3 H, s), 4.32 (2 H, s), 7.08 (1 H, s), 7.31 (1 H, s), 7.36-7.54 (4 H, m), 7.58 (1 H, s), 7.64-7.71 (2 H, m).

Reference Example 95

1-methyl-3-{2-[3-(trifluoromethyl)benzyl]-1-benzothiophen-7-yl}-1H-pyrazole-5-carboxylic acid

To a solution of methyl 1-methyl-3-{2-[3-(trifluoromethyl)benzyl]-1-benzothiophen-7-yl}-1H-pyrazole-5-carboxylate obtained in Reference Example 94 (0.14 g, 0.33 mmol) in THF (10 mL)-methanol (20 mL) was added 8N aqueous sodium hydroxide solution (0.12 mL, 0.98 mmol), and the mixture was stirred for 3 hr at 85°C. The reaction mixture was neutralized with 6N hydrochloric acid, and diluted with water. The mixture was extracted with ethyl acetate. The organic layer was washed with water, dried over anhydrous magnesium sulfate, and concentrated under reduced pressure. To the precipitated crystals was added isopropyl alcohol, and the crystals were collected by filtration to give the title compound (0.13 g, yield 96%) as crystals.
$^1$H-NMR ($CDCl_3$) 5: 4.31 (3 H, s), 4.33 (2 H, s), 7.09 (1 H, s), 7.37-7.47 (3 H, m), 7.48-7.54 (2 H, m), 7.59 (1 H, brs), 7.65-7.73 (2 H, m), 1 H, unconfirmed.

Example 1

N-(2-hydroxyethyl)-2-[2-[3-(trifluoromethyl)benzyl]-1-benzothiophen-7-yl]-1,3-thiazole-5-carboxamide

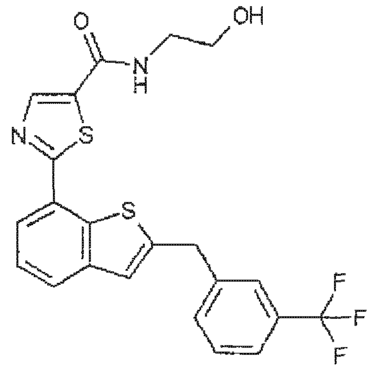

A mixture of 2-[2-[3-(trifluoromethyl)benzyl]-1-benzothiophen-7-yl]-1,3-thiazole-5-carboxylic acid obtained in Reference Example 2 (25 mg, 0.060 mmol), 2-aminoethanol (0.0054 mL, 0.089 mmol), WSC (17 mg, 0.089 mmol), HOBt (12 mg, 0.089 mmol) and THF (1 mL) was stirred for 3 hr. The reaction mixture was diluted with ethyl acetate, and washed with 1N hydrochloric acid, saturated aqueous sodium hydrogen carbonate solution and saturated brine. The organic layer was dried over anhydrous magnesium sulfate, and concentrated under reduced pressure. The residue was crystallized from hexane-ethyl acetate to give the title compound (18 mg, yield 65 %) as crystals. melting point 182-184°C.
$^1$H-NMR ($CDCl_3$) $\delta$: 2.21 (1 H, t, J = 4.9 Hz), 3. 61 - 3.72 (2 H, m), 3.83 - 3.93 (2 H, m), 4.33 (2 H, s), 6.42 - 6.54 (1 H, m), 7.10 (1 H, s), 7.39 - 7.60 (5 H, m), 7.77 - 7.87 (2 H, m), 8.28 (1 H, s).

Example 2

N-(2-methoxyethyl)-2-[2-[3-(trifluoromethyl)benzyl]-1-benzothiophen-7-yl]-1,3-thiazole-5-carboxamide

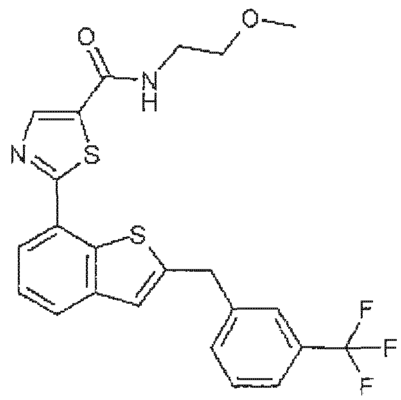

In the same manner as in Example 1 and using 2-[2-[3-(trifluoromethyl)benzyl]-1-benzothiophen-7-yl]-1,3-thiazole-5-carboxylic acid obtained in Reference Example 2 and 2-methoxyethanamine, the title compound was obtained as crystals. yield 79% melting point 172 - 174°C (ethanol-water). $^1$H-NMR ($CDCl_3$) $\delta$: 3.41 (3 H, s), 3.54 - 3.71 (4 H, m), 4.33 (2 H, s), 6.37 - 6.49 (1 H, m), 7.09 - 7.11 (1 H, m), 7.38 - 7.60 (5 H, m), 7.77 - 7.88 (2 H, m), 8.27 (1 H, s).

Example 3

N-(2-amino-2-oxoethyl)-2-[2-[3-(trifluoromethyl)benzyl]-1-benzothiophen-7-yl]-1,3-thiazole-5-carboxamide

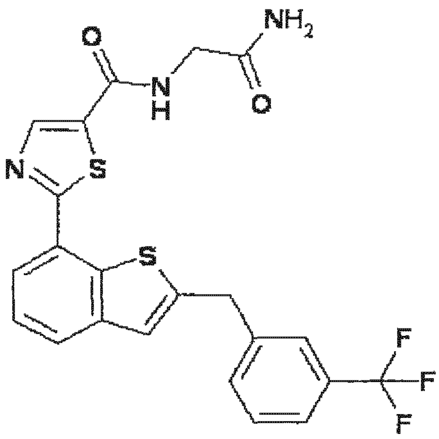

In the same manner as in Example 1 and using 2-[2-[3-(trifluoromethyl)benzyl]-1-benzothiophen-7-yl]-1,3-thiazole-5-carboxylic acid obtained in Reference Example 2 and glycinamide hydrochloride, the title compound was obtained as crystals. yield 45% melting point 214 - 216°C (hexane). $^1$H-NMR (DMSO-$d_6$) δ: 3.82 (2 H, d, J = 5.8 Hz), 4.42 (2 H, s), 7.09 (1 H, br s), 7.34 (1 H, s), 7.41 - 7.79 (6 H, m), 7.95 (2 H, d, J = 7.7 Hz), 8.54 (1 H, s), 8.97 - 9.11 (1 H, m).

Example 4

N-(2-hydroxyethyl)-1-[2-[3-(trifluoromethyl)benzyl]-1-benzothiophen-7-yl]-1H-pyrazole-4-carboxamide

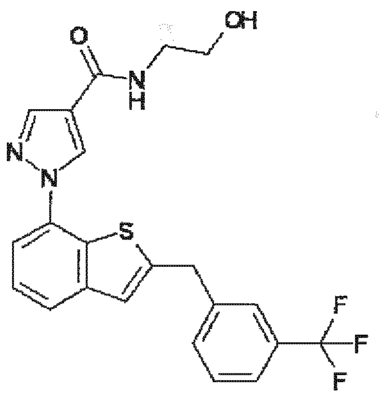

In the same manner as in Example 1 and using 1-[2-[3-(trifluoromethyl)benzyl]-1-benzothiophen-7-yl]-1H-pyrazole-4-carboxylic acid obtained in Reference Example 4 and 2-aminoethanol, the title compound was obtained as crystals. yield 54% melting point 226 - 228°C (hexane). $^1$H-NMR ($CDCl_3$) δ: 3.27 - 3.37 (2 H, m), 3.45 - 3.59 (2 H, m), 4.33 - 4.47 (2 H, m), 4.69 - 4.82 (1 H, m), 7.28 - 7.38 (1 H, m), 7.41 - 7.83 (7 H, m), 8.16 - 8.35 (2 H, m), 8.95 - 9.07 (1 H, m).

Example 5

N-(2-methoxyethyl)-1-[2-[3-(trifluoromethyl)benzyl]-1-benzothiophen-7-yl]-1H-pyrazole-4-carboxamide

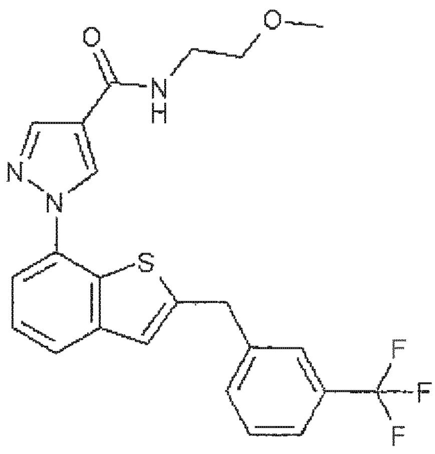

In the same manner as in Example 1 and using 1-[2-[3-(trifluoromethyl)benzyl]-1-benzothiophen-7-yl]-1H-pyrazole-4-carboxylic acid obtained in Reference Example 4 and 2-methoxyethanamine, the title compound was obtained as crystals. yield 71% melting point 157 - 159°C (hexane-ethyl acetate). $^1$H-NMR ($CDCl_3$) δ: 3.41 (3 H, s), 3.53 - 3.69 (4 H, m), 4.29 (2 H, s), 6.22 (1 H, br s), 7.08 - 7.11 (1 H, m), 7.40 - 7.58 (6 H, m), 7.66 - 7.70 (1 H, m), 8.02 (1 H, d, J = 0.8 Hz), 8.47 (1 H, d, J = 0.8 Hz).

Example 6

N-(2-amino-2-oxoethyl)-1-[2-[3-(trifluoromethyl)benzyl]-1-benzothiophen-7-yl]-1H-pyrazole-4-carboxamide

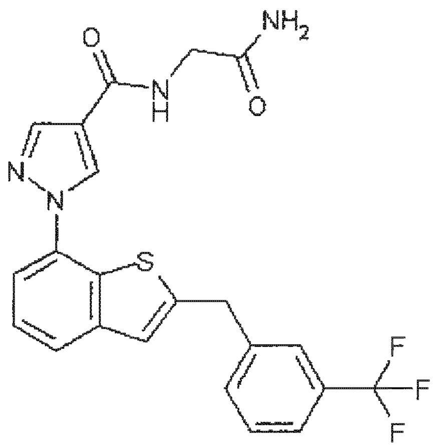

In the same manner as in Example 1 and using 1-[2-[3-(trifluoromethyl)benzyl]-1-benzothiophen-7-yl]-1H-pyrazole-4-carboxylic acid obtained in Reference Example 4 and glycinamide hydrochloride, the title compound was obtained as crystals. yield 74% melting point 227 - 230°C (hexane-THF). $^1$H-NMR (DMSO-$d_6$) δ: 3.82 (2 H, d, J = 6.0 Hz), 4.40 (2 H, s), 7.07 (1 H, br s), 7.32 - 7.83 (9 H, m), 8.24 (1 H, s), 8.50 (1 H, t, J = 6.0 Hz), 9.06 (1 H, s).

Example 7

N-(2-hydroxyethyl)-1-[2-[3-(trifluoromethyl)benzyl]-1-benzothiophen-7-yl]-1H-imidazole-4-carboxamide

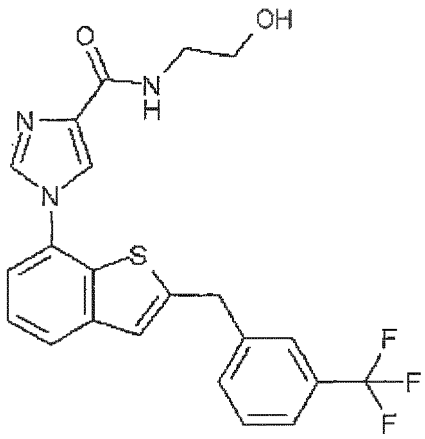

In the same manner as in Example 1 and using 1-[2-[3-(trifluoromethyl)benzyl]-1-benzothiophen-7-yl]-1H-imidazole-4-carboxylic acid obtained in Reference Example 7 and 2-aminoethanol, the title compound was obtained as an oil. yield 73%. $^1$H-NMR ($CDCl_3$) δ: 3.31 (1 H, br s), 3.59 - 3.68 (2 H, m), 3.81 - 3.88 (2 H, m), 4.28 (2 H, s), 7.14 (1 H, t, J = 1.1 Hz), 7.22 - 7.28 (1H, m), 7.40 - 7.64 (6 H, m), 7.75 (1 H, dd, J = 7.9, 0.8 Hz), 7.81 (1 H, d, J = 1.1 Hz), 7.98 (1 H, d, J = 1.1 Hz).

Example 8

N-(2-methoxyethyl)-1-[2-[3-(trifluoromethyl)benzyl]-1-benzothiophen-7-yl]-1H-imidazole-4-carboxamide

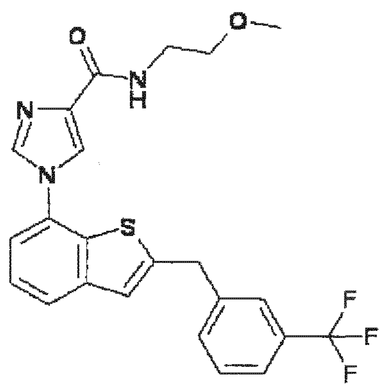

In the same manner as in Example 1 and using 1-[2-[3-(trifluoromethyl)benzyl]-1-benzothiophen-7-yl]-1H-imidazole-4-carboxylic acid obtained in Reference Example 7 and 2-methoxyethanamine, the title compound was obtained as crystals. yield 76% melting point 123 - 126°C (hexane-ethyl acetate). $^1$H-NMR ($CDCl_3$) δ: 3.40 (3 H, s), 3.54 - 3.70 (4 H, m), 4.28 (2 H, s), 7.12 - 7.16 (1 H, m), 7.23 - 7.28 (1 H, m), 7.41 - 7.57 (6 H, m), 7.75 (1 H, dd, J = 7.9, 1.1 Hz), 7.82 (1 H, d, J = 1.5 Hz), 7.97 (1 H, d, J = 1.5 Hz).

Example 9

N-(2-amino-2-oxoethyl)-1-[2-[3-(trifluoromethyl)benzyl]-benzothiophen-7-yl]-1H-imidazole-4-carboxamide

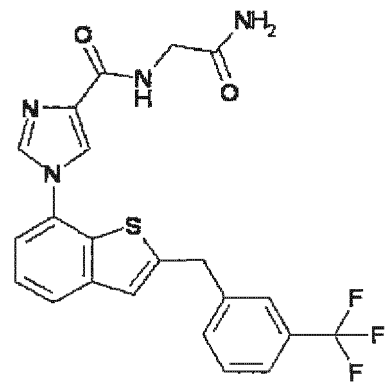

In the same manner as in Example 1 and using 1-[2-[3-(trifluoromethyl)benzyl]-1-benzothiophen-7-yl]-1H-imidazole-4-carboxylic acid obtained in Reference Example 7 and glycinamide hydrochloride, the title compound was obtained as crystals. yield 75% melting point 214 - 218°C (hexane-THF). $^1$H-NMR (DMSO-$d_6$) 5: 3.84 (2 H, d, J = 5.7 Hz), 4.41 (2 H, s), 7.08 (1 H, br s), 7.35 - 7.76 (8 H, m), 7.91 (1 H, dd, J = 6.8, 1.9 Hz), 8.08 - 8.16 (2 H, m), 8.21 (1 H, d, J = 1.5 Hz).

Example 10

N-(2-methoxyethyl)-4-methyl-2-[2-[3-(trifluoromethyl)benzyl]-1-benzothiophen-7-yl]-1,3-oxazole-5-carboxamide

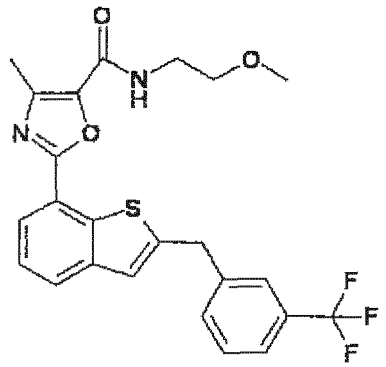

To a solution of ethyl 4-methyl-2-[2-[3-(trifluoromethyl)benzyl]-1-benzothiophen-7-yl]-1,3-oxazole-5-carboxylate obtained in Reference Example 10 (0.10 g, 0.22 mmol) in ethanol (5 mL) was added 1N aqueous sodium hydroxide solution (0.67 mL, 0.67 mmol), and the mixture was stirred for 1 hr at 50°C. The reaction mixture was neutralized with 1N hydrochloric acid (0.67 mL), and evaporated under reduced pressure. A mixture of the residue, 2-methoxyethylamine (0.02 mL, 0.27 mmol), WSC (58 mg, 0.34 mmol), HOBt (46 mg, 0.34 mmol) and DMF (5 mL) was stirred for 2 hr. The reaction mixture was diluted with water, and extracted with ethyl acetate. The extract was washed with water, dried over anhydrous magnesium sulfate, and evaporated under reduced pressure. The residue was purified by silica gel column chromatography (hexane:ethyl acetate=1:1) to give crystals, and the obtained crystals were recrystallized from hexane-diethyl ether to give the title compound (74.9 mg, yield 70%) as crystals. melting point 84 - 85°C.
$^1$H NMR ($CDCl_3$) δ: 2.63 (3 H, s), 3.41 (3 H, s), 3.57 (2 H, t, J = 5.0 Hz), 3.66 (2 H, q, J = 5.4 Hz), 4.33 (2 H, s), 6.69 (1 H, m), 7.09 (1 H, s), 7.40 - 7.58 (4 H, m), 7.57 (1 H, s), 7.82 (1 H, d, J = 8.1 Hz), 8.04 (1 H, d, J = 7.5 Hz).

Example 11

N-(2-amino-2-oxoethyl)-4-methyl-2-[2-[3-(trifluoromethyl)benzyl]-1-benzothiophen-7-yl]-1,3-oxazole-5-carboxamide

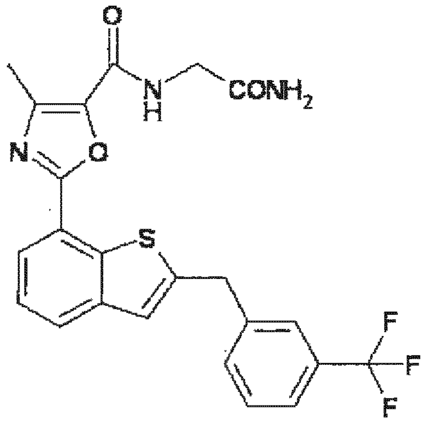

To a solution of ethyl 4-methyl-2-[2-[3-(trifluoromethyl)benzyl]-1-benzothiophen-7-yl]-1,3-oxazole-5-carboxylate obtained in Reference Example 10 (0.10 g, 0.22 mmol) in ethanol (3 mL) was added 1N aqueous sodium hydroxide solution (0.67 mL, 0.67 mmol), and the mixture was stirred for 30 min at 50°C. The reaction mixture was neutralized with 1N hydrochloric acid (0.67 mL), and evaporated under reduced pressure. A mixture of the residue, glycinamide hydrochloride (30 mg, 0.27 mmol), WSC (58 mg, 0.34 mmol), HOBt (46 mg, 0.34 mmol) and DMF (7 mL) was stirred for 2 hr. The reaction mixture was diluted with water, and extracted with ethyl acetate. The extract was washed with water, dried over anhydrous magnesium sulfate, and evaporated under reduced pressure. The residue was purified by silica gel column chromatography (ethyl acetate), and the obtained crystals were recrystallized from hexane-ethyl acetate to give the title compound (73.3 mg, yield 69%) as crystals.
melting point 242 - 243°C.
$^1$H NMR ($CDCl_3$ - $d_6$-DMSO(1 drop)) δ: 2.61 (1 H, br s), 4.12 (2 H, d, J = 5.1 Hz), 4.34 (2 H, s), 6.10 (1 H, br s), 7.06 (1 H, br s), 7.12 (1 H, s), 7.40 - 7.60 (5 H, m), 7.62 (1 H, m), 7.84 (1 H, d, J = 8.1 Hz), 8.12 (1 H, d, J = 7.5 Hz).

Example 12

N-(2-amino-2-oxoethyl)-4-methyl-1-[2-[3-(trifluoromethyl)benzyl]-1-benzothiophen-7-yl]-1H-pyrrole-3-carboxamide

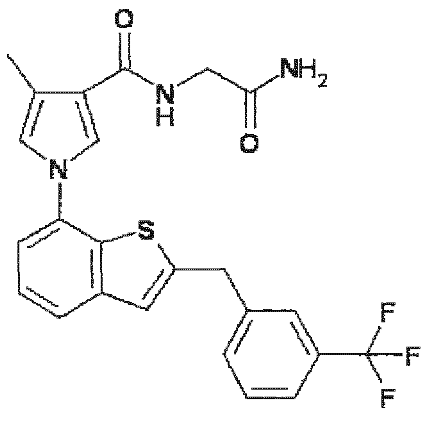

A mixture of ethyl 4-methyl-1H-pyrrole-3-carboxylate (184 mg, 1.2 mmol), 4,4,5,5-tetramethyl-2-[2-[3-(trifluoromethyl)benzyl]-1-benzothiophen-7-yl]-1,3,2-dioxaborolane (602 mg, 1.44 mmol), copper acetate (109 mg, 0.6 mmol) and pyridine (6.0 mL) was stirred for 2 hr at 50°C, and stirred for 4 hr at 80°C. The reaction mixture was allowed to cool to room temperature, and concentrated under reduced pressure. The residue was purified by silica gel column chromatography (hexane-ethyl acetate 95:5→90:10) to give crude ethyl 4-methyl-1-[2-[3-(trifluoromethyl)benzyl]-1-benzothiophen-7-yl]-1H-pyrrole-3-carboxylate (84 mg).
To a solution of the compound (84 mg) in THF (2 mL)-methanol (1 mL) was added 2N aqueous sodium hydroxide solution (284 μL, 0.568 mmol), and the mixture was stirred for 2 hr.at room temperature. 2N Aqueous sodium hydroxide solution (0.5 mL, 1.00 mmol) was added thereto. The mixture was stirred for 1.5 hr at room temperature, stirred for 1 hr at 50°C,

and heated under reflux overnight. The reaction mixture was acidified with 1N hydrochloric acid, and extracted with ethyl acetate. The organic layer was washed with saturated brine, dried over anhydrous sodium sulfate, and evaporated under reduced pressure to give crude 4-methyl-1-[2-[3-(trifluoromethyl)benzyl]-1-benzothiophen-7-yl]-1H-pyrrole-3-carboxylic acid (80 mg). A solution of the obtained crude 4-methyl-1-[2-[3-(trifluoromethyl)benzyl]-1-benzothiophen-7-yl]-1H-pyrrole-3-carboxylic acid (80 mg), glycinamide hydrochloride (25 mg, 0.227 mmol), WSC (44 mg, 0.227 mmol), HOBt (31 mg, 0.227 mmol) and N,N-diisopropylethylamine (39 μL, 0.227 mmol) in DMF (2.0 mL) was stirred for 5 hr at room temperature. The reaction mixture was diluted with saturated aqueous sodium hydrogen carbonate solution, and extracted with ethyl acetate. The extract was washed with saturated brine, dried over anhydrous magnesium sulfate, and evaporated under reduced pressure. The residue was purified by silica gel column chromatography (ethyl acetate:methanol= 100:0→95:5) and NH silica gel column chromatography (ethyl acetate:methanol= 95:5). The obtained crystals were recrystallized from hexane-ethyl acetate to give the title compound (27 mg, yield 5%, 3 steps) as crystals. melting point 152-153°C.

$^1$H-NMR ($CDCl_3$) δ: 2.39 (3 H, s), 4.14 (2 H, d, J = 5.2 Hz), 4.28 (2 H, s), 5.40 (1 H, br s), 6.24 (1 H, br s), 6.43 - 6.54 (1 H, m), 6.89 - 6.96 (1 H, m), 7.11 (1 H, s), 7.21 (1 H, dd, J = 7.6, 0.7 Hz), 7.39 (1 H, t, J = 7.8 Hz), 7.43 - 7.57 (4 H, m), 7.58 (1 H, d, J = 2.5 Hz), 7.65 (1 H, d, J = 7.4 Hz).

Example 13

N-(2-methoxyethyl)-5-methyl-1-[2-[3-(trifluoromethyl)benzyl]-1-benzothiophen-7-yl]-1H-pyrazole-4-carboxamide

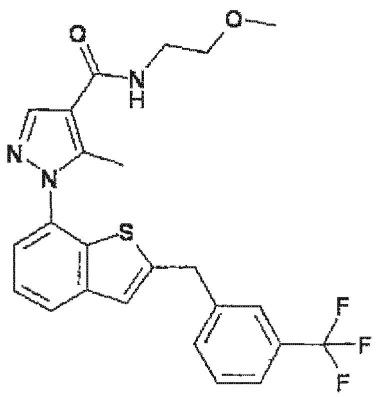

In the same manner as in Example 1 and using 5-methyl-1-[2-[3-(trifluoromethyl)benzyl]-1-benzothiophen-7-yl]-1H-pyrazole-4-carboxylic acid obtained in Reference Example 13 and 2-methoxyethanamine, the title compound was obtained as an oil. yield 92%.

$^1$H-NMR ($CDCl_3$) 5: 2.53 (3 H, s), 3.40 (3 H, s), 3.51 - 3.70 (4 H, m), 4.25 (2 H, s), 6.23 (1 H, t, J = 4.5 Hz), 7.10 (1 H, s), 7.19 - 7.24 (1 H, m), 7.37 - 7.57 (5 H, m), 7.73 - 7.78 (1 H, m), 7.88 (1 H, s).

Example 14

N-(2-methoxyethyl)-3-methyl-1-[2-[3-(trifluoromethyl)benzyl]-1-benzothiophen-7-yl]-1H-pyrazole-4-carboxamide

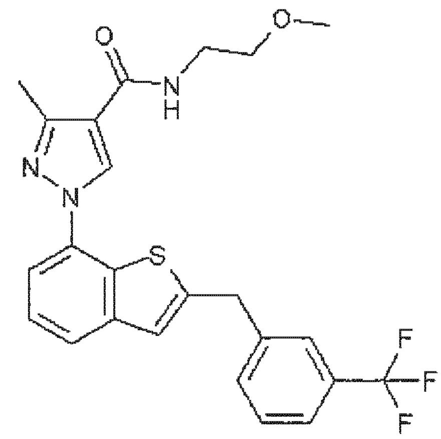

In the same manner as in Example 1 and using 3-methyl-1-[2-[3-(trifluoromethyl)benzyl]-1-benzothiophen-7-yl]-1H-pyrazole-4-carboxylic acid obtained in Reference Example 14 and 2-methoxyethanamine, the title compound was obtained as crystals. yield 89% melting point 97 - 100°C (hexane-ethyl acetate).
$^1$H-NMR ($CDCl_3$) δ: 2.61 (3 H, s), 3.39 (3 H, s), 3.51 - 3. 68 (4 H, m), 4.28 (2 H, s), 6.26 (1 H, t, J = 4.5 Hz), 7.06 (1 H, t, J = 1.1 Hz), 7.33 - 7.67 (7 H, m), 8.34 (1 H, s).

Example 15

N-(2-hydroxyethyl)-3-methyl-1-[2-[3-(trifluoromethyl)benzyl]-1-benzothiophen-7-yl]-1H-pyrazole-4-carboxamide

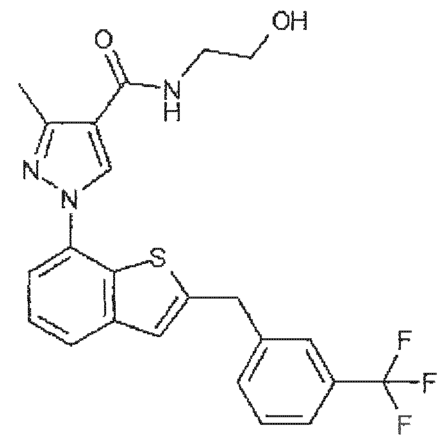

A mixture of 3-methyl-1-[2-[3-(trifluoromethyl)benzyl]-1-benzothiophen-7-yl]-1H-pyrazole-4-carboxyl acid obtained in Reference Example 14 (125 mg, 0.30 mmol), 2-aminoethanol (0.027 mL, 0.45 mmol), WSC (86 mg, 0.45 mmol), HOBt (61 mg, 0.45 mmol), THF (3 mL) and DMF (1 mL) was stirred for 3 days. The reaction mixture was diluted with ethyl acetate, and washed with 1N hydrochloric acid, saturated aqueous sodium hydrogen carbonate solution and saturated brine. The organic layer was dried over anhydrous magnesium sulfate, and concentrated under reduced pressure. The residue was purified by silica gel column chromatography (ethyl acetate-ethanol=9:1). The obtained solid was crystallized from hexane-ethyl acetate to give the title compound (107 mg, yield 78 %) as crystals. melting point 122-124°C.
$^1$H-NMR ($CDCl_3$) δ: 2.47 (1 H, t, J = 4.9 Hz), 2.62 (3 H, s), 3.58 - 3.67 (2 H, m), 3.80 - 3.89 (2 H, m), 4.29 (2 H, s), 6.24 (1 H, br s), 7.06 - 7.10 (1 H, m), 7.35 - 7.68 (7 H, m), 8.35 (1 H, s).

Example 16

N-(2-amino-2-oxoethyl)-3-methyl-1-[2-[3-(trifluoromethyl)benzyl]-1-benzothiophen-7-yl]-1H-pyrazole-4-carboxamide

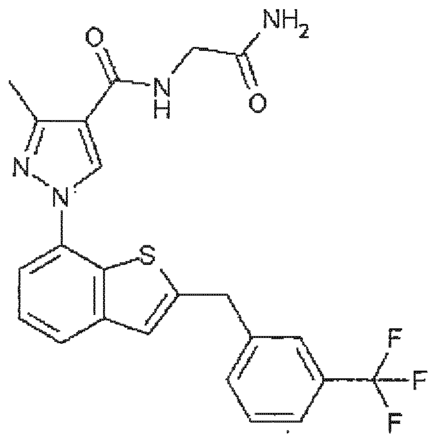

In the same manner as in Example 1 and using 3-methyl-1-[2-[3-(trifluoromethyl}benzyl]-1-benzothiophen-7-yl]-1H-pyrazole-4-carboxylic acid obtained in Reference Example 14 and glycinamide hydrochloride, the title compound was obtained as crystals. yield 81% melting point 206 - 209°C (hexane-ethyl acetate).
$^1$H-NMR ($CDCl_3$) δ: 2.64 (3 H, s), 4.15 (2 H, d, J = 4.9 Hz), 4.29 (2 H, s), 5.45 (1 H, br s), 5.99 (1 H, br s), 6.61 (1 H, br s), 7.07 (1 H, t, J = 0.9 Hz), 7.36 - 7.68 (7 H, m), 8.39 (1 H, s).

Example 17

3-methyl-1-[2-[3-(trifluoromethyl)benzyl]-1-benzothiophen-7-yl]-1H-pyrazole-4-carboxamide

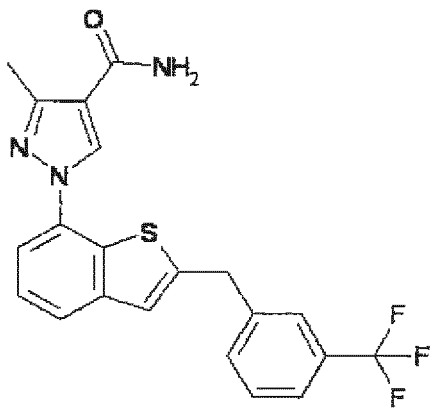

In the same manner as in Example 1 and using 3-methyl-1-[2-[3-(trifluoromethyl)benzyl]-1-benzothiophen-7-yl]-1H-pyrazole-4-carboxylic acid obtained in Reference Example 14 and HOBt ammonium salt, the title compound was obtained as crystals. yield 75% melting point 162 - 164°C (hexane-ethyl acetate).
$^1$H-NMR ($CDClo_3$) δ: 2.62 (3 H, s), 4.30 (2 H, s), 5.58 (2 H, br s), 7.09 (1 H, t, J = 1.1 Hz), 7.37 - 7.69 (7 H, m), 8.38 (1 H, s).

Example 18

N-(2-amino-2-oxoethyl)-1-methyl-4-[2-[3-(trifluoromethyl)benzyl]-1-benzothiophen-7-yl]-1H-imidazole-2-carboxamide

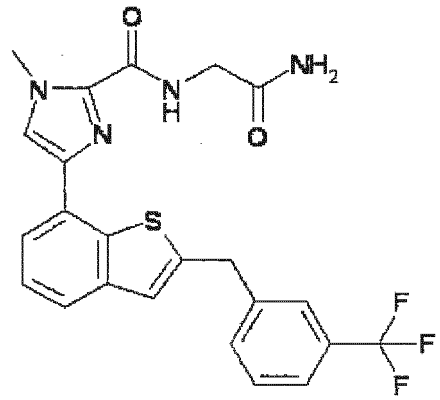

To a solution of ethyl 1-methyl-4-[2-[3-(trifluoromethyl)benzyl]-1-benzothiophen-7-yl]-1H-imidazole-2-carboxylate obtained in Reference Example 19 (95 mg, 0.213 mmol) in THF (2 mL)-methanol (1 mL) was added 2N aqueous sodium hydroxide solution (374 μL, 0.748 mmol), and the mixture was stirred for 4 hr at room temperature. The reaction mixture was neutralized with 1N hydrochloric acid, and extracted with ethyl acetate. The organic layer was washed with saturated brine, dried over anhydrous sodium sulfate, and evaporated under reduced pressure to give crude 1-methyl-4-[2-[3-(trifluoxomethyl)benzyl]-1-benzothiophen-7-yl]-1H-imidazole-2-carboxylic acid (87 mg). A solution of the compound, glycinamide hydrochloride (28 mg, 0.251 mmol), WSC (48 mg, 0.251 mmol), HOBt (34 mg, 0.251 mmol) and N, N-diisopropylethylamine (43.2 μL, 0.251 mmol) in DMF (2.0 mL) was stirred overnight at room temperature. The reaction mixture was diluted with saturated aqueous sodium hydrogen carbonate solution, and extracted with ethyl acetate. The extract was washed with saturated brine, dried over anhydrous sodium sulfate, and evaporated under reduced pressure. The residue was purified by silica gel column chromatography (hexane:ethyl acetate= 30:70→0:100, ethyl acetate: methanol= 100:0→95:5). The obtained crystals were washed with hexane-diethyl ether to give the title compound (7 mg, yield 7%, 2 steps) as crystals. melting point 208-211°C.

$^1$H-NMR ($CDCl_3$) δ: 4.09 - 4.25 (5 H, m), 4.34 (2 H, s), 5.79 (1 H, br s), 6.17 (1 H, br s), 7.08 (1 H, s), 7.34 - 7.57 (5 H, m), 7.59 (1 H, s), 7.65 (1 H, dd, J = 8.0, 1.1 Hz), 7.70 (1 H, dd, J = 7.7, 1.1 Hz), 7.96 - 8.10 (1 H, m).

Example 19

N-(2-methoxyethyl)-3-(trifluoromethyl)-1-[2-[3-(trifluoromethyl)benzyl]-1-benzothiophen-7-yl]-1H-pyrazole-4-carboxamide

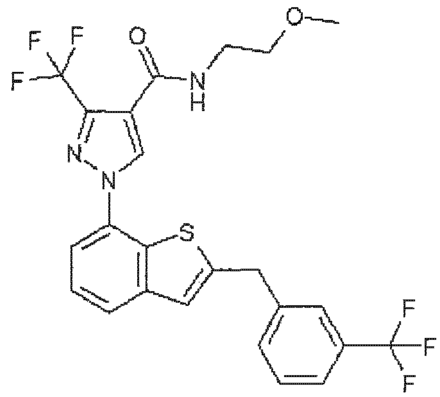

In the same manner as in Example 1 and using 3-(trifluoromethyl)-1-[2-[3-(trifluoromethyl)benzyl]-1-benzothiophen-7-yl]-1H-pyrazole-4-carboxylic acid obtained Reference Example 16 and 2-methoxyethanamine, the title compound was obtained as crystals. yield 70% melting point 120 - 123°C (hexane).

$^1$H-NMR ($CDCl_3$) δ: 3.40 (3 H, s), 3.52 - 3.69 (4 H, m), 4.30 (2 H, s), 6.54 (1 H, br s), 7.08 (1 H, t, J = 0.9 Hz), 7.40 - 7.59 (6 H, m), 7.72 (1 H, dd, J = 7.0, 2.1 Hz), 8.53 - 8.57 (1 H, m).

Example 20

N-(2-hydroxyethyl)-3-(trifluoromethyl)-1-[2-[3-(trifluoromethyl)benzyl]-1-benzothiophen-7-yl]-1H-pyrazole-4-carboxamide

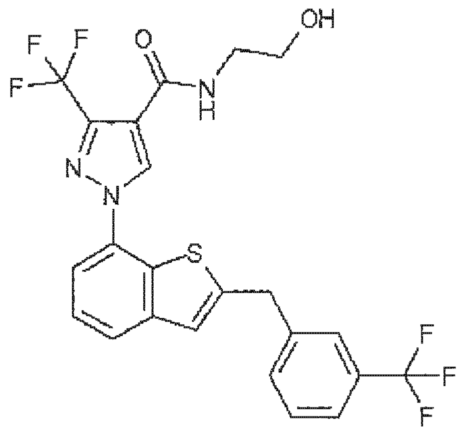

In the same manner as in Example 1 and using 3-(trifluoromethyl)-1-[2-[3-(trifluoromethyl)benzyl]-1-benzothiophen-7-yl]-1H-pyrazole-4-carboxylic acid obtained Reference Example 16 and 2-aminoethanol, the title compound was obtained as crystals. yield 84% melting point 174 - 177°C (ethanol-water).
$^1$H-NMR ($CDCl_3$) δ: 2.23 (1 H, t, J = 4.9 Hz), 3. 60 - 3.89 (4 H, m), 4.30 (2 H, s), 6.59 (1 H, br s), 7.09 (1 H, t, J = 1.1 Hz), 7.39 - 7.59 (6 H, m), 7.72 (1 H, dd, J = 7.2, 1.9 Hz), 8.55 - 8.60 (1 H, m).

Example 21

N-(2-amino-2-oxoethyl)-3-(trifluoromethyl)-1-[2-[3-(trifluoromethyl)benzyl]-1-benzothiophen-7-yl]-1H-pyrazole-4-carboxamide

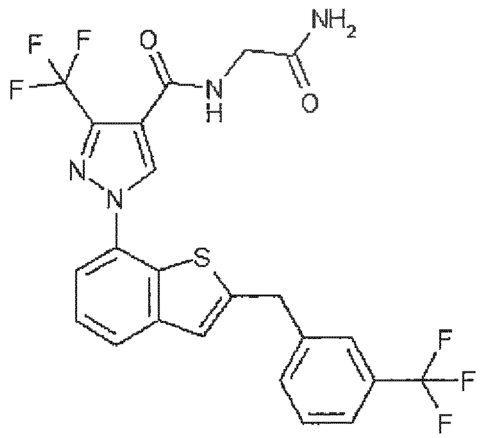

In the same manner as in Example 1 and using 3-(trifluoromethyl)-1-[2-[3-(trifluoromethyl)benzyl]-1-benzothiophen-7-yl]-1H-pyrazole-4-carboxylic acid obtained Reference Example 16 and glycinamide hydrochloride, the title compound was obtained as crystals. yield 87% melting point 222 - 225°C (ethanol-water).
$^1$H-NMR ($CDCl_3$) 5: 3.83 (2 H, d, J = 5.9 Hz), 4.43 (2 H, s), 7.14 (1 H, br s), 7.37 (1 H, s), 7.43 (1 H, br s), 7.53 - 7.77 (6 H, m), 7.90 (1 H, dd, J = 7.9, 0.8 Hz), 8.59 (1 H, t, J = 5.9 Hz), 9.28 (1 H, s).

Example 22

3-(trifluoromethyl)-1-[2-[3-(trifluoromethyl)benzyl] benzothiophen-7-yl]-1H-pyrazole-4-carboxamide

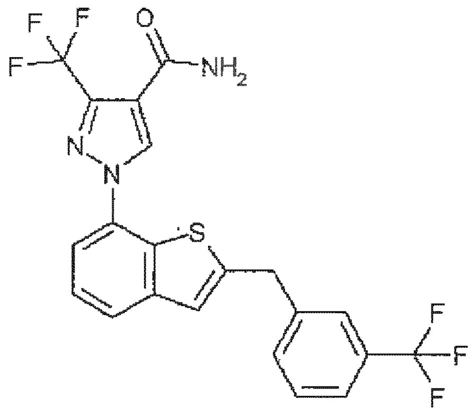

In the same manner as in Example 1 and using 3-(trifluoromethyl)-1-[2-[3-(trifluoromethyl)benzyl]-1-benzothi6phen-7-yl]-1H-pyrazole-4-carboxylic acid obtained. Reference Example 16 and HOBt ammonium salt, the title compound was obtained as crystals. yield 85% melting point 179 - 182°C (ethanol-water).
$^1$H-NMR ($CDCl_3$) 5: 4.31 (2 H, s), 6.00 (2 H, br s), 7.09 (1 H, s), 7.39 - 7.60 (6 H, m), 7.73 (1 H, dd, J = 7.2, 1.9 Hz), 8.61 - 8.68 (1 H, m).

Example 23

N-(2-amino-2-oxoethyl)-1-methyl-2-[2-[3-(trifluoromethyl)benzyl]-1-benzothiophen-7-yl]-1H-imidazole-5-carboxamide

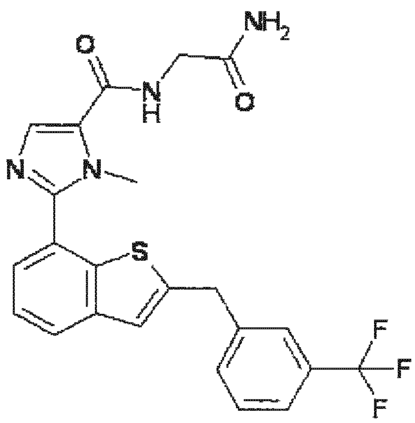

In the same manner as in Example 1 and using 1-methyl-2-[2-[3-(trifluoromethyl)benzyl]-1-benzothiophen-7-yl]-1H-imidazole-5-carboxylic acid obtained in Reference Example 21 and glycinamide hydrochloride, the title compound was obtained as crystals. yield 73% melting point 197 - 198°C (ethyl acetate-hexane)
$^1$H-NMR (DMSO-$d_6$) $\delta$: 3.78 (2 H, d, J=5.7 Hz), 3.86 (3 H, s), 4.38 (2 H, s), 7.05 (1 H, br s), 7.34 (1 H, s), 7.38 (1 H, br s), 7.45 - 7.53 (1 H, m), 7.53 - 7.69 (4 H, m), 7.71 (1 H, s), 7.78 (1 H, s), 7.85 - 7.92 (1 H, m), 8.60 (1 H, t, J=5.7 Hz).

Example 24

N-(2-amino-2-oxoethyl)-5-(1-methylethyl)-1-[2-[3-(trifluoromethyl)benzyl]-1-benzothiophen-7-yl]-1H-pyrazole-4-carboxamide

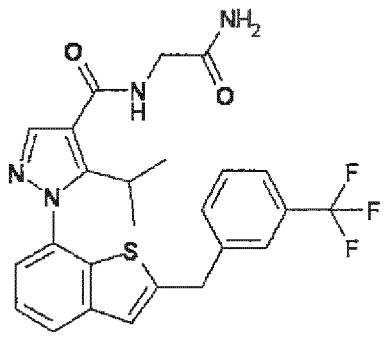

To a solution of 5-(1-methylethyl)-1-[2-[3-(trifluoromethyl)benzyl]-1-benzothiophen-7-yl]-1H-pyrazole-4-carboxylic acid synthesized in Reference Example 25 (20 mg, 0.045 mmol) in DMF (1 mL) were added WSC (13 mg, 0.067 mmol) and HOBt (10 mg, 0.067 mmol), and the mixture was stirred for 5 min at room temperature. To the mixture were added glycinamide hydrochloride (7.4 mg, 0.067 mmol) and diisopropylethylamine (17 mg, 0.14 mmol), and the mixture was stirred overnight. To the reaction mixture was added water, and the mixture was extracted with ethyl acetate. The organic layer was washed with water, saturated aqueous sodium hydrogen carbonate solution, saturated aqueous ammonium chloride solution and saturated brine, dried over anhydrous magnesium sulfate, and concentrated under reduced pressure. The residue was purified by silica gel column chromatography (ethyl acetate:methanol=95:5) to give the title compound (12 mg, yield 53%) as an oil.
$^1$H-NMR ($CDCl_3$) $\delta$: 1.31 (6 H, d, J = 7.1 Hz), 3.07-3.21 (1 H, m), 4.13 (2 H, d, J = 5.2 Hz), 4.25 (2 H, s), 6.56 (1 H, br s), 7.10 (1 H, s), 7.18-7.30 (2 H, m), 7.39-7.58 (6 H, m), 7.78 (1 H, d, J = 8.2 Hz), 7.90 (1 H, s).

Example 25

N-(2-amino-2-oxoethyl)-3-(1-methylethyl)-1-[2-[3-(trifluoromethyl)benzyl]-1-benzothiophen-7-yl]-1H-pyrazole-4-carboxamide

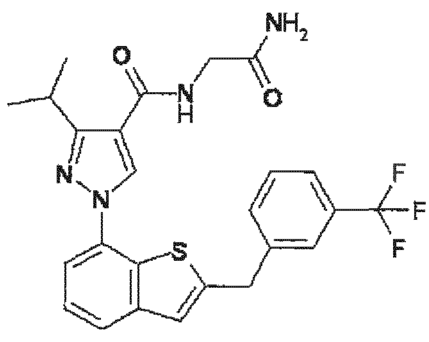

To a solution of (1-methylethyl)-1-[2-[3-(trifluoromethyl)benzyl]-1-benzothiophen-7-yl]-1H-pyrazole-4-carboxylic acid synthesized in Reference Example 24 (0.1 g, 0.23 mmol) in DMF (2 mL) were added WSC (65 mg, 0.34 mmol) and HOBt (52 mg, 0.34 mmol), and the mixture was stirred for 5 min at room temperature. To the mixture were added glycinamide hydrochloride (37 mg, 0.34 mmol) and diisopropylethylamine (87 mg, 0.68 mmol), and the mixture was stirred overnight. To the reaction mixture was added water, and the mixture was extracted with ethyl acetate. The organic layer was washed with water, saturated aqueous sodium hydrogen carbonate solution, saturated aqueous ammonium chloride solution and saturated brine, dried over anhydrous magnesium sulfate, and concentrated under reduced pressure. The crude crystals were recrystallized from ethanol-ethyl acetate to give the title compound (12 mg, yield 11%) as

crystals. melting point 189 - 190°C.
$^1$H-NMR (DMSO-$d_6$)δ: 1.28 (6 H, d, J = 6.8 Hz), 3.52-3.64 (1 H, m), 3.79 (2 H, d, J = 5.7 Hz), 4.40 (2 H, s), 7.07 (1 H, br s), 7.27 (1 H, s), 7.36 (1 H, br s), 7.45-7.53 (1 H, m), 7.54-7.69 (4 H, m), 7.71-7.78 (2 H, m), 8.18-8.30 (1 H, m), 9.02 (1 H, s).

Example 26

N-(2-hydroxyethyl)-3-(1-methylethyl)-1-[2-[3-(trifluoromethyl)benzyl]-1-benzothiophen-7-yl]-1H-pyrazole-4-carboxamide

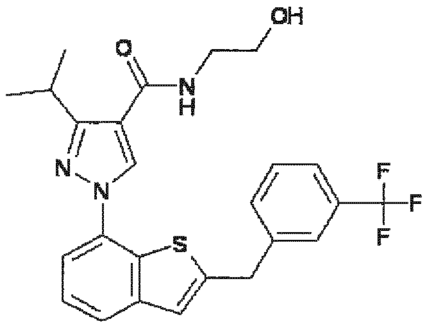

To a solution of 3-(1-methylethyl)-1-[2-[3-(trifluoromethyl)benzyl]-1-benzothiophen-7-yl]-1H-pyrazole-4-carboxylic acid synthesized in Reference Example 24 (0.1 g, 0.23 mmol) in DMF (3 mL) were added WSC (65 mg, 0.34 mmol) and HOBt (52 mg, 0.34 mmol), the mixture was stirred for 5 min at room temperature. 2-Aminoethanol (21 mg, 0.34 mmol) was added thereto, and the mixture was stirred overnight. To the reaction mixture was added water, and the mixture was extracted with ethyl acetate. The organic layer was washed with water, saturated aqueous sodium hydrogen carbonate solution, saturated aqueous ammonium chloride solution and saturated brine, dried over anhydrous magnesium sulfate, and concentrated under reduced pressure. The crude crystals were recrystallized from ethanol-diethyl ether to give the title compound (52 mg, yield 47%) as crystals. melting point 137 - 138°C.
$^1$H-NMR ($CDCl_3$) 5: 1.41 (6 H, d, J = 6.9 Hz), 3.07 (1 H, br s), 3.44-3.68 (3 H, m), 3.80 (2 H, t, J = 4.9 Hz), 4.27 (2 H, s), 6.49 (1 H, br s), 6.99 (1 H, s), 7.23-7.65 (6 H, m), 8.29 (1 H, s), 1 H, unconfirmed.

Example 27

N-(2-amino-2-oxoethyl)-5-cyclopropyl-1-[2-[3-(trifluoromethyl)benzyl]-1-benzothiophen-7-yl]-1H-pyrazole-4-carboxamide

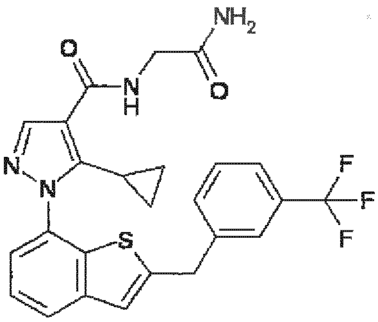

To a solution of 5-cyclopropyl-1-[2-[3-(trifluoromethyl)benzyl]-1-benzothiophen-7-yl]-1H-pyrazole-4-carboxylic acid synthesized in Reference Example 29 (5 mg, 0-011 mmol) in DMF (1 mL) were added WSC (3.3 mg, 0.017 mmol) and HOBt (2.6 mg, 0.017 mmol), and the mixture was stirred for 5 min at room temperature. To the mixture were added glycinamide hydrochloride (1.9 mg, 0.017 mmol) and diisopropylethylamine (4.4 mg, 0.034 mmol), and the mixture was

stirred overnight. To the reaction mixture was added water, and the mixture was extracted with ethyl acetate. The organic layer was washed with water, saturated aqueous sodium hydrogen carbonate solution, saturated aqueous ammonium chloride solution and saturated brine, dried over anhydrous magnesium sulfate, and concentrated under reduced pressure. The residue was purified by silica gel column chromatography (ethyl acetate:methanol=95:5) to give the title compound (4 mg, yield 73%) as an oil.
$^1$H-NMR ($CDCl_3$) δ: 0.46-0.60 (2 H, m), 0.84-1.03 (2 H, m), 1.92-2.09 (1 H, m), 4.20 (2 H, d, J = 4.9 Hz), 4.26 (2 H, s), 5.46 (1 H, br s), 6.10 (1 H, br s), 7.02-7.13 (2 H, m), 7.22-7.61 (6 H, m), 7.70-7.80 (1 H, m), 8.09 (1 H, s).

Example 28

N-(2-amino-2-oxoethyl)-3-cyclopropyl-1-[2-[3-(trifluoromethyl)benzyl]-1-benzothiophen-7-yl]-1H-pyrazole-4-carboxamide

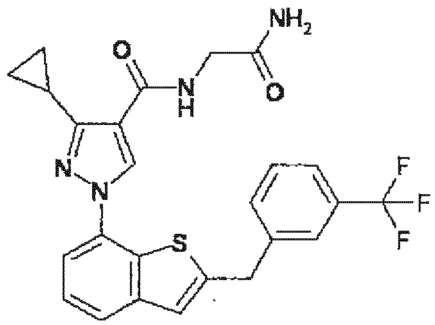

To a solution of 3-cyclopropyl-1-[2-[3-(trifluoromethyl)benzyl]-1-benzothiophen-7-yl]-1H-pyrazole-4-carboxylic acid synthesized in Reference Example 28 (0.1 g, 0.23 mmol) in DMF (3 mL) were added WSC (65 mg, 0.34 mmol) and HOBt (52 mg, 0.34 mmol), and the mixture was stirred for 5 min at room temperature. To the mixture were added glycinamide hydrochloride (37 mg, 0.34 mmol) and diisopropylethylamine (87 mg, 0.68 mmol), and the mixture was stirred overnight. To the reaction mixture was added water, and the mixture was extracted with ethyl acetate. The organic layer was washed with water, saturated aqueous sodium hydrogen carbonate solution, saturated aqueous ammonium chloride solution and saturated brine, dried over anhydrous magnesium sulfate, and concentrated under reduced pressure. The crude crystals were recrystallized from ethanol-ethyl acetate to give the title compound (88 mg, yield 78%) as crystals. melting point 239 - 240°C.
$^1$H-NMR (DMSO-$d_6$)δ: 0.90-1.02 (4 H, m), 2.59-2.75 (1 H, m), 3.82 (2 H, d, J = 5.7 Hz), 4.40 (2 H, s), 7.09 (1 H, br s), 7.26 (1 H, s), 7.40 (1 H, br s), 7.43-7.79 (7 H, m), 8.22 (1 H, t, J = 5.7 Hz), 8.99 (1 H, s).

Example 29

3-cyclopropyl-N-(2-hydroxyethyl)-1-[2-[3-(trifluoromethyl)benzyl]-1-benzothiophen-7-yl]-1H-pyrazole-4-carboxamide

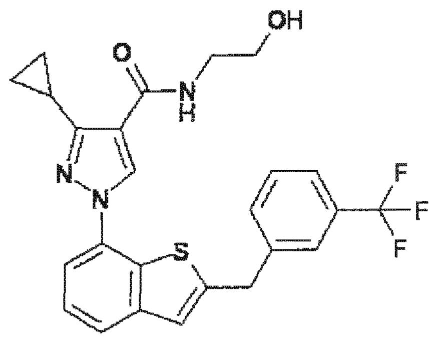

In the same manner as in Example 1 and using 3-cyclopropyl-1-[2-[3-(trifluoromethyl)benzyl]-1-benzothiophen-7-yl]-1H-pyrazole-4-carboxy acid obtained in Reference Example 28 and 2-aminoethanol, the title compound was obtained as crystals. yield 12% solid.
$^1$H-NMR ($CDCl_3$) δ: 1.03-1.21 (4 H, m), 2.14-2.27 (1 H, m), 2.36 (1 H, br s), 3.61-3.71 (2 H, m), 3.81-3.91 (2 H, m), 4.29 (2 H, s), 6.91 (1 H, br s), 7.03 (1 H, s), 7.15-7.65 (7 H, m), 8.43 (1 H, s).

Example 30

N-(2-amino-2-oxoethyl)-4-methyl-1-[2-[3-(trifluoromethyl)benzyl]-1-benzothiophen-7-yl]-1H-pyrazole-3-carboxamide

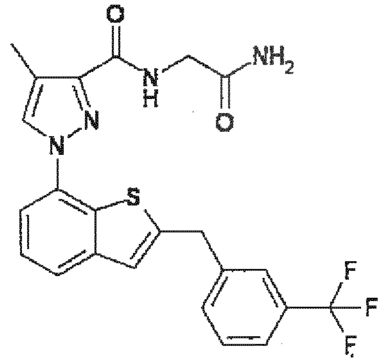

To a solution of ethyl 4-methyl-1-[2-[3-(trifluoromethyl)benzyl]-1-benzothiophen-7-yl]-1H-pyrazole-3-carboxylate obtained in Reference Example 30 (42 mg, 0.094 mmol) in THF (1 mL)-methanol (0.5 mL) was added 2N aqueous sodium hydroxide solution (165 μL, 0.331 mmol), and the mixture was stirred for 5 hr at room temperature. The reaction mixture was acidified with 1N hydrochloric acid, and the mixture was extracted with ethyl acetate. The organic layer was washed with saturated brine, dried over anhydrous sodium sulfate, and evaporated under reduced pressure to give crude 4-methyl-1-[2-[3-(trifluoromethyl)benzyl]-1-benzothiophen-7-yl]-1H-pyrazole-3-carboxylic acid (44 mg). A solution of the compound, glycinamide hydrochloride (12.5 mg, 0.113 mmol), WSC (21.6 mg, 0.113 mmol), HOBt (15.2 mg, 0.113 mmol) and N,N-diisopropylethylamine (19.4 μL, 0.113 mmol) in DMF (1.5 mL) was stirred overnight at room temperature. The reaction mixture was diluted with saturated aqueous sodium hydrogen carbonate solution, and extracted with ethyl acetate. The extract was washed with saturated brine, dried over anhydrous magnesium sulfate, and evaporated under reduced pressure. The residue was purified by silica gel column chromatography (ethyl acetate:methanol= 100:0→95:5), and the obtained crystals were recrystallized from hexane-ethyl acetate to give the title compound (30 mg, yield 68%) as crystals. melting point 177-178°C.
$^1$H-NMR ($CDCl_3$) δ : 2.45 (3 H, s), 4.16 (2 H, d, J = 6.0 Hz), 4.33 (2 H, s), 5.38 (1H, brs), 6.22 (1 H, br s), 7.08 (1 H, s), 7.38 - 7.71 (8 H, m), 7.88 (1 H, s).

Example 31

N-(2-amino-2-oxoethyl)-3-methyl-1-[2-[3-(trifluoromethyl)benzyl]-1-benzothiophen-7-yl]-1H-pyrazole-5-carboxamide

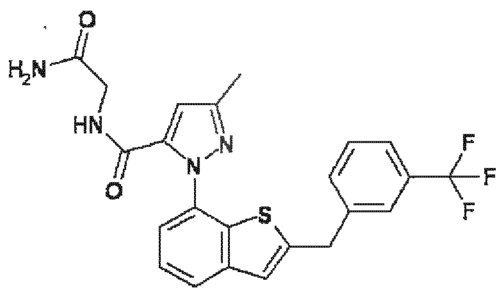

To a solution of 3-methyl-1-[2-[3-(trifluoromethyl)benzyl]-1-benzothiophen-7-yl]-1H-pyrazole-5-carboxylic acid

synthesized in Reference Example 33 (0.12 g, 0.29 mmol) in DMF (4 mL) were added WSC (0.17 g, 0.86 mmol) and HOBt (0.13 g, 0.86 mmol), and the mixture was stirred for 5 min at room temperature. To the mixture were added glycinamide hydrochloride (48 mg, 0.43 mmol) and diisopropylethylamine (0.11 g, 0.86 mmol), and the mixture was stirred overnight. To the reaction mixture was added water, and the mixture was extracted with ethyl acetate. The organic layer was washed with water, saturated aqueous sodium hydrogen carbonate solution, saturated aqueous ammonium chloride solution and saturated brine, dried over anhydrous magnesium sulfate, and concentrated under reduced pressure. The crude crystals were recrystallized from ethyl acetate-diisopropyl ether to give the title compound (79 mg, yield 58%) as crystals. melting point 88 - 89°C.
$^1$H-NMR ($CDCl_3$) δ: 2.37 (3 H, s), 3.90 (2 H, d, J = 4.9 Hz), 4.23 (2 H, s), 5.10 (1 H, br s), 5.81 (1 H, br s), 6.60 (1 H, br s), 6.65 (1 H, s), 7.07 (1 H, s), 7.20-7.57 (6 H, m), 7.71 (1 H, d, J = 8.0 Hz).

Example 32

3-methyl-1-[2-[3-(trifluoromethyl)benzyl]-1-benzothiophen-7-yl]-1H-pyrazole-5-carboxamide

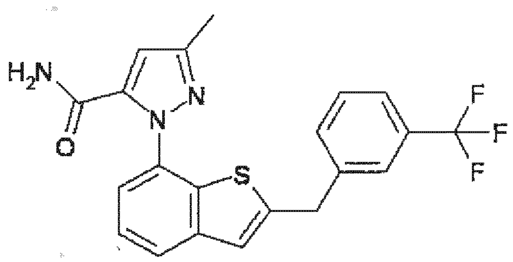

To a solution of 3-methyl-1-[2-[3-(trifluoromethyl)benzyl]-1-benzothiophen-7-yl]-1H-pyrazole-5-carboxylic acid synthesized in Reference Example 33 (0.12 g, 0.29 mmol) in DMF (4 mL) were added WSC (0.17 g, 0.86 mmol) and HOBt (0.13 g, 0.86 mmol), and the mixture was stirred for 5 min at room temperature. Aqueous ammonia (0.5 mL) was added thereto, and the mixture was stirred overnight. To the reaction mixture was added water, and the mixture was extracted with ethyl acetate. The organic layer was washed with water, saturated aqueous sodium hydrogen carbonate solution, saturated aqueous ammonium chloride solution and saturated brine, dried over anhydrous magnesium sulfate, and concentrated under reduced pressure. The crude crystals were recrystallized from ethyl acetate-hexane to give the title compound (69 mg, yield 58%) as crystals. melting point 126 - 127°C.
$^1$H-NMR ($CDCl_3$) 5: 2.31 (3 H, s), 4.10 (2 H, d, J = 6.0 Hz), 4.28 (2 H, s), 5.40 (1 H, br s), 6.24 (1 H, br s), 6.78 (1 H, s), 7.10 (1 H, s), 7.20-7.63 (4 H, m), 7.76 (1 H, d, J = 8.0 Hz).

Example 33

N-(2-hydroxyethyl)-3-methyl-1-[2-[3-(trifluoromethyl)benzyl]-1-benzothiophen-7-yl]-1H-pyrazole-5-carboxamide

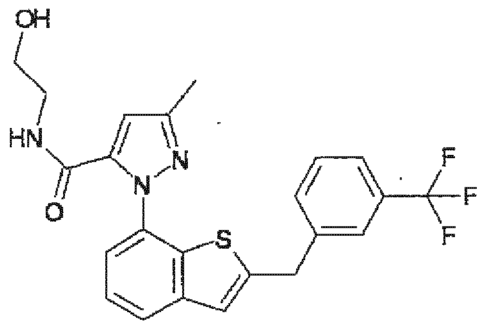

To a solution of 3-methyl-1-[2-[3-(trifluoromethyl)benzyl]-1-benzothiophen-7-yl]-1H-pyrazole-5-carboxylic acid synthesized in Reference Example 33 (0.12 g, 0.29 mmol) in DMF (3 mL) were added WSC (0.17 g, 0.86 mmol) and

HOBt (0.13 g, 0.86 mmol), and the mixture was stirred for 5 min at room temperature. 2-Aminoethanol (53 mg, 0.86 mmol) was added thereto, and the mixture was stirred overnight. To the reaction mixture was added water, and the mixture was extracted with ethyl acetate. The organic layer was washed with water, saturated aqueous sodium hydrogen carbonate solution, saturated aqueous ammonium chloride solution and saturated brine, dried over anhydrous magnesium sulfate, and concentrated under reduced pressure. The residue was purified by silica gel column chromatography (ethyl acetate:methanol=95:5) to give the title compound (20 mg, yield 15%) as an oil.
$^1$H-NMR ($CDCl_3$) δ: 2.11 (1 H, br s), 2.35 (3 H, s), 3.24-3.40 (2 H, m), 3.52 (2 H, br s), 4.23 (2 H, s), 6.57 (1 H, s), 7.06 (1 H, s), 7.21-7.56 (7 H, m), 7.69 (1 H, d, J = 7.7 Hz).

Example 34

N-(2-amino-2-oxoethyl)-5-methyl-1-[2-[3-(trifluoromethyl)benzyl]-1-benzothiophen-7-yl]-1H-pyrazole-3-carboxamide

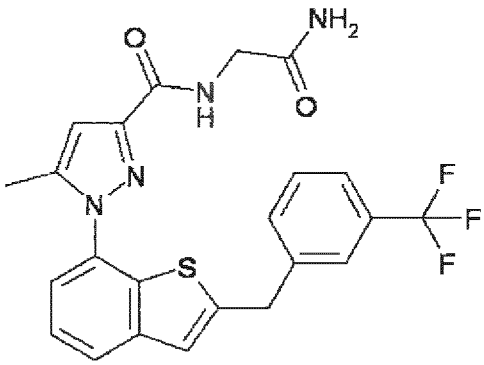

In the same manner as in Example 31 and using 5-methyl-1-[2-[3-(trifluoromethyl)benzyl]-1-benzothiophen-7-yl]-1H-pyrazole-3-carboxylic acid obtained in Reference Example 34, the title compound was obtained as crystals. yield 46% melting point 189 - 190°C.
$^1$H-NMR ($CDCl_3$) δ: 2.30 (3 H, s), 4.10 (2 H, d, J = 6.1 Hz), 4.28 (2 H, s), 5.32 (1 H, br s), 6.22 (1 H, br s), 6.78 (1 H, s), 7.11 (1 H, s), 7.37-7.58 (7 H, m), 7.77 (1 H, d, J = 8.0 Hz).

Example 35

N-(2-hydroxyethyl)-5-methyl-1-[2-[3-(trifluoromethyl)benzyl]-1-benzothiophen-7-yl]-1H-pyrazole-3-carboxamide

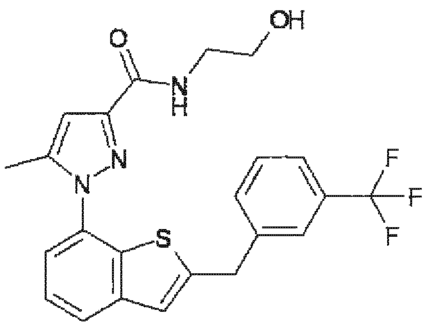

In the same manner as in Example 33 and using 5-methyl-1-[2-[3-(trifluoromethyl)benzyl]-1-benzothiophen-7-yl]-1H-pyrazole-3-carboxylic acid obtained in Reference Example 34, the title compound was obtained as crystals. yield 58% oil.
$^1$H-NMR ($CDCl_3$) δ: 0.89 (1 H, br s), 2.29 (3 H, s), 3.45-3.65 (2 H, m), 3.73-3.85 (2 H, m), 4.27 (2 H, s), 6. 77 (1 H, s), 7.11 (1 H, s), 7.19-7.34 (3 H, m), 7.36-7.59 (4 H, m), 7. 76 (1 H, d, J = 7.7 Hz).

Example 36

4-methyl-2-[2-[3-(trifluoromethyl)benzyl]-1-benzothiophen-7-yl]-1,3-thiazole-5-carboxamide

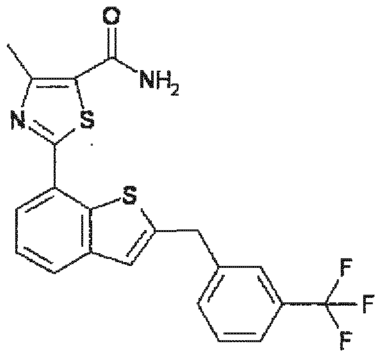

To a solution of ethyl 4-methyl-2-[2-[3-(trifluoromethyl)benzyl]-1-benzothiophen-7-yl]-1,3-thiazole-5-carboxylate obtained in Reference Example 37 (749 mg, 1.62 mmol) in THF (8 mL)-methanol (4 mL) was added 2N aqueous sodium hydroxide solution (2.84 mL, 5.67 mmol), and the mixture was stirred for 5 hr at 40°C, stirred for 2.5 days at room temperature, and stirred for 1.5 hr at 45°C. To the reaction mixture was added 2N aqueous sodium hydroxide solution (2.0 mL, 4.00 mmol), and the mixture was heated under reflux for 1 hr. To the mixture was added again 2N aqueous sodium hydroxide solution (2.0 mL, 4.00 mmol), and the mixture was heated under reflux for 7 hr. The reaction mixture was neutralized with 1N hydrochloric acid, and extracted with ethyl acetate. The organic layer was washed with saturated brine, dried over anhydrous sodium sulfate, and evaporated under reduced pressure to synthesize crude 4-methyl-2-[2-[3-(trifluoromethyl)benzyl]-1-benzyl]-1-benzothiophen-7-yl]-1,3-thiazole-5-carboxylic acid (708 mg). To a solution of the compound (200 mg) in DMF (4 mL) were WSC (106 mg, 0.554 mmol) and HOBt (75 mg, 0.554 mmol), and the mixture was stirred for 2 hr at room temperature. Aqueous ammonia (2.5 mL) was added thereto, and the mixture was stirred for 2.5 hr at room temperature. To the reaction mixture was added saturated aqueous sodium hydrogen carbonate solution, and the mixture was extracted with ethyl acetate. The organic layer was washed with saturated aqueous ammonium chloride solution, water and saturated brine, dried over anhydrous sodium sulfate, and evaporated under reduced pressure. The residue was purified by silica gel column chromatography (hexane:ethyl acetate= 60:40→10:90). The obtained crystals were recrystallized from hexane-ethyl acetate to give the title compound (130 mg, yield 66%, 2 steps) as crystals. melting point 206-207°C.
$^1$H-NMR ($CDCl_3$) δ : 2.84 (3 H, s), 4.33 (2 H, s), 5.67 (2 H, br s), 7.08 (1 H, t, J = 1.0 Hz), 7.36 -7.62 (5 H, m), 7.80 (2 H, td, J = 7.4, 1.1 Hz).

Example 37

N-(2-amino-2-oxoethyl)-4-methyl-2-[2-[3-(trifluoromethyl)benzyl]-1-benzothiophen-7-yl]-1,3-thiazole-5-carboxamide

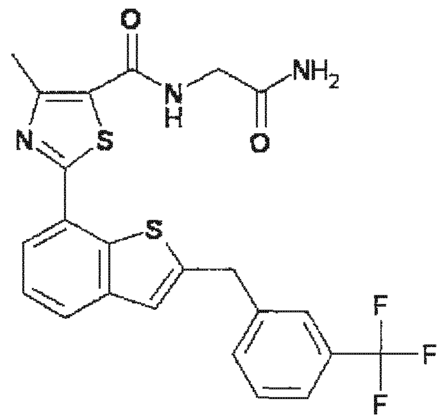

A solution of the crude 4-methyl-2-[2-[3-(trifluoromethyl)benzyl]-1-benzothiophen-7-yl]-1,3-thiazole-5-carboxylic acid obtained in Example 36 (199 mg), glycinamide hydrochloride (61 mg, 0.551 mmol), WSC (106 mg, 0.551 mmol), HOBt (74 mg, 0.551 mmol) and N,N-diisopropylethylamine (95.0 μL, 0.551 mmol) in DMF (4.6 mL) was stirred for 7 hr at room temperature. The reaction mixture was diluted with saturated aqueous 'sodium hydrogen carbonate solution, and extracted with ethyl acetate. The extract was washed with saturated brine, dried over anhydrous magnesium sulfate, and evaporated under reduced pressure. The residue was purified by silica gel column chromatography (ethyl acetate: methanol= 100:0→90:10). The obtained crystals were recrystallized from hexane-ethanol to give the title compound (127 mg, yield 56%, 2 steps) as crystals. melting point 211-212°C.
$^1$H-NMR (DMSO-$d_6$) δ : 2.67 (3 H, s), 3.79 (2 H, d, J = 5.8 Hz), 4.43 (2 H, s), 7.06 (1 H, s), 7.32 (1 H, s), 7.41 (1 H, br s), 7.45 - 7.70 (4 H, m), 7.73 (1 H, s), 7.84 - 7.98 (2 H, m), 8.38 (1 H, t, J = 5.4 Hz).

Example 38

N-(2-cyanoethyl)-4-methyl-2-[2-[3-(trifluoromethyl)benzyl]-1-benzothiophen-7-yl]-1,3-thiazole-5-carboxamide

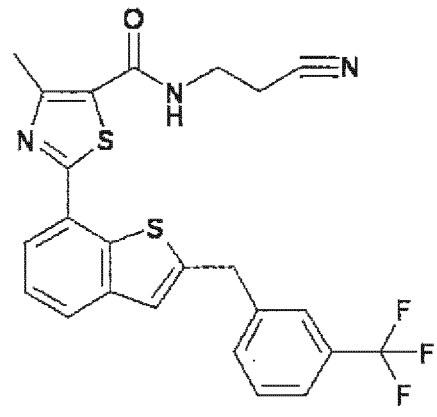

In the same manner as in Example 37 and using the crude 4-methyl-2-[2-[3-(trifluoromethyl)benzyl]-1-benzothiophen-7-yl]-2,3-thiazole-5-carboxylic acid obtained in Example 36 and 3-aminopropionitrile, the title compound was obtained as crystals. yield 67%. melting point 164-165°C.
$^1$H-NMR ($CDCl_3$) δ : 2.78 (2 H, t, J = 6.3 Hz), 2.84 (3 H, s), 3.73 (2 H, q, J = 6.3 Hz), 4.34 (2 H, s), 6.23 (1 H, s), 7.08 (1 H, s), 7.38 - 7.56 (4 H, m), 7.58 (1 H, s), 7.81 (2 H, ddd, J = 7.7, 5.4, 1.0 Hz).

Example 39

N-(2-hydroxyethyl)-4-methyl-2-[2-[3-(trifluoromethyl)benzyl]-1-benzothiophen-7-yl]-1,3-thiazole-5-carboxamide

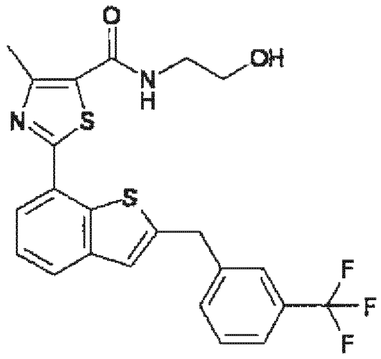

In the same manner as in Example 37 and using the crude 4-methyl-2-[2-[3-(trifluoromethyl)benzyl]-1-benzothiophen-7-yl]-1,3-thiazole-5-carboxylic acid obtained in Example 36 and 2-aminoethanol, the title compound was obtained as crystals. yield 82%. melting point 147-148°C.
$^1$H-NMR ($CDCl_3$) $\delta$ : 2.21 (1 H, t, J = 4.9 Hz), 2.83 (3 H, s), 3.64 (2 H, q, J = 5.1 Hz), 3.87 (2 H, q, J = 4.9 Hz), 4.33 (2 H, s), 6.29 (1 H, br s), 7.08 (1 H, t, J = 1.1 Hz), 7.36 - 7.63 (5 H, m), 7.80 (2 H, ddd, J = 7.4, 6.7, 1.0 Hz).

Example 40

N-(2-methoxyethyl)-4-methyl-2-[2-[3-(trifluoromethyl)benzyl]-1-benzothiophen-7-yl]-1,3-thiazole-5-carboxamide

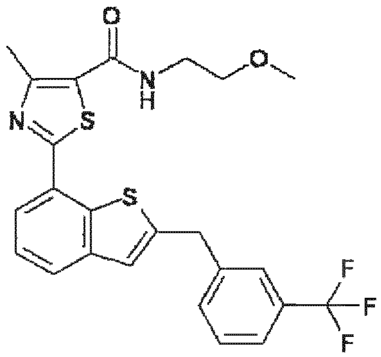

In the same manner as in Example 37 and using the crude 4-methyl-2-[2-[3-(trifluoromethyl)benzyl]-1-benzothiophen-7-yl]-1,3-thiazole-5-carboxylic acid obtained in Example 36 and 2-methoxyethylamine, the title compound was obtained as crystals. yield 56%. melting point 123-124°C. $^1$H-NMR ($CDCl_3$) $\delta$ : 2.81 (3 H, s), 3.42 (3 H, s), 3.53 - 3.61 (2 H, m), 3.61 - 3.71 (2 H, m), 4.33 (2 H, s), 6.23 (1 H, br s), 7.05 - 7.11 (1 H, m), 7.36 - 7.62 (5 H, m), 7.74 - 7.84 (2 H, m).

Example 41

4-methyl-2-[2-[3-(trifluoromethyl)benzyl]-2H-indazol-4-yl]-1,3-thiazole-5-carboxamide

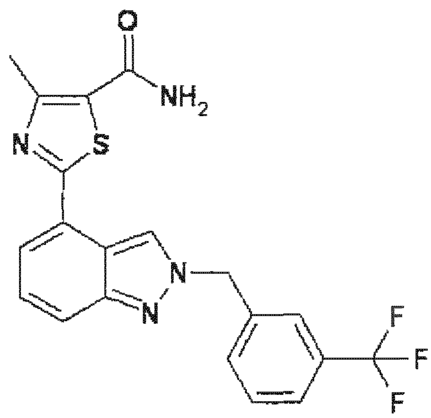

In the same manner as in Example 36, crude 4-methyl-2-[2-[3-(trifluoromethyl)benzyl]-2H-indazol-4-yl]-1,3-thiazole-5-carboxylic acid was synthesized from ethyl 4-methyl-2-[2-[3-(trifluoromethyl)benzyl]-2H-indazol-4-yl]-1,3-thiazole-5-carboxylate obtained in Reference Example 40, and using the obtained crude product and 28% aqueous ammonia solution, the title compound was obtained as crystals. yield 73%. melting point 202-203°C.
$^{1}$H-NMR ($CDCl_3$) δ : 2.81 (3 H, s), 5.66 (2 H, br s), 5.72 (2 H, s), 7.36 (1 H, dd, J = 8.7, 7.0 Hz), 7.44 - 7.50 (2 H, m), 7.56 - 7.64 (2 H, m), 7.67 (1 H, d, J = 7.1 Hz), 7.86 (1 H, d, J = 8.5 Hz).

Example 42

N-(2-amino-2-oxoethyl)-4-methyl-2-[2-[3-(trifluoromethyl)benzyl]-2H-indazol-4-yl]-1,3-thiazole-5-carboxamide

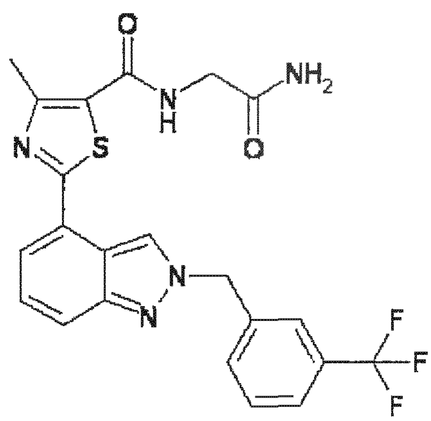

In the same manner as in Example 37 and using the crude 4-methyl-2-[2-[3-(trifluoromethyl)benzyl]-2H-indazol-4-yl]-1,3-thiazole-5-carboxylic acid obtained in Example 41 and glycinamide hydrochloride, the title compound was obtained as crystals. yield 8%. melting point 179-181°C. $^{1}$H-NMR (DMSO-$d_6$)δ: 2.71 (3 H, s), 3.80 (2 H, d, J = 5.5 Hz), 5.87 (2 H, s), 7.06 (1 H, br s), 7.37 (1 H, dd, J = 8.5, 7.1 Hz), 7.41 (1 H, br s), 7.56 - 7.65 (2 H, m), 7.65 - 7.75 (2 H, m), 7.76 - 7.87 (2 H, m), 8.28 - 8-41 (1 H, m), 9.09 (1 H, d, J = 0.8 Hz).

Example 43

N-(2-hydroxyethyl)-4-methyl-2-[2-[3-(trifluoromethyl)benzyl]-2H-indazol-4-yl]-1,3-thiazole-5-carboxamide

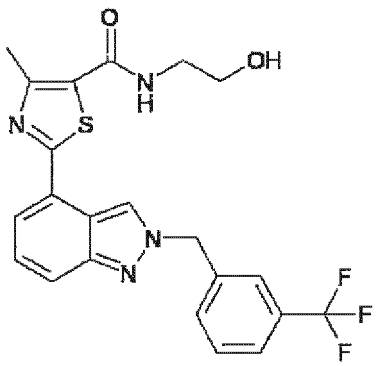

In the same manner as in Example 37 and using the crude 4-methyl-2-[2-[3-(trifluoromethyl)benzyl]-2H-indazol-4-yl]-1,3-thiazole-5-carboxylic acid obtained in Example 41 and 2-aminoethanol, the title compound was obtained as crystals. yield 61%. melting point 187-188°C.
$^1$H-NMR ($CDCl_3$) δ : 2.26 (1 H, t, J = 4.9 Hz), 2.80 (3 H, s), 3.59 - 3.70 (2 H, m), 3.87 (2 H, q, J = 4.8 Hz), 5.71 (2 H, s), 6.28 (1 H, br s), 7.35 (1 H, dd, J = 8.5, 7.1 Hz), 7.43 - 7.53 (2 H, m), 7.54 - 7.69 (3 H, m), 7.85 (1 H, d, J = 8.8 Hz), 8.78 (1 H, d, J = 0.8 Hz).

Example 44

4-methyl-2-[2-[3-(trifluoromethyl)benzyl]-1-benzofuran-4-yl]-1,3-thiazole-5-carboxamide

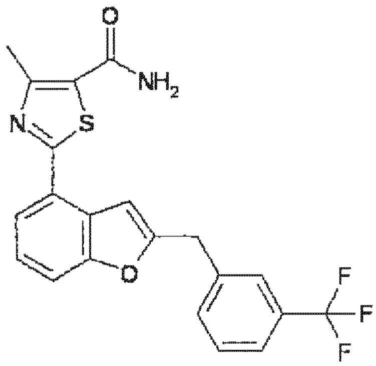

In the same manner as in Example 36, crude 4-methyl-2-[2-[3-(trifluoromethyl)benzyl]-1-benzofuran-4-yl]-1,3-thiazole-5-carboxylic acid was synthesized from ethyl 4-methyl-2-[2-[3-(trifluoromethyl)benzyl]-1-benzofuran-4-yl]-1,3-thiazole-5-carboxylate obtained in Reference Example 39, and using the obtained crude product and 28% aqueous ammonia solution, the title compound was obtained as crystals. yield 61%. melting point 191-192°C.
$^1$H-NMR ($CDCl_3$) δ : 2.81 (3 H, s), 4.24 (2 H, s), 5.69 (2 H, br s), 7.24 - 7.33 (3 H, m), 7.40 - 7.57 (3 H, m), 7.60 (1 H, s), 7.74 (1 H, dd, J = 7.4, 0.8 Hz).

Example 45

N-(2-amino-2-oxoethyl)-4-methyl-2-[2-[3-(trifluoromethyl)benzyl]-1-benzofuran-4-yl]-1,3-thiazole-5-carboxamide

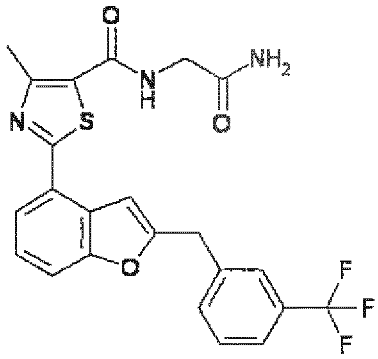

In the same manner as in Example 37 and using the crude 4-methyl-2-[2-[3-(trifluoromethyl)benzyl]-1-benzofuran-4-yl]-1,3-thiazole-5-carboxylic acid obtained in Example 44 and glycinamide hydrochloride, the title compound was obtained as crystals. yield 51%. melting point 179-181°C. $^1$H-NMR ($CDCl_3$)$\delta$ : 2.82 (3 H, s), 4.17 (2 H, d, J = 4.9 Hz), 4.24 (2 H, s), 5.48 (1 H, br s), 5.87 (1 H, br s), 6.62 (1 H, br s), 7.26 - 7.34 (2 H, m), 7.41 - 7.57 (4 H, m), 7.60 (1 H, s), 7.75 (1 H, dd, J = 7.7, 0.8 Hz).

Example 46

N-(2-hydroxyethyl)-4-methyl-2-[2-[3-(trifluoromethyl)benzyl]-1-benzofuran-4-yl]-1,3-thiazole-5-carboxamide

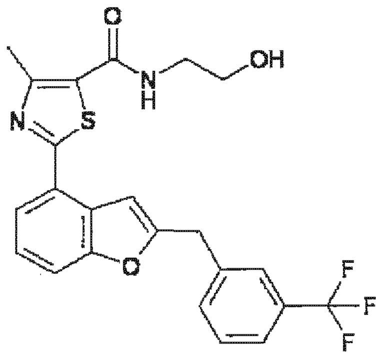

In the same manner as in Example 37 and using the crude 4-methyl-2-[2-[3-(trifluoromethyl)benzyl]-1-benzofuran-4-yl]-1,3-thiazole-5-carboxylic acid obtained in Example 44 and 2-aminoethanol, the title compound was obtained as crystals. yield 55%. melting point 146-147°C.
$^1$H-NMR ($CDCl_3$) $\delta$ : 2.25 (1H, td, J = 4.9, 1.8 Hz), 2.80 (3 H, s), 3.60 - 3.68 (2 H, m), 3.87 (2 H, q, J = 5.1 Hz), 4.23 (2 H, s), 6.28 (1 H, br s), 7.27 - 7.34 (2 H, m), 7.40 - 7.57 (4 H, m), 7.60 (1 H, s), 7.73 (1 H, d, J = 7.7 Hz).

Example 47

N-(2-amino-2-oxoethyl)-1,4-dimethyl-2-[2-[3-(trifluoromethyl)benzyl]-1-benzothiophen-7-yl]-1H-imidazole-5-carboxamide

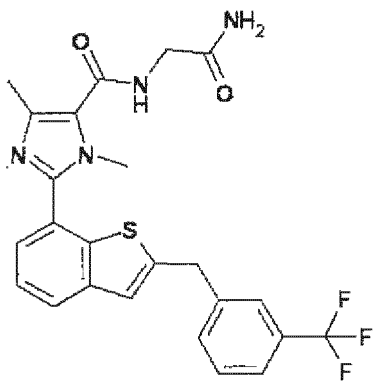

In the same manner as in Example 1 and using 1,4-dimethyl-2-[2-[3-(trifluoromethyl)benzyl]-1-benzothiophen-7-yl]-1H-imidazole-5-carboxylic acid obtained in Reference Example 43 and glycinamide hydrochloride, the title compound was obtained as crystals. yield 67% melting point 169 - 170°C (ethyl acetate-hexane)
$^1$H-NMR (DMSO-$d_6$) $\delta$: 2.37 (3 H, s), 3.70 (3 H, s), 3.83 (2 H, d, J=6.1 Hz), 4.38 (2 H, s), 7.05 (1 H, br s), 7.32 (1 H, s), 7.40 (1 H, br s), 7.43 - 7.68 (5 H, m), 7.71 (1 H, s), 7.82 - 7.90 (1 H, m), 8.09 (1 H, t, J=6.1 Hz).

Example 48

1,4-dimethyl-2-[2-[3-(trifluoromethyl)benzyl]-1-benzothiophen-7-yl]-1H-imidazole-5-carboxamide

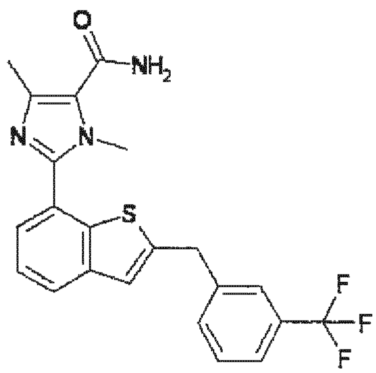

In the same manner as in Example 1 and using 1,4-dimethyl-2-[2-[3-(trifluoromethyl)benzyl]-1-benzothiophen-7-yl]-1H-imidazole-5-carboxylic acid obtained in Reference Example 43 and 28% aqueous ammonia, the title compound was obtained as crystals. yield 74% melting point 161 - 162°C (ethyl acetate-hexane)
$^1$H-NMR (DMSO-$d_6$) $\delta$: 2.34 (3 H, s), 3.70 (3 H, s), 4.38 (2 H, s), 7.31 (1 H, s), 7.42 - 7.67 (7 H, m), 7.71 (1 H, s), 7.86 (1 H, dd, J=7.4, 1.7 Hz).

Example 49

N-(2-amino-2-oxoethyl)-1,5-dimethyl-2-[2-[3-(trifluoromethyl)benzyl]-1-benzothiophen-7-yl]-1H-imidazole-4-carboxamide

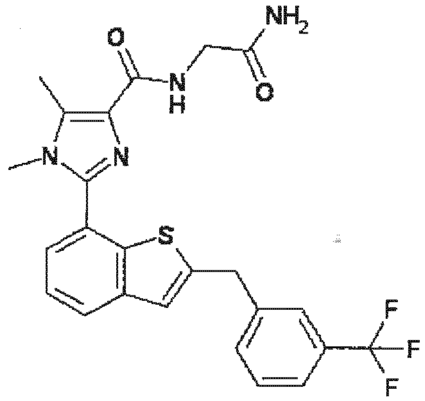

In the same manner as in Example 1 and using 1,5-dimethyl-2-[2-[3-(trifluoromethyl)benzyl]-1-benzothiophen-7-yl]-1H-imidazole-4-carboxylic acid obtained in Reference Example 46 and glycinamide hydrochloride, the title compound was obtained as crystals. yield 48% melting point 248 - 249°C (ethyl acetate-hexane)
$^1$H-NMR (DMSO-$d_6$) δ: 2.57 (3 H, s), 3.57 (3 H, s), 3.84 (2 H, d, J=5.7 Hz), 4.38 (2 H, s), 7.09 (1 H, br s), 7.31 (1 H, s), 7.41 (1 H, br s), 7.44 - 7.69 (5 H, m), 7.71 (1 H, s), 7.79 (1 H, t, J=5.7 Hz), 7.87 (1 H, dd, J=7.5, 1.5 Hz).

Example 50

1,5-dimethyl-2-[2-[3-(trifluoromethyl)benzyl]-1-benzothiophen-7-yl]-1H-imidazole-4-carboxamide

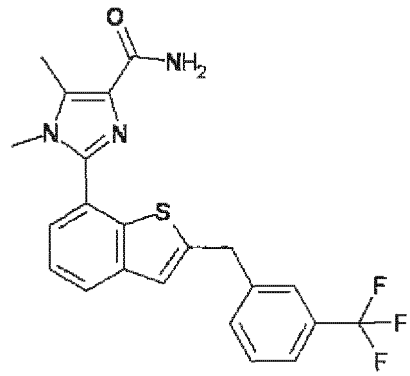

In the same manner as in Example 1 and using 1,5-dimethyl-2-[2-[3-(trifluoromethyl)benzyl]-1-benzothiophen-7-yl]-1H-imidazole-4-carboxylic acid obtained in Reference Example 46 and 28% aqueous ammonia, the title compound was obtained as crystals. yield 40% melting point 202 - 203°C (ethyl acetate-hexane)
$^1$H-NMR (DMSO-$d_6$) δ: 2.56 (3 H, s), 3.58 (3 H, s), 4.38 (2 H, s), 7.01 (1 H, br s), 7.09 (1 H, br s), 7.31 (1 H, s), 7.44 - 7.68 (5 H, m), 7.71 (1 H, s), 7.82 - 7.89 (1 H, m).

Example 51

N-(2-methoxyethyl)-3,5-dimethyl-1-[2-[3-(trifluoromethyl)benzyl]-1-benzothiophen-7-yl]-1H-pyrazole-4-carboxamide

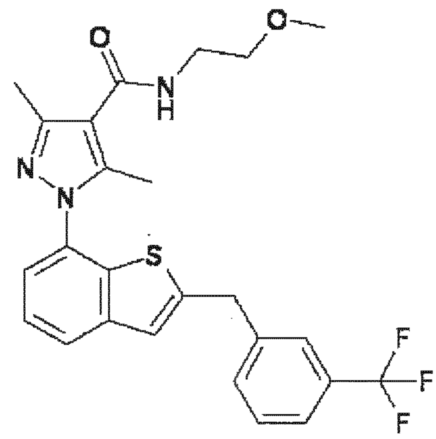

In the same manner as in Example 1 and using 3,5-dimethyl-1-[2-[3-(trifluoromethyl)benzyl]-1-benzothiophen-7-yl]-1H-pyrazole-4-carboxylic acid obtained in Reference Example 48 and 2-methoxyethanamine, the title compound was obtained as an oil. yield 85%.
$^1$H-NMR ($CDCl_3$) $\delta$: 2.43 (3 H, s), 2.50 (3 H, s), 3.40 (3 H, s), 3.53 - 3.68 (4 H, m), 4.25 (2 H, s), 6.06 - 6.16 (1 H, m), 7.05 - 7.09 (1 H, m), 7.18 (1 H, dd, J = 7.7, 0.8 Hz), 7.35 - 7.54 (5 H, m), 7.71 (1 H, dd, J = 8.0, 0.8 Hz).

Example 52

N-(2-hydroxyethyl)-3,5-dimethyl-1-[2-[3-(trifluoromethyl)benzyl]-1-benzothiophen-7-yl]-1H-pyrazole-4-carboxamide

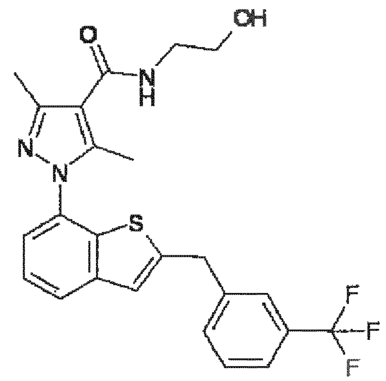

In the same manner as in Example 1 and using 3,5-dimethyl-1-[2-[3-(trifluoromethyl)benzyl]-1-benzothiophen-7-yl]-1H-pyrazole-4-carboxylic acid obtained in Reference Example 48 and 2-aminoethanol, the title compound was obtained as crystals. yield 70% melting point 137 - 139°C (hexane-ethyl acetate).
$^1$H-NMR ($CDCl_3$) $\delta$: 2.44 (3 H, s), 2.52 (3 H, s), 2.63 (1 H, t, J = 4.9 Hz), 3.59 - 3.68 (2 H, m), 3.81 - 3.89 (2 H, m), 4.26 (2 H, s), 6.15 (1 H, br s), 7.09 (1 H, t, J = 1.1 Hz), 7.19 (1 H, dd, J = 7.5, 0.8 Hz), 7.38 - 7.55 (5 H, m), 7.74 (1 H, dd, J = 7.9, 1.1 Hz).

Example 53

N-(2-amino-2-oxoethyl)-3,5-dimethyl-1-[2-[3-(trifluoromethyl)benzyl]-1-benzothiophen-7-yl]-1H-pyrazole-4-carboxamide

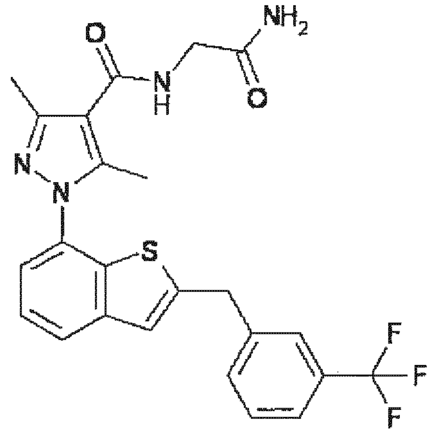

In the same manner as in Example 1 and using 3,5-dimethyl-1-[2-[3-(trifluoromethyl)benzyl]-1-benzothiophen-7-yl]-1H-pyrazole-4-carboxylic acid obtained in Reference Example 48 and glycinamide hydrochloride, the title compound was obtained as crystals. yield 63% melting point 184 - 187°C (hexane-ethyl acetate).
$^1$H-NMR ($CDCl_3$) δ: 2.46 (3 H, s), 2.56 (3 H, s), 4.18 (2 H, d, J = 4.9 Hz), 4.26 (2 H, s), 5.50 (1 H, br s), 6.11 (1 H, br s), 6.50 (1 H, t, J = 4.7 Hz), 7.09 (1 H, t, J = 1.1 Hz), 7.20 (1 H, dd, J = 7.5, 1.1 Hz), 7.39 - 7.55 (5 H, m), 7.74 (1 H, dd, J = 7.9, 0.8 Hz).

Example 54

3,5-dimethyl-1-[2-[3-(trifluoromethyl)benzyl]-1-benzothiophen-7-yl]-1H-pyrazole-4-carboxamide

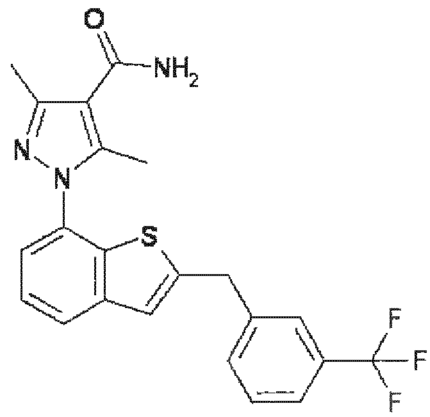

In the same manner as in Example 1 and using 3,5-dimethyl-1-[2-[3-(trifluoromethyl)benzyl]-1-benzothiophen-7-yl]-1H-pyrazole-4-carboxylic acid obtained in Reference Example 48 and HOBt ammonium salt, the title compound was obtained as crystals. yield 59% melting point 171 - 175°C (hexane-ethyl acetate).
$^1$H-NMR ($CDCl_3$) δ: 2.46 (3 H, s), 2.54 (3 H, s), 4.26 (2 H, s), 5.57 (2 H, br s), 7.09 (1 H, t, J = 1.1 Hz), 7.20 (1 H, dd, J = 7.5, 1.1 Hz), 7.38 - 7.56 (5 H, m), 7.74 (1 H, dd, J = 7.9, 0.8 Hz).

Example 55

N-(2-amino-2-oxoethyl)-1-[2-(3-chloro-5-fluorobenzyl)-1-benzothiophen-7-yl]-3,5-dimethyl-1H-pyrazole-4-carboxamide

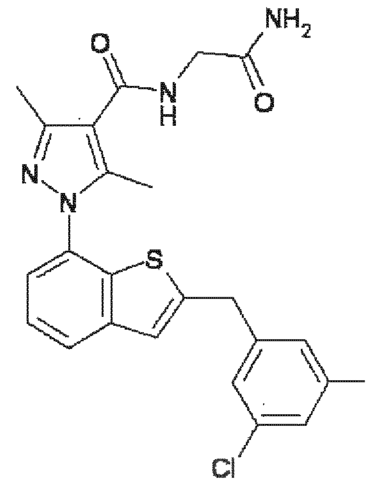

In the same manner as in Example 1 and using 1-[2-(3-chloro-5-fluorobenzyl)-1-benzothiophen-7-yl]-3,5-dimethyl-1H-pyrazole-4-carboxylic acid obtained in Reference Example 49 and glycinamide hydrochloride, the title compound was obtained as crystals. yield 30% melting point 132 - 133°C (ethyl acetate-hexane).
$^1$H-NMR (DMSO-$d_6$) $\delta$. 2.34 (3 H, s), 2.36 (3 H, s), 3.81 (2 H, d, J=6.1 Hz), 4.29 (2 H, s), 7.03 (1 H, br s), 7.17 - 7.26 (1 H, m), 7.27 - 7.41 (5 H, m), 7.50 (1 H, t, J=7.8 Hz), 7.69 (1 H, t, J=5.7 Hz), 7.88 (1 H, d, J=8.0 Hz).

Example 56

1-[2-(3-chloro-5-fluorobenzyl)-1-benzothiophen-7-yl]-N-(2-hydroxyethyl)-3,5-dimethyl-1H-pyrazole-4-carboxamide

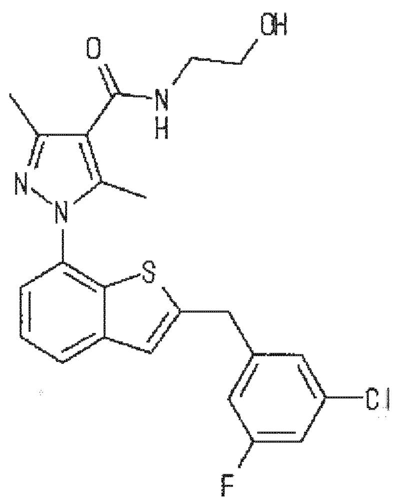

A mixture of 1-[2-(3-chloro-5-fluorobenzyl)-1-benzothiophen-7-yl]-3,5-dimethyl-1H-pyrazole-4-carboxylic acid obtained in Reference Example 49 (200 mg, 0.48 mmol), 2-aminoethanol (32 $\mu$L, 0.53 mmol), WSC (110 mg, 0.57 mmol) and HOBt (78 mg, 0.58 mmol) in DMF (4 mL) was reacted for 18 hr at room temperature. To the reaction mixture was added water, and the mixture was extracted with ethyl acetate. The organic layer was dried over magnesium sulfate, and evaporated under reduced pressure. The obtained residue was purified by column chromatography, and crystallized to give the title compound (77 mg, yield 35%) as crystals. melting point 148 - 149°C (ethyl acetate-hexane).
$^1$H-NMR (DMSO-$d_6$) $\delta$: 2.29 (3 H, s), 2.33 (3 H, s), 3.24 - 3.36 (2 H, m), 3.50 (2 H, q, J=5.9 Hz), 4.29 (2 H, s), 4.69 (1 H, t, J=5.3 Hz), 7.21 (1 H, d, J=9.5 Hz), 7.26 - 7.40 (4 H, m), 7.49 (1 H, t, J=7.8 Hz), 7.60 (1 H, t, J=5.5 Hz), 7.87 (1 H, d, J=7. 6 Hz).

Example 57

1-[2-(3-chloro-5-fluorobenzyl)-1-benzothiophen-7-yl]-N-(2-methoxyethyl)-3,5-dimethyl-1H-pyrazole-4-carboxamide

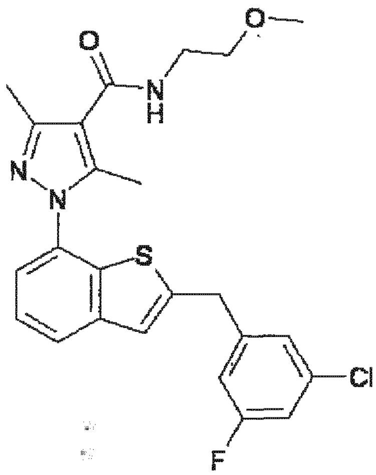

In the same manner as in Example 1 and using 1-[2-(3-chloro-5-fluorobenzyl)-1-benzothiophen-7-yl]-3,5-dimethyl-1H-pyrazole-4-carboxylic acid obtained in Reference Example 49 and 2-methoxyethanamine, the title compound was obtained as a solid. yield 18% melting point 138 - 139°C (ethyl acetate-hexane).
$^1$H-NMR (DMSO-$d_6$) $\delta$: 2.29 (3 H, s), 2.32 (3 H, s), 3.28 (3 H, s), 3.35 - 3.50 (4 H, m), 4.29 (2 H, s), 7.21 (1 H, dt, J=9.5, 1.7 Hz), 7.27 - 7.39 (4 H, m), 7.49 (1 H, t, J=7.8 Hz), 7.71 (1 H, t, J=5.3 Hz), 7.87 (1 H, d, J=7.2 Hz).

Example 58

N-(2-amino-2-oxoethyl)-1-[4-fluoro-2-[3-(trifluoromethyl)benzyl]-1-benzothiophen-7-yl]-3,5-dimethyl-1H-pyrazole-4-carboxamide

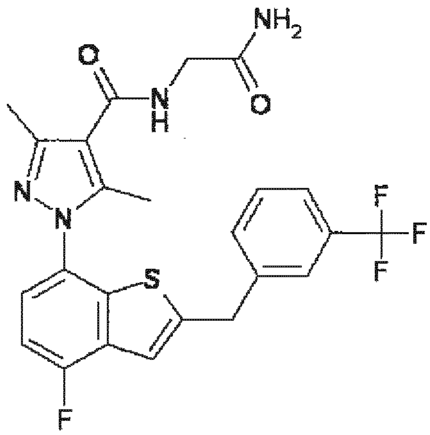

To a solution of 1-[4-fluoro-2-[3-(trifluoromethyl)benzyl]-1-benzothiophen-7-yl]-3,5-dimethyl-1H-pyrazole-4-carboxylic acid synthesized in Reference Example 53 (0.1 g, 0.23 mmol) in DMF (3 mL) were added WSC (65 mg, 0.34 mmol) and HOBt (52 mg, 0.34 mmol), and the mixture was stirred for 5 min at room temperature. Glycinamide hydrochloride (37 mg, 0.34 mmol) and diisopropylethylamine (87 mg, 0.68 mmol) were added thereto, and the mixture was stirred overnight. To the reaction mixture was added water, and the mixture was extracted with ethyl acetate. The organic layer was washed with water, saturated aqueous sodium hydrogen carbonate solution, saturated aqueous ammonium chloride solution and saturated brine, dried over anhydrous magnesium sulfate, and concentrated under reduced pressure. The crude crystals were recrystallized from ethanol-ethyl acetate to give the title compound (51 mg, yield 45%) as crystals. melting point 154 - 155°C.
$^1$H-NMR ($CDCl_3$) δ: 2.43 (3 H, s), 2.54 (3 H, s), 4.17 (2 H, d, J = 4.7 Hz), 4.26 (2 H, s), 5.55 (1 H, br s), 6.16 (1 H, br s), 6.52 (1 H, br s), 7.02-7.22 (3 H, m), 7.39-7.58 (4 H, m).

Example 59

N-(2-amino-2-oxoethyl)-1-[2-(3-chloro-5-fluorobenzyl)-4-fluoro-1-benzothiophen-7-yl]-3,5-dimethyl-1H-pyrazole-4-carboxamide

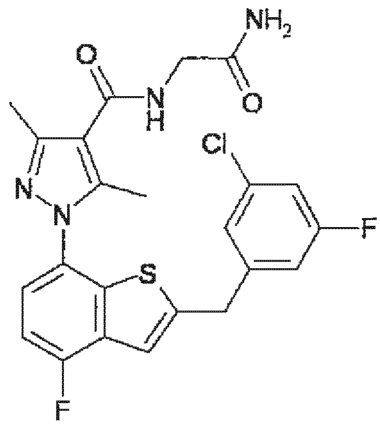

In the same manner as in Example 58 and using 1-[2-(3-chloro-5-fluorobenzyl)-4-fluoro-1-benzothiophen-7-yl]-3,5-dimethyl-1H-pyrazole-4-carboxylic acid obtained in Reference Example 55, the title compound was obtained as crystals. yield 29% solid. melting point 135 - 136°C.
$^1$H-NMR ($CDCl_3$) δ: 2.44 (3 H, s), 2.55 (3 H, s), 4.13-4.23 (4 H, m), 5.71 (1 H, br s), 6.22 (1 H, br s), 6.55 (1 H, t, J = 4.5 Hz), 6.87 (1 H, d, J = 9.0 Hz), 6.99 (1 H, dt, J = 8.3, 2.1 Hz), 7.03-7.24 (4 H, m).

Example 60

1-[2-(3-chloro-5-fluorobenzyl)-4-fluoro-1-benzothiophen-7-yl]-N-(2-hydroxyethyl)-3,5-dimethyl-1H-pyrazole-4-carboxamide

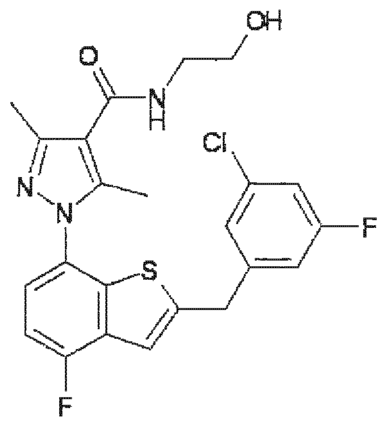

In the same manner as in Example 1 and using 1-[2-(3-chloro-5-fluorobenzyl)-4-fluoro-1-benzothiophen-7-yl]-3,5-dimethyl-1H-pyrazole-4-carboxylic acid obtained in Reference Example 55, the title compound was obtained as crystals. yield 33% solid. melting point 119 - 120°C.

$^1$H-NMR ($CDCl_3$) δ: 2.41 (3 H, s), 2.49-2.55 (4 H, m), 3.59-3.73 (2 H, m), 3.88 (2 H, t, J = 4.7 Hz), 4.17 (2 H, s), 6.29 (1 H, br s), 6.81-6.93 (1 H, m), 6.94-7.32 (5 H, m).

Example 61

1-[4-fluoro-2-[3-(trifluoromethyl)benzyl]-1-benzothiophen-7-yl]-3,5-dimethyl-1H-pyrazole-4-carboxamide

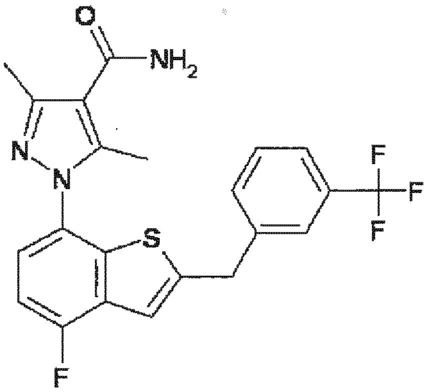

To a solution of 1-[4-fluoro-2-[3-(trifluoromethyl)benzyl]-1-benzothiophen-7-yl]-3,5-dimethyl-1H-pyrazole-4-carboxylic acid synthesized in Reference Example 53 (0.1 g, 0.23 mmol) in methanol (3 mL) was added DMTMM (77 mg, 0.28 mmol), and the mixture was stirred for 5 min at room temperature. Aqueous ammonia (1 mL) was added thereto, and the mixture was stirred overnight. To the reaction mixture was added water, and the mixture was extracted with ethyl acetate. The organic layer was washed with water, saturated aqueous sodium hydrogen carbonate solution, saturated aqueous ammonium chloride solution and saturated brine, dried over anhydrous magnesium sulfate, and concentrated under reduced pressure. The crude crystals were recrystallized from ethanol-ethyl acetate to give the title compound (45 mg, yield 43%) as crystals. melting point 201 - 202°C.
$^1$H-NMR ($CDCl_3$) δ: 2.44 (3 H, s), 2.52 (3 H, s), 4.26 (2 H, s), 5.44 (1 H, br s), 5.73 (1 H, br s), 7.03-7.24 (3 H, m), 7.39-7.59 (4 H, m).

Example 62

1-[4-fluoro-2-[3-(trifluoromethyl)benzyl]-1-benzothiophen-7-yl]-N-(2-hydroxyethyl)-3,5-dimethyl-1H-pyrazole-4-carboxamide

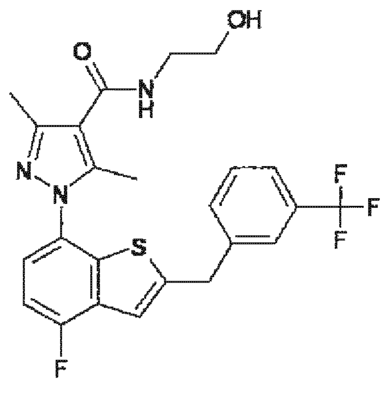

To a solution of 1-[4-fluoro-2-[3-(trifluoromethyl)benzyl]-1-benzothiophen-7-yl]-3,5-dimethyl-1H-pyrazole-4-carboxylic acid synthesized in Reference Example 53 (0.15 g, 0.34 mmol) in DMF (2 mL) were added WSC (96 mg, 0.50 mmol) and HOBt (77 mg, 0.50 mmol), and the mixture was stirred for 5 min at room temperature. 2-Aminoethanol

(61 mg, 1.00 mmol) was added thereto, and the mixture was stirred overnight. To the reaction mixture was added water, and the mixture was extracted with ethyl acetate. The organic layer was washed with water, saturated aqueous sodium hydrogen carbonate solution, saturated aqueous ammonium chloride solution and saturated brine, dried over anhydrous magnesium sulfate, and concentrated under reduced pressure. The crude crystals were recrystallized from ethanol-ethyl acetate to give the title compound (52 mg, yield 53%) as crystals. melting point 157 - 158°C.
$^1$H-NMR ($CDCl_3$) δ: 2.42 (3 H, s), 2.51 (3 H, s), 2.54-2.60 (1 H, m), 3.58-3.69 (2 H, m), 3.80-3.89 (2 H, m), 4.26 (2 H, s), 6.14 (1 H, br s), 7.04-7.31 (3 H, m), 7.40-7.58 (4 H, m).

Example 63

N-(2-amino-2-oxoethyl)-5-(hydroxymethyl)-3-methyl-1-[2-[3-(trifluoromethyl)benzyl]-1-benzothiophen-7-yl]-1H-pyrazole-4-carboxamide

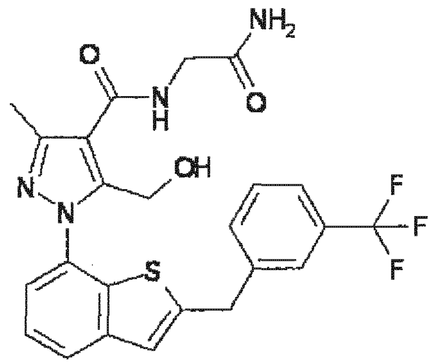

To a solution of 5-(hydroxymethyl)-3-methyl-1-[2-[3-(trifluoromethyl)benzyl]-1-benzothiophen-7-yl]-1H-pyrazole-4-carboxylic acid synthesized in Reference Example 57 (30 mg, 0.067 mmol) in DMF (1 mL) were added WSC (19 mg, 0.101 mmol) and HOBt (15 mg, 0.101 mmol), and the mixture was stirred for 5 min at room temperature. Glycinamide hydrochloride (11 mg, 0.101 mmol) and diisopropylethylamine (26 mg, 0.202 mmol) were added thereto, and the mixture was stirred overnight. To the reaction mixture was added water, and the mixture was extracted with ethyl acetate. The organic layer was washed with water, saturated aqueous sodium hydrogen carbonate solution, saturated aqueous ammonium chloride solution and saturated brine, dried over anhydrous magnesium sulfate, and concentrated under reduced pressure. The residue was purified by silica gel column chromatography (ethyl acetate:methanol=95:5) to give the title compound (19 mg, yield 56%) as a solid.
$^1$H-NMR ($CDCl_3$) δ: 2.55 (3 H, s), 4.11 (2 H, d, J = 4.9 Hz), 4.22 (2 H, s), 4.58 (2 H, s), 6.04 (1 H, br s), 6.57 (1 H, br s), 7.05 (1 H, s), 7.21-7.58 (8 H, m), 7.70 (1 H, d, J = 7.1 Hz).

Example 64

5-(hydroxymethyl)-3-methyl-1-[2-[3-(trifluoromethyl)benzyl]-1-benzothiophen-7-yl]-1H-pyrazole-4-carboxamide

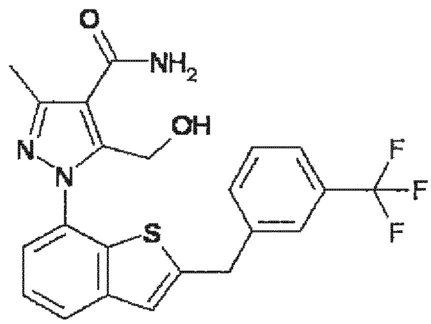

To a solution of 5-(hydroxymethyl)-3-methyl-1-[2-[3-(trifluoromethyl)benzyl]-1-benzothiophen-7-yl]-1H-pyrazole-4-carboxylic acid synthesized in Reference Example 57 (40 mg, 0.090 mmol) in DMF (1 mL) were added WSC (27 mg, 0.134 mmol) and HOBt (21 mg, 0.134 mmol), and the mixture was stirred for 5 min at room temperature. 28% Aqueous ammonia (0.3 mL) was added thereto, and the mixture was stirred overnight. To the reaction mixture was added water, and the mixture was extracted with ethyl acetate. The organic layer was washed with water, saturated aqueous sodium hydrogen carbonate solution, saturated aqueous ammonium chloride solution and saturated brine, dried over anhydrous magnesium sulfate, and concentrated under reduced pressure. The residue was purified by silica gel column chromatography (ethyl acetate:methanol=95:5) to give the title compound (4 mg, yield 10%) as an oil.
$^1$H-NMR ($CDCl_3$) δ: 2.57 (3 H, s), 4.26 (2 H, s), 4.60 (2 H, d, J = 6.3 Hz), 4.98 (1H, br s), 6.02 (2 H, br s), 7.09 (1 H, s), 7.24-7.31 (1 H, m), 7.38-7.57 (5 H, m), 7.75 (1 H, d, J = 7.1 Hz).

Example 65

N-(2-hydroxyethyl)-5-(hydroxymethyl)-3-methyl-1-[2-[3-(trifluoromethyl)benzyl]-1-benzothiophen-7-yl]-1H-pyrazole-4-carboxamide

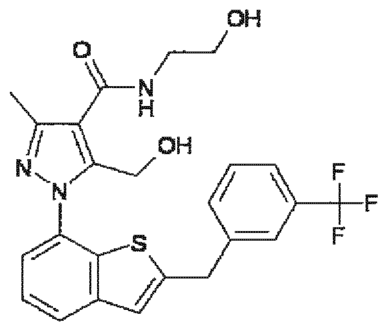

To a solution of 5-(tert-butoxymethyl)-3-methyl-1-[2-[3-(trifluoromethyl)benzyl]-1-benzothiophen-7-yl]-1H-pyrazole-4-carboxylic acid synthesized in Reference Example 60 (283 mg, 0.562 mmol) in DMF (2 mL) were added WSC (0.32 g, 1.69 mmol) and HOBt (0.26 g, 1.69 mmol), and the mixture was stirred for 5 min at room temperature. 2-Aminoethanol (0.103 g, 1.69 mmol) was added thereto, and the mixture was stirred overnight. To the reaction mixture was added water, and the mixture was extracted with ethyl acetate. The organic layer was washed with water, saturated aqueous sodium hydrogen carbonate solution, saturated aqueous ammonium chloride solution and saturated brine, dried over anhydrous magnesium sulfate, and concentrated under reduced pressure. To a solution of the residue in toluene (2 mL) was added TFA (2 mL), and the mixture was stirred overnight at room temperature. The mixture was concentrated under reduced pressure, and the residue was purified by silica gel column chromatography (ethyl acetate: methanol=95:5). The crude crystals were recrystallized from ethyl acetate-hexane to give the title compound (149 mg, yield 54%) as crystals. melting point 112 - 113°C. $^1$H-NMR ($CDCl_3$) δ: 2.39 (1 H, br s), 2.57 (3 H, s), 3.61-3.71 (2 H, m), 3.86 (2 H, br s), 4.26 (2 H, s), 4.60 (2 H, d, J = 6.4 Hz), 4.98 (1 H, t, J = 7.0 Hz), 6.62 (1 H, br s), 7.09 (1 H, s), 7.25-7.31 (1 H, m), 7.38-7.47 (3 H, m), 7.49-7.55 (2 H, m), 7.75 (1 H, d, J = 7.9 Hz).

Example 66

5-(hydroxymethyl)-N-(2-methoxyethyl)-3-methyl-1-[2-[3-(trifluoromethyl)benzyl]-1-benzothiophen-7-yl]-1H-pyrazole-4-carboxamide

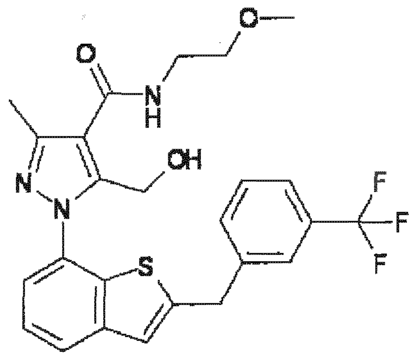

To a solution of 5-(tert-butoxymethyl)-3-methyl-1-[2-[3-(trifluoromethyl)benzyl]-1-benzothiophen-7-yl]-1H-pyrazole-4-carboxylic acid synthesized in Reference Example 60 (0.28 g, 0.56 mmol) in DMF (2 mL) were added WSC (0.32 g, 1.69 mmol) and HOBt (0.26 g, 1.69 mmol), and the mixture was stirred for 5 min at room temperature. 2-Methoxyethanamine (0.13 g, 1. 69 mmol) was added thereto, and the mixture was stirred overnight. To the reaction mixture was added water, and the mixture was extracted with ethyl acetate. The organic layer was washed with water, saturated aqueous sodium hydrogen carbonate solution, saturated aqueous ammonium chloride solution and saturated brine, dried over anhydrous magnesium sulfate, and concentrated under reduced pressure. To the obtained residue was added 4M hydrochloric acid/ethyl acetate (5 mL), and the mixture was stirred overnight at room temperature. The reaction mixture was evaporated solvents under reduced pressure. The residue was purified by silica gel column chromatography (methanol:ethyl acetate=5:95) to give the title compound (47 mg, yield 17%) as an oil.
$^1$H-NMR ($CDC1_3$) δ: 2.56 (3 H, s), 3.43 (3 H, s), 3.55-3.74 (4 H, m), 4.26 (2 H, s), 4.59 (2 H, d, J = 6.6 Hz), 5.18-5.28 (1 H, m), 6.49 (1 H, br s), 7.09 (1 H, s), 7.24-7.58 (6 H, m), 7.74 (1 H, d, J = 8.0 Hz).

Example 67

N-(2-hydroxyethyl)-3-(hydroxymethyl)-5-methyl-1-[2-[3-(trifluoromethyl)benzyl]-1-benzothiophen-7-yl]-1H-pyrazole-4-carboxamide

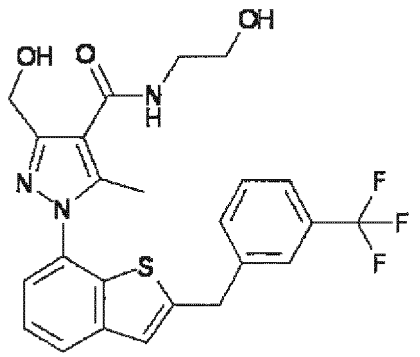

To a solution of 3-(tert-butoxymethyl)-5-methyl-1-[2-[3-(trifluoromethyl)benzyl]-1-benzothiophen-7-yl]-1H-pyrazole-4-carboxylic acid synthesized in Reference Example 61 (0.37 g, 0.74 mmol) in DMF (3 mL) were added WSC (0.43 g, 2.22 mmol) and HOBt (0.34 g, 2.22 mmol), and the mixture was stirred for 5 min at room temperature. 2-Aminoethanol (0.14 g, 2.22 mmol) was added thereto, and the mixture was stirred overnight. To the reaction mixture was added water, and the mixture was extracted with ethyl acetate. The organic layer was washed with water, saturated aqueous sodium hydrogen carbonate solution, saturated aqueous ammonium chloride solution and saturated brine, dried over anhydrous magnesium sulfate, and concentrated under reduced pressure. To the obtained residue was added 4M hydrochloric acid/ethyl acetate (5 mL), and the mixture was stirred overnight at room temperature. The reaction mixture was evaporated under reduced pressure. The residue was purified by silica gel column chromatography (methanol:ethyl acetate=5:95) to give the title compound (41 mg, yield 12%) as an oil.
$^1$H-NMR ($CDCl_3$) 5: 2.48 (3 H, s), 3.08 (1 H, br s), 3.32 (1 H, br s), 3.55-3.67 (2 H, m), 3.78-3.90 (2 H, m), 4.26 (2 H, s), 4.78-4.91 (2 H, m), 7.06-7.59 (7 H, m), 7.76 (1 H, d, J = 8.2 Hz), 7.96 (1 H, br s).

Example 68

N-(2-amino-2-oxoethyl)-3-(hydroxymethyl)-5-methyl-1-[2-[3-(trifluoromethyl)benzyl]-1-benzothiophen-7-yl]-1H-pyrazole-4-carboxamide

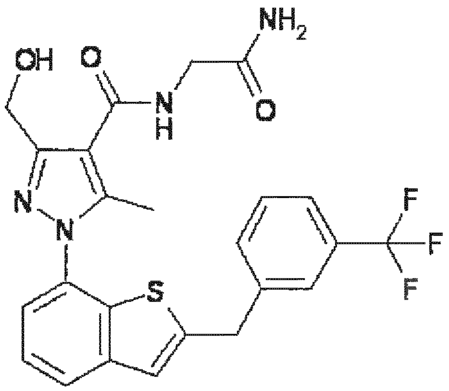

To a solution of 3-(tert-butoxymethyl)-5-methyl-1-[2-[3-(trifluoromethyl)benzyl]-1-benzothiophen-7-yl]-1H-pyrazole-4-carboxylic acid synthesized in Reference Example 61 (0.2 g, 0.40 mmol) in DMF (2 mL) were added WSC (0.23 g, 1.319 mmol) and HOBt (0.18 g, 1.19 mmol), and the mixture was stirred for 5 min at room temperature. Glycinamide hydrochloride (66 mg, 0.60 mmol) and diisopropylethylamine (0.15 g, 1.19 mmol) were added thereto, and the mixture was stirred overnight. To the reaction mixture was added water, and the mixture was extracted with ethyl acetate. The organic layer was washed with water, saturated aqueous sodium hydrogen carbonate solution, saturated aqueous ammonium chloride solution and saturated brine, dried over anhydrous magnesium sulfate, and concentrated under reduced pressure. To the obtained residue was added 4M hydrochloric acid/ethyl acetate (10 mL), and the mixture was stirred overnight at room temperature. The reaction mixture was evaporated under reduced pressure. The residue was purified by silica gel column chromatography (methanol:ethyl acetate=5:95), and the obtained crude crystals were recrystallized from ethyl acetate-hexane to give the title compound (94 mg, yield 47%) as crystals. melting point 154 - 155°C. $^1$H-NMR ($CDCl_3$) 5: 2.49 (3 H, s), 3.68 (1 H, br s), 4.13 (2 H, s), 4.25 (2 H, s), 4.84 (2 H, s), 5.52 (1 H, br s), 6.29 (1 H, br s), 7.03-7.33 (2 H, m), 7.36-7.62 (5 H, m), 7.68-7.82 (1 H, m), 8.19-8.29 (1 H, m).

Example 69

3-(hydroxymethyl)-5-methyl-1-[2-[3-(trifluoromethyl)benzyl]-1-benzothiophen-7-yl]-1H-pyrazole-4-carboxamide

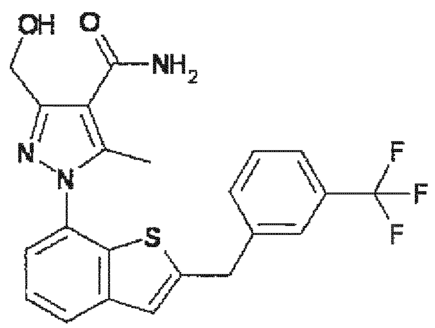

To 3-(tert-butoxymethyl)-5-methyl-1-[2-[3-(trifluoromethyl)benzyl]-1-benzothiophen-7-yl]-1H-pyrazole-4-carboxylic acid synthesized in Reference Example 61 (0.2 g, 0.40 mmol) was added 4M hydrochloric acid/ethyl acetate (5 mL), and the mixture was stirred overnight at room temperature. The reaction mixture was evaporated under reduced pressure. To a solution of the obtained residue in DMF (2 mL) were added WSC (0.23 g, 1.319 mmol) and HOBt (0.18 g, 1.19 mmol), and the mixture was stirred for 5 min at room temperature. 28% Aqueous ammonia (1.5 mL) was added

thereto, and the mixture was stirred overnight. To the reaction mixture was added water, and the mixture was extracted with ethyl acetate. The organic layer was washed with water, saturated aqueous sodium hydrogen carbonate solution, saturated aqueous ammonium chloride solution and saturated brine, dried over anhydrous magnesium sulfate, and concentrated under reduced pressure. The residue was purified by silica gel column chromatography (methanol:ethyl acetate= 5:95), and the obtained crude crystals were recrystallized from ethyl acetate-hexane to give the title compound (79 mg, yield 46%) as crystals. melting point 102 - 103°C.
$^1$H-NMR ($CDCl_3$) δ: 2.50 (3 H, s), 4.26 (2 H, s), 4.86 (2 H, s), 7.07-7.16 (1 H, m), 7.16-7.57 (8 H, m), 7.72-7.83 (1 H, m), 1 H, unconfirmed.

Example 70

N-(2-hydroxyethyl)-5-(methoxymethyl)-3-methyl-1-[2-[3-(trifluoromethyl)benzyl]-1-benzothiophen-7-yl]-1H-pyrazole-4-carboxamide

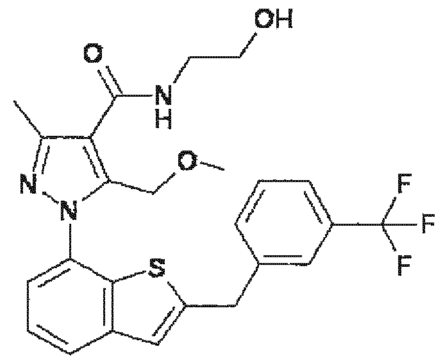

To a solution of 5-(hydroxymethyl)-3-methyl-1-[2-[3-(trifluoromethyl)benzyl]-1-benzothiophen-7-yl]-1H-pyrazole-4-carboxylic acid obtained in Reference Example 57 (0.30 g, 0.63 mmol) in DMF (1 mL) were added methyl iodide (0.13 g, 0.95 mmol) and sodium hydride (60%oil, 38 mg, 0.95 mmol) at 0°C, and the mixture was stirred for 3 hr at room temperature. To the reaction mixture was added saturated aqueous ammonium chloride solution, and the mixture was extracted with ethyl acetate. The organic layer was washed with water, dried over anhydrous magnesium sulfate, and concentrated under reduced pressure. The residue was simple-purified by silica gel column chromatography (ethyl acetate:hexane=1:9). To a solution of the obtained compound in THF (3 mL)-ethanol (3 mL) was added 8N aqueous sodium hydroxide solution (0.09 mL, 0.71 mmol), and the mixture was stirred for 4 hr at 85°C. The reaction mixture was neutralized with 1N hydrochloric acid, and diluted with water. The mixture was extracted with ethyl acetate. The organic layer was washed with water, dried over anhydrous magnesium sulfate, and concentrated under reduced pressure. To a solution of the obtained residue in DMF (3 mL) were added WSC (0.12 g, 0.63 mmol) and HOBt (96 mg, 0.63 mmol), and the mixture was stirred for 5 min at room temperature. 2-Aminoethanol (38 mg, 0.63 mmol) was added thereto, and the mixture was stirred overnight. To the reaction mixture was added water, and the mixture was extracted with ethyl acetate. The organic layer was washed with water, saturated aqueous sodium hydrogen carbonate solution, saturated aqueous ammonium chloride solution and saturated brine, dried over anhydrous magnesium sulfate, and concentrated under reduced pressure. The residue was purified by silica gel column chromatography (methanol:ethyl acetate=5:95) to give the title compound (24 mg, yield 20%) as an oil.
$^1$H-NMR ($CDCl_3$) 5: 2.51-2.62 (4 H, m), 3.34 (3 H, s), 3.56-3.66 (2 H, m), 3.79-3.86 (2 H, m), 4.26 (2 H, s), 4.42 (2 H, s), 7.08 (1 H, s), 7.23-7.31 (2 H, m), 7.37-7.59 (5 H, m), 7.75 (1 H, d, J = 8.0 Hz).

Example 71

1-[4-fluoro-2-[3-(trifluoromethyl)benzyl]-1-benzothiophen-7-yl]-N-(2-hydroxyethyl)-5-(hydroxymethyl)-3-methyl-1H-pyrazole-4-carboxamide

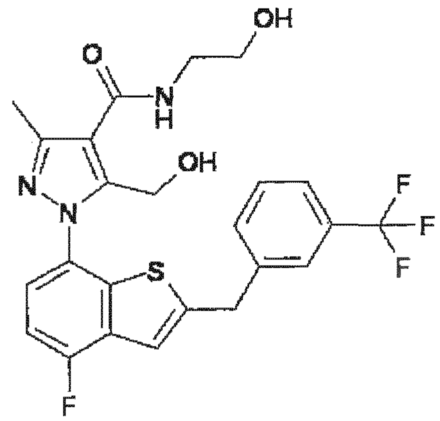

To a solution of 1-[4-fluoro-2-[3-(trifluoromethyl)benzyl]-1-benzothiophen-7-yl]-5-(hydroxymethyl)-3-methyl-1H-pyrazole-4-carboxylic acid synthesized in Reference Example 63 (0.1 g, 0.22 mmol) in DMF (3 mL) were added WSC (0.13 g, 0.65 mmol) and HOBt (99 mg, 0.65 mmol), and the mixture was stirred for 5 min at room temperature. 2-Aminoethanol (39 mg, 0.65 mmol) was added thereto, and the mixture was stirred overnight. To the reaction mixture was added water, and the mixture was extracted with ethyl acetate. The organic layer was washed with water, saturated aqueous sodium hydrogen carbonate solution, saturated aqueous ammonium chloride solution and saturated brine, dried over anhydrous magnesium sulfate, and concentrated under reduced pressure. The crude crystals were recrystallized from ethyl acetate-hexane to give the title compound (51 mg, yield 47%) as crystals. melting point 120 - 121°C.
$^1$H-NMR ($CDCl_3$) δ: 2.56 (3 H, s), 2.60 (1 H, br s), 3.61-3.70 (2 H, m), 3.83-3.90 (2 H, m), 4.26 (2 H, s), 4.57 (2 H, s), 6.58 (1 H, br s), 7.04-7.14 (1 H, m), 7.18-7.30 (3 H, m), 7.41-7.58 (4 H, m).

Example 72

N-(2-amino-2-oxoethyl)-1-[4-fluoro-2-[3-(trifluoromethyl)benzyl]-1-benzothiophen-7-yl]-5-(hydroxymethyl)-3-methyl-1H-pyrazole-4-carboxamide

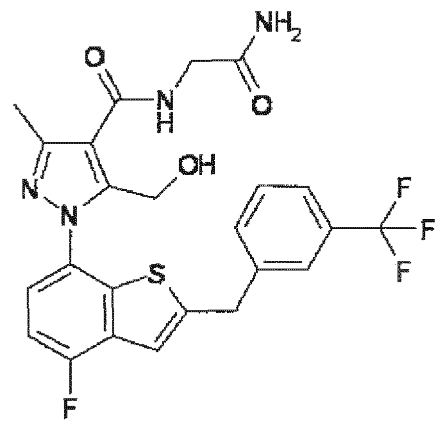

To a solution of 1-[4-fluoro-2-[3-(trifluoromethyl)benzyl]-1-benzothiophen-7-yl]-5-(hydroxymethyl)-3-methyl-1H-pyrazole-4-carboxylic acid synthesized in Reference Example 63 (0.1 g, 0.22 mmol) in DMF (3 mL) were added WSC (0.13 g, 0.65 mmol) and HOBt (99 mg, 0.65 mmol), and the mixture was stirred for 5 min at room temperature. Glycinamide hydrochloride (36 mg, 0.32 mmol) and diisopropylethylamine (83 mg, 0.65 mmol) were added thereto, and the mixture was stirred overnight. To the reaction mixture was added water, and the mixture was extracted with ethyl acetate. The organic layer was washed with water, saturated aqueous sodium hydrogen carbonate solution, saturated aqueous ammonium chloride solution and saturated brine, dried over anhydrous magnesium sulfate, and concentrated under reduced pressure. The crude crystals were recrystallized from ethanol-ethyl acetate the title compound (41 mg, yield 37%) as crystals. melting point 190 - 191°C.
$^1$H-NMR ($CDCl_3$) δ: 2.60 (3 H, s), 4.18 (2 H, d, J = 4.9 Hz), 4.26 (2 H, s), 4.58 (2 H, d, J = 6.4 Hz), 4.83-4.90 (1 H, m),

5.51 (1 H, br s), 5.88 (1 H, br s), 7.00 (1 H, br s), 7.05-7.14 (1 H, m), 7.17-7.30 (2 H, m), 7.42-7.56 (4 H, m).

Example 73

1-[4-fluoro-2-[3-(trifluoromethyl)benzyl]-1-benzothiophen-7-yl]-5-(hydroxymethyl)-3-methyl-1H-pyrazole-4-carboxamide

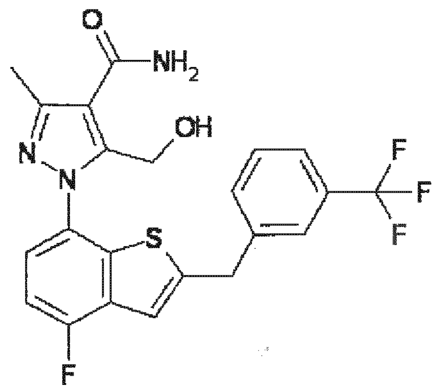

To a solution of 1-[4-fluoro-2-[3-(trifluoromethyl)benzyl]-1-benzothiophen-7-yl]-5-(hydroxymethyl)-3-methyl-1H-pyrazole-4-carboxylic acid synthesized in Reference Example 63 (0.1 g, 0.22 mmol) in DMF (3 mL) were added WSC (0.13 g, 0.65 mmol) and HOBt (99 mg, 0.65 mmol), and the mixture was stirred for 5 min at room temperature. 28% Aqueous ammonia (0.5 mL) was added thereto, and the mixture was stirred overnight. To the reaction mixture was added water, and the mixture was extracted with ethyl acetate. The organic layer was washed with water, saturated aqueous sodium hydrogen carbonate solution, saturated aqueous ammonium chloride solution and saturated brine, dried over anhydrous magnesium sulfate, and concentrated under reduced pressure. The crude crystals were recrystallized from ethyl acetate-hexane to give the title compound (54 mg, yield 54%) as crystals. melting point 123 - 124°C. $^{1}$H-NMR ($CDCl_3$) δ: 2.56 (3 H, s), 4.27 (2 H, s), 4.58 (2 H, d, J = 6.8 Hz), 4.88 (1 H, t, J = 6.8 Hz), 5.86 (2 H, br s), 7.05-7.16 (1 H, m), 7.19-7.30 (2 H, m), 7.42-7.56 (4 H, m).

Example 74

N-(2-amino-2-oxoethyl)-3,5-dimethyl-1-[2-[3-(trifluoromethyl)benzyl]-1-benzofuran-4-yl]-1H-pyrazole-4-carboxamide

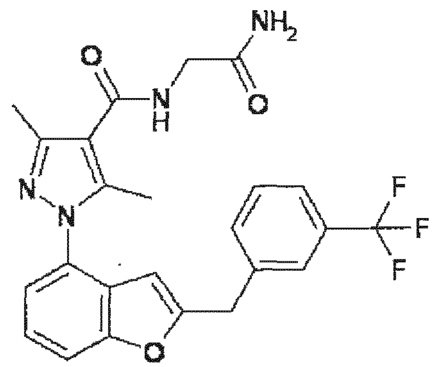

To a solution of 3,5-dimethyl-1-[2-[3-(trifluoromethyl)benzyl]-1-benzofuran-4-yl]-1H-pyrazole-4-carboxylic acid synthesized in Reference Example 65 (0.25 g, 0.60 mmol) in DMF (5 mL) were added WSC (0.35 g, 1.81 mmol) and HOBt (0.28 mg, 1.81 mmol), and the mixture was stirred for 5 min at room temperature. Glycinamide hydrochloride (0.1 g, 0.90 mmol) and diisopropylethylamine (0.23 g, 1.81 mmol) were added thereto, and the mixture was stirred overnight. To the reaction mixture was added water, and the mixture was extracted with ethyl acetate. The organic layer was

washed with water, saturated aqueous sodium hydrogen carbonate solution, saturated aqueous ammonium chloride solution and saturated brine, dried over anhydrous magnesium sulfate, and concentrated under reduced pressure. The crude crystals were recrystallized from ethyl acetate to give the title compound (122 mg, yield 43%) as crystals. melting point 187 - 188°C.

$^1$H-NMR ($CDCl_3$) δ: 2.45 (3 H, s), 2.55 (3 H, s), 4.08-4.30 (4 H, m), 5.72 (1 H, br s), 6.36 (1 H, s), 6.50 (1 H, br s), 6.61 (1 H, br s), 7.14 (1 H, d, J = 7.7 Hz), 7.22-7.38 (1 H, m), 7.40-7.63 (5 H, m).

Example 75

N-(2-hydroxyethyl)-3,5-dimethyl-1-[2-[3-(trifluoromethyl)benzyl]-1-benzofuran-4-yl]-1H-pyrazole-4-carboxamide

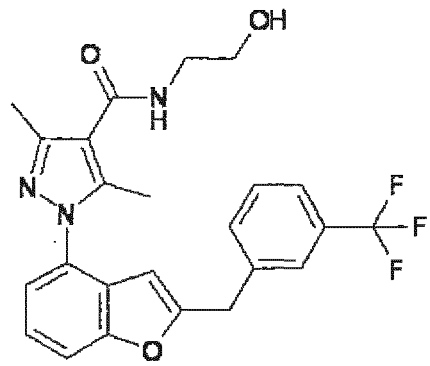

To a solution of 3,5-dimethyl-1-[2-[3-(trifluoromethyl)benzyl]-1-benzofuran-4-yl]-1H-pyrazole-4-carboxylic acid synthesized in Reference Example 65 (0.25 g, 0.60 mmol) in DMF (5 mL) were added WSC (0.35 g, 1.81 mmol) and HOBt (0.28 mg, 1.81 mmol), and the mixture was stirred for 5 min at room temperature. 2-Aminoethanol (0.11 g, 1.81 mmol) was added thereto, and the mixture was stirred overnight. To the reaction mixture was added water, and the mixture was extracted with ethyl acetate. The organic layer was washed with water, saturated aqueous sodium hydrogen carbonate solution, saturated aqueous ammonium chloride solution and saturated brine, dried over anhydrous magnesium sulfate, and concentrated under reduced pressure. The crude crystals were recrystallized from ethyl acetate-hexane to give the title compound (165 mg, yield 60%) as crystals. melting point 125 - 126°C.

$^1$H-NMR ($CDCl_3$) δ: 2.45 (3 H, s), 2.52 (3 H, s), 2. 64 (1 H, t, J = 4.8 Hz), 3.58-3.70 (2 H, m), 3.81-3.91 (2 H, m), 4.16 (2 H, s), 6.15 (1 H, br s), 6.34 (1 H, s), 7.15 (1 H, d, J = 6.9 Hz), 7.27-7.35 (1 H, m), 7.40-7.60 (5 H, m).

Example 76

3,5-dimethyl-1-[2-[3-(trifluoromethyl)benzyl]-1-benzofuran-4-yl]-1H-pyrazole-4-carboxamide

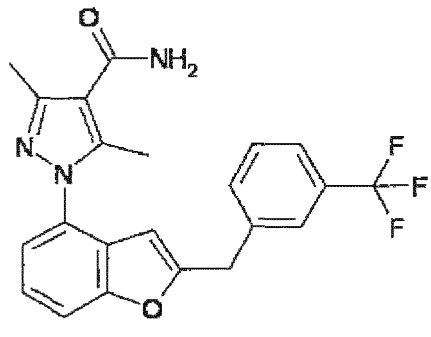

To a solution of 3,5-dimethyl-1-[2-[3-(trifluoromethyl)benzyl]-1-benzofuran-4-yl]-1H-pyrazole-4-carboxylic acid synthesized in Reference Example 65 (0.25 g, 0.60 mmol) in DMF (5 mL) were added WSC (0.35 g, 1.81 mmol) and HOBt (0.28 mg, 1.81 mmol), and the mixture was stirred for 5 min at room temperature. 28% Aqueous ammonia (0.5

mL) was added thereto, and the mixture was stirred overnight. To the reaction mixture was added water, and the mixture was extracted with ethyl acetate. The organic layer was washed with water, saturated aqueous sodium hydrogen carbonate solution, saturated aqueous ammonium chloride solution and saturated brine, dried over anhydrous magnesium sulfate, and concentrated under reduced pressure. The crude crystals were recrystallized from ethyl acetate to give the title compound (81 mg, yield 33%) as crystals. melting point 196 - 197°C. $^1$H-NMR ($CDCl_3$) δ: 2.47 (3 H, s), 2.53 (3 H, s), 4.16 (2 H, s), 5.57 (2 H, br s), 6.35 (1 H, s), 7.16 (1 H, d, J = 7.7 Hz), 7.32 (1 H, t, J = 8.0 Hz), 7.39-7.59 (5 H, m).

Example 77

3,5-dimethyl-N-[2-(1-methylethoxy)ethyl]-1-[2-3-(trifluoromethyl)benzyl]-1-benzofuran-4-yl]-1H-pyrazole-4-carboxamide

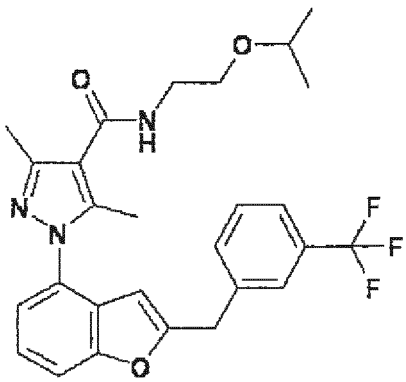

To a solution of 3,5-dimethyl-1-[2-[3-(trifluoromethyl)benzyl]-1-benzofuran-4-yl]-1H-pyrazole-4-carboxylic acid synthesized in Reference Example 65 (0.2 g, 0.48 mmol) in DMF (3 mL) were added WSC (0.28 g, 1.45 mmol) and HOBt (0.22 mg, 1.45 mmol), and the mixture was stirred for 5 min at room temperature. 2-(1-Methylethoxy)ethanamine (0.15 g, 1.45 mmol) was added thereto, and the mixture was stirred overnight. To the reaction mixture was added water, and the mixture was extracted with ethyl acetate. The organic layer was washed with water, saturated aqueous sodium hydrogen carbonate solution, saturated aqueous ammonium chloride solution and saturated brine, dried over anhydrous magnesium sulfate, and concentrated under reduced pressure. The residue was purified by silica gel column chromatography (ethyl acetate:methanol=95:5) to give the title compound (213 mg, yield 88%) as an oil.
$^1$H-NMR ($CDCl_3$) 5: 1.18 (3 H, s), 1.20 (3 H, s), 2.44 (3 H, s), 2.51 (3 H, s), 3.52-3.72 (5 H, m), 4.16 (2 H, s), 6.19 (1 H, br s), 6.36 (1 H, s), 7.15 (1 H, d, J = 6.8 Hz), 7.31 (1 H, t, J = 8.0 Hz), 7.40-7.59 (5 H, m).

Example 78

3,5-dimethyl-1-[2-[3-(trifluoromethyl)benzyl]-2H-indazol-4-yl]-1H-pyrazole-4-carboxamide

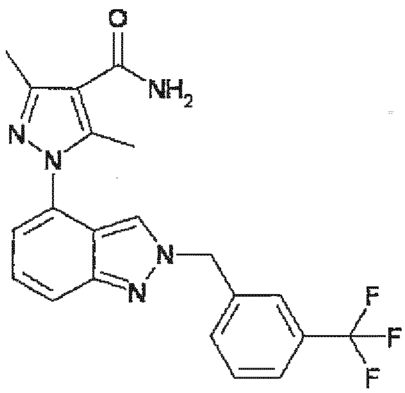

To a solution of ethyl 3,5-dimethyl-1-[2-[3-(trifluoromethyl)benzyl]-2H-indazol-4-yl]-1H-pyrazole-4-carboxylate obtained in Reference Example 71 (460 mg, 1.04 mmol) in THF (4 mL)-methanol (2 mL) was added 2N aqueous sodium hydroxide solution (1.85 mL, 3.64 mmol), and the mixture was stirred for 1 hr at room temperature, and stirred for 21 hr at 40°C. The reaction mixture was neutralized with 1N hydrochloric acid, and the mixture was extracted with ethyl acetate. The organic layer was washed with saturated brine, dried over anhydrous sodium sulfate, and evaporated under reduced pressure to give crude 3,5-dimethyl-1-[2-[3-(trifluoromethyl)benzyl]-2H-indazol-4-yl]-1H-pyrazole-4-carboxylic acid (391 mg). A solution of a part (130 mg) of the obtained crude product, WSC (72 mg, 0.376 mmol) and HOBt (51 mg, 0.376 mmol) in DMF (1.5 mL) was stirred for 5 hr at room temperature. To the reaction mixture was added 28% aqueous ammonia solution (2.0 mL), and the mixture was stirred for 2 hr at room temperature. The mixture was diluted with saturated aqueous sodium hydrogen carbonate solution, and extracted with ethyl acetate. The extract was washed with saturated brine, dried over anhydrous magnesium sulfate, and evaporated under reduced pressure. The residue was purified by silica gel column chromatography (ethyl acetate:methanol= 100:0→95:5), and the obtained crystals were recrystallized from hexane-ethyl acetate to give the title compound (105 mg, yield 73%) as crystals. melting point 199-200°C.
$^1$H-NMR ($CDCl_3$) $\delta$ : 2.54 (3 H, s), 2.55 (3 H, s), 5.58 (2 H, br s), 5.64 (2 H, s), 7.01 (1H, dd, J = 7.1, 0.5 Hz), 7.36 (1 H, dd, J = 8.8, 7.1 Hz), 7.42 - 7.52 (2 H, m), 7.56 - 7.63(2 H, m), 7.75 - 7.81 (1 H, m), 7.99 (1 H, d, J = 0.8 Hz).

Example 79

N-(2-hydroxyethyl)-3,5-dimethyl-1-[2-[3-(trifluoromethyl)benzyl]-2H-indazol-4-yl]-1H-pyrazole-4-carboxamide

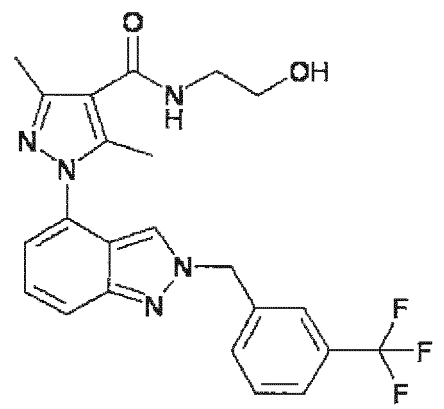

A solution of a part (130 mg) of the crude 3,5-dimethyl-1-[2-[3-(trifluoromethyl)benzyl]-2H-indazol-4-yl]-1H-pyrazole-4-carboxylic acid obtained in Example 78, 2-aminoethanol (15.8 µL, 0. 376 mmol), WSC (72 mg, 0.376 mmol) and HOBt (51 mg, 0.376 mmol) in DMF (3.0 mL) was stirred for 15 hr at room temperature. The reaction mixture was diluted with saturated aqueous sodium hydrogen carbonate solution, and extracted with ethyl acetate. The extract was washed with saturated brine, dried over anhydrous magnesium sulfate, and evaporated under reduced pressure. The residue was purified by silica gel column chromatography (ethyl acetate:methanol= 100:0→95:5), and the obtained crystals were recrystallized from hexane-ethyl acetate to give the title compound (112 mg, yield 71%) as crystals. melting point 150-151°C.
$^1$H-NMR ($CDCl_3$) $\delta$ : 2.52 (6 H, d, J = 0.8 Hz), 2.54 - 2.62 (1 H, m), 3.59 - 3.70 (2 H m), 3.85 (2 H, q, J = 5.0 Hz), 5.63 (2 H, s), 6.15 (1 H, br s), 7.00 (1 H, dd, J = 7.1, 0.5 Hz), 7.36 (1 H, dd, J = 8.8, 7.1 Hz), 7.42 - 7.52 (2 H, m), 7.56 - 7.63 (2 H, m), 7.74 - 7.81 (1 H, m), 7.99 (1 H, d, J = 0.8 Hz).

Example 80

N-(2-amino-2-oxoethyl)-3,5-dimethyl-1-[2-[3-(trifluoromethyl)benzyl]-2H-indazol-4-yl]-1H-pyrazole-4-carboxamide

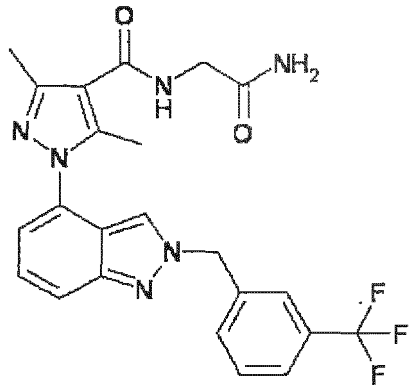

A solution of a part (130 mg) of the crude 3,5-dimethyl-1-[2-[3-(trifluoromethyl)benzyl]-2H-indazol-4-yl]-1H-pyrazole-4-carboxylic acid obtained in Example 78, glycinamide hydrochloride (42 mg, 0.376 mmol), WSC (72 mg, 0.376 mmol), HOBt (51 mg, 0.376 mmol) and N,N-diisopropylethylamine (65 μL, 0.376 mmol) in DMF (3.0 mL) was stirred for 15 hr at room temperature. The reaction mixture was diluted with saturated aqueous sodium hydrogen carbonate solution, and extracted with ethyl acetate. The extract was washed with saturated brine, dried over anhydrous magnesium sulfate, and evaporated under reduced pressure. The residue was purified by silica gel column chromatography (ethyl acetate: methanol= 100:0→95:5) and the obtained crystals were recrystallized from hexane-ethyl acetate to give the title compound (119 mg, yield 73%) as crystals. melting point 205-206°C.
$^1$H-NMR (DMSO-$d_6$) δ : 2.39 (3 H, s), 2.41 (3 H, s), 3.82 (2 H d, J = 5.8 Hz), 5.77 (2 H, s), 6.99 - 7.05 (1 H, m), 7.08 (1 H, d, J = 7.1 Hz), 7.29 - 7.40 (2 H, m), 7.52 - 7.72 (5 H, m), 7.76 (1 H, s), 8.51 (1 H, s).

Example 81

1-[2-(3-chloro-5-fluorobenzyl)-2H-indazol-4-yl]-3,5-dimethyl-1H-pyrazole-4-carboxamide

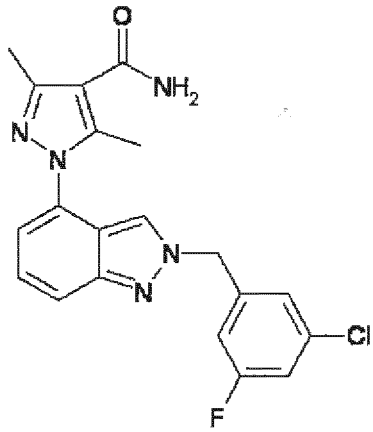

In the same manner as in Example 78, crude 1-[2-(3-chloro-5-fluorobenzyl)-2H-indazol-4-yl]-3,5-dimethyl-1H-pyrazole-4-carboxylic acid was synthesized from ethyl 1-[2-(3-chloro-5-fluorobenzyl)-2H-indazol-4-yl]-3,5-dimethyl-1H-pyrazole-4-carboxylate obtained in Reference Example 72, and using the obtained crude product and 28% aqueous ammonia solution, the title compound was obtained as crystals. yield 46%. melting point 245-246°C.
$^1$H-NMR ($CDCl_3$) δ : 2.55 (3 H, s), 2.57 (3 H, s), 5.49 - 5.65 (4 H, m), 6.84 - 6.92 (1 H, m), 6.99 - 7.11 (3 H, m), 7.37 (1 H, dd, J = 8.8, 7.1 Hz), 7.78 (1 H, d, J = 8.8 Hz), 8.00 (1 H, d, J = 0.5 Hz).

Example 82

1-[2-(3-chloro-5-fluorobenzyl)-2H-indazol-4-yl]-N-(2-hydroxyethyl)-3,5-dimethyl-1H-pyrazole-4-carboxamide

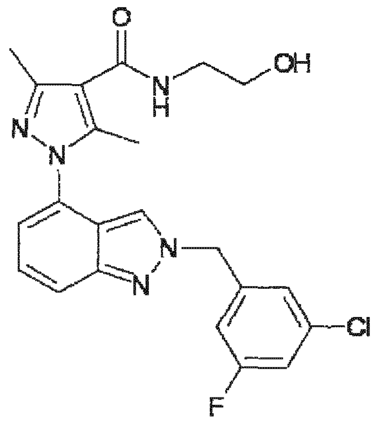

In the same manner as in Example 79 and using the crude 1-[2-(3-chloro-5-fluorobenzyl)-2H-indazol-4-yl]-3,5-dimethyl-1H-pyrazole-4-carboxylic acid obtained in Example 81 and 2-aminoethanol, the title compound was obtained as crystals. yield 73%. melting point 160-163°C.
$^1$H-NMR ($CDCl_3$) δ : 2.49 - 2.62 (7 H, m), 3. 60 - 3.71 (2 H m), 3.86 (2 H, q, J = 4.8 Hz), 5.54 (2 H, s), 6.16 (1 H, br s), 6.88 (1 H, d, J = 8.5 Hz), 6.97 - 7.11 (3 H, m), 7.37 (1 H, dd, J = 8.9, 7.3 Hz), 7.77 (1 H, d, J = 8.2 Hz), 7.99 (1 H, s).

Example 83

1-[2-[3-fluoro-5-(trifluoromethyl)benzyl]-2H-indazol-4-yl]-3,5-dimethyl-1H-pyrazole-4-carboxamide

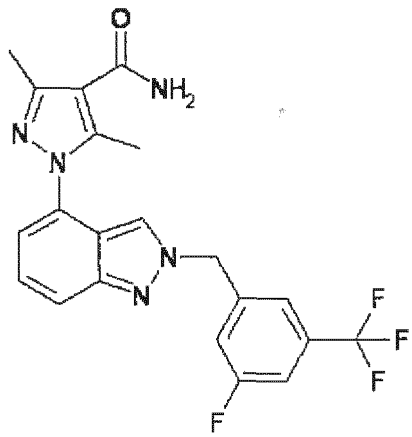

In the same manner as in Example 36, crude 1-[2-[3-fluoro-5-(trifluoromethyl)benzyl]-2H-indazol-4-yl]-3,5-dimethyl-1H-pyrazole-4-carboxylic acid was synthesized from ethyl 1-[2-[3-fluoro-5-(trifluoromethyl)benzyl]-2H-indazol-4-yl]-3,5-dimethyl-1H-pyrazole-4-carboxylate obtained in Reference Example 73, and using the obtained crude product and 28% aqueous ammonia solution, the title compound was obtained as crystals. yield 57%. melting point 203-204°C. $^1$H-NMR ($CDCl_3$) δ : 2.55 (3 H, s), 2.57 (3 H, s), 5.53 - 5.68 (4 H, m), 7.03 (1 H, dd, J = 7.1, 0.8 Hz), 7.15 (1 H, dt, J = 8.7, 1.5 Hz), 7.27 - 7.32 (1 H, m), 7.33 - 7.42 (2 H, m), 7.78 (1 H, d, J = 8.8 Hz), 8.03 (1 H, s).

Example 84

1-[2-[3-fluoro-5-(trifluoromethyl)benzyl]-2H-indazol-4-yl]-N-(2-hydroxyethyl)-3,5-dimethyl-1H-pyrazole-4-carboxamide

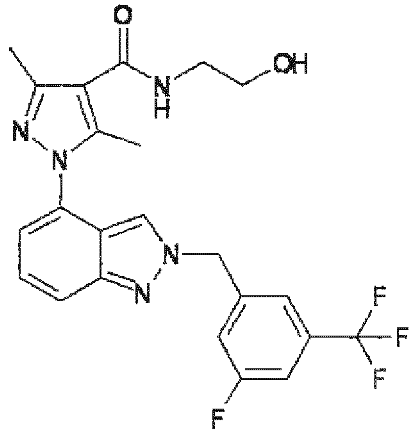

In the same manner as in Example 37 and using the crude 1-[2-[3-fluoro-5-(trifluoromethyl)benzyl]-2H-indazol-4-yl]-3,5-dimethyl-1H-pyrazole-4-carboxylic acid obtained in Example 83 and 2-aminoethanol, the title compound was obtained as crystals. yield 76%. melting point 176-177°C.
$^1$H-NMR ($CDCl_3$) δ : 2.49 - 2.59 (7 H, m), 3.65 (2 H q, J = 5.1 Hz), 3.86 (2 H, q, J = 4.8 Hz), 5.62 (2 H, s), 6.15 (1 H, br s), 7.02 (1 H, d, J = 7.1 Hz), 7.12 - 7.19 (1 H, m), 7.27 - 7.32 (1 H, m), 7.33 - 7.42 (2 H, m), 7.77 (1 H, d, J = 8.8 Hz), 8.03 (1 H, s).

Example 85

2-[2-(3-chloro-5-fluorobenzyl)-2H-indazol-4-yl]-4-methyl-1,3-thiazole-5-carboxamide

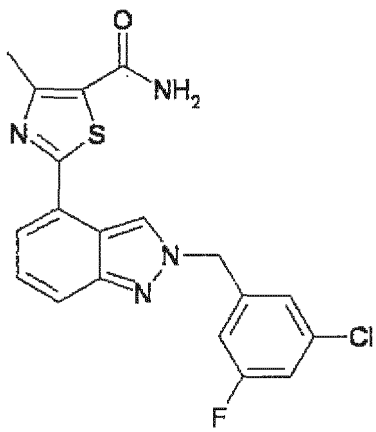

In the same manner as in Example 36, crude 2-[2-(3-chloro-5-fluorobenzyl)-2H-indazol-4-yl]-4-methyl-1,3-thiazole-5-carboxylic acid was synthesized from ethyl 2-[2-(3-chloro-5-fluorobenzyl)-2H-indazol-4-yl]-4-methyl-1,3-thiazole-5-carboxylate obtained in Reference Example 74, and using the obtained crude product and 28% aqueous ammonia solution, the title compound was obtained as crystals. yield 54%. melting point 250-251°C.
$^1$H-NMR (DMSO-$d_6$) δ: 2.70 (3 H, s), 5.79 (2 H, s), 7.15 - 7.26 (1 H, m), 7.29 (1 H, s), 7.33 - 7.45 (2 H, m), 7.56 - 7.76 (3 H, m), 7.81 (1 H, d, J = 8.5 Hz), 9.07 (1 H, s).

Example 86

2-[2-[3-fluoro-5-(trifluoromethyl)benzyl]-2H-indazol-4-yl]-4-methyl-1,3-thiazole-5-carboxamide

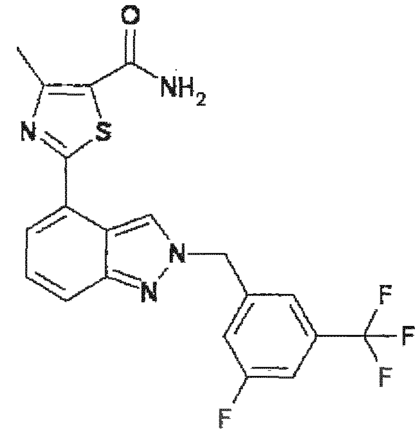

In the same manner as in Example 36, crude 2-[2-[3-fluoro-5-(trifluoromethyl)benzyl]-2H-indazol-4-yl]-4-methyl-1,3-thiazole-5-carboxylic acid was synthesized from ethyl 2-[2-[3-fluoro-5-(trifluoromethyl)benzyl]-2H-indazol-4-yl]-4-methyl-1,3-thiazole-5-carboxylate obtained in Reference Example 75, and using the obtained crude product and 28% aqueous ammonia solution, the title compound was obtained as crystals. yield 73%. melting point 226-228°C.
$^1$H-NMR ($CDCl_3$) δ : 2.82 (3 H, s), 5.66 (2 H, br s), 5.71 (2 H, s), 7.10 - 7.18 (1 H, m), 7.27 - 7.33 (1 H, m), 7.34 - 7.44 (2 H, m), 7.68 (1 H, dd, J = 7.1, 0.5 Hz), 7.86 (1 H, d, J = 8.8 Hz), 8.82 (1 H, s).

Example 87

N-(2-hydroxyethyl)-4-(trifluoromethyl)-2-[2-[3-(trifluoromethyl)benzyl]-1-benzothiophen-7-yl]-1,3-thiazole-5-carboxamide

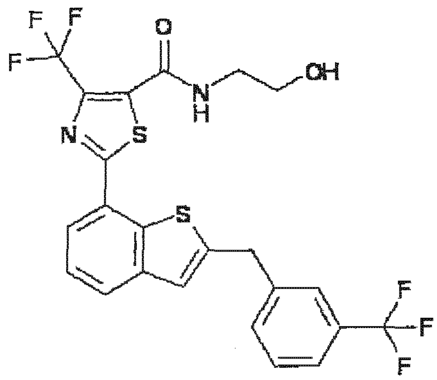

To a solution of ethyl 4-(trifluoromethyl)-2-[2-[3-(trifluoromethyl)benzyl]-1-benzothiophen-7-yl]-1,3-thiazole-5-carboxylate obtained in Reference Example 41 (82 mg, 0.159 mmol) in THF (1 mL)-methanol (0.5 mL) was added 2N aqueous sodium hydroxide solution (240 μL, 0.477 mmol), and the mixture was stirred for 7 hr at room temperature. The reaction mixture was acidified with 1N hydrochloric acid, and extracted with ethyl acetate. The organic layer was washed with saturated brine, dried over anhydrous sodium sulfate, and evaporated under reduced pressure to give crude 4-(trifluoromethyl)-2-[2-[3-(trifluoromethyl)benzyl]-1-benzothiophen-7-yl]-1,3-thiazole-5-carboxylic acid (78 mg). A solution of the compound, WSC (37 mg, 0.191 mmol), HOBt (26 mg, 0.191 mmol) and 2-aminoethanol (8.0 μL, 0.191 mmol) in DMF (2.0 mL) was stirred overnight room temperature. The reaction mixture was diluted with saturated aqueous sodium hydrogen carbonate solution, and extracted with ethyl acetate. The extract was washed with saturated brine, dried over anhydrous magnesium sulfate, and evaporated under reduced pressure. The residue was purified by silica gel column chromatography (hexane:ethyl acetate= 50:50→10:90), and the obtained crystals were recrystallized from hexane-ethyl acetate to give the title compound (36 mg, yield 45%) as crystals. melting point 155-156°C.
$^1$H-NMR ($CDCl_3$) δ : 1.95 (1 H, t, J = 4.9 Hz), 3.63 - 3.71 (2 H, m), 3.81 - 3.93 (2 H, m), 4.34 (2 H, s), 6.70 (1 H, br s), 7.07 (1 H, t, J = 1.1 Hz), 7.38 - 7.62 (5 H, m), 7.77 - 7.88 (2 H, m).

Example 88

N-(2-hydroxyethyl)-4-(trifluoromethyl)-2-[2-[3-(trifluoromethyl)benzyl]-2H-indazol-4-yl]-1,3-thiazole-5-carboxamide

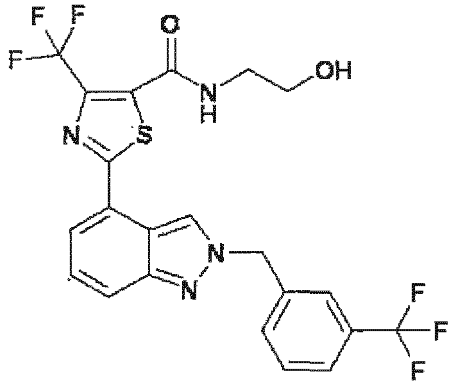

A mixture of ethyl 2-bromo-4-(trifluoromethyl)-1,3-thiazole-5-carboxylate obtained in Reference Example 36 (304 mg, 1.0 mmol), 4-(4,4,5,5-tetramethyl-1,3,2-dioxaborolan-2-yl)-2-[3-(trifluoromethyl)benzyl]-2H-indazole (483 mg, 1.2 mmol), tetrakis(triphenylphosphine)palladium(0) (46 mg, 0.04 mmol) and 2N aqueous sodium carbonate solution (2.0 mL)-1,2-dimethoxyethane (10 mL) was heated under reflux overnight under a nitrogen atmosphere. The reaction mixture was diluted with saturated brine and ethyl acetate, and the mixture was extracted with ethyl acetate. The organic layer was dried over anhydrous sodium sulfate, and concentrated under reduced pressure. The residue was purified by silica gel column chromatography (hexane-ethyl acetate 90:10→67:33, 80:20→ 67:33) to give crude ethyl 4-(trifluoromethyl)-2-[2-[3-(trifluoromethyl)benzyl]-2H-indazol-4-yl]-1,3-thiazole-5-carboxylate (240 mg). To a solution of the compound in THF (3 mL)-methanol (1.5 mL) was added 2N aqueous sodium hydroxide solution (720 μL, 1.44 mmol), and the mixture was stirred for 7 hr at room temperature. The reaction mixture was acidified with 1N hydrochloric acid, and extracted with ethyl acetate. The organic layer was washed with saturated brine, dried over anhydrous sodium sulfate, and evaporated under reduced pressure to give crude 4-(trifluoromethyl)-2-[2-[3-(trifluoromethyl)benzyl]-2H-indazol-4-yl]-1,3-thiazole-5-carboxylic acid (234 mg). A solution of the compound, WSC (110 mg, 0.576 mmol), HOBt (78 mg, 0.576 mmol) and 2-aminoethanol (24.0 μL, 0.576 mmol) in DMF (5.0 mL) was stirred overnight at room temperature. The reaction mixture was diluted with saturated aqueous sodium hydrogen carbonate solution, and extracted with ethyl acetate. The extract was washed with saturated brine, dried over anhydrous magnesium sulfate, and evaporated under reduced pressure. The residue was purified by silica gel column chromatography (hexane:ethyl acetate= 30:70→ 0:100), and the obtained crystals were recrystallized from hexane-ethyl acetate to give the title compound (77 mg, yield 15%, 3 steps) as crystals. melting point 209-210°C.
$^1$H-NMR ($CDCl_3$) δ : 1.99 (1 H, t, J = 4.8 Hz), 3.66 (2 H, q, J = 5.1 Hz), 3.87 (2 H, q, J = 5.1 Hz), 5.72 (2 H, s), 6.69 (1 H, br s), 7.37 (1 H, dd, J = 8.8, 7.1 Hz), 7.43 - 7.53 (2 H, m), 7.55 - 7.70 (3 H, m), 7.90 (1 H, d, J = 8.2 Hz), 8.74 (1 H, s).

Example 89

N-(2-amino-2-oxoethyl)-1-[2-(3-chloro-5-fluorobenzyl)-4-fluoro-1-benzothiophen-7-yl]-5-(hydroxymethyl)-3-methyl-1H-pyrazole-4-carboxamide

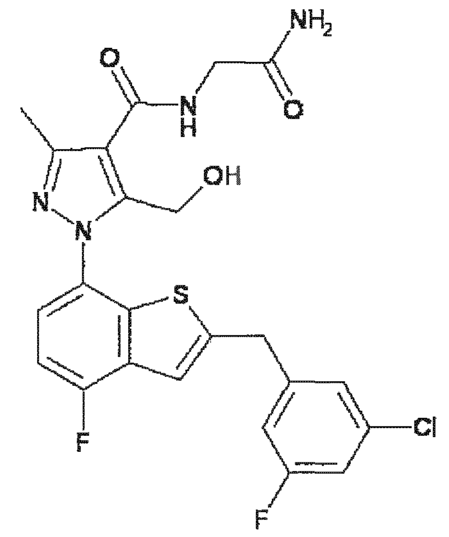

To a solution of 1-[2-(3-chloro-5-fluorobenzyl)-4-fluoro-1-benzothiophen-7-yl]-5-(hydroxymethyl)-3-methyl-1H-pyrazole-4-carboxylic acid synthesized in Reference Example 81 (0.1 g, 0.23 mmol) in DMF (2 mL) were added WSC (0.13 g, 0.67 mmol) and HOBt (0.1 g, 0.67 mmol), and the mixture was stirred for 5 min at room temperature. Glycinamide hydrochloride (37 mg, 0.34 mmol) and diisopropylethylamine (87 mg, 0.68 mmol) were added thereto, and the mixture was stirred overnight. To the reaction mixture was added water, and the mixture was extracted with ethyl acetate. The organic layer was washed with water, saturated aqueous sodium hydrogen carbonate solution, saturated aqueous ammonium chloride solution and saturated brine, dried over anhydrous magnesium sulfate, and concentrated under reduced pressure. The crude crystals were recrystallized from ethyl acetate-hexane to give the title compound (72 mg, yield 64%) as crystals. melting point 207 - 208°C.
$^1$H-NMR ($CDCl_3$) 5: 2.61 (3 H, s), 4.14-4.22 (4 H, m), 4.59 (2 H, d, J = 6.8 Hz), 4.83-4.90 (1 H, m), 5.50 (1 H, brs), 5.84 (1 H, brs), 6.83-6.91 (1 H, m), 6.94-7.02 (2 H, m), 7.03-7.15 (2 H, m), 7.22 (1 H, s), 7.24-7.30 (1 H, m).

Example 90

1-[2-(3-chloro-5-fluorobenzyl)-4-fluoro-1-benzothiophen-7-yl]-N-(2-hydroxyethyl)-5-(hydroxymethyl)-3-methyl-1H-pyrazole-4-carboxamide

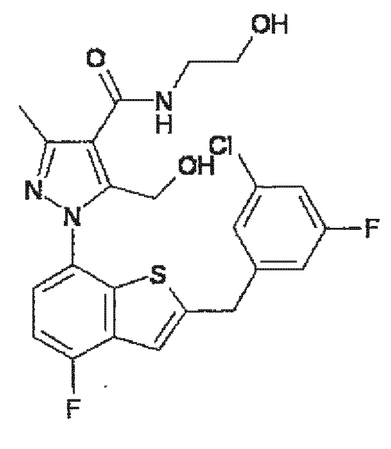

To a solution of 1-[2-(3-chloro-5-fluorobenzyl)-4-fluoro-1-benzothiophen-7-yl]-5-(hydroxymethyl)-3-methyl-1H-pyrazole-4-carboxylic acid synthesized in Reference Example 81 (0.1 g, 0.23 mmol) in DMF (2 mL) were added WSC (0.13 g, 0.67 mmol) and HOBt (0.1 g, 0.67 mmol), and the mixture was stirred for 5 min at room temperature. 2-Aminoethanol (41 mg, 0.67 mmol) was added thereto, and the mixture was stirred overnight. To the reaction mixture was added water, and the mixture was extracted with ethyl acetate. The organic layer was washed with water, saturated aqueous sodium hydrogen carbonate solution, saturated aqueous ammonium chloride solution and saturated brine,

dried over anhydrous magnesium sulfate, and concentrated under reduced pressure. The crude crystals were purified by basic silica gel column chromatography (ethyl acetate:methanol=95:5), and recrystallized from ethyl acetate-hexane to give the title compound (21 mg, yield 19%) as crystals. melting point 196 - 197°C.
$^1$H-NMR ($CDCl_3$) δ: 2.26 (1 H, s), 2.57 (3 H, s), 3.61-3.71 (2 H, m), 3.82-3.92 (2 H, m), 4.17 (2 H, s), 4.58 (2 H, d, J = 6.8 Hz), 4.95 (1 H, t, J = 7.0 Hz), 6.56 (1 H, brs), 6.83-6.92 (1 H, m), 6.95-7.15 (3 H, m), 7.19-7.31 (2 H, m).

Example 91

1-[2-(3-chloro-5-fluorobenzyl)-4-fluoro-1-benzothiophen-7-yl]-5-(hydroxymethyl)-3-methyl-1H-pyrazole-4-carboxamide

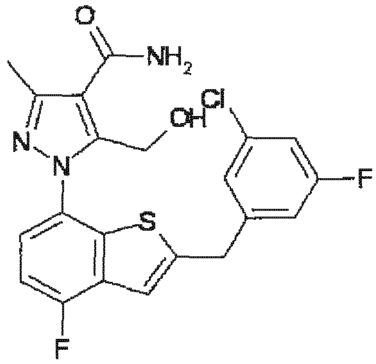

To a solution of 1-[2-(3-chloro-5-fluorobenzyl)-4-fluoro-1-benzothiophen-7-yl]-5-(hydroxymethyl)-3-methyl-1H-pyrazole-4-carboxylic acid synthesized in Reference Example 81 (70 mg, 0.17 mmol) in DMF (2 mL) were added WSC (90 mg, 0.47 mmol) and HOBt (72 mg, 0.47 mmol), and the mixture was stirred for 5 min at room temperature. Aqueous ammonia (1 mL) was added thereto, and the mixture was stirred overnight. To the reaction mixture was added water, and the mixture was extracted with ethyl acetate. The organic layer was washed with water, saturated aqueous sodium hydrogen carbonate solution, saturated aqueous ammonium chloride solution and saturated brine, dried over anhydrous magnesium sulfate, and concentrated under reduced pressure. The residue was purified by basic silica gel column chromatography (ethyl acetate:methanol=95:5), and recrystallized from ethyl acetate-hexane to give the title compound (16 mg, yield 13%) as crystals. melting point 197 - 198°C.
$^1$H-NMR ($CDCl_3$) δ: 2.57 (3 H, s), 4.17 (2 H, s), 4.59 (2 H, d, J = 7.0 Hz), 4.89 (1 H, t, J = 7.0 Hz), 6.84-6.92 (1 H, m), 6.99 (1 H, dt, J = 8.5, 2.0 Hz), 7.05 (1 H, s), 7.07-7.15 (1 H, m), 7.21-7.31 (4 H, m).

Example 92

N-(1-[4-fluoro-2-[3-(trifluoromethyl)benzyl]-1-benzothiophen-7-yl]-3,5-dimethyl-1H-pyrazol-4-yl)propanamide

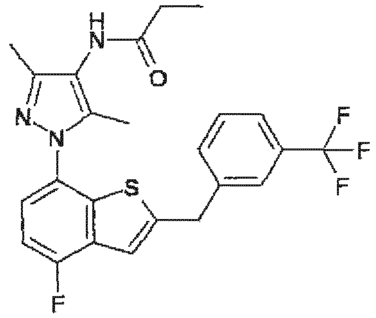

To a solution of 1-[4-fluoro-2-[3-(trifluoromethyl)benzyl]-1-benzothiophen-7-yl]-3,5-dimethyl-1H-pyrazole-4-carboxylic acid synthesized in Reference Example 53 (0.5 g, 1.00 mmol) in anhydrous toluene (5 mL) were added triethylamine (0.14 g, 1.34 mmol), diphenylphosphoryl azide (0.37 g, 1.34 mmol) and t-butyl alcohol (0.10 g, 1.34 mmol),

and the mixture was heated under reflux for 3 hr. To the reaction mixture were added water and saturated aqueous sodium hydrogen carbonate solution, and the mixture was extracted with ethyl acetate. The organic layer was washed with water and saturated brine, dried over anhydrous magnesium sulfate, and concentrated under reduced pressure. To a solution of the obtained residue in ethanol (1 mL) was added 2N hydrochloric acid-2-propyl alcohol solution (2 mL). The mixture was stirred overnight at room temperature, and evaporated under reduced pressure. To a solution of the obtained residue in THF (5 mL) were added triethylamine (0.36 g, 3.59 mmol) and propionyl chloride (28 mg, 0.31 mmol), and the mixture was stirred for 3 hr at room temperature. To the reaction mixture were added water and saturated aqueous ammonium chloride solution, and the mixture was extracted with ethyl acetate. The organic layer was washed with water and saturated brine, dried over anhydrous magnesium sulfate, and concentrated under reduced pressure. The residue was purified by silica gel column chromatography (ethyl acetate:hexane=1:9), and recrystallized from ethyl acetate-hexane to give the title compound (27 mg, yield 2%) as crystals. melting point 157 - 158°C. $^1$H-NMR($CDCl_3$) $\delta$: 1.29 (3 H, t, J = 7.6 Hz), 2.13 (3 H, s), 2.22 (3 H, s), 2.43 (2 H, q, J = 7.6 Hz), 4.25 (2 H, s), 6.51 (1 H, s), 7.02-7.10 (1 H, m), 7.13-7.21 (2 H, m), 7.41-7.56 (4 H, m).

Example 93

N-(2-amino-2-oxoethyl)-3,5-dimethyl-1-[2-(3-methylbenzyl)-1-benzothiophen-7-yl]-1H-pyrazole-4-carboxamide

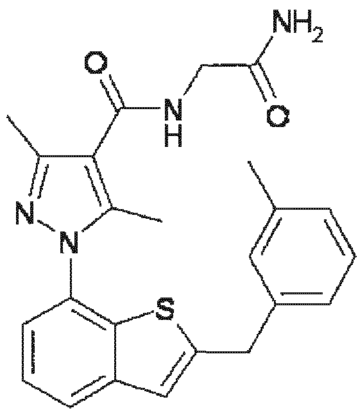

In the same manner as in Example 89 and using 3,5-dimethyl-1-[2-(3-methylbenzyl)-1-benzothiophen-7-yl]-1H-pyrazole-4-carboxylic acid obtained in Reference Example 86 and glycinamide hydrochloride, the title compound was obtained as crystals. yield 46% melting point 154 - 155°C $^1$H-NMR($CDCl_3$) 5: 2.32 (3 H, s), 2.43 (3 H, s), 2.55 (3 H, s), 4.15 (2 H, s), 4.19 (2 H, d, J = 4.9 Hz), 5.68 (1 H, brs), 6.47 (1 H, brs), 6.60 (1 H, t, J = 4.7 Hz), 7.01-7.10 (4 H, m), 7.12-7.23 (2 H, m), 7.39 (1 H, t, J = 8.0 Hz), 7.71 (1 H, d, J = 8.0 Hz).

Example 94

N-(2-hydroxyethyl)-3,5-dimethyl-1-[2-(3-methylbenzyl)-1-benzothiophen-7-yl]-1H-pyrazole-4-carboxamide

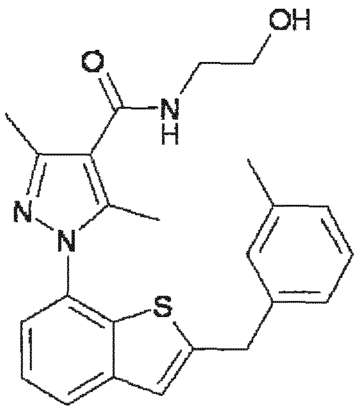

In the same manner as in Example 90 and using 3,5-dimethyl-1-[2-(3-methylbenzyl)-1-benzothiophen-7-yl]-1H-pyrazole-4-carboxylic acid obtained in Reference Example 86 and 2-aminoethanol, the title compound was obtained as crystals. yield 38% melting point 150 - 151°C $^1$H-NMR($CDCl_3$) 5: 2.32 (3 H, s), 2.43 (3 H, s), 2.52 (3 H, s), 2.63 (1 H, t, J = 5.1 Hz), 3.58-3.69 (2 H, m), 3.80-3.91 (2 H, m), 4.15 (2 H, s), 6.15 (1 H, brs), 6.99-7.10 (4 H, m), 7.13-7.23 (2 H, m), 7.39 (1 H, t, J = 7.8 Hz), 7.71 (1 H, d, J = 8.0 Hz).

Example 95

3,5-dimethyl-1-[2-(3-methylbenzyl)-1-benzothiophen-7-yl]-1H-pyrazole-4-carboxamide

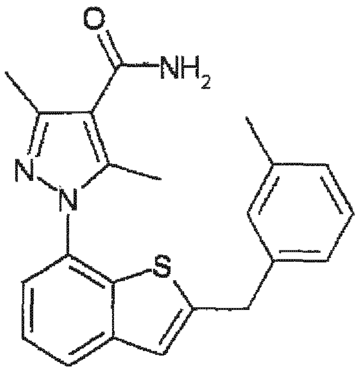

In the same manner as in Example 91 and using 3,5-dimethyl-1-[2-(3-methylbenzyl)-1-benzothiophen-7-yl]-1H-pyrazole-4-carboxylic acid obtained in Reference Example 86 and aqueous ammonia, the title compound was obtained as crystals. yield 56% melting point 200 - 201°C $^1$H-NMR ($CDCl_3$) $\delta$: 2.33 (3 H, s), 2.45 (3 H, s), 2.53 (3 H, s), 4.16 (2 H, s), 5.55 (2 H, brs), 7.02-7.10 (4 H, m), 7.15-7.22 (2 H, m), 7.40 (1 H, t, J = 7.8 Hz), 7.72 (1 H, d, J = 6.8 Hz).

Example 96

N-(2-amino-2-oxoethyl)-1-[2-(4-fluoro-3-methoxybenzyl)-1-benzothiophen-7-yl]-3,5-dimethyl-1H-pyrazole-4-carboxamide

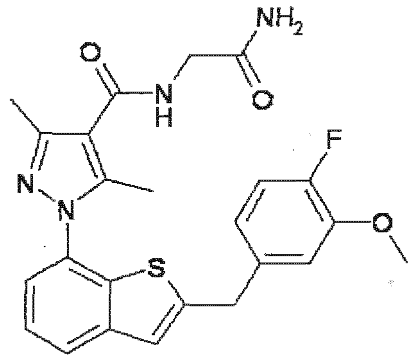

In the same manner as in Example 89 and using 1-[2-(4-fluoro-3-methoxybenzyl)-1-benzothiophen-7-yl]-3,5-dimethyl-1H-pyrazole-4-carboxylic acid obtained in Reference Example 88 and glycinamide hydrochloride, the title compound was obtained as crystals. yield 65% melting point 170 - 171°C $^1$H-NMR($CDCl_3$) δ: 2.45 (3 H, s), 2.56 (3 H, s), 3.85 (3 H, s), 4.11-4.19 (4 H, m), 5.46 (1 H, brs), 5.97 (1 H, brs), 6.33-6.56 (1 H, m), 6.75-6.88 (2 H, m), 6.96-7.09 (2 H, m), 7.19 (1 H, d, J = 7.6 Hz), 7.41 (1 H, t, J = 7.8 Hz), 7.73 (1 H, d, J = 8.0 Hz).

Example 97

1-[2-(4-fluoro-3-methoxybenzyl)-1-benzothiophen-7-yl]-N-(2-hydroxyethyl)-3,5-dimethyl-1H-pyrazole-4-carboxamide

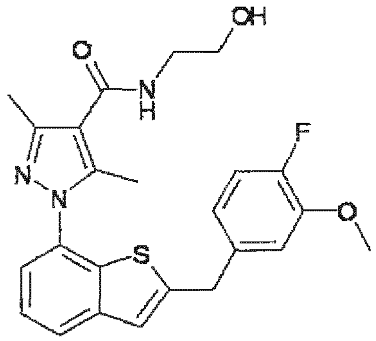

In the same manner as in Example 90 and using 1-[2-(4-fluoro-3-methoxybenzyl)-1-benzothiophen-7-yl]-3,5-dimethyl-1H-pyrazole-4-carboxylic acid obtained in Reference Example 88 and 2-aminoethanol, the title compound was obtained as crystals. yield 41% melting point 163 - 164°C $^1$H-NMR ($CDCl_3$) δ: 2.44 (3 H, s), 2.52 (3 H, s), 2.61 (1 H, t, J = 4.9 Hz), 3.60-3.69 (2 H, m), 3.81-3.89 (5H, m), 4.15 (2 H, s), 6.15 (1 H, brs), 6.75-6.88 (2 H, m), 7.01 (1 H, dd, J = 11.4, 8.3 Hz), 7.07 (1 H, s), 7.18 (1 H, d, J = 6.8 Hz), 7.41 (1 H, t, J = 7.8 Hz), 7.72 (1 H, d, J = 7.2 Hz).

Example 98

1-[2-(4-fluoro-3-methoxybenzyl)-1-benzothiophen-7-yl]-3,5-dimethyl-1H-pyrazole-4-carboxamide

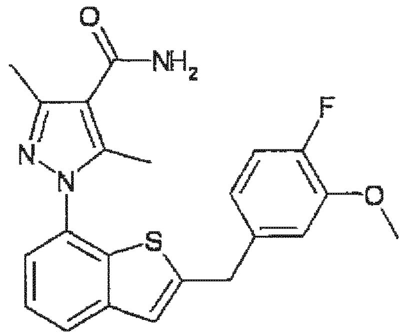

In the same manner as in Example 91 and using 1-[2-(4-fluoro-3-methoxybenzyl)-1-benzothiophen-7-yl]-3,5-dimethyl-1H-pyrazole-4-carboxylic acid obtained in Reference Example 88 and aqueous ammonia, the title compound was obtained as crystals. yield 40% melting point 164 - 165°C $^1$H-NMR ($CDCl_3$) δ: 2.46 (3 H, s), 2.54 (3 H, s), 3.85 (3 H, s), 4.16 (2 H, s), 5.55 (2 H, brs), 6.75-6.88 (2 H, m), 7.01 (1 H, dd, J = 11.2, 8.1 Hz), 7.07 (1 H, s), 7.19 (1 H, d, J = 6.8 Hz), 7.41 (1 H, t, J = 7.8 Hz), 7.73 (1 H, d, J = 8.0 Hz).

Example 99

1-[2-(3,5-difluorobenzyl)-1-benzothiophen-7-yl]-N-(2-hydroxyethyl)-3,5-dimethyl-1H-pyrazole-4-carboxamide

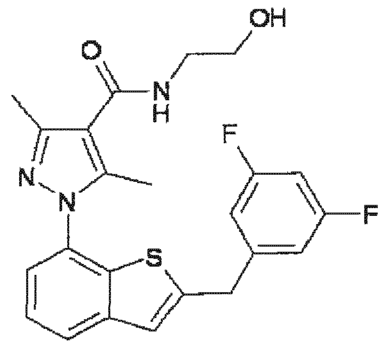

In the same manner as in Example 90 and using 1-[2-(3,5-difluorobenzyl)-1-benzothiophen-7-yl]-3,5-dimethyl-1H-pyrazole-4-carboxylic acid obtained in Reference Example 87 and 2-aminoethanol, the title compound was obtained as crystals. yield 39% melting point 159 - 160°C $^1$H-NMR ($CDCl_3$) δ: 2.45 (3 H, s), 2.53 (3 H, s), 2.61 (1 H, t, J = 4.9 Hz), 3.59-3.69 (2 H, m), 3.80-3.92 (2 H, m), 4.17 (2 H, s), 6.16 (1 H, brs), 6.65-6.74 (1 H, m), 6.74-6.83 (2 H, m), 7.11 (1 H, s), 7.20 (1 H, d, J = 7.6 Hz), 7.42 (1 H, t, J = 7.8 Hz), 7.74 (1 H, d, J = 8.0 Hz) .

Example 100

1-[2-(3,5-difluorobenzyl)-1-benzothiophen-7-yl]-3,5-dimethyl-1H-pyrazole-4-carboxamide

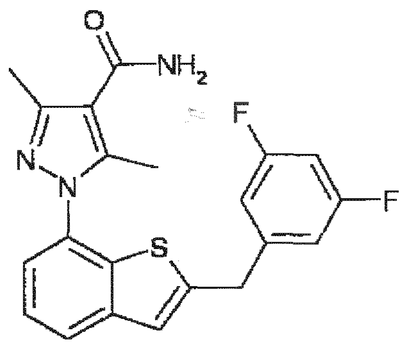

In the same manner as in Example 91 and using 1-[2-(3,5-difluorobenzyl)-1-benzothiophen-7-yl]-3,5-dimethyl-1H-pyrazole-4-carboxylic acid obtained in Reference Example 87 and aqueous ammonia, the title compound was obtained as crystals. yield 39% melting point 208 - 209°C $^1$H-NMR ($CDCl_3$) 5: 2.47 (3 H, s), 2.54 (3 H, s), 4.18 (2 H, s), 5.56 (2 H, brs), 6.64-6.75 (1 H, m), 6.76-6.84 (2 H, m), 7.12 (1 H, s), 7.21 (1 H, d, J = 6.4 Hz), 7.43 (1 H, t, J = 7.8 Hz), 7.75 (1 H, d, J = 8.0 Hz).

Example 101

N-(2-amino-2-oxoethyl)-1-[2-(3,5-difluorobenzyl)-1-benzothiophen-7-yl]-3,5-dimethyl-1H-pyrazole-4-carboxamide

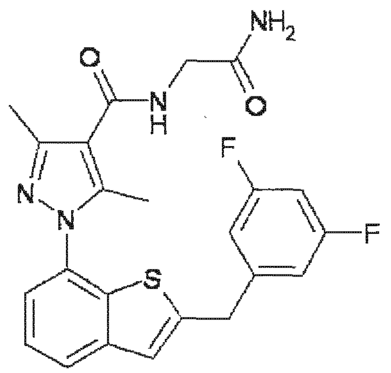

In the same manner as in Example 89 and using 1-[2-(3,5-difluorobenzyl)-1-benzothiophen-7-yl]-3,5-dimethyl-1H-pyrazole-4-carboxylic acid obtained in Reference Example 87 and glycinamide hydrochloride, the title compound was obtained as crystals. yield 47% melting point 149 - 150°C $^1$H-NMR ($CDCl_3$) $\delta$: 2.46 (3 H, s), 2.57 (3 H, s), 4.15-4.19 (4 H, m), 5.46 (1 H, brs), 6.01 (1 H, brs), 6.44-6.52 (1 H, m), 6.64-6.74 (1 H, m), 6.74-6.84 (2 H, m), 7.11 (1 H, s), 7.21 (1 H, d, J = 7.6 Hz), 7.43 (1 H, t, J = 7.8 Hz), 7.75 (1 H, d, J = 6.8 Hz).

Example 102

1-[4-fluoro-2-[3-(trifluoromethyl)benzyl]-1-benzothiophen-7-yl]-N,3,5-trimethyl-1H-pyrazole-4-carboxamide

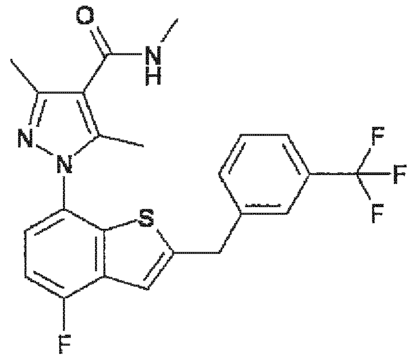

To a solution of 1-[4-fluoro-2-[3-(trifluoromethyl)benzyl]-1-benzothiophen-7-yl]-3,5-dimethyl-1H-pyrazole-4-carboxylic acid obtained in Reference Example 53 (0.1 g, 0.22 mmol) in DMF (3 mL) were added WSC (0.13 g, 0.67 mmol) and HOBt (0.1 g, 0.67 mmol), and the mixture was stirred for 5 min at room temperature. Methylamine (21 mg, 0.67 mmol) was added thereto, and the mixture was stirred overnight. To the reaction mixture was added water, and the mixture was extracted with ethyl acetate. The organic layer was washed with water, saturated aqueous sodium hydrogen carbonate solution, saturated aqueous ammonium chloride solution and saturated brine, dried over anhydrous magnesium sulfate, and concentrated under reduced pressure. The crude crystals were purified by basic silica gel column chromatography (ethyl acetate:methanol=95:5), and recrystallized from ethyl acetate-hexane to give the title compound (11 mg, yield 11%) as crystals. melting point 177 - 178°C.
$^1$H-NMR ($CDCl_3$) δ: 2.41 (3 H, s), 2.49 (3 H, s), 3.01 (3 H, d, J = 4.9 Hz), 4.26 (2 H, s), 5.65 (1 H, brs), 7.03-7.22 (3 H, m), 7.41-7.58 (4 H, m).

Example 103

N-cyclopropyl-1-[4-fluoro-2-[3-(trifluoromethyl)benzyl]-1-benzothiophen-7-yl]-3,5-dimethyl-1 H-pyrazole-4-carboxamide

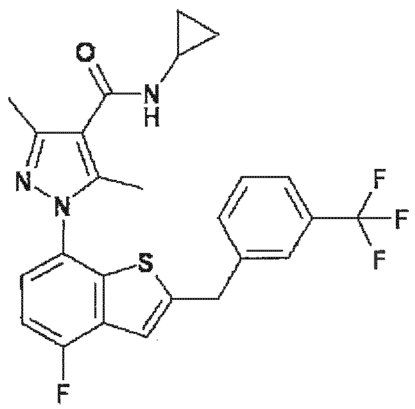

To a solution of 1-[4-fluoro-2-[3-(trifluoromethyl)benzyl]-1-benzothiophen-7-yl]-3,5-dimethyl-1H-pyrazole-4-carboxylic acid obtained in Reference Example 53 (0.1 g, 0.22 mmol) in DMF (3 mL) were added WSC (0.13 g, 0.67 mmol) and HOBt (0.1 g, 0.67 mmol), and the mixture was stirred for 5 min at room temperature. Cyclopropylamine (38 mg, 0.67 mmol) was added thereto, and the mixture was stirred overnight. To the reaction mixture was added water, and the mixture was extracted with ethyl acetate. The organic layer was washed with water, saturated aqueous sodium hydrogen carbonate solution, saturated aqueous ammonium chloride solution and saturated brine, dried over anhydrous magnesium sulfate, and concentrated under reduced pressure. The crude crystals were purified by basic silica gel column chromatography (ethyl acetate:methanol=95:5), and recrystallized from ethyl acetate-hexane to give the title compound (24 mg, yield 22%) as crystals. melting point 169 - 170°C.
$^1$H-NMR ($CDCl_3$) 5: 0.52-0.68 (2 H, m), 0.81-0.94 (2 H, m), 2.41 (3 H, s), 2.45 (3 H, s), 2.80-2.94 (1 H, m), 4.26 (2 H, s), 5.80 (1 H, brs), 7.02-7.22 (3 H, m), 7.41-7.59 (4 H, m).

Example 104

3,5-dimethyl-1-[2-[3-(trifluoromethyl)benzyl]-1-benzothiophen-4-yl]-1H-pyrazole-4-carboxamide

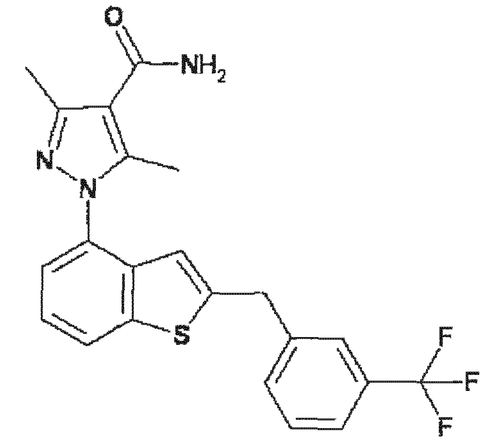

In the same manner as in Example 91 and using 3,5-dimethyl-1-[2-[3-(trifluoromethyl)benzyl]-1-benzothiophen-4-yl]-1H-pyrazole-4-carboxylic acid obtained in Reference Example 92 and aqueous ammonia, the title compound was obtained as crystals. yield 34% melting point 204 - 205°C $^1$H-NMR ($CDCl_3$) 5: 2.42 (3 H, s), 2.55 (3 H, s), 4.26 (2 H, s), 5.58 (2 H, brs), 6.83 (1 H, s), 7.21-7.29 (1 H, m), 7.37 (1 H, t, J = 7.9 Hz), 7.41-7.47 (2 H, m), 7.48-7.56 (2 H, m), 7.82 (1 H, d, J = 7.9 Hz).

Example 105

N-(2-amino-2-oxoethyl)-3,5-dimethyl-1-[2-[3-(trifluoromethyl)benzyl]-1-benzothiophen-4-yl]-1H-pyrazole-4-carboxamide

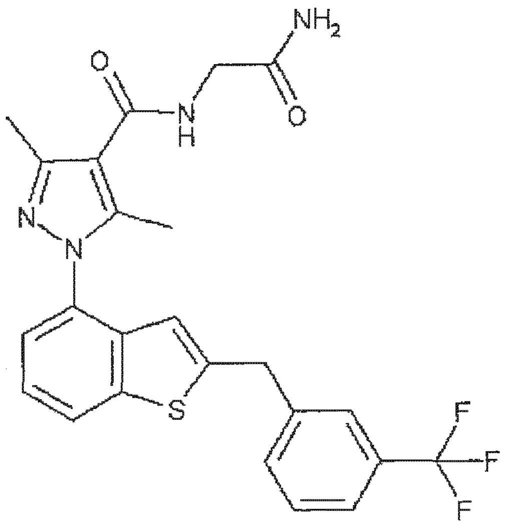

In the same manner as in Example 89 and using 3,5-dimethyl-1-[2-[3-(trifluoromethyl)benzyl]-1-benzothiophen-4-yl]-1H-pyrazole-4-carboxylic acid obtained in Reference Example 92 and glycinamide hydrochloride, the title compound was obtained as crystals. yield 63% melting point 185 - 186°C $^1$H-NMR ($CDCl_3$) $\delta$: 2.42 (3 H, s), 2.57 (3 H, s), 4.18 (2 H, d, J = 4.9 Hz), 4.26 (2 H, s), 5.46 (1 H, brs), 5.98 (1 H, brs), 6.48 (1 H, brs), 6.84 (1 H, s), 7.21-7.30 (1 H, m), 7.37 (1 H, t, J = 7.9 Hz), 7.41-7.47 (2 H, m), 7.48-7.56 (2 H, m), 7.82 (1 H, d, J = 7.9 Hz).

Example 106

N-(2-hydroxyethyl)-3,5-dimethyl-1-[2-[3-(trifluoromethyl)benzyl]-1-benzothiophen-4-yl]-1H-pyrazole-4-carboxamide

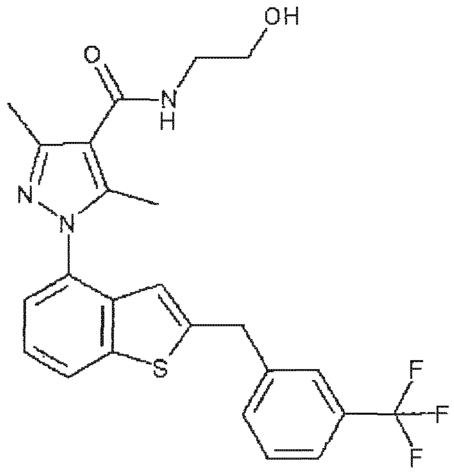

In the same manner as in Example 90 and using 3,5-dimethyl-1-[2-[3-(trifluoromethyl)benzyl]-1-benzothiophen-4-yl]-1H-pyrazole-4-carboxylic acid obtained in Reference Example 92 and 2-aminoethanol, the title compound was obtained as crystals. yield 15% melting point 109 - 110°C $^1$H-NMR ($CDCl_3$) δ: 2.40 (3 H, s), 2.54 (3 H, s), 2.63 (1 H, t, J = 4.9 Hz), 3.60-3.72 (2 H, m), 3.81-3.92 (2 H, m), 4.26 (2 H, s), 6.18 (1 H, brs), 6.83 (1 H, s), 7.21-7.31 (1 H, m), 7.37 (1 H, t, J = 7.9 Hz), 7.41-7.48 (2 H, m), 7.48-7.58 (2 H, m), 7.82 (1 H, d, J = 8.3 Hz).

Example 107

N-(2-amino-2-oxoethyl)-1-[2-(3-chloro-5-fluorobenzyl)-1-benzothiophen-4-yl]-3,5-dimethyl-1H-pyrazole-4-carboxamide

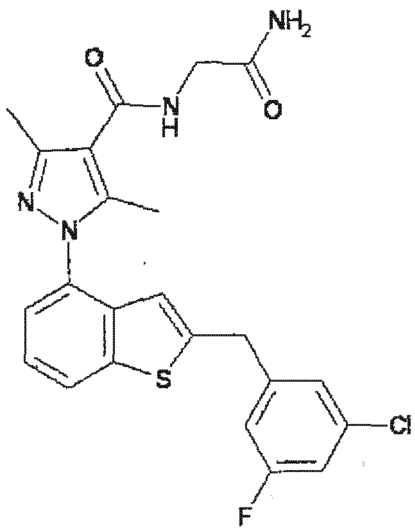

In the same manner as in Example 89 and using 1-[2-(3-chloro-5-fluorobenzyl)-1-benzothiophen-4-yl]-3,5-dimethyl-1H-pyrazole-4-carboxylic acid obtained in Reference Example 93 and glycinamide hydrochloride, the title compound was obtained as crystals. yield 39% melting point 139 - 140°C $^1$H-NMR ($CDCl_3$) 5: 2.42 (3 H, s), 2.58 (3 H, s), 4.14-4.21 (4 H, m), 5.46 (1 H, brs), 5.98 (1 H, brs), 6.49 (1 H, brs), 6.82-6.92 (2 H, m), 6.98 (1 H, dt, J = 8.3, 2.1 Hz), 7.05 (1 H, s), 7.22-7.30 (1 H, m), 7.35-7.42 (1 H, m), 7.83 (1 H, d, J = 8.0 Hz).

Example 108

1-[2-(3-chloro-5-fluorobenzyl)-1-benzothiophen-4-yl]-N-(2-hydroxyethyl)-3,5-dimethyl-1H-pyrazole-4-carboxamide

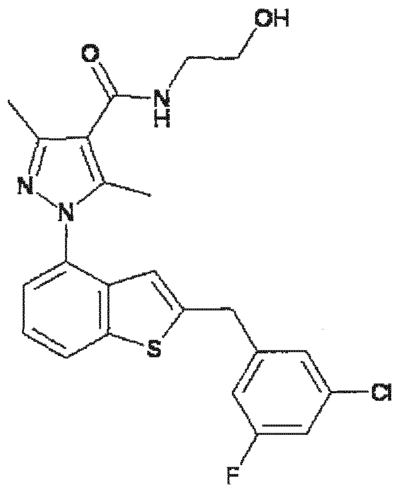

In the same manner as in Example 90 and using 1-[2-(3-chloro-5-fluorobenzyl)-1-benzothiophen-4-yl]-3,5-dimethyl-1H-pyrazole-4-carboxylic acid obtained in Reference Example 93 and 2-aminoethanol, the title compound was obtained as crystals. yield 22% melting point 142 - 143°C
$^1$H-NMR ($CDCl_3$) δ: 2.41 (3 H, s), 2.54 (3 H, s), 2.63 (1 H, t, J = 4.9 Hz), 3.60-3.71 (2 H, m), 3.81-3.94 (2 H, m), 4.16 (2 H, s), 6.18 (1 H, brs), 6.80-6.92 (2 H, m), 6.94-7.01 (1 H, m), 7.05 (1 H, s), 7.22-7.30 (1 H, m), 7.33-7.43 (1 H, m), 7.83 (1 H, d, J = 7.6 Hz).

Example 109

1-[2-(3-chloro-5-fluorobenzyl)-1-benzothiophen-4-yl]-3,5-dimethyl-1H-pyrazole-4-carboxamide

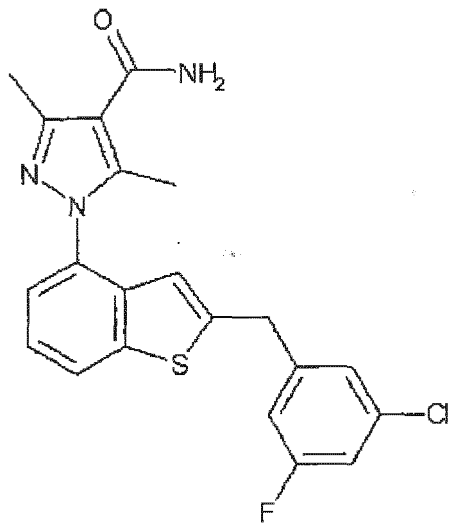

In the same manner as in Example 91 and using 1-[2-(3-chloro-5-fluorobenzyl)-1-benzothiophen-4-yl]-3,5-dimethyl-1H-pyrazole-4-carboxylic acid obtained in Reference Example 93 and aqueous ammonia, the title compound was obtained as crystals. yield 28% melting point 186 - 187°C
$^1$H-NMR ($CDCl_3$) δ: 2.43 (3 H, s), 2.56 (3 H, s), 4.16 (2 H, s), 5.57 (2 H, brs), 6.80-6.91 (3 H, m), 6.98 (1 H, d, J = 8.3

Hz), 7.05 (1 H, s), 7.33-7.44 (1 H, m), 7.83 (1 H, d, J = 8.0 Hz).

Example 110

N-(2-amino-2-oxoethyl)-1-methyl-3-[2-[3-(trifluoromethyl)benzyl]-1-benzothiophen-7-yl]-1H-pyrazole-5-carboxamide

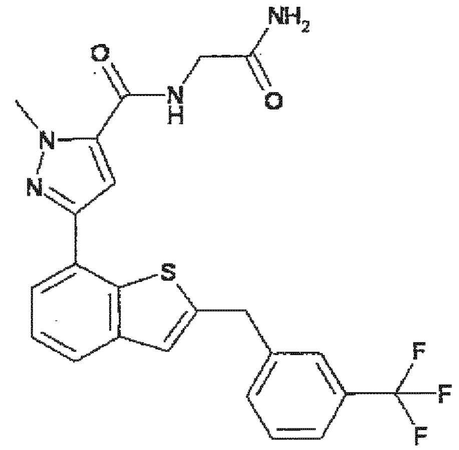

In the same manner as in Example 89 and using 1-methyl-3-[2-[3-(trifluoromethyl)benzyl]-1-benzothiophen-7-yl]-1H-pyrazole-5-carboxylic acid obtained in Reference Example 95 and glycinamide hydrochloride, the title compound was obtained as crystals. yield 51% melting point 196 - 197°C $^1$H-NMR ($CDCl_3$) δ: 4.15 (2 H, d, J = 4.9 Hz), 4.27 (3 H, s), 4.32 (2 H, s), 5.52 (1 H, brs), 5.82 (1 H, brs), 6.84 (1 H, brs), 7.07 (2 H, s), 7.35-7.48 (2 H, m), 7.48-7.55 (2 H, m), 7.58 (1 H, s), 7.66 (2 H, dd, J = 11.2, 7.8 Hz).

Example 111

N-(2-hydroxyethyl)-1-methyl-3-[2-[3-(trifluoromethyl)benzyl]-1-benzathiophen-7-yl]-1H-pyrazole-5-carboxamide

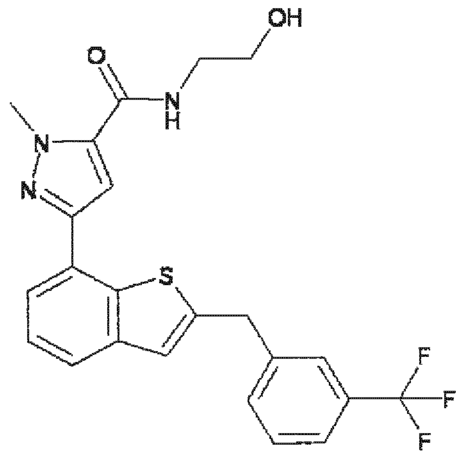

In the same manner as in Example 90 and using 1-methyl-3-[2-[3-(trifluoromethyl)benzyl]-1-benzothiophen-7-yl]-1H-pyrazole-5-carboxylic acid obtained in Reference Example 95 and 2-aminoethanol, the title compound was obtained as crystals. yield 67% melting point 127 - 128°C

$^1$H-NMR ($CDCl_3$) δ: 2.14 (1 H, t, J = 4.9 Hz), 3.63 (2 H, q, J = 5.3 Hz), 3.86 (2 H, q, J = 4.9 Hz), 4.27 (3 H, s), 4.32 (2 H, s), 6.51 (1 H, brs), 6.99 (1 H, s), 7.08 (1 H, s), 7.35-7.47 (2 H, m), 7.48-7.55 (2 H, m), 7.58 (1 H, s), 7.66 (2 H, dd, J = 12.1, 7.2 Hz).

Experimental Example 1

Increase of intracellular cAMP concentration in human GPR52-expressing CHO cell

Using OptiPlate-384 (PerkinElmer Inc.), $1 \times 10^4$ human GPR52-expressing CHO(dhfr-) cells were incubated together with various concentration($10^{-5}\mu M$ - $10\mu m$) of a test compound in an assay buffer (30 μL, HBSS (containing $Ca^{2+}$ and $Mg^{2+}$), 0.5% BSA, 100 μM IBMX, 100 μM Ro20-1724, 5 mM HEPES (pH 7.55)) at 37°C for 30 min. Thereafter, according to the protocol of AlphaScreen CAMP Assay Kit (PerkinElmer Inc.), the intracellular cAMP concentration was measured by EnVision (PerkinElmer Inc.). The GPR52 agonist activity was calculated, assuming the intracellular cAMP concentration in the presence of 1 μN N-(2-hydroxyethyl)-3-[2-[3-(trifluoromethyl)benzyl]-1-benzofuran-4-yl]benzamide (Reference Example 79) to be 100% and assuming the intracellular cAMP concentration when DMSO was added instead of the test compound to be 0%. The results of GPR52 agonist activity (%) of 1μM test compound are shown in Table 1-1 and Table 1-2. In addition, EC50 of some of the compounds was calculated as the concentration of the test compound at which it shows 50% activity. The results are shown in Table 2.

Table 1-1

| Example No. | GPR52 agonist activity (%) |
|---|---|
| 11 | 70 |
| 12 | 107 |
| 13 | 79 |
| 14 | 87 |
| 15 | 102 |
| 16 | 94 |
| 17 | 96 |
| 20 | 79 |
| 21 | 94 |
| 22 | 93 |
| 26 | 96 |
| 28 | 95 |
| 29 | 97 |
| 30 | 96 |
| 31 | 84 |
| 33 | 79 |
| 36 | 96 |
| 37 | 97 |
| 38 | 101 |
| 39 | 93 |
| 40 | 90 |
| 44 | 92 |
| 46 | 90 |
| 47 | 93 |
| 48 | 89 |
| 50 | 79 |
| 51 | 102 |

(continued)

| Example No. | GPR52 agonist activity (%) |
|---|---|
| 52 | 107 |
| 53 | 102 |
| 54 | 101 |
| 55 | 96 |
| 56 | 105 |
| 57 | 96 |
| 58 | 92 |
| 59 | 93 |
| 60 | 99 |
| 61 | 93 |
| 62 | 105 |
| 63 | 94 |
| 64 | 97 |
| 65 | 99 |
| 66 | 89 |
| 67 | 83 |

Table 1-2

| Example No. | GPR52 agonist activity (%) |
|---|---|
| 68 | 88 |
| 69 | 78 |
| 70 | 91 |
| 71 | 114 |
| 72 | 96 |
| 73 | 97 |
| 74 | 98 |
| 75 | 101 |
| 76 | 95 |
| 78 | 104 |
| 79 | 102 |
| 80 | 98 |
| 81 | 102 |
| 82 | 105 |
| 83 | 104 |
| 84 | 105 |
| 86 | 85 |
| 86 | 102 |

(continued)

| Example No. | GPR52 agonist activity (%) |
|---|---|
| 87 | 92 |
| 88 | 83 |
| 89 | 99 |
| 90 | 104 |
| 91 | 99 |
| 93 | 70 |
| 94 | 96 |
| 95 | 95 |
| 96 | 84 |
| 97 | 94 |
| 98 | 98 |
| 99 | 99 |
| 100 | 104 |
| 101 | 96 |
| 102 | 99 |
| 103 | 97 |
| 104 | 93 |
| 105 | 94 |
| 106 | 97 |
| 107 | 100 |
| 108 | 93 |
| 109 | 89 |
| 110 | 94 |
| 111 | 89 |

Table 2

| Example No. | GPR52 agonist activity EC50 (nM) |
|---|---|
| 11 | 290 |
| 14 | 14 |
| 15 | 5.7 |
| 17 | 11 |
| 41 | 49 |
| 56 | 6.9 |
| 58 | 40 |
| 64 | 18 |
| 68 | 170 |
| 71 | 27 |

(continued)

| Example No. | GPR52 agonist activity EC50 (nM) |
|---|---|
| 75 | 30 |
| 90 | 14 |
| 99 | 13 |
| 111 | 38 |

Experimental Example 2

Inhibition test of methamphetamine-induced locomotor hyperactivity

The compound of the present invention can be evaluated by assessing its capacity to inhibit methamphetamine-induced locomotor hyperactivity in mice. The method of Okuyama et al (Life Science, Vol. 65, p. 2109-2125 (1999)) was modified and used for testing the inhibition of methamphetamine-induced locomotor hyperactivity.

Test Method

Locomotor activity was measured using a 32-channel locomotor activity measurement device MDC-LT (Brain-Science Idea Co., Ltd.), which emits infrared beams from the top of a cage (30 x 40 x 20 cm). 6- to 8-Week-old male ICR mice were acclimated for at least 45 min in the measurement cages, and then either test compound (10 mg/kg body weight) dissolved in vehicle (0.5% aqueous methylcellulose solution) or the vehicle alone was orally administered. This oral administration was followed 60 min later by subcutaneous administration of methamphetamine (2 mg/kg body weight).

Activity Calculation

The number of times the laboratory animals passed through the infrared beams emitted inside the measurement cages in 150 min after administration of the test compound or vehicle was counted to quantify locomotor activity. The methamphetamine-induced locomotor hyperactivity inhibition rate (activity inhibition rate (%)) was calculated by the following equation.

Activity inhibition rate (%) =
{1−(locomotor activity of test compound treatment group after treatment with methamphetamine ÷ locomotor activity of vehicle treatment group after treatment with methamphetamine)} x 100

The results are shown in Table 3
Table 3

| Example No. | Activity inhibition rate (%) |
|---|---|
| 14 | 53 |
| 15 | 48 |
| 17 | 43 |
| 58 | 47 |
| 65 | 51 |

Formulation Example 1

| | |
|---|---|
| (1) Compound of Example 1 | 10.0 g |
| (2) Lactose | 70.0 g |
| (3) Cornstarch | 50.0 g |
| (4) Soluble starch | 7.0 g |
| (5) Magnesium stearate | 3.0 g |

The compound of Example 1 (10.0 g) and magnesium stearate (3.0 g) are granulated with an aqueous solution (70 ml) of soluble starch (7.0 g as soluble starch), dried, and the resulting mixture is mixed with lactose (70.0 g) and cornstarch (50.0 g) (lactose, cornstarch, soluble starch and magnesium stearate are all products on the Japanese Pharmacopoeia 14th ed.). The mixture is compressed to give tablets.

**Industrial Applicability**

The medicament of the present invention is useful as a medicament for the prophylaxis or treatment of mental disorders such as schizophrenia and the like.

This application is based on patent application No. 2009-293279 filed in Japan, the contents of which are encompassed in full herein.

**Claims**

1. A compound represented by the formula (I)

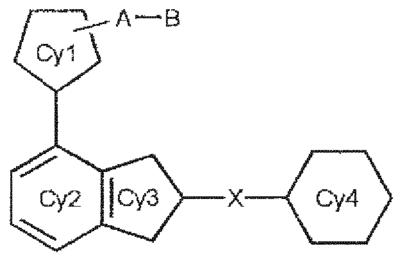

(I)

wherein
ring Cy1 is a 5-membered aromatic heterocycle optionally further substituted besides a group represented by -A-B,
ring Cy2 is an optionally substituted benzene ring,
ring Cy3 is an optionally substituted 5-membered aromatic heterocycle,
ring Cy4 is an optionally substituted 6-membered aromatic ring, A is -CONR$^a$- or -NR$^a$CO-,
R$^a$ is a hydrogen atom or a substituent,
B is a hydrogen atom or an optionally substituted $C_{1-6}$ alkyl group,
X is an optionally substituted $C_{1-2}$ alkylene, -Y-, -Y-$CH_2$- or - $CH_2$-Y-,
Y is an oxygen atom, -NR$^b$- or -S(O)$_m$-,
R$^b$ is a hydrogen atom or a substituent, and
m is 0, 1 or 2,
provided that N-methyl-4-[2-[(3,4,5-trimethoxyphenyl)amino]-1,3-benzoxazol-7-yl]thiophene-2-carboxamide is excluded,
or a salt thereof.

2. The compound or salt of claim 1, wherein R$^b$ is a substituent.

3. The compound or salt of claim 1, wherein ring Cy1 is a 5-membered nitrogen-containing aromatic heterocycle optionally further substituted by substituent (s) selected from

(i) optionally substituted lower alkyl, and
(ii) optionally substituted $C_{3-8}$ cycloalkyl,

besides a group represented by -A-B.

**4.** The compound or salt of claim 3, wherein the 5-membered nitrogen-containing aromatic heterocycle is pyrrole, imidazole, pyrazole, thiazole or oxazole.

**5.** The compound or salt of claim 1, wherein ring Cy2 is a benzene ring optionally substituted by substituent(s) selected from (i) a halogen atom and (ii) $C_{1-6}$ alkyl.

**6.** The compound or salt of claim 1, wherein ring Cy3 is a 5-membered aromatic heterocycle optionally substituted by $C_{1-6}$ alkyl.

**7.** The compound or salt of claim 6, wherein the 5-membered aromatic heterocycle is thiophene, furan, pyrazole or thiazole.

**8.** The compound or salt of claim 1, wherein ring Cy4 is a 6-membered aromatic ring optionally substituted by substituent (s) selected from

(i) a halogen atom,
(ii) cyano,
(iii) optionally substituted lower alkyl,
(iv) optionally substituted lower alkoxy, and
(v) optionally substituted lower alkylsulfonyl.

**9.** The compound or salt of claim 8, wherein the 6-membered aromatic ring is benzene, pyridine, pyridazine, pyrimidine, pyrazine or triazine.

**10.** The compound or salt of claim 8, wherein the 6-membered aromatic ring is benzene.

**11.** The compound or salt of claim 1, wherein A is $-CONR^a-$wherein $R^a$ is a hydrogen atom, an optionally substituted $C_{1-6}$ alkyl group or $C_{3-8}$ cycloalkyl, or -NHCO-.

**12.** The compound or salt of claim 1, wherein B is

(i) a hydrogen atom, or
(ii) a $C_{1-6}$ alkyl group optionally substituted by substituent(s) selected from

(a) hydroxy,
(b) $C_{1-6}$ alkoxy, and
(c) carbamoyl.

**13.** The compound or salt of claim 1, wherein X is an $C_{1-2}$ alkylene.

**14.** N-(2-Methoxyethyl)-3-methyl-1-[2-[3-(trifluoromethyl)benzyl]-1-benzothiophen-7-yl]-1H-pyrazole-4-carboxamide or a salt thereof.

**15.** N-(2-Hydroxyethyl)-3-methyl-1-[2-[3-(trifluoromethyl)benzyl]-1-benzothiophen-7-yl]-1H-pyrazole-4-carboxamide or a salt thereof.

**16.** 3-Methyl-1-[2-[3-(trifluoromethyl)benzyl]-1-benzothiophen-7-yl]-1H-pyrazole-4-carboxamide or a salt thereof.

**17.** N-(2-Amino-2-oxoethyl)-1-[4-fluoro-2-[3-(trifluoromethyl)benzyl]-1-benzothiophen-7-yl]-3,5-dimethyl-1H-pyrazole-4-carboxamide or a salt thereof.

**18.** N-(2-Hydroxyethyl)-5-(hydroxlmethyl)-3-methyl-1-[2-[3-(trifluoromethyl)benzyl]-1-benzothiophen-7-yl]-1H-pyrazole-4-carboxamide or a salt thereof.

**19.** A medicament comprising the compound or salt of claim 1.

**20.** A GPR52 activating agent comprising the compound or salt of claim 1.

**21.** An agent for the prophylaxis or treatment of schizophrenia, which comprises the compound or salt of claim 1.

**22.** A method of activating GPR52, which comprises administering an effective amount of the compound or salt of claim 1 to a mammal.

**23.** A method for the prophylaxis or treatment of schizophrenia, which comprises administering an effective amount of the compound or salt of claim 1 to a mammal.

**24.** Use of the compound or salt of claim 1 for the production of a GPR52 activating agent.

**25.** Use of the compound or salt of claim 1 for the production of an agent for the prophylaxis or treatment of schizophrenia.

**26.** The compound or salt of claim 1 for use in the prophylaxis or treatment of schizophrenia.

**INTERNATIONAL SEARCH REPORT**

International application No. PCT/JP2010/073426

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
C07D231/56, A61K31/4025, A61K31/4155, A61K31/4178, A61K31/422, A61K31/427, A61P21/02, A61P25/00, A61P25/14, A61P25/16, A61P25/18, A61P25/22, A61P25/24, A61P25/28, A61P43/00, C07D405/04, C07D409/04,

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br><br>A | JP 2008-539247 A (Takeda Chemical Industries, Ltd.),<br>13 November 2008 (13.11.2008),<br>compound of each example (particularly, examples 42, 46, 59, 65 to 70)<br>& EP 1874736 A & WO 2006/116412 A2<br>& AR 53859 A & CA 2605453 A<br>& KR 10-2008-0000625 A & NO 20075936 A<br>& CN 101166729 A & ZA 200708844 A<br>& NZ 562235 A & IL 186407 D | 1-13,19-21, 24-26<br>14-18 |
| A | WO 2009/107391 A1 (Takeda Chemical Industries, Ltd.),<br>03 September 2009 (03.09.2009),<br>& CA 2716898 A | 1-21,24-26 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
19 January, 2011 (19.01.11)

Date of mailing of the international search report
01 February, 2011 (01.02.11)

Name and mailing address of the ISA/
Japanese Patent Office

Facsimile No.

Authorized officer

Telephone No.

Form PCT/ISA/210 (second sheet) (July 2009)

**INTERNATIONAL SEARCH REPORT**

International application No.
PCT/JP2010/073426

C (Continuation). DOCUMENTS CONSIDERED TO BE RELEVANT

| Category* | Citation of document, with indication, where appropriate, of the relevant passages | Relevant to claim No. |
|---|---|---|
| A | JP 2007-514768 A (BRISTOL-MYERS SQUIBB CO.), 07 June 2007 (07.06.2007), & JP 2007-514756 A & JP 2007-514770 A & US 2005/0171110 A1 & EP 1699796 A & EP 1697370 A & EP 1697371 A & WO 2005/061509 A1 & WO 2005/063761 A1 & WO 2005/063762 A1 & DE 602004006165 D & DE 602004006166 D & CA 2550375 A & CA 2550435 A & NO 20062689 A | 1-21,24-26 |
| A | WO 2006/138695 A1 (BRISTOL-MYERS SQUIBB CO.), 28 December 2006 (28.12.2006), & EP 1891068 A & AR 54789 A | 1-21,24-26 |
| A | WO 2008/031594 A1 (NOVARTIS AG.), 20 March 2008 (20.03.2008), & JP 2010-503629 A & US 2010/0009978 A & EP 1900729 A1 & EP 2066647 A & UY 30587 A & AR 62786 A & CL 26692007 A & CA 2660987 A & NO 20091469 A & KR 10-2009-0064389 A & CN 101516860 A & CR 10639 A & EA 200900388 A & PA 8748101 A & SM 200900023 A | 1-21,24-26 |

Form PCT/ISA/210 (continuation of second sheet) (July 2009)

**INTERNATIONAL SEARCH REPORT**

International application No. PCT/JP2010/073426

Continuation of A. CLASSIFICATION OF SUBJECT MATTER
(International Patent Classification (IPC))

*C07D231/56*(2006.01)i, *A61K31/4025*(2006.01)i, *A61K31/4155*(2006.01)i, *A61K31/4178*(2006.01)i, *A61K31/422*(2006.01)i, *A61K31/427*(2006.01)i, *A61P21/02*(2006.01)i, *A61P25/00*(2006.01)i, *A61P25/14*(2006.01)i, *A61P25/16*(2006.01)i, *A61P25/18*(2006.01)i, *A61P25/22*(2006.01)i, *A61P25/24*(2006.01)i, *A61P25/28*(2006.01)i, *A61P43/00*(2006.01)i, *C07D405/04*(2006.01)i, *C07D409/04*(2006.01)i, *C07D413/04*(2006.01)i, *C07D417/04*(2006.01)i

(According to International Patent Classification (IPC) or to both national classification and IPC)

Continuation of B. FIELDS SEARCHED
Minimum documentation searched (International Patent Classification (IPC))

C07D413/04, C07D417/04

Minimum documentation searched (classification system followed by classification symbols)

Form PCT/ISA/210 (extra sheet) (July 2009)

**INTERNATIONAL SEARCH REPORT**

International application No.
PCT/JP2010/073426

**Box No. II Observations where certain claims were found unsearchable (Continuation of item 2 of first sheet)**

This international search report has not been established in respect of certain claims under Article 17(2)(a) for the following reasons:

1. [X] Claims Nos.: 22-23
because they relate to subject matter not required to be searched by this Authority, namely:
Claims 22 to 23 involve inventions which pertain to methods for treatment of human being by therapy using medicines and methods for prevention.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying additional fees, this Authority did not invite payment of additional fees.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**

[ ] The additional search fees were accompanied by the applicant's protest and, where applicable, the payment of a protest fee.

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006098520 A
- WO 2009107391 A
- WO 2010018874 A
- WO 2009157196 A
- WO 2006116412 A
- WO 2006138695 A
- US 2005143381 A
- WO 2008031594 A
- JP 2009293279 A


### Non-patent literature cited in the description


- **SAWZDARGO et al.** *Molecular Brain Research,* 1999, vol. 64, 193-198
- 4th Edition Jikken Kagaku Kouza. MARUZEN, vol. 19
- 4th Edition Jikken Kagaku Kouza. MARUZEN, vol. 25
- Design of Molecules. IYAKUHIN no KAIHATSU. HIROKAWA SHOTEN, 1990, vol. 7, 163-198
- **OKUYAMA et al.** *Life Science,* 1999, vol. 65, 2109-2125
- Japanese Pharmacopoeia